(19) Europäisches Patentamt
European Patent Office
Office européen des brevets

(11) **EP 3 926 047 A1**

(12) **EUROPEAN PATENT APPLICATION**
published in accordance with Art. 153(4) EPC


(43) Date of publication:
**22.12.2021 Bulletin 2021/51**

(21) Application number: **19872655.6**

(22) Date of filing: **30.09.2019**

(51) Int Cl.:
*C12N 15/115* (2010.01) *A61K 47/56* (2017.01)
*A61K 31/713* (2006.01) *C07H 21/04* (2006.01)

(86) International application number:
**PCT/CN2019/109616**

(87) International publication number:
**WO 2020/078219 (23.04.2020 Gazette 2020/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**16.10.2018 CN 201811205135**
**16.10.2018 CN 201811204337**
**16.10.2018 CN 201811204156**
**16.10.2018 CN 201811204164**
**16.10.2018 CN 201811204344**
**16.10.2018 CN 201811204336**
**25.10.2018 CN 201811253117**



(71) Applicant: **Bai Yao Zhi Da (Beijing) Nanobio Technology Co., Ltd.**
**Beijing 100080 (CN)**

(72) Inventors:
- **WANG, Liyuan**
**Beijing 100080 (CN)**
- **WANG, Meng**
**Beijing 100080 (CN)**

(74) Representative: **Basck Ltd**
**WeWork**
**50-60 Station Rd**
**Cambridge CB1 2JH (GB)**


(54) **NUCLEIC ACID NANO-CARRIER DRUG, PREPARATION METHOD THEREFOR, AND PHARMACEUTICAL COMPOSITION AND APPLICATION THEREOF**


(57) Provided are a nucleic acid nanocarrier drug, a preparation method thereof, a pharmaceutical composition and an application thereof. The nucleic acid nanocarrier drug includes a nucleic acid nanoparticle carrier and a drug, the drug is loaded on the nucleic acid nanoparticle, and the drug includes one or more of paclitaxel, lenalidomide, cytarabine, docetaxel, idarubicin and mitoxantrone; and the nucleic acid nanoparticle includes a nucleic acid domain, the nucleic acid domain includes a sequence a, a sequence b and a sequence c, the sequence a includes a sequence a1 or a sequence obtained by insertion, deletion or substitution of at least one base in the sequence a1, the sequence b includes a sequence b1 or a sequence obtained by insertion, deletion or substitution of at least one base in the sequence b1, and the sequence c includes a sequence c1 or a sequence obtained by insertion, deletion or substitution of at least one base in the sequence c1. After the nucleic acid domain thereof is modified by a target head, the nucleic acid nanocarrier drug of the present application has better targeting properties, may stably deliver the drug, and is very high in reliability.


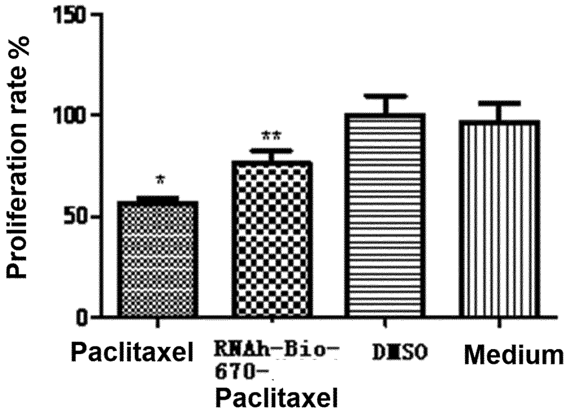


Fig. 13

EP 3 926 047 A1

SEQUENCE LISTING

<110> BAI YAO ZHI DA (Beijing) NanoBio Technology Co., Ltd

<120> Nucleic acid nanocarrier drug and preparation method thereof, pharmaceutical composition and use thereof

<130> PN114617BYZD

<141> 2019-09-30

<150> 201811205135.6
<151> 2018-10-16

<150> 201811204337.9
<151> 2018-10-16

<150> 201811204344.9
<151> 2018-10-16

<150> 201811204336.4
<151> 2018-10-16

<150> 201811253117.5
<151> 2018-10-25

<150> 201811204156.6
<151> 2018-10-16

<150> 201811204164.0
<151> 2018-10-16

<160> 180

<170> SIPOSequenceListing 1.0

<210> 1
<211> 10
<212> DNA/RNA
<213> Artificial Sequence

<220>
<221> misc_feature
<222> (1)..(10)
<223> a1 sequence

<400> 1
ccagcguucc 10

<210> 2
<211> 10
<212> DNA/RNA
<213> Artificial Sequence

<220>
<221> mRNA
<222> (1)..(10)
<223> a1 sequence

<400> 2
ccagcgttcc 10

<210> 3
<211> 9
<212> DNA/RNA
<213> Artificial Sequence

<220>
<221> misc_feature
<222> (1)..(9)
<223> b1 sequence

<400> 3
gguucgccg 9

<210> 4
<211> 9
<212> DNA/RNA
<213> Artificial Sequence

<220>
<221> misc_feature
<222> (1)..(9)
<223> b1 sequence

<400> 4
ggttcgccg 9

<210> 5
<211> 12
<212> DNA/RNA
<213> Artificial Sequence

<220>
<221> misc_feature
<222> (1)..(12)
<223> c1 sequence

<400> 5
cggccauagc gg 12

<210> 6
<211> 12
<212> DNA/RNA
<213> Artificial Sequence

<220>
<221> misc_feature
<222> (1)..(12)
<223> c1 sequence

<400> 6
cggccatagc gg 12

<210> 7
<211> 10
<212> DNA/RNA
<213> Artificial Sequence

<220>

<221> misc_feature
<222> (1)..(10)
<223> a sequence

<400> 7
ggagcguugg 10

<210> 8
<211> 9
<212> DNA/RNA
<213> Artificial Sequence

<220>
<221> misc_feature
<222> (1)..(9)
<223> b sequence

<400> 8
ccuucgccg 9

<210> 9
<211> 12
<212> DNA/RNA
<213> Artificial Sequence

<220>
<221> misc_feature
<222> (1)..(12)
<223> c sequence

<400> 9
cggccauagc cc 12

<210> 10
<211> 10
<212> DNA/RNA
<213> Artificial Sequence

<220>
<221> misc_feature
<222> (1)..(10)
<223> a sequence

<400> 10
gcagcguucg 10

<210> 11
<211> 9
<212> DNA/RNA
<213> Artificial Sequence

<220>
<221> misc_feature
<222> (1)..(9)
<223> b sequence

<400> 11
cguucgccg 9

<210>  12
<211>  12
<212>  DNA/RNA
<213>  Artificial Sequence

<220>
<221>  misc_feature
<222>  (1)..(12)
<223>  c sequence

<400>  12
cggccauagc gc    12

<210>  13
<211>  10
<212>  DNA/RNA
<213>  Artificial Sequence

<220>
<221>  misc_feature
<222>  (1)..(10)
<223>  a sequence

<400>  13
cgagcguugc    10

<210>  14
<211>  9
<212>  DNA/RNA
<213>  Artificial Sequence

<220>
<221>  misc_feature
<222>  (1)..(9)
<223>  b sequence

<400>  14
gcuucgccg    9

<210>  15
<211>  12
<212>  DNA/RNA
<213>  Artificial Sequence

<220>
<221>  misc_feature
<222>  (1)..(12)
<223>  c sequence

<400>  15
cggccauagc cg    12

<210>  16
<211>  10
<212>  DNA/RNA
<213>  Artificial Sequence

<220>

<221> misc_feature
<222> (1)..(10)
<223> a sequence

<400> 16
ggagcguugg 10

<210> 17
<211> 9
<212> DNA/RNA
<213> Artificial Sequence

<220>
<221> misc_feature
<222> (1)..(9)
<223> b sequence

<400> 17
ccuucgggg 9

<210> 18
<211> 12
<212> DNA/RNA
<213> Artificial Sequence

<220>
<221> misc_feature
<222> (1)..(12)
<223> c sequence

<400> 18
cccccauagc cc 12

<210> 19
<211> 10
<212> DNA/RNA
<213> Artificial Sequence

<220>
<221> misc_feature
<222> (1)..(10)
<223> a sequence

<400> 19
gcagcguucg 10

<210> 20
<211> 9
<212> DNA/RNA
<213> Artificial Sequence

<220>
<221> misc_feature
<222> (1)..(9)
<223> b sequence

<400> 20
cguucggcg 9

<210> 21
<211> 12
<212> DNA/RNA
<213> Artificial Sequence

<220>
<221> misc_feature
<222> (1)..(12)
<223> c sequence

<400> 21
cgcccauagc gc 12

<210> 22
<211> 10
<212> DNA/RNA
<213> Artificial Sequence

<220>
<221> misc_feature
<222> (1)..(10)
<223> a sequence

<400> 22
gcagcguucg 10

<210> 23
<211> 9
<212> DNA/RNA
<213> Artificial Sequence

<220>
<221> misc_feature
<222> (1)..(9)
<223> b sequence

<400> 23
cguucggcc 9

<210> 24
<211> 12
<212> DNA/RNA
<213> Artificial Sequence

<220>
<221> misc_feature
<222> (1)..(12)
<223> c sequence

<400> 24
ggcccauagc gc 12

<210> 25
<211> 10
<212> DNA/RNA
<213> Artificial Sequence

<220>

<221> misc_feature
<222> (1)..(10)
<223> a sequence

<400> 25
cgagcguugc 10

<210> 26
<211> 9
<212> DNA/RNA
<213> Artificial Sequence

<220>
<221> misc_feature
<222> (1)..(9)
<223> b sequence

<400> 26
gcuucggcg 9

<210> 27
<211> 12
<212> DNA/RNA
<213> Artificial Sequence

<220>
<221> misc_feature
<222> (1)..(12)
<223> c sequence

<400> 27
cgcccauagc cg 12

<210> 28
<211> 10
<212> DNA/RNA
<213> Artificial Sequence

<220>
<221> misc_feature
<222> (1)..(10)
<223> a sequence

<400> 28
ggagcgttgg 10

<210> 29
<211> 9
<212> DNA/RNA
<213> Artificial Sequence

<220>
<221> misc_feature
<222> (1)..(9)
<223> b sequence

<400> 29
ccttcgccg 9

<210> 30
<211> 12
<212> DNA/RNA
<213> Artificial Sequence

<220>
<221> misc_feature
<222> (1)..(12)
<223> c sequence

<400> 30
cggccatagc cc 12

<210> 31
<211> 10
<212> DNA/RNA
<213> Artificial Sequence

<220>
<221> misc_feature
<222> (1)..(10)
<223> a sequence

<400> 31
gcagcgttcg 10

<210> 32
<211> 9
<212> DNA/RNA
<213> Artificial Sequence

<220>
<221> misc_feature
<222> (1)..(9)
<223> b sequence

<400> 32
cgttcgccg 9

<210> 33
<211> 12
<212> DNA/RNA
<213> Artificial Sequence

<220>
<221> misc_feature
<222> (1)..(12)
<223> c sequence

<400> 33
cggccatagc gc 12

<210> 34
<211> 10
<212> DNA/RNA
<213> Artificial Sequence

<220>

<221> misc_feature
<222> (1)..(10)
<223> a sequence

<400> 34
cgagcgttgc 10

<210> 35
<211> 9
<212> DNA/RNA
<213> Artificial Sequence

<220>
<221> misc_feature
<222> (1)..(9)
<223> b sequence

<400> 35
gcttcgccg 9

<210> 36
<211> 12
<212> DNA/RNA
<213> Artificial Sequence

<220>
<221> misc_feature
<222> (1)..(12)
<223> c sequence

<400> 36
cggccatagc cg 12

<210> 37
<211> 10
<212> DNA/RNA
<213> Artificial Sequence

<220>
<221> misc_feature
<222> (1)..(10)
<223> a sequence

<400> 37
ggagcgttgg 10

<210> 38
<211> 9
<212> DNA/RNA
<213> Artificial Sequence

<220>
<221> misc_feature
<222> (1)..(9)
<223> b sequence

<400> 38
ccttcgggg 9

<210> 39
<211> 12
<212> DNA/RNA
<213> Artificial Sequence

<220>
<221> misc_feature
<222> (1)..(12)
<223> c sequence

<400> 39
cccccatagc cc 12

<210> 40
<211> 10
<212> DNA/RNA
<213> Artificial Sequence

<220>
<221> misc_feature
<222> (1)..(10)
<223> a sequence

<400> 40
gcagcgttcg 10

<210> 41
<211> 9
<212> DNA/RNA
<213> Artificial Sequence

<220>
<221> misc_feature
<222> (1)..(9)
<223> b sequence

<400> 41
cgttcggcg 9

<210> 42
<211> 12
<212> DNA/RNA
<213> Artificial Sequence

<220>
<221> misc_feature
<222> (1)..(12)
<223> c sequence

<400> 42
cgcccatagc gc 12

<210> 43
<211> 10
<212> DNA/RNA
<213> Artificial Sequence

<220>

<221> misc_feature
<222> (1)..(10)
<223> a sequence

<400> 43
gcagcgttcg 10

<210> 44
<211> 9
<212> DNA/RNA
<213> Artificial Sequence

<220>
<221> misc_feature
<222> (1)..(9)
<223> b sequence

<400> 44
cgttcggcc 9

<210> 45
<211> 12
<212> DNA/RNA
<213> Artificial Sequence

<220>
<221> misc_feature
<222> (1)..(12)
<223> c sequence

<400> 45
ggcccatagc gc 12

<210> 46
<211> 10
<212> DNA/RNA
<213> Artificial Sequence

<220>
<221> misc_feature
<222> (1)..(10)
<223> a sequence

<400> 46
cgagcgttgc 10

<210> 47
<211> 9
<212> DNA/RNA
<213> Artificial Sequence

<220>
<221> misc_feature
<222> (1)..(9)
<223> b sequence

<400> 47
gcttcggcg 9

<210> 48
<211> 12
<212> DNA/RNA
<213> Artificial Sequence

<220>
<221> misc_feature
<222> (1)..(12)
<223> c sequence

<400> 48
cgcccatagc cg 12

<210> 49
<211> 77
<212> DNA/RNA
<213> Artificial Sequence

<220>
<221> misc_feature
<222> (1)..(77)
<223> a sequence

<220>
<221> misc_feature
<222> (1)..(77)
<223> m is u or t

<400> 49
cgcgcgaaaa aacgcgcgaa aaaacgcgcg cccaccagcg mmccgggcgc gcgaaaaaac 60
gcgcgaaaaa acgcgcg 77

<210> 50
<211> 75
<212> DNA/RNA
<213> Artificial Sequence

<220>
<221> misc_feature
<222> (1)..(75)
<223> b sequence

<220>
<221> misc_feature
<222> (1)..(75)
<223> m is u or t

<400> 50
cgcgcgmmmm mmcgcgcgmm mmmmcgcgcg cccggmmcgc cgccagccgc cmmmmmmgcc 60
gccmmmmmmg ccgcc 75

<210> 51
<211> 78
<212> DNA/RNA
<213> Artificial Sequence

<220>

<221> misc_feature
<222> (1)..(78)
<223> c sequence

<220>
<221> misc_feature
<222> (1)..(78)
<223> m is u or t

<400> 51
ggcggcaaaa aaggcggcaa aaaaggcggc aggcggcama gcggmgggcg cgcgmmmmmm 60
cgcgcgmmmm mmcgcgcg 78

<210> 52
<211> 29
<212> RNA
<213> Artificial Sequence

<220>
<221> misc_feature
<222> (1)..(29)
<223> a strand

<220>
<221> modified_base
<222> (1)..(1)
<223> 5'Biotin

<400> 52
cgcgcgccca ccagcguucc gggcgccgc 29

<210> 53
<211> 27
<212> RNA
<213> Artificial Sequence

<220>
<221> misc_feature
<222> (1)..(27)
<223> b strand

<220>
<221> modified_base
<222> (1)..(1)
<223> 5'Biotin

<400> 53
gcggcgcccg guucgccgcc aggcggc 27

<210> 54
<211> 31
<212> RNA
<213> Artificial Sequence

<220>
<221> misc_feature
<222> (1)..(31)

<223>  c strand

<220>
<221>  modified_base
<222>  (1)..(1)
<223>  5′CY3

<400>  54
gccgccaggc ggccauagcg gugggcgcgc g 31

<210>  55
<211>  10
<212>  RNA
<213>  Artificial Sequence

<220>
<221>  misc_feature
<222>  (1)..(10)
<223>  a strand

<400>  55
ggagcguugg 10

<210>  56
<211>  9
<212>  RNA
<213>  Artificial Sequence

<220>
<221>  misc_feature
<222>  (1)..(9)
<223>  b strand

<400>  56
ccuucgccg 9

<210>  57
<211>  12
<212>  RNA
<213>  Artificial Sequence

<220>
<221>  misc_feature
<222>  (1)..(12)
<223>  c strand

<400>  57
cggccauagc cc 12

<210>  58
<211>  10
<212>  RNA
<213>  Artificial Sequence

<220>
<221>  misc_feature
<222>  (1)..(10)
<223>  a strand

<400> 58
gcagcguucg 10

<210> 59
<211> 9
<212> RNA
<213> Artificial Sequence

<220>
<221> misc_feature
<222> (1)..(9)
<223> b strand

<400> 59
cguucgccg 9

<210> 60
<211> 12
<212> RNA
<213> Artificial Sequence

<220>
<221> misc_feature
<222> (1)..(12)
<223> c strand

<400> 60
cggccauagc gc 12

<210> 61
<211> 10
<212> RNA
<213> Artificial Sequence

<220>
<221> misc_feature
<222> (1)..(10)
<223> a strand

<400> 61
cgagcguugc 10

<210> 62
<211> 9
<212> RNA
<213> Artificial Sequence

<220>
<221> misc_feature
<222> (1)..(9)
<223> b strand

<400> 62
gcuucgccg 9

<210> 63
<211> 12

<212> RNA
<213> Artificial Sequence

<220>
<221> misc_feature
<222> (1)..(12)
<223> c strand

<400> 63
cggccauagc cg 12

<210> 64
<211> 10
<212> RNA
<213> Artificial Sequence

<220>
<221> misc_feature
<222> (1)..(10)
<223> a strand

<400> 64
ggagcguugg 10

<210> 65
<211> 9
<212> RNA
<213> Artificial Sequence

<220>
<221> misc_feature
<222> (1)..(9)
<223> b strand

<400> 65
ccuucgggg 9

<210> 66
<211> 12
<212> RNA
<213> Artificial Sequence

<220>
<221> misc_feature
<222> (1)..(12)
<223> c strand

<400> 66
cccccauagc cc 12

<210> 67
<211> 10
<212> RNA
<213> Artificial Sequence

<220>
<221> misc_feature
<222> (1)..(10)
<223> a strand

<400> 67
gcagcguucg 10

<210> 68
<211> 9
<212> RNA
<213> Artificial Sequence

<220>
<221> misc_feature
<222> (1)..(9)
<223> b strand

<400> 68
cguucggcg 9

<210> 69
<211> 12
<212> RNA
<213> Artificial Sequence

<220>
<221> misc_feature
<222> (1)..(12)
<223> c strand

<400> 69
cgcccauagc gc 12

<210> 70
<211> 10
<212> RNA
<213> Artificial Sequence

<220>
<221> misc_feature
<222> (1)..(10)
<223> a strand

<400> 70
gcagcguucg 10

<210> 71
<211> 9
<212> RNA
<213> Artificial Sequence

<220>
<221> misc_feature
<222> (1)..(9)
<223> b strand

<400> 71
cguucggcc 9

<210> 72
<211> 12

<212> RNA
<213> Artificial Sequence

<220>
<221> misc_feature
<222> (1)..(12)
<223> c strand

<400> 72
ggcccauagc gc 12

<210> 73
<211> 10
<212> RNA
<213> Artificial Sequence

<220>
<221> misc_feature
<222> (1)..(10)
<223> a strand

<400> 73
cgagcguugc 10

<210> 74
<211> 9
<212> RNA
<213> Artificial Sequence

<220>
<221> misc_feature
<222> (1)..(9)
<223> b strand

<400> 74
gcuucggcg 9

<210> 75
<211> 12
<212> RNA
<213> Artificial Sequence

<220>
<221> misc_feature
<222> (1)..(12)
<223> c strand

<400> 75
cgcccauagc cg 12

<210> 76
<211> 29
<212> RNA
<213> Artificial Sequence

<220>
<221> misc_feature
<222> (1)..(29)
<223> a strand

<400> 76
cgcgcgccca ggagcguugg cgggcggcg 29

<210> 77
<211> 27
<212> RNA
<213> Artificial Sequence

<220>
<221> misc_feature
<222> (1)..(27)
<223> b strand

<400> 77
cgccgcccgc cuucgccgcc agccgcc 27

<210> 78
<211> 31
<212> RNA
<213> Artificial Sequence

<220>
<221> misc_feature
<222> (1)..(31)
<223> c strand

<400> 78
ggcggcaggc ggccauagcc cugggcgcgc g 31

<210> 79
<211> 29
<212> RNA
<213> Artificial Sequence

<220>
<221> misc_feature
<222> (1)..(29)
<223> a strand

<400> 79
cgcgcgccca gcagcguucg cgggcggcg 29

<210> 80
<211> 27
<212> RNA
<213> Artificial Sequence

<220>
<221> misc_feature
<222> (1)..(27)
<223> b strand

<400> 80
cgccgcccgc guucgccgcc agccgcc 27

<210> 81
<211> 31

<212> RNA
<213> Artificial Sequence

<220>
<221> misc_feature
<222> (1)..(31)
<223> c strand

<400> 81
ggcggcaggc ggccauagcg cugggcgcgc g 31

<210> 82
<211> 29
<212> RNA
<213> Artificial Sequence

<220>
<221> misc_feature
<222> (1)..(29)
<223> a strand

<400> 82
cgcgcgccca cgagcguugc ggggcggcg 29

<210> 83
<211> 27
<212> RNA
<213> Artificial Sequence

<220>
<221> misc_feature
<222> (1)..(27)
<223> b strand

<400> 83
cgccgccccg cuucgccgcc agccgcc 27

<210> 84
<211> 31
<212> RNA
<213> Artificial Sequence

<220>
<221> misc_feature
<222> (1)..(31)
<223> c strand

<400> 84
ggcggcaggc ggccauagcc gugggcgcgc g 31

<210> 85
<211> 29
<212> RNA
<213> Artificial Sequence

<220>
<221> misc_feature
<222> (1)..(29)
<223> a strand

<400> 85
cgcgcgccca ggagcguugg cccgcggcg 29

<210> 86
<211> 27
<212> RNA
<213> Artificial Sequence

<220>
<221> misc_feature
<222> (1)..(27)
<223> b strand

<400> 86
cgccgcgggc cuucggggcc agccgcc 27

<210> 87
<211> 31
<212> RNA
<213> Artificial Sequence

<220>
<221> misc_feature
<222> (1)..(31)
<223> c strand

<400> 87
ggcggcaggc ccccauagcc cugggcgcgc g 31

<210> 88
<211> 29
<212> RNA
<213> Artificial Sequence

<220>
<221> misc_feature
<222> (1)..(29)
<223> a strand

<400> 88
cgcgcgccca gcagcguucg ccccgccgc 29

<210> 89
<211> 27
<212> RNA
<213> Artificial Sequence

<220>
<221> misc_feature
<222> (1)..(27)
<223> b strand

<400> 89
gcggcggggc guucggcggc aggcggc 27

<210> 90
<211> 31

<212> RNA
<213> Artificial Sequence

<220>
<221> misc_feature
<222> (1)..(31)
<223> c strand

<400> 90
gccgccagcc gcccauagcg cugggcgcgc g 31

<210> 91
<211> 29
<212> RNA
<213> Artificial Sequence

<220>
<221> misc_feature
<222> (1)..(29)
<223> a strand

<400> 91
cgcgcgccca gcagcguucg gggcgccgc 29

<210> 92
<211> 28
<212> RNA
<213> Artificial Sequence

<220>
<221> misc_feature
<222> (1)..(28)
<223> b strand

<400> 92
gcggcgcccc guucggccgg caggcggc 28

<210> 93
<211> 32
<212> RNA
<213> Artificial Sequence

<220>
<221> misc_feature
<222> (1)..(32)
<223> c strand

<400> 93
gccgccagcc ggcccauagc gcugggcgcg cg 32

<210> 94
<211> 40
<212> DNA/RNA
<213> Artificial Sequence

<220>
<221> misc_feature
<222> (1)..(40)
<223> a strand

<400> 94
cgcgcgcgag cguugcaaug acagauaagg aaccugcutt 40

<210> 95
<211> 36
<212> RNA
<213> Artificial Sequence

<220>
<221> misc_feature
<222> (1)..(36)
<223> b strand

<400> 95
ggcagguucc uuaucuguca aagcuucggc ggcagc 36

<210> 96
<211> 23
<212> RNA
<213> Artificial Sequence

<220>
<221> misc_feature
<222> (1)..(23)
<223> c strand

<400> 96
gcagccgccc auagccgcgc gcg 23

<210> 97
<211> 39
<212> DNA
<213> Artificial Sequence

<220>
<221> misc_feature
<222> (1)..(39)
<223> EGFRapt

<400> 97
gccttagtaa cgtgctttga tgtcgattcg acaggaggc 39

<210> 98
<211> 41
<212> DNA
<213> Artificial Sequence

<220>
<221> misc_feature
<222> (1)..(41)
<223> PSMAapt

<400> 98
gggccgaaaa agacctgact tctatactaa gtctacgtcc c 41

<210> 99
<211> 68

<212> DNA
<213> Artificial Sequence

<220>
<221> misc_feature
<222> (1)..(68)
<223> a strand

<400> 99
cgcgcgccca ggagcgttgg cgggcggcgg ccttagtaac gtgctttgat gtcgattcga 60
caggaggc 68

<210> 100
<211> 27
<212> DNA
<213> Artificial Sequence

<220>
<221> misc_feature
<222> (1)..(27)
<223> b strand

<400> 100
cgccgcccgc cttcgccgcc agccgcc 27

<210> 101
<211> 31
<212> DNA
<213> Artificial Sequence

<220>
<221> misc_feature
<222> (1)..(31)
<223> c strand

<400> 101
ggcggcaggc ggccatagcc ctgggcgcgc g 31

<210> 102
<211> 68
<212> DNA
<213> Artificial Sequence

<220>
<221> misc_feature
<222> (1)..(68)
<223> a strand

<400> 102
cgcgcgccca gcagcgttcg cgggcggcgg ccttagtaac gtgctttgat gtcgattcga 60
caggaggc 68

<210> 103
<211> 27
<212> DNA
<213> Artificial Sequence

<220>
<221> misc_feature

<222> (1)..(27)
<223> b strand

<400> 103
cgccgcccgc gttcgccgcc agccgcc 27

<210> 104
<211> 31
<212> DNA
<213> Artificial Sequence

<220>
<221> misc_feature
<222> (1)..(31)
<223> c strand

<400> 104
ggcggcaggc ggccatagcg ctgggcgcgc g 31

<210> 105
<211> 68
<212> DNA
<213> Artificial Sequence

<220>
<221> misc_feature
<222> (1)..(68)
<223> a strand

<400> 105
cgcgcgccca cgagcgttgc ggggcggcgg ccttagtaac gtgctttgat gtcgattcga 60
caggaggc 68

<210> 106
<211> 27
<212> DNA
<213> Artificial Sequence

<220>
<221> misc_feature
<222> (1)..(27)
<223> b strand

<400> 106
cgccgccccg cttcgccgcc agccgcc 27

<210> 107
<211> 31
<212> DNA
<213> Artificial Sequence

<220>
<221> misc_feature
<222> (1)..(31)
<223> c strand

<400> 107
ggcggcaggc ggccatagcc gtgggcgcgc g 31

<210> 108
<211> 71
<212> DNA
<213> Artificial Sequence

<220>
<221> misc_feature
<222> (1)..(71)
<223> a strand

<400> 108
cgcgcgccca ggagcgttgg cccgcggcgt gggccgaaaa agacctgact tctatactaa 60
gtctacgtcc c 71

<210> 109
<211> 27
<212> DNA
<213> Artificial Sequence

<220>
<221> misc_feature
<222> (1)..(27)
<223> b strand

<400> 109
cgccgcgggc cttcggggcc agccgcc 27

<210> 110
<211> 31
<212> DNA
<213> Artificial Sequence

<220>
<221> misc_feature
<222> (1)..(31)
<223> c strand

<400> 110
ggcggcaggc ccccatagcc ctgggcgcgc g 31

<210> 111
<211> 71
<212> DNA
<213> Artificial Sequence

<220>
<221> misc_feature
<222> (1)..(71)
<223> a strand

<400> 111
cgcgcgccca gcagcgttcg ccccgccgct gggccgaaaa agacctgact tctatactaa 60
gtctacgtcc c 71

<210> 112
<211> 27
<212> DNA
<213> Artificial Sequence

<220>
<221> misc_feature
<222> (1)..(27)
<223> b strand

<400> 112
gcggcggggc gttcggcggc aggcggc 27

<210> 113
<211> 31
<212> DNA
<213> Artificial Sequence

<220>
<221> misc_feature
<222> (1)..(31)
<223> c strand

<400> 113
gccgccagcc gcccatagcg ctgggcgcgc g 31

<210> 114
<211> 29
<212> DNA
<213> Artificial Sequence

<220>
<221> misc_feature
<222> (1)..(29)
<223> a strand

<400> 114
cgcgcgccca gcagcgttcg ggcgccgc 29

<210> 115
<211> 28
<212> DNA
<213> Artificial Sequence

<220>
<221> misc_feature
<222> (1)..(20)
<223> b strand

<400> 115
gcggcgcccc gttcggccgg caggcggc 28

<210> 116
<211> 32
<212> DNA
<213> Artificial Sequence

<220>
<221> misc_feature
<222> (1)..(32)
<223> c strand

<400> 116
gccgccagcc ggcccatagc gctgggcgcg cg 32

<210> 117
<211> 29
<212> DNA
<213> Artificial Sequence

<220>
<221> misc_feature
<222> (1)..(29)
<223> a strand

<400> 117
cgcgcgccca cgagcgttgc gggcgccgc 29

<210> 118
<211> 27
<212> DNA
<213> Artificial Sequence

<220>
<221> misc_feature
<222> (1)..(27)
<223> b strand

<400> 118
gcggcgcccg cttcggcggc aggcggc 27

<210> 119
<211> 31
<212> DNA
<213> Artificial Sequence

<220>
<221> misc_feature
<222> (1)..(31)
<223> c strand

<400> 119
gccgccagcc gcccatagcc gtgggcgcgc g 31

<210> 120
<211> 37
<212> RNA
<213> Artificial Sequence

<220>
<221> misc_feature
<222> (1)..(37)
<223> a strand

<400> 120
gcggcgagcg gcgaggagcg uuggggccgg aggccgg 37

<210> 121
<211> 31
<212> RNA
<213> Artificial Sequence

<220>
<221> misc_feature
<222> (1)..(37)
<223> b strand

<400> 121
ccggccuccg gccccuucgg ggccagccgc c 31

<210> 122
<211> 35
<212> RNA
<213> Artificial Sequence

<220>
<221> misc_feature
<222> (1)..(35)
<223> c strand

<400> 122
ggcggcaggc ccccauagcc cucgccgcuc gccgc 35

<210> 123
<211> 37
<212> RNA
<213> Artificial Sequence

<220>
<221> misc_feature
<222> (1)..(37)
<223> a strand

<400> 123
gcggcgagcg gcgagcagcg uucgggccgg aggccgg 37

<210> 124
<211> 31
<212> RNA
<213> Artificial Sequence

<220>
<221> misc_feature
<222> (1)..(31)
<223> b strand

<400> 124
ccggccuccg gcccguucgc cgccagccgc c 31

<210> 125
<211> 35
<212> RNA
<213> Artificial Sequence

<220>
<221> misc_feature
<222> (1)..(35)
<223> c strand

<400> 125
ggcggcaggc ggccauagcg cucgccgcuc gccgc 35

<210> 126
<211> 37
<212> RNA
<213> Artificial Sequence

<220>
<221> misc_feature
<222> (1)..(37)
<223> a strand

<400> 126
gcggcgagcg gcgaggagcg uuggggccgg aggccgg 37

<210> 127
<211> 31
<212> RNA
<213> Artificial Sequence

<220>
<221> misc_feature
<222> (1)..(31)
<223> b strand

<400> 127
ccggccuccg gccccuucgc cgccagccgc c 31

<210> 128
<211> 35
<212> RNA
<213> Artificial Sequence

<220>
<221> misc_feature
<222> (1)..(35)
<223> c strand

<400> 128
ggcggcaggc ggccauagcc cucgccgcuc gccgc 35

<210> 129
<211> 37
<212> RNA
<213> Artificial Sequence

<220>
<221> misc_feature
<222> (1)..(37)
<223> a strand

<400> 129
gcggcgagcg gcgagcagcg uucgggccgg aggccgg 37

<210> 130
<211> 31
<212> RNA
<213> Artificial Sequence

<220>
<221> misc_feature
<222> (1)..(31)
<223> b strand

<400> 130
ccggccuccg gcccguucgg cgccagccgc c 31

<210> 131
<211> 35
<212> RNA
<213> Artificial Sequence

<220>
<221> misc_feature
<222> (1)..(35)
<223> c strand

<400> 131
ggcggcaggc gcccauagcg cucgccgcuc gccgc 35

<210> 132
<211> 37
<212> RNA
<213> Artificial Sequence

<220>
<221> misc_feature
<222> (1)..(37)
<223> a strand

<400> 132
gcggcgagcg gcgagcagcg uucgggccgg aggccgg 37

<210> 133
<211> 31
<212> RNA
<213> Artificial Sequence

<220>
<221> misc_feature
<222> (1)..(31)
<223> b strand

<400> 133
ccggccuccg gcccguucgg ccccagccgc c 31

<210> 134
<211> 35
<212> RNA
<213> Artificial Sequence

<220>
<221> misc_feature
<222> (1)..(35)
<223> c strand

<400> 134
ggcggcaggg gcccauagcg cucgccgcuc gccgc 35

<210> 135
<211> 37
<212> RNA
<213> Artificial Sequence

<220>
<221> misc_feature
<222> (1)..(37)
<223> a strand

<400> 135
gcggcgagcg gcgacgagcg uugcggccgg aggccgg 37

<210> 136
<211> 31
<212> RNA
<213> Artificial Sequence

<220>
<221> misc_feature
<222> (1)..(31)
<223> b strand

<400> 136
ccggccuccg gccgcuucgc cgccagccgc c 31

<210> 137
<211> 35
<212> RNA
<213> Artificial Sequence

<220>
<221> misc_feature
<222> (1)..(35)
<223> c strand

<400> 137
ggcggcaggc ggccauagcc gucgccgcuc gccgc 35

<210> 138
<211> 37
<212> RNA
<213> Artificial Sequence

<220>
<221> misc_feature
<222> (1)..(37)
<223> a strand

<400> 138
gcggcgagcg gcgacgagcg uugcggccgg aggccgg 37

<210> 139
<211> 31
<212> RNA
<213> Artificial Sequence

<220>
<221> misc_feature
<222> (1)..(31)
<223> b strand

<400> 139
ccggccuccg gccgcuucgg cgccagccgc c 31

<210> 140
<211> 35
<212> RNA
<213> Artificial Sequence

<220>
<221> misc_feature
<222> (1)..(35)
<223> c strand

<400> 140
ggcggcaggc gcccauagcc gucgccgcuc gccgc 35

<210> 141
<211> 37
<212> DNA
<213> Artificial Sequence

<220>
<221> misc_feature
<222> (1)..(37)
<223> a strand

<400> 141
gcggcgagcg gcgaggagcg ttggggccgg aggccgg 37

<210> 142
<211> 31
<212> DNA
<213> Artificial Sequence

<220>
<221> misc_feature
<222> (1)..(31)
<223> b strand

<400> 142
ccggcctccg gccccttcgg ggccagccgc c 31

<210> 143
<211> 35
<212> DNA
<213> Artificial Sequence

<220>
<221> misc_feature
<222> (1)..(35)
<223> c strand

<400> 143
ggcggcaggc ccccatagcc ctcgccgctc gccgc 35

<210> 144
<211> 37
<212> DNA
<213> Artificial Sequence

<220>
<221> misc_feature
<222> (1)..(37)
<223> a strand

<400> 144
gcggcgagcg gcgagcagcg ttcgggccgg aggccgg 37

<210> 145
<211> 31
<212> DNA
<213> Artificial Sequence

<220>
<221> misc_feature
<222> (1)..(31)
<223> b strand

<400> 145
ccggcctccg gcccgttcgc cgccagccgc c 31

<210> 146
<211> 35
<212> DNA
<213> Artificial Sequence

<220>
<221> misc_feature
<222> (1)..(35)
<223> c strand

<400> 146
ggcggcaggc ggccatagcg ctcgccgctc gccgc 35

<210> 147
<211> 37
<212> DNA
<213> Artificial Sequence

<220>
<221> misc_feature
<222> (1)..(37)
<223> a strand

<400> 147
gcggcgagcg gcgaggagcg ttggggccgg aggccgg 37

<210> 148
<211> 31
<212> DNA
<213> Artificial Sequence

<220>
<221> misc_feature
<222> (1)..(31)
<223> b strand

<400> 148
ccggcctccg gccccttcgc cgccagccgc c 31

<210> 149
<211> 35
<212> DNA
<213> Artificial Sequence

<220>
<221> misc_feature
<222> (1)..(35)
<223> c strand

<400> 149
ggcggcaggc ggccatagcc ctcgccgctc gccgc 35

<210> 150
<211> 37
<212> DNA
<213> Artificial Sequence

<220>
<221> misc_feature
<222> (1)..(37)
<223> a strand

<400> 150
gcggcgagcg gcgagcagcg ttcgggccgg aggccgg 37

<210> 151
<211> 31
<212> DNA
<213> Artificial Sequence

<220>
<221> misc_feature
<222> (1)..(31)
<223> b strand

<400> 151
ccggcctccg gcccgttcgg cgccagccgc c 31

<210> 152
<211> 35
<212> DNA
<213> Artificial Sequence

<220>
<221> misc_feature
<222> (1)..(35)
<223> c strand

<400> 152
ggcggcaggc gcccatagcg ctcgccgctc gccgc 35

<210> 153
<211> 37
<212> DNA
<213> Artificial Sequence

<220>
<221> misc_feature
<222> (1)..(37)
<223> a strand

<400> 153
gcggcgagcg gcgagcagcg ttcgggccgg aggccgg 37

<210> 154
<211> 31
<212> DNA
<213> Artificial Sequence

<220>
<221> misc_feature
<222> (1)..(31)
<223> b strand

<400> 154
ccggcctccg gcccgttcgg ccccagccgc c 31

<210> 155
<211> 35
<212> DNA
<213> Artificial Sequence

<220>
<221> misc_feature
<222> (1)..(35)
<223> c strand

<400> 155
ggcggcaggg gcccatagcg ctcgccgctc gccgc 35

<210> 156
<211> 37
<212> DNA
<213> Artificial Sequence

<220>
<221> misc_feature
<222> (1)..(37)
<223> a strand

<400> 156
gcggcgagcg gcgacgagcg ttgcggccgg aggccgg 37

<210> 157
<211> 31
<212> DNA
<213> Artificial Sequence

<220>
<221> misc_feature
<222> (1)..(31)
<223> b strand

<400> 157
ccggcctccg gccgcttcgc cgccagccgc c 31

<210> 158
<211> 35
<212> DNA
<213> Artificial Sequence

<220>
<221> misc_feature
<222> (1)..(35)
<223> c strand

<400> 158
ggcggcaggc ggccatagcc gtcgccgctc gccgc 35

<210> 159
<211> 37
<212> DNA
<213> Artificial Sequence

<220>
<221> misc_feature
<222> (1)..(37)
<223> a strand

<400> 159
gcggcgagcg gcgacgagcg ttgcggccgg aggccgg 37

<210> 160
<211> 31
<212> DNA
<213> Artificial Sequence

<220>
<221> misc_feature
<222> (1)..(31)
<223> b strand

<400> 160
ccggcctccg gccgcttcgg cgccagccgc c 31

<210> 161
<211> 35
<212> DNA
<213> Artificial Sequence

<220>
<221> misc_feature
<222> (1)..(35)
<223> c strand

<400> 161
ggcggcaggc gcccatagcc gtcgccgctc gccgc 35

<210> 162
<211> 14
<212> DNA/RNA
<213> Artificial Sequence

<220>
<221> misc_feature
<222> (1)..(14)
<223> first elongating fragment

<400> 162
gcggcgagcg gcga 14

<210> 163
<211> 14
<212> DNA/RNA
<213> Artificial Sequence

<220>
<221> misc_feature
<222> (1)..(14)
<223> first elongating fragment

<400> 163
ucgccgcucg ccgc 14

<210> 164
<211> 13
<212> DNA/RNA
<213> Artificial Sequence

<220>
<221> misc_feature
<222> (1)..(13)
<223> first elongating fragment

<400> 164
ggccggaggc cgg 13

<210> 165
<211> 13
<212> DNA/RNA
<213> Artificial Sequence

<220>
<221> misc_feature
<222> (1)..(13)
<223> first elongating fragment

<400> 165
ccggccuccg gcc 13

<210> 166
<211> 9
<212> DNA/RNA
<213> Artificial Sequence

<220>
<221> misc_feature
<222> (1)..(9)
<223> first elongating fragment

<400> 166
ccagccgcc 9

<210> 167
<211> 9
<212> DNA/RNA
<213> Artificial Sequence

<220>
<221> misc_feature
<222> (1)..(9)
<223> first elongating fragment

<400> 167
ggcggcagg 9

<210> 168
<211> 14
<212> DNA/RNA
<213> Artificial Sequence

<220>
<221> misc_feature
<222> (1)..(14)
<223> first elongating fragment

<400> 168
gcggcgagcg gcga 14

<210> 169
<211> 14
<212> DNA/RNA
<213> Artificial Sequence

<220>
<221> misc_feature
<222> (1)..(14)
<223> first elongating fragment

<400> 169
tcgccgctcg ccgc 14

<210> 170
<211> 13
<212> DNA/RNA
<213> Artificial Sequence

<220>
<221> misc_feature
<222> (1)..(13)
<223> first elongating fragment

<400> 170
ggccggaggc cgg 13

<210> 171
<211> 13
<212> DNA/RNA
<213> Artificial Sequence

<220>
<221> misc_feature
<222> (1)..(13)
<223> first elongating fragment

<400> 171
ccggcctccg gcc 13

<210> 172
<211> 65
<212> DNA
<213> Artificial Sequence

<220>
<221> misc_feature
<222> (1)..(65)
<223> a strand

<400> 172
cgcgcgccca cgagcgttcc gggcgcgcct tagtaacgtg ctttgatgtc gattcgacag 60
gaggc 65

<210> 173
<211> 26
<212> DNA
<213> Artificial Sequence

<220>
<221> misc_feature
<222> (1)..(26)
<223> b strand

<400> 173
gcgcccggtt cgccgccagc cgccgc 26

<210> 174
<211> 33
<212> DNA
<213> Artificial Sequence

<220>
<221> misc_feature
<222> (1)..(33)
<223> c strand

<400> 174
gcggcggcag gcggccatag ccgtgggcgc gcg 33

<210> 175
<211> 10

<212> DNA/RNA
<213> Artificial Sequence

<220>
<221> misc_feature
<222> (1)..(10)
<223> a sequence

<400> 175
cgagcgttcc 10

<210> 176
<211> 9
<212> DNA/RNA
<213> Artificial Sequence

<220>
<221> misc_feature
<222> (1)..(9)
<223> b sequence

<400> 176
ggttcgccg 9

<210> 177
<211> 12
<212> DNA/RNA
<213> Artificial Sequence

<220>
<221> misc_feature
<222> (1)..(12)
<223> c sequence

<400> 177
cggccatagc cg 12

<210> 178
<211> 34
<212> DNA
<213> Artificial Sequence

<220>
<221> misc_feature
<222> (1)..(34)
<223> a strand

<400> 178
cgcgcgcgcc cacgagcgtt ccgggcgccg ccgc 34

<210> 179
<211> 33
<212> DNA
<213> Artificial Sequence

<220>
<221> misc_feature
<222> (1)..(33)
<223> b strand

```
<400>  179
gcggcggcgc ccggttcgcc gccagccgcc gcc                                    33

<210>  180
<211>  36
<212>  DNA
<213>  Artificial Sequence

<220>
<221>  misc_feature
<222>  (1)..(36)
<223>  c strand


<400>  180
ggcggcggca ggcggccata gccgtgggcg cgcgcg                                 36
```

## Description

### Technical Field

The present application relates to the field of medicines, and in particular to a nucleic acid nanocarrier drug, a preparation method, a pharmaceutical composition and an application thereof.

### Background

Paclitaxel, also known as taxol, is the best natural anti-cancer drug that is already discovered at present. It is clinically widely used already in the treatment of breast cancer, ovarian cancer, and some head and neck cancers and lung cancers. As a diterpene alkaloid compound with anti-cancer activity, the paclitaxel has a novel and complex chemical structure, extensive and significant biological activity, a brand-new and unique mechanism of action, and scarce natural resources, it is greatly favored by botanists, chemists, pharmacologists, and molecular biologists, so it becomes world-renowned anti-cancer star and research focus in the second half of the 20th century.

A lenalidomide compound is a yellow solid, and a chemical name thereof is 3- (7-amino-3-oxo-1H-isoindol-2-yl) piperidine-2,6-dione, anti-tumor drug lenalidomide prepared from this compound is an anti-tumor drug developed by Celgene Biopharmaceutical Company of the United States. A chemical structure thereof is similar to that of thalidomide, and it has multiple effects such as anti-tumor, immune regulation and anti-angiogenesis.

Cytarabine is firstly synthesized in 1959 by Richard Walwick, Walden Roberts and Charles Dekker of the University of California, Berkeley. The U.S. Food and Drug Administration approves the cytarabine into the market in June 1969; it is originally sold by Upjohn Company in a trade name Cytosar-U. A chemical structure of this drug is a nucleoside formed by combining cytosine and arabinose, thus it is named as "cytarabine". In a normal case, the cytosine is combined with another sugar (deoxyribose) to form deoxycytidine, one of components of a DNA. However, some organisms in porifera may use the arabinose to combine with the cytosine to form another compound (not a component of the DNA). People find this compound in these organisms, namely the cytarabine. The cytarabine is very similar to the deoxycytidine so that it may be incorporated into a human DNA instead of the latter. However, a structural difference makes the DNA unable to replicate, and affected cells are killed. When being administered, the cytarabine kills cancer cells with this mechanism of action. It is the first chemotherapeutic drug which changes the nucleoside itself -- other earlier similar drugs (such as 5-fluorouracil) change a base.

Docetaxel has the same effect as the paclitaxel (PTX). It is an M-period cycle-specific drug, promotes the polymerization of tubules into stable microtubules and inhibits aggregation thereof, thereby the number of the tubules is significantly reduced, and a reticular structure of the microtubules may be destroyed. It is indicated from an experiment in vitro that it has a cytotoxic effect on a variety of mouse and human tumor cell strains, and has a broader anti-tumor spectrum than the PTX.

Idarubicin (molecular formula: $C_{26}H_{27}NO_9$, and molecular weight: 497.49) is an anthracycline cell cycle non-specific anticancer drug, it may inhibit DNA synthesis, interfere with RNA polymerase, and inhibit topoisomerase II. Potency thereof is stronger than that of daunorubicin and adriamycin. It is mainly used for the treatment of acute non-lymphocytic leukemia, advanced breast cancer, but also for the treatment of myelodysplastic syndrome and non-Hodgkin's lymphoma.

Mitoxantrone (CSA: 65271-80-9, molecular formula: $C_{22}H_{28}N_4O_6$, and molecular weight: 444.481) is an anti-tumor antibiotic, a mechanism of action thereof is similar to that of the doxorubicin. Because it has not an amino sugar structure, it does not generate a free radical, and has an effect of inhibiting lipid peroxidation, so it is less toxic to a heart. It is mainly used to treat cancers such as breast cancer, malignant lymphoma, gastric cancer, bowel cancer, leukemia, bladder cancer, liver cancer, multiple myeloma, malignant mesothelioma and ovarian cancer.

At present, anti-tumor antibiotics, including the paclitaxel, lenalidomide, cytarabine, docetaxel, idarubicin and mitoxantrone, must use large doses of chemotherapeutics in order to achieve an effective treatment level at a tumor site, but the large doses of systemic administration may damage healthy normal cells, and cause adverse reactions in a series of tissues and organs. These adverse reactions include suppression of an immune system (myelosuppression), inflammation and ulcers of mucous membranes of a digestive tract (mucositis), hair loss (alopecia), and organ-specific toxicity, such as cardiotoxicity and neurotoxicity. In order to avoid the occurrence of these adverse reactions, it is necessary to replace the traditional systemic administration with a local tumor administration mode to achieve effects of increasing the local drug concentration of the tumor and reducing the systemic drug concentration. Therefore, how to achieve such local drug delivery and controlled release in vitro becomes a focus of a cancer chemotherapy research.

In order to alleviate side effects caused by poor targeting of active ingredients of a drug, a drug delivery carrier is generated at the right moment, and a function thereof is mainly to carry the active ingredients of the drug, and deliver the active ingredients into blood or tissue cells so as to treat diseases. There are already a variety of methods to achieve the targeted delivery of the different drugs. It may be achieved with instruments or devices, such as a gene gun, and an

electroporator. These methods do not need to use a gene carrier, but the transfection efficiency is generally very low, the operation is complicated, and the damage to a tissue is relatively large. It is also mediated by viral carriers, such as an adenovirus, and a lentivirus. Although the viral carriers have the higher transfection activity in vitro, the immunogenicity and disadvantages thereof which may easily cause mutations bring huge safety risks to in vivo delivery. There are also non-viral carriers, especially a biodegradable polymer material, to achieve the targeted delivery of the drug. A main advantage of the non-viral carriers is that the immunogenicity and many inflammatory reactions brought by the viral carriers may be greatly reduced under a condition of guaranteeing the expected transfection activity.

In the above multiple targeted delivery modes, more researches are focused on the field of the non-viral carriers at present, and it is generally a plurality of the following carrier designs: (a) a cationic liposome; and (b) a polycationic gene carrier. At present, the more researches focus on modification of the polycationic gene carrier and the cationic liposome, so that it is suitable for the targeted delivery of the genetic substances. The cationic liposome has the higher transfection activity in vivo and in vitro. However, because normal distribution thereof in vivo is affected by positive charge on a surface, at the same time, the cationic liposome may cause the immunogenicity and the inflammatory responses in animal experiments. The development of the polycationic gene carrier is relatively mature at present, but it is difficult to guarantee that a targeting group is on a surface of a structure in a structural design, and there is an own design contradiction between the toxicity and the transfection activity, at the same time, connection thereof is difficult to achieve non-toxic degradation in vivo.

Therefore, how to improve the delivery reliability of an existing small molecular drug of the paclitaxel, lenalidomide, cytarabine, docetaxel, idarubicin and mitoxantrone is one of the difficulties in solving a limited clinical application of the drug of the paclitaxel, lenalidomide, cytarabine, docetaxel, idarubicin and mitoxantrone.

## Summary

A main purpose of the present application is to provide a nucleic acid nanocarrier drug, a preparation method thereof, a pharmaceutical composition and an application thereof, as to improve the delivery reliability of a drug.

In order to achieve the above purpose, according to one aspect of the present application, a nucleic acid nanocarrier drug is provided, and it includes nucleic acid nanoparticles and a drug, the drug is loaded on the nucleic acid nanoparticle, and the drug includes one or more of paclitaxel, lenalidomide, cytarabine, docetaxel, idarubicin and mitoxantrone; and the nucleic acid nanoparticles includes a nucleic acid domain, the nucleic acid domain includes a sequence a, a sequence b, and a sequence c, the sequence a includes a sequence a1 or a sequence obtained by insertion, deletion or substitution of at least one base in the sequence a1, the sequence b includes a sequence b1 or a sequence obtained by insertion, deletion or substitution of at least one base in the sequence b1, and the sequence c includes a sequence c1 or a sequence obtained by insertion, deletion or substitution of at least one base in the sequence c1, herein, the sequence a1 is SEQ ID NO: 1: 5'-CCAGCGUUCC-3' or SEQ ID NO: 2: 5'-CCAGCGTTCC-3'; the sequence b1 is SEQ ID NO: 3: 5'-GGUUCGCCG-3' or SEQ ID NO: 4: 5'-GGTTCGCCG-3'; and the sequence c1 is SEQ ID NO: 5: 5'-CGGCCAUAGCGG-3' or SEQ ID NO: 6: 5'-CGGCCATAGCGG-3'.

Further, when the sequence a1 is the SEQ ID NO: 1, the sequence b1 is the SEQ ID NO: 3, and the sequence c1 is the SEQ ID NO: 5, at least one sequence of the sequence a, the sequence b and the sequence c includes a sequence obtained by insertion, deletion or substitution of at least one base.

Further, the insertion, deletion or substitution of at least one base is occurred:

(1) on 1, 2, 4 or 5-th base starting from a 5 '-end of the sequence shown in the SEQ ID NO:1 or the SEQ ID NO:2; and/or

(2) between 8-th and 10-th bases starting from the 5 '-end of the sequence shown in the SEQ ID NO:1 or the SEQ ID NO:2; and/or

(3) between 1-th and 3-th bases starting from a 5 '-end of the sequence shown in the SEQ ID NO:3 or the SEQ ID NO:4; and/or

(4) between 6-th and 9-th bases starting from the 5 '-end of the sequence shown in the SEQ ID NO:3 or the SEQ ID NO:4; and/or

(5) between 1-th and 4-th bases starting from a 5 '-end of the sequence shown in the SEQ ID NO:5 or the SEQ ID NO:6; and/or

(6) between 9-th and 12-th bases starting from the 5 '-end of the sequence shown in the SEQ ID NO:5 or the SEQ ID NO:6.

Further, the sequence a, the sequence b and the sequence c are self-assembled into a structure shown in Formula (1):

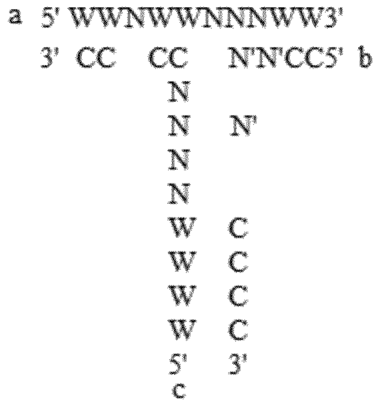


Formula (1),

Herein, W-C represents Watson-Crick pairing, N and N' represent non-Watson-Crick pairing, the W-C in any one position is independently selected from C-G or G-C; in the sequence a, the first N from the 5'-end is A, the second N is G, the third N is U or T, and the fourth N is any one of U, T, A, C or G; in the sequence b, the first N' from the 5'-end is any one of U, T, A, C or G, the second N' is U or T, and the third N' is C; and in the sequence c, a sequence NNNN along a direction from the 5'-end to the 3'-end is CAUA or CATA.

Further, the sequence a, the sequence b and the sequence c are any one of the following groups: (1) sequence a: 5'-GGAGCGUUGG-3', sequence b: 5'-CCUUCGCCG-3', sequence c: 5'-CGGCCAUAGCCC-3'; (2) sequence a: 5'-GCAGCGUUCG-3', sequence b: 5'-CGUUCGCCG-3', sequence c: 5'-CGGCCAUAGCGC-3'; (3) sequence a: 5'-CGAGCGUUGC-3', sequence b: 5'-GCUUCGCCG-3', sequence c: 5'-CGGCCAUAGCCG-3'; (4) sequence a: 5'-GGAGCGUUGG-3', sequence b: 5'-CCUUCGGGG-3', sequence c: 5'-CCCCCAUAGCCC-3'; (5) sequence a: 5'-GCAGCGUUCG-3', sequence b: 5'-CGUUCGGCG-3', sequence c: 5'-CGCCCAUAGCGC-3'; (6) sequence a: 5'-GCAGCGUUCG-3', sequence b: 5'-CGUUCGGCC-3', sequence c: 5'-GGCCCAUAGCGC-3'; (7) sequence a: 5'-CGAGCGUUGC-3', sequence b: 5'-GCUUCGGCG-3', sequence c: 5'-CGCCCAUAGCCG-3'; (8) sequence a: 5'-GGAGCGTTGG-3', sequence b: 5'-CCTTCGCCG-3', sequence c: 5'-CGGCCATAGCCC-3'; (9) sequence a: 5'-GCAGCGTTCG-3', sequence b: 5'-CGTTCGCCG-3', sequence c: 5'-CGGCCATAGCGC-3'; (10) sequence a: 5'-CGAGCGTTGC-3', sequence b: 5'-GCTTCGCCG-3', sequence c: 5'-CGGCCATAGCCG-3'; (11) sequence a: 5'-GGAGCGTTGG-3', sequence b: 5'-CCTTCGGGG-3', sequence c: 5'-CCCCCATAGCCC-3'; (12) sequence a: 5'-GCAGCGTTCG-3', sequence b: 5'-CGTTCGGCG-3', sequence c: 5'-CGCCCATAGCGC-3'; (13) sequence a: 5'-GCAGCGTTCG-3', sequence b: 5'-CGTTCGGCC-3', sequence c: 5'-GGCCCATAGCGC-3'; (14) sequence a: 5'-CGAGCGTTGC-3', sequence b: 5'-GCTTCGGCG-3', sequence c: 5'-CGCCCATAGCCG-3'; and (15) sequence a: 5'-CGAGCGTTCC -3', sequence b: 5'-GGTTCGCCG -3', sequence c: 5'-CGGCCATAGCCG-3'.

Further, the nucleic acid domain further includes a first extension fragment, the first extension fragment is an extension fragment of Watson-Crick pairing, and the first extension fragment is positioned at the 5'-end and/or the 3'-end of any one sequence of the sequence a, the sequence b or the sequence c; preferably, the first extension fragment is selected from any one of the following groups: (1): a-strand 5'-end: 5'-CCCA-3', c-strand 3'-end: 5'-UGGG-3'; (2): a-strand 3'-end: 5'-GGG-3', b-strand 5'-end: 5'-CCC-3'; (3): b-strand 3'-end: 5'-CCA-3', c-strand 5'-end: 5'-UGG-3'; (4): a-strand 5'-end: 5'-CCCG-3', c-strand 3'-end: 5'-CGGG-3'; (5): a-strand 5'-end: 5'-CCCC-3', c-strand 3'-end: 5'-GGGG-3'; (6): b-strand 3'-end: 5'-CCC-3', c-strand 5'-end: 5'-GGG-3'; (7): b-strand 3'-end: 5'-CCG-3', c-strand 5'-end: 5'-CGG-3'; (8): a-strand 5'-end: 5'-CCCA-3', c-strand 3'-end: 5'-TGGG-3'; and (9): b-strand 3'-end: 5'-CCA-3', c-strand 5'-end: 5'-TGG-3'.

Further, the nucleic acid domain further includes a second extension fragment, the second extension fragment is positioned at the 5'-end and/or the 3'-end of any one sequence of the sequence a, the sequence b or the sequence c, and the second extension fragment is an extension fragment of Watson-Crick pairing; preferably, the second extension fragment is an extension sequence of a CG base pair; more preferably, the second extension fragment is an extension sequence of 1-10 CG base pairs.

Further, the nucleic acid domain further includes at least one group of the following second extension fragments: a first group: a-strand 5'-end: 5'-CGCGCG-3', c-strand 3'-end: 5'-CGCGCG-3'; a second group: a-strand 3'-end: 5'-CGCCGC-3', b-strand 5'-end: 5'-GCGGCG-3'; and a third group: b-strand 3'-end: 5'-GGCGGC-3', c-strand 5'-end: 5'-GCCGCC-3'.

Further, the second extension fragment is an extension sequence containing both CG base pair and AT/AU

base pair, and preferably the second extension fragment is an extension sequence of 2-50 base pairs.

Further, the second extension fragment is an extension sequence in which sequences of 2-8 continuous CG base pairs and sequences of 2-8 continuous AT/AU base pairs are alternately arranged; or the second extension fragment is an extension sequence in which a sequence of 1 CG base pair and a sequence of 1 AT/AU base pair are alternately arranged.

Further, a base, a ribose and a phosphate in the sequence a, the sequence b and the sequence c have at least one modifiable site, and any one of the modifiable sites is modified by any one of the following modification adapters: -F, a methyl, an amino, a disulfide, a carbonyl, a carboxyl, a sulfhydryl and a formyl; and preferably, the base C or U in the sequence a, the sequence b and the sequence c has 2'-F modification.

Further, the drug is loaded on the nucleic acid nanoparticle in modes of physical linkage and/or covalent linkage, and a molar ratio between the drug and the nucleic acid nanoparticles is 2-300:1, preferably 10-50:1, and more preferably 15-25:1.

Further, the nucleic acid nanoparticles further include a bioactive substance, the bioactive substance is linked with the nucleic acid domain, and the bioactive substance is one or more of a target head, a fluorescein, an interfering nucleic acid siRNA, a miRNA, a ribozyme, a riboswitch, an aptamer, a RNA antibody, a protein, a polypeptide, a flavonoid, a glucose, a natural salicylic acid, a monoclonal antibody, a vitamin, phenol, a lecithin, and a small molecular drug except the paclitaxel, lenalidomide, cytarabine, docetaxel, idarubicin and mitoxantrone.

Further, a relative molecular weight of the nucleic acid domain is marked as N1, and a total relative molecular weight of the drug and the bioactive substance is marked as N2, $N_1/N_2 \geq 1:1$.

Further, the bioactive substance is one or more of the target head, the fluorescein and the miRNA, herein the target head is positioned on any one sequence of the sequence a, the sequence b and the sequence c, preferably the 5'-end or the 3'-end of any one sequence of the sequence a, the sequence b and the sequence c, or inserted between GC bonds of the nucleic acid domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and preferably, the target head is a folic acid or a biotin, the fluorescein is any one or more of FAM, CY5 and CY3, and the anti-miRNA is anti-miR-21.

Further, the small molecular drug except the paclitaxel, lenalidomide, cytarabine, docetaxel, idarubicin and mitoxantrone is a drug containing any one or more of the following groups: an amino group, a hydroxyl group, a carboxyl group, a mercapto group, a benzene ring group and an acetamido group.

Further, the protein is one or more of SOD, survivin, hTERT, EGFR and PSMA; the vitamin is L-Vc and/or esterified Vc; and the phenol is a tea polyphenols and/or a grape polyphenol.

Further, a particle size of the nucleic acid nanoparticle is 1-100 nm, preferably 5-50 nm; more preferably 10-30 nm; and further preferably 10-15 nm.

According to another aspect of the present application, a method for preparing a nucleic acid nanocarrier drug is further provided, and it includes the following steps: providing the above nucleic acid nanoparticles; and enabling a drug to be loaded on the nucleic acid nanoparticle in modes of physical linkage and/or covalent linkage, to obtain the nucleic acid nanocarrier drug.

Further, the step of loading the drug in the physical linkage mode includes: mixing and stirring the drug, the nucleic acid nanoparticle and a first solvent, to obtain a premixed system; and precipitating the premixed system, to obtain the nucleic acid nanocarrier drug; preferably, the first solvent is selected from one or more of DCM, DCC, DMAP, Py, DMSO, PBS and glacial acetic acid; preferably, the step of precipitating the premixed system, to obtain the nucleic acid nanocarrier drug includes: precipitating the premixed system, to obtain a precipitation; and washing the precipitation to remove impurities, as to obtain the nucleic acid nanocarrier drug; more preferably, mixing the premixed system with absolute ethyl alcohol, and precipitating at a temperature condition lower than 10 DEG C, to obtain the precipitation; and more preferably, precipitating at a temperature condition of 0-5 DEG C, to obtain the precipitation; and more preferably, washing the precipitation to remove the impurities with 6-12 times of the absolute ethyl alcohol in volume, as to obtain the nucleic acid nanocarrier drug.

Further, the step of loading the drug in the covalent linkage mode includes: preparing a drug solution; enabling the drug solution to react with the G-exocyclic amino of the nucleic acid nanoparticles under a mediating effect of the formaldehyde, to obtain a reaction system; and purifying the reaction system, to obtain the nucleic acid nanocarrier drug; preferably, the reaction step includes: mixing the drug solution with paraformaldehyde solution and the nucleic acid nanoparticles, and reacting in a dark condition, to obtain the reaction system; herein the concentration of the paraformaldehyde solution is preferably 3.7-4wt%, and the paraformaldehyde solution is preferably a solution formed by mixing paraformaldehyde and a second solvent, and the second solvent is one or more of DCM, DCC, DMAP, Py, DMSO, PBS and glacial acetic acid.

Further, the preparation method further includes a step of preparing the nucleic acid nanoparticles, the step includes: self-assembling a single strand corresponding to the above nucleic acid domain, to obtain the nucleic acid domain; preferably, after the nucleic acid domain is obtained, the preparation method further includes: loading the above

bioactive substance on the nucleic acid domain in the modes of physical linkage and/or covalent linkage, to obtain the nucleic acid nanoparticle.

Further, in a process of loading the bioactive substance in the covalent linkage mode, the loading is performed through solvent covalent linkage, linker covalent linkage or click-linkage; preferably, a third solvent used in the solvent covalent linkage is served as a linkage medium, and the third solvent is selected from one or more of paraformaldehyde, DCM, DCC, DMAP, Py, DMSO, PBS and glacial acetic acid; preferably, the linker is selected from a disulfide bond, a p-phenylazide, bromopropyne or PEG; preferably, the click-linkage is that a bioactive substance precursor and the nucleic acid domain are modified by alkynyl or azide modification simultaneously, and then linked through a click reaction.

Further, the bioactive substance is linked with the nucleic acid domain in the click-linkage mode, a site, for performing the alkynyl or azide modification, of the bioactive substance precursor is selected from a 2'-hydroxyl, a carboxyl or an amino, and a site, for performing the alkynyl or azide modification, of the nucleic acid domain is selected from a G-exocyclic amino, a 2'-hydroxyl, an $\alpha$-amino or a 2'-hydroxyl.

According to a third aspect of the present application, a pharmaceutical composition is further provided, and the pharmaceutical composition includes any one of the above nucleic acid nanocarrier drugs.

According to a fourth aspect of the present application, use of any one of the above nucleic acid nanocarrier drugs in preparing a drug for treating a tumor is further provided.

Further, when the drug is the paclitaxel, the tumor is breast cancer or ovarian cancer; when the drug is the lenalidomide, the tumor is acute leukemia or multiple myeloma; when the drug is the cytarabine, the tumor is any one or more of acute leukemia, malignant lymphoma, lung cancer, digestive tract cancer, colorectal cancer, and head and neck cancer; when the drug is the docetaxel, the tumor is any one or more of breast cancer, ovarian cancer, non-small cell lung cancer, head and neck cancer, pancreatic cancer, small cell lung cancer, gastric cancer, melanoma, and soft tissue sarcoma; when the drug is the idarubicin, the tumor is any one or more of acute pulmonary lymphocytic leukemia, advanced breast cancer and non-Hodgkin's lymphoma; and when the drug is the mitoxantrone, the tumor is any one or more of breast cancer, malignant lymphoma, gastric cancer, bowel cancer, leukemia, bladder cancer, liver cancer, multiple myeloma, malignant mesothelioma, and ovarian cancer.

Further, use of the nucleic acid nanocarrier drug in preparing a drug for treating viral keratitis and epidemic conjunctivitis is provided, herein the drug contained in the nucleic acid nanocarrier drug is the cytarabine.

Further, use of the nucleic acid nanocarrier drug in preparing a drug for treating myelodysplastic syndrome is provided, herein the drug contained in the nucleic acid nanocarrier drug is the idarubicin.

According to a fifth aspect of the present application, a method for preventing and/or treating a tumor is further provided, the method includes: providing any one of the above nucleic acid nanocarrier drugs or pharmaceutical compositions; and administering an effective dose of the above nucleic acid nanocarrier drug or pharmaceutical composition to a tumor patient.

Further, the tumor is the breast cancer or the ovarian cancer.

The nucleic acid nanocarrier drug provided by the present application includes the nucleic acid nanoparticles and the drug, and the drug is loaded on the nucleic acid nanoparticle in the modes of the physical linkage and/or the covalent linkage, herein, the drug includes one or more of the paclitaxel, lenalidomide, cytarabine, docetaxel, idarubicin and mitoxantrone. The nucleic acid nanoparticles, through including the three sequences provided by the present application or the variant sequences thereof, not only may be self-assembled to form the nucleic acid domain, but also may be served as a carrier to link the drug at the arbitrary 5'-end and/or 3'-end of the three strands, or enable these drugs to be stably inserted between the strands of the nucleic acid domain. The present application is capable of, through loading the small molecular drug on the nucleic acid nanoparticles, using internal hydrophobicity, external hydrophilicity and a base stacking effect of the nucleic acid nanoparticles to have a "coating effect" on the drug, and the drug is not dissolved within a certain period of time through the coating effect or the covalent linkage, so the delivery stability is improved. In addition, when the nucleic acid domain is modified by the target head, it may have the better targeting property, and may deliver the drug stably, the reliability is very high; at the same time, it may reduce a chance of the drug in contact with non-target cells or tissues, toxic side effects are reduced.

## Brief Description of the Drawings

Drawings of the description for constituting a part of the present application are used to provide further understanding of the disclosure, exemplary embodiments of the disclosure and descriptions thereof are used to explain the present application, and do not constitute improper limitation to the present application. In the drawings:

Fig. 1 shows an electrophoresis detection result of RNA nanoparticles formed by self-assembly in Embodiment 1 of the present application.

Fig. 2 shows an electrophoresis detection result of DNA nanoparticles formed by self-assembly in Embodiment 1

of the present application.

Fig. 3 shows a 2% agarose gel electrophoresis detection result of 7 groups of short-sequence RNA nanoparticles formed by self-assembly in Embodiment 2 of the present application.

Fig. 4 shows a 4% agarose gel electrophoresis detection result of 7 groups of short-sequence RNA nanoparticles formed by self-assembly in Embodiment 2 of the present application.

Fig. 5 shows a 2% agarose gel electrophoresis detection result of 7 groups of conventional sequence RNA nanoparticles formed by self-assembly in Embodiment 3 of the present application.

Fig. 6 shows a 4% agarose gel electrophoresis detection result of 7 groups of conventional sequence RNA nanoparticles formed by self-assembly in Embodiment 3 of the present application.

Fig. 7 shows a 2% agarose gel electrophoresis detection result of 7 groups of conventional sequence DNA nanoparticles formed by self-assembly in Embodiment 4 of the present application.

Fig. 8 shows a 4% agarose gel electrophoresis detection result of 7 groups of conventional sequence DNA nanoparticles formed by self-assembly in Embodiment 4 of the present application.

Fig. 9 shows a 2% agarose gel electrophoresis detection result of 8-th and 9-th groups of DNA nanoparticles formed by self-assembly in Embodiment 4 of the present application.

Fig. 10 shows a transmission electron microscope picture of conventional sequence DNA nanoparticles D-7 formed by self-assembly in Embodiment 4 of the present application.

Fig. 11a shows a standard curve of a paclitaxel absorbance in a RNA nanoparticle loading rate detection process in Embodiment 5 of the present application.

Fig. 11b shows a standard curve of a paclitaxel absorbance in a DNA nanoparticle loading rate detection process in Embodiment 5 of the present application.

Fig. 12 shows an electrophoresis detection result of DNAh-Bio-EGFRapt-Cy5-paclitaxel nanoparticles after being incubated in serum for different times in Embodiment 7 of the present application.

Fig. 13 shows detection results of small molecular drug paclitaxel and RNAh-Biotin-quasar670-paclitaxel nanoparticles for inhibiting proliferation of U87MG cells in Embodiment 8 of the present application.

Fig. 14a to Fig. 14d show detection results of DNAh-Bio-EGFRapt-Cy5-paclitaxel nanoparticles for inhibiting proliferation of SKOV3 cells in Embodiment 9, herein, Fig. 14a is a proliferation inhibition situation of the small molecular drug paclitaxel to the SKOV3 cells,

Fig. 14b is a proliferation inhibition situation of the DNAh-Bio-EGFRapt-Cy5-paclitaxel (targeted drug) to the SKOV3 cells, Fig. 14c is a proliferation inhibition situation of DNAh-Bio-EGFRapt-Cy5 (targeted fluorescent carrier) to the SKOV3 cells, and Fig. 14d is a proliferation inhibition situation of a blank control of DMSO to the SKOV3 cells.

Fig. 15 shows non-denaturing PAGE gel electrophoresis detection results of 7 groups of extension fragment deformation + core short-sequence RNA self-assembly products in Embodiment 10 of the disclosure.

Fig. 16 shows a solubility curve of RNA nanoparticles R-15 in Embodiment 10 of the disclosure.

Fig. 17 shows a solubility curve of RNA nanoparticles R-16 in Embodiment 10 of the disclosure.

Fig. 18 shows a solubility curve of RNA nanoparticles R-17 in Embodiment 10 of the disclosure.

Fig. 19 shows a solubility curve of RNA nanoparticles R-18 in Embodiment 10 of the disclosure.

Fig. 20 shows a solubility curve of RNA nanoparticles R-19 in Embodiment 10 of the disclosure.

Fig. 21 shows a solubility curve of RNA nanoparticles R-20 in Embodiment 10 of the disclosure.

Fig. 22 shows a solubility curve of RNA nanoparticles R-21 in Embodiment 10 of the disclosure.

Fig. 23 shows non-denaturing PAGE gel electrophoresis detection results of 7 groups of extension fragment deformation + core short-sequence DNA self-assembly products in Embodiment 11 of the disclosure.

Fig. 24 shows a solubility curve of DNA nanoparticles D-8 in Embodiment 11 of the disclosure.

Fig. 25 shows a solubility curve of DNA nanoparticles D-9 in Embodiment 11 of the disclosure.

Fig. 26 shows a solubility curve of DNA nanoparticles D-10 in Embodiment 11 of the disclosure.

Fig. 27 shows a solubility curve of DNA nanoparticles D-11 in Embodiment 11 of the disclosure.

Fig. 28 shows a solubility curve of DNA nanoparticles D-12 in Embodiment 11 of the disclosure.

Fig. 29 shows a solubility curve of DNA nanoparticles D-13 in Embodiment 11 of the disclosure.

Fig. 30 shows a solubility curve of DNA nanoparticles D-14 in Embodiment 11 of the disclosure.

Fig. 31 shows an electrophoresis detection result of RNA nanoparticles R-15 after being incubated in serum for different times in Embodiment 12 of the disclosure.

Fig. 32 shows an electrophoresis detection result of RNA nanoparticles R-16 after being incubated in serum for different times in Embodiment 12 of the disclosure.

Fig. 33 shows an electrophoresis detection result of RNA nanoparticles R-17 after being incubated in serum for different times in Embodiment 12 of the disclosure.

Fig. 34 shows an electrophoresis detection result of RNA nanoparticles R-18 after being incubated in serum for different times in Embodiment 12 of the disclosure.

Fig. 35 shows an electrophoresis detection result of RNA nanoparticles R-19 after being incubated in serum for different times in Embodiment 12 of the disclosure.

Fig. 36 shows an electrophoresis detection result of RNA nanoparticles R-20 after being incubated in serum for different times in Embodiment 12 of the disclosure.

Fig. 37 shows an electrophoresis detection result of RNA nanoparticles R-21 after being incubated in serum for different times in Embodiment 12 of the disclosure.

Fig. 38 shows an electrophoresis detection result of DNA nanoparticles D-8 after being incubated in serum for different times in Embodiment 13 of the disclosure.

Fig. 39 shows an electrophoresis detection result of DNA nanoparticles D-9 after being incubated in serum for different times in Embodiment 13 of the disclosure.

Fig. 40 shows an electrophoresis detection result of DNA nanoparticles D-10 after being incubated in serum for different times in Embodiment 13 of the disclosure.

Fig. 41 shows an electrophoresis detection result of DNA nanoparticles D-11 after being incubated in serum for different times in Embodiment 13 of the disclosure.

Fig. 42 shows an electrophoresis detection result of DNA nanoparticles D-12 after being incubated in serum for different times in Embodiment 13 of the disclosure.

Fig. 43 shows an electrophoresis detection result of DNA nanoparticles D-13 after being incubated in serum for

different times in Embodiment 13 of the disclosure.

Fig. 44 shows an electrophoresis detection result of DNA nanoparticles D-14 after being incubated in serum for different times in Embodiment 13 of the disclosure.

Fig. 45a, Fig. 45b, Fig. 45c, Fig. 45d, Fig. 45e, Fig. 45f, Fig. 45g, and Fig. 45h respectively show cell survival rate curves corresponding to DMSO and original drug doxorubicin, D-8 and D-8-doxorubicin, D-9 and D-9-doxorubicin, D-10 and D-10-doxorubicin, D-11 and D-11-doxorubicin, D-12 and D-12-doxorubicin, D-13 and D-13-doxorubicin, and D-14 and D-14-doxorubicin in Embodiment 16 of the disclosure.

Fig. 46 shows a standard curve of a daunorubicin absorbance used in a loading rate detection process of Embodiment 17.

Fig. 47a shows a standard curve of a lenalidomide absorbance in a RNA nanoparticle loading rate detection process in Embodiment 18 of the present application.

Fig. 47b shows a standard curve of a lenalidomide absorbance in a DNA nanoparticle loading rate detection process in Embodiment 18 of the present application.

Fig. 48 shows an electrophoresis detection result of DNAh-Bio-EGFRapt-Cy5-lenalidomide nanoparticles after being incubated in serum for different times in Embodiment 20 of the present application.

Fig. 49a to Fig. 49d show detection results of DNAh-Bio-EGFRapt-Cy5-lenalidomide nanoparticles for inhibiting proliferation of RPMI 8226 cells in Embodiment 21, herein, Fig. 49a is a proliferation inhibition situation of the small molecular drug lenalidomide to the RPMI 8226 cells, Fig. 49b is a proliferation inhibition situation of the DNAh-Bio-EGFRapt-Cy5-lenalidomide (targeted drug) to the RPMI 8226 cells, Fig. 49c is a proliferation inhibition situation of DNAh-Bio-EGFRapt-Cy5 (targeted fluorescent carrier) to the RPMI 8226 cells, and Fig. 49d is a proliferation inhibition situation of a blank control of DMSO to the RPMI 8226 cells.

Fig. 50 shows a standard curve of a cytarabine absorbance in a DNA nanoparticle loading rate detection process in Embodiment 22 of the present application.

Fig. 51 shows an electrophoresis detection result of DNAh-Bio-EGFRapt-Cy5-cytarabine nanoparticles after being incubated in serum for different times in Embodiment 24 of the present application.

Fig. 52a to Fig. 52d show detection results of DNAh-Bio-EGFRapt-Cy5-cytarabine nanoparticles for inhibiting proliferation of MCF-7 cells in Embodiment 25, herein, Fig. 52a is a proliferation inhibition situation of the small molecular drug cytarabine to the MCF-7 cells,

Fig. 52b is a proliferation inhibition situation of the DNAh-Bio-EGFRapt-Cy5-cytarabine (targeted drug) to the MCF-7 cells, Fig. 52c is a proliferation inhibition situation of DNAh-Bio-EGFRapt-Cy5 (targeted fluorescent carrier) to the MCF-7 cells, and Fig. 52d is a proliferation inhibition situation of a blank control of DMSO to the MCF-7 cells.

Fig. 53a shows a standard curve of a docetaxel absorbance in a RNA nanoparticle loading rate detection process in Embodiment 26 of the present application.

Fig. 53b shows a standard curve of a docetaxel absorbance in a DNA nanoparticle loading rate detection process in Embodiment 26 of the present application.

Fig. 54 shows an electrophoresis detection result of DNAh-Bio-EGFRapt-Cy5-docetaxel nanoparticles after being incubated in serum for different times in Embodiment 28 of the present application.

Fig. 55 shows detection results of small molecular drug docetaxel and RNAh-Biotin-quasar670-docetaxel nanoparticles for inhibiting proliferation of HCT116 cells in Embodiment 29 of the present application.

Fig. 56a to Fig. 56d show detection results of DNAh-Bio-EGFRapt-Cy5-docetaxel nanoparticles for inhibiting proliferation of MCF-7 cells in Embodiment 30, herein, Fig. 56a is a proliferation inhibition situation of the small molecular drug docetaxel to the MCF-7 cells,

Fig. 56b is a proliferation inhibition situation of the DNAh-Bio-EGFRapt-Cy5-docetaxel (targeted drug) to the MCF-7 cells, Fig. 56c is a proliferation inhibition situation of DNAh-Bio-EGFRapt-Cy5 (targeted fluorescent carrier) to the MCF-7 cells, and Fig. 56d is a proliferation inhibition situation of a blank control of DMSO to the MCF-7 cells.

Fig. 57a to Fig. 57d show detection results of DNAh-Bio-EGFRapt-Cy5-docetaxel nanoparticles for inhibiting proliferation of SKOV3 cells in Embodiment 30, herein, Fig. 57a is a proliferation inhibition situation of the small molecular drug docetaxel to the SKOV3 cells,

Fig. 57b is a proliferation inhibition situation of the DNAh-Bio-EGFRapt-Cy5-docetaxel (targeted drug) to the SKOV3 cells, Fig. 57c is a proliferation inhibition situation of DNAh-Bio-EGFRapt-Cy5 (targeted fluorescent carrier) to the SKOV3 cells, and Fig. 57d is a proliferation inhibition situation of a blank control of DMSO to the SKOV3 cells.

Fig. 58 shows a standard curve of a mitoxantrone absorbance in a DNA nanoparticle loading rate detection process in Embodiment 31 of the present application.

Fig. 59 shows an electrophoresis detection result of DNAh-Bio-EGFRapt-Cy5-Mit nanoparticles after being incubated in serum for different times in Embodiment 32 of the present application.

Fig. 60 is a proliferation inhibition situation of the small molecular drug mitoxantrone to the MCF-7 cells in Embodiment 33.

Fig. 61 is a proliferation inhibition situation of the DNAh-Bio-EGFRapt-Cy5-Mit (targeted drug) to the MCF-7 cells in Embodiment 33.

Fig. 62 is a proliferation inhibition situation of the DNAh-Bio-EGFRapt-Cy5 (targeted fluorescent carrier) to the MCF-7 cells in Embodiment 33.

Fig. 63 is a proliferation inhibition situation of the blank control of the DMSO to the MCF-7 cells in Embodiment 33.

Fig. 64 shows a standard curve of a idarubicin absorbance in a DNA nanoparticle loading rate detection process in Embodiment 34 of the present application.

Fig. 65 shows an electrophoresis detection result of DNAh-Bio-EGFRapt-Cy5-idarubicin nanoparticles after being incubated in serum for different times in Embodiment 36 of the present application.

Fig. 66a to Fig. 66d show detection results of DNAh-Bio-EGFRapt-Cy5-idarubicin nanoparticles for inhibiting proliferation of MCF-7 cells in Embodiment 37, herein, Fig. 66a is a proliferation inhibition situation of the small molecular drug idarubicin to the MCF-7 cells,

Fig. 66b is a proliferation inhibition situation of the DNAh-Bio-EGFRapt-Cy5-idarubicin (targeted drug) to the MCF-7 cells, Fig. 66c is a proliferation inhibition situation of DNAh-Bio-EGFRapt-Cy5 (targeted fluorescent carrier) to the MCF-7 cells, and Fig. 66d is a proliferation inhibition situation of a blank control of DMSO to the MCF-7 cells.

Fig. 67a to Fig. 67d show detection results of DNAh-Bio-EGFRapt-Cy5-idarubicin nanoparticles for inhibiting proliferation of MV4-11 cells in Embodiment 37, herein, Fig. 67a is a proliferation inhibition situation of the small molecular drug idarubicin to the MV4-11 cells,

Fig. 67b is a proliferation inhibition situation of the DNAh-Bio-EGFRapt-Cy5-idarubicin (targeted drug) to the MV4-11 cells, Fig. 67c is a proliferation inhibition situation of DNAh-Bio-EGFRapt-Cy5 (targeted fluorescent carrier) to the MV4-11 cells, and Fig. 67d is a proliferation inhibition situation of a blank control of DMSO to the MV4-11 cells.

## Detailed Description of the Embodiments

It is to be noted that embodiments in the present application and features in the embodiments may be combined with each other under a condition without conflicting. The present application is described in detail below with reference to the embodiments.

Term explanation:

RNAh, DNAh or Blank carrier: refers to a blank nucleic acid nanoparticle carrier without containing any bioactive substances, such as an RNAh or a DNAh.

Targeting carrier: refers to a nucleic acid nanoparticle carrier which contains a target head but does not contain a fluorescent substance, such as a Biotin-RNAh or a Biotin-DNAh.

Fluorescent carrier: refers to a nucleic acid nanoparticle carrier which contains the fluorescent substance but does not contain the target head, such as a Cy3-RNAh or a Cy3-DNAh.

Targeted fluorescent carrier: refers to a nucleic acid nanoparticle carrier containing the target head and the fluorescent substance, such as an RNAh-Biotin-FAM or a DNAh-Biotin-FAM. Targeted drug: refers to a nucleic acid nanoparticle carrier containing the target head, the fluorescent substance and a chemical, such as a RNAh-Biotin-quasar670-paclitaxel or a DNAh-Biotin-quasar670-paclitaxel.

It is to be noted that there is no special format for a naming rule of each carrier or bioactive substance in the present application, and front and rear positions thereof in the expression do not mean that it is at a 5'end or a 3'end of the RNAh or the DNAh, but only mean that the bioactive substance is contained.

As mentioned in the background, although there are a variety of drug carriers for improving drug delivery efficiency in the prior art, it is still difficult to solve the problem of the limited clinical applications of the drugs. In order to improve this situation, the inventor of the present application researches on all existing materials which may be used as the drug carriers, and deeply investigates and analyzes the various carriers in aspects, such as cell/tissue targeting of the carrier, stability in a delivery process, activity and efficiency of entering target cells, drug release ability after reaching the target cells and toxicity to cells, it is discovered that an emerging nanostructure formed by self-assembly of DNA and/or RNA molecules, such as a DNA in a self-assembly system of DNA dendrimers, is used to have a significant obstruction effect to degradation of a nuclease, and have a very important application value in the fields of gene therapy and biomedicine.

Through analyzing the existing reported nanoparticles formed by the self-assembly of the DNA and RNA, it is discovered that, compared with the relatively rigid DNA nanoparticles, the RNA nanoparticles have larger flexibility and stronger tension because there are a large number of stem-loop structures within or between molecules, so it has more advantages in an aspect as a candidate drug carrier. However, the RNA nanoparticles in a natural state are relatively poor in stability, and the existing improvements based on application aspects of the RNA nanocarriers are mostly focused on improving the stability and reliability thereof. Although research results at present provide the possibility of loading the drug to a certain extent, they are more focused on researching the possibility and effectiveness of loading nucleic acid drugs, especially a siRNA drug or a miRNA drug and the like. There are few reports at present on whether non-nucleic acid drugs are equally effective. In addition, the existing self-assembled nanoparticles, especially the self-assembled nanoparticles used as carriers, are self-assembled by using a RNA strand, and a very few is self-assembled in a mode of a RNA strand and DNA strand combination, but the self-assembly is achieved without using a pure DNA strand.

In order to provide a new RNA nanoparticle carrier with good reliability and self-assembly, the existing RNA nanoparticles are compared and improved by the applicant, a series of new RNA nanoparticles are developed, and in view of improving applicability and reducing cost, the self-assembly is further tried to be performed by using the pure DNA strand, it is unexpectedly discovered that after being changed, these DNA single strands may not only self-assemble into the DNA nanoparticles, but also have the same excellent performance as the RNA nanoparticles. In addition, the self-assembly of the DNA nanoparticles also has advantages of low price and easy operation. After being verified by experiments, both the RNA nanoparticles and the DNA nanoparticles improved by the inventor may be loaded with various drugs, and may exist stably in serum; and further verified by the experiments, it may carry the drugs into the cells, and the separate carrier is non-toxic to the cells. However, the drug-carried carrier may have alleviating and treating effects to corresponding diseases.

On the basis of the above research result, the applicant provides a technical scheme of the present application. The present application provides a nucleic acid nanocarrier drug, the drug includes nucleic acid nanoparticles and a drug, the drug is loaded on the nucleic acid nanoparticle; and the nucleic acid nanoparticle includes a nucleic acid domain, the nucleic acid domain includes a sequence a, a sequence b and a sequence c, the sequence a includes a sequence a1 or a sequence obtained by insertion, deletion or substitution of at least one base in the sequence a1, the sequence b includes a sequence b1 or a sequence obtained by insertion, deletion or substitution of at least one base in the sequence b1, and the sequence c includes a sequence c1 or a sequence obtained by insertion, deletion or substitution of at least one base in the sequence c1, herein the sequence a1 is SEQ ID NO:1: 5'-CCAGCGUUCC-3'or SEQ ID NO:2: 5'-CCAGCGTTCC-3'; the sequence b1 is SEQ ID NO:3: 5'-GGUUCGCCG-3'or SEQ ID NO:4: 5'-GGTTCGCCG-3'; and the sequence c1 is SEQ ID NO:5: 5'-CGGCCAUAGCGG-3'or SEQ ID NO:6: 5'-CGGCCATAGCGG-3'.

The nucleic acid nanocarrier drug provided by the present application includes the nucleic acid nanoparticles and the drug, the drug is loaded on the nucleic acid nanoparticle in the modes of physical linkage and/or covalent linkage, and the drug includes one or more of paclitaxel, lenalidomide, cytarabine, docetaxel, idarubicin and mitoxantrone. The nucleic acid nanoparticles, through including the above three sequences or the variant sequences thereof, not only may be self-assembled to form the nucleic acid domain, but also may be served as a carrier to link the drug at the arbitrary 5'-end and/or 3'-end of the three strands, or enable these drugs to be stably inserted between the strands of the nucleic acid domain. The present application is capable of, through loading the small molecular drug on the nucleic acid nanoparticles, using internal hydrophobicity, external hydrophilicity and a base stacking effect of the nucleic acid nanoparticles to have a "coating effect" on the drug, and the drug is not dissolved within a certain period of time through the coating effect or the covalent linkage, so the delivery stability is improved. In addition, when the nucleic acid domain is modified by the target head, it may have the better targeting property, and may deliver the drug stably, the reliability is very high; at the same time, it may reduce a chance of the drug in contact with non-target cells or tissues, toxic side effects are reduced.

The above self-assembly refers to a technology that basic structural units spontaneously form an ordered structure. In a process of the self-assembly, the basic structural units spontaneously organize or aggregate into a stable structure with a certain regular geometric appearance under an interaction based on a non-covalent bond. The self-assembly process is not a simple superposition of weak interaction forces (herein the "weak interaction forces" refer to a hydrogen bond, a Van der Waals force, an electrostatic force, a hydrophobic action force and the like) between a large number of atoms, ions or molecules, but a tight and orderly whole formed by simultaneously spontaneously parallel connection and aggregation between a plurality of individuals, and is an overall complicated synergistic effect.

The production of the self-assembly requires two conditions: self-assembly power and guiding effect. The self-assembly power refers to the synergistic effect of the weak interaction forces between the molecules, and it provides energy for molecular self-assembly. The guiding effect of the self-assembly refers to complementary of the molecules in space, namely the production of the self-assembly needs to meet requirements of molecular rearrangement in size and direction of the space.

A DNA nanotechnology is a bottom-up molecular self-assembly mode, a stable structure is spontaneously formed by using a molecular structure as a starting point on the basis of physical and chemical properties of the nucleic acid nanoparticles, and a strict nucleic acid base pairing principle is followed. Multiple DNA fragments are linked together in vitro in a correct sequence, a sub-assembly structure is established through the base complementary pairing principle, and finally a complicated multi-level structure is formed. Unlike the DNA, the structure of the RNA may exceed limitation of double-helix. The RNA may form a series of different base pairs, and at least two hydrogen bonds are formed between the base pairs. The different bases may be divided into two types, including a standard Waston-Crick base pair type and a non-Waston-Crick base pair type, so that the RNA forms a large number and multiple types of cyclic structure modules, and these modules are basic units for forming a folded RNA three-level structure. The RNA nanotechnology may make use of these natural existing 3D modules and predictable interactions thereof, herein, many RNA structures with biological activity may have an atomic-level resolution, such as a ribosome, various ribozymes and a natural RNA aptamer existing in a riboswitch. A superior feature of the RNA nanotechnology is that a structure which is comparable in size and complexity to a natural RNA substance may be designed. A unique assembly property of the RNA in a natural RNA complex may also be utilized.

The above nucleic acid nanoparticles of the present application include three sequences shown in sequences SEQ ID NO:1, SEQ ID NO:3 and SEQ ID NO:5 or variant sequences thereof, or include three sequences shown in sequences SEQ ID NO:2, SEQ ID NO:4 and SEQ ID NO:6 or variant sequences thereof, and all of the sequences are subject to an ability to form the nucleic acid nanoparticles through the self-assembly, the specific variant sequence may be obtained on the basis of the sequences of the SEQ ID NO:1, the SEQ ID NO:2, the SEQ ID NO:3, the SEQ ID NO:4, the SEQ ID NO:5 and the SEQ ID NO:6 by rationally selecting a variant site and a variant type thereof, or obtained by extending a suitable fragment.

The nanoparticles formed by the self-assembly of the SEQ ID NO:1, the SEQ ID NO:3 and the SEQ ID NO:5 are the RNA nanoparticles, and the nanoparticles formed by the self-assembly of the SEQ ID NO:2, the SEQ ID NO:4 and the SEQ ID NO:6 are the DNA nanoparticles. In a preferred embodiment, when the above nucleic acid nanoparticles are the RNA nanoparticles, and at least one of the sequence a, the sequence b and the sequence c includes the sequence obtained by insertion, deletion or substitution of at least one base, a specific position and a base type of the variant sequence in the RNA nanoparticles may be modified into the nanoparticles of improving a drug loading amount or improving stability according to the needs under a precondition of achieving the self-assembly.

In order to make the prepared nucleic acid nanoparticles have the relatively higher stability, when the base insertion, deletion or substitution is performed on the sequences shown in the above SEQ ID NO:1/2, SEQ ID NO:3/4 and SEQ ID NO:5/6, it may be performed on bases in some specific positions of the above sequences. On the one hand, the variant sequence is the same as the original sequence, and may be self-assembled into the nanoparticles, and on the other hand, the variation retains at least 50%, 55%, 60%, 65%, 70%, 75%, 80%, 85% 90% or 95% of homology with

the original sequence, so that it has the same drug-loading features and similar stability as the nanoparticles formed by the self-assembly of the above sequences, the drug may be loaded and delivered well.

In a preferred embodiment, the above insertion, deletion or substitution of at least one base is occurred: (1) on 1, 2, 4 or 5-th base starting from a 5 '-end of the sequence a shown in the SEQ ID NO:1 or 2; and/or (2) between 8-th and 10-th bases starting from the 5 '-end of the sequence a shown in the SEQ ID NO:1 or 2; and/or (3) between 1-th and 3-th bases starting from a 5 '-end of the sequence b shown in the SEQ ID NO:3 or 4; and/or (4) between 6-th and 9-th bases starting from the 5 '-end of the sequence b shown in the SEQ ID NO:3 or 4; and/or (5) between 1-th and 4-th bases starting from a 5 '-end of the sequence c shown in the SEQ ID NO:5 or 6; and/or (6) between 9-th and 12-th bases starting from the 5 '-end of the sequence c shown in the SEQ ID NO:5 or 6.

In the above preferred embodiment, the defined base position in which the variation happens is a non-classical Watson-Crick paired base position or a bulged unpaired base position in the nanostructure formed by the sequences shown in the SEQ ID NO:1, SEQ ID NO:2, SEQ ID NO:3, SEQ ID NO:4, SEQ ID NO:5 and SEQ ID NO:6, thus the formation of these bulges or loop structures is not affected, thereby the flexibility and tension of the nanostructure formed by the above sequences are maintained, and it is helpful to maintain the stability thereof as a carrier.

In order to further improve the stability of the above nucleic acid nanoparticles, and improve the stability of the drug after drug loading, in a preferred embodiment, the sequence a, the sequence b and the sequence c are self-assembled into a structure shown in Formula (1):

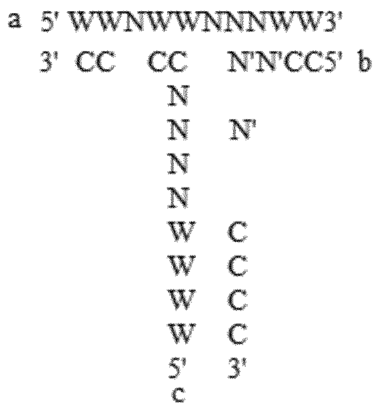


Formula (1).

Herein, W-C represents Watson-Crick pairing, N and N' represent non-Watson-Crick pairing, the W-C in any one position is independently selected from C-G or G-C; in the sequence a, the first N from the 5'-end is A, the second N is G, the third N is U or T, and the fourth N is any one of U, T, A, C or G; in the sequence b, the first N' from the 5'-end is any one of U, T, A, C or G, the second N' is U or T, and the third N' is C; and in the sequence c, a sequence NNNN along a direction from the 5'-end to the 3'-end is CAUA or CATA.

In the above preferred embodiment, the sequences a, b and c are self-assembled to form the nucleic acid domain shown in Formula (1), herein, except the non-Watson-Crick paired bases defined by N and N', the bases in the rest positions all form classical Watson-Crick pairing, and the bases of the above Watson-Crick pairing all choose G-C or C-G base pairs. Because an action force of hydrogen bonds between the G-C or C-G base pairs is greater than an action force of hydrogen bonds between the A-U/T or U/T-A base pairs, the nucleic acid nanostructure is more stable. Rather than the bulges or loop structures formed by the non-Watson-Crick paired bases, the greater tension is brought to the nucleic acid nanocarrier, so that it has the stronger adaptation to a micro-environmental change, thus the stability of the nucleic acid nanoparticles is higher.

In the nanoparticles in the above structure of Formula (1), the specific sequence formation of the sequence a, the sequence b and the sequence c is not specially limited, as long as the above structure may be formed. In view of the self-assembly of the nucleic acid sequence, in order to further improve the efficiency of the self-assembly of the above three sequences into the nanoparticles in the above structure of Formula (1), when the Watson-Crick paired bases are selected, the selection of the bases in the different positions is best to follow the following principles: (1) the sequence a, the sequence b and the sequence c, when a single sequence is selected, it is not complementary-paired by itself to form a two-level structure; and (2) the sequence a, the sequence b and the sequence c, one end of arbitrary two sequences is complementary-paired to form a double-strand, and the other end is not complementary-paired, to form a Y-type or T-type structure. The above principle of the base selection is to most efficiently enable two ends of any one strand to be respectively complementary-paired with two ends of the other two strands, thereby the self-assembly efficiency is improved. Certainly, in addition to the Y-type or T-type structure, it may also be other deformation modes such as a quadrangle rather than a trigeminal shape, as long as it meets the principle that one end of arbitrary two

sequences is complementary-paired to form the double-strand, and the other end is not complementary-paired.

In the nanoparticles in the above structure of Formula (1), in the non-Watson-Crick paired bases, the fourth N starting from the 5'-end in the sequence a and the first N' starting from the 5'-end paired in the sequence b may be non-Watson-Crick paired U-U, or may be T, A, C or G improved for following the Watson-Crick pairing principle. A binding force between the strands is relatively improved by the Watson-Crick pairing, the stability is improved, and the nanoparticles are endowed with the larger softness and flexibility by the non-Watson-Crick pairing, in the face of the micro-environmental change, it is also helpful to improve the stability of the nanoparticles.

In a preferred embodiment, the sequence a, the sequence b and the sequence c are any one of the following groups: (1) sequence a (SEQ ID NO:7): 5'-GGAGCGUUGG-3', sequence b (SEQ ID NO:8): 5'-CCUUCGCCG-3', sequence c (SEQ ID NO:9): 5'-CGGCCAUAGCCC-3'; (2) sequence a (SEQ ID NO:10): 5'-GCAGCGUUCG-3', sequence b (SEQ ID NO:11): 5'-CGUUCGCCG-3', sequence c (SEQ ID NO:12): 5'-CGGCCAUAGCGC-3'; (3) sequence a (SEQ ID NO:13): 5'-CGAGCGUUGC-3', sequence b (SEQ ID NO:14): 5'-GCUUCGCCG-3', sequence c (SEQ ID NO:15): 5'-CGGCCAUAGCCG-3'; (4) sequence a (SEQ ID NO:16): 5'-GGAGCGUUGG-3', sequence b (SEQ ID NO:17): 5'-CCUUCGGGG-3', sequence c (SEQ ID NO:18): 5'-CCCCCAUAGCCC-3'; (5) sequence a (SEQ ID NO:19): 5'-GCAGCGUUCG-3', sequence b (SEQ ID NO:20): 5'-CGUUCGGCG-3', sequence c (SEQ ID NO:21): 5'-CGCCCAUAGCGC-3'; (6) sequence a (SEQ ID NO:22): 5'-GCAGCGUUCG-3', sequence b (SEQ ID NO:23): 5'-CGUUCGGCC-3', sequence c (SEQ ID NO:24): 5'-GGCCCAUAGCGC-3'; (7) sequence a (SEQ ID NO:25): 5'-CGAGCGUUGC-3', sequence b (SEQ ID NO:26): 5'-GCUUCGGCG-3', sequence c (SEQ ID NO:27): 5'-CGCCCAUAGCCG-3'; (8) sequence a (SEQ ID NO:28): 5'-GGAGCGTTGG-3', sequence b (SEQ ID NO:29): 5'-CCTTCGCCG-3', sequence c (SEQ ID NO:30): 5'-CGGCCATAGCCC-3'; (9) sequence a (SEQ ID NO:31): 5'-GCAGCGTTCG-3', sequence b (SEQ ID NO:32): 5'-CGTTCGCCG-3', sequence c (SEQ ID NO:33): 5'-CGGCCATAGCGC-3'; (10) sequence a (SEQ ID NO:34): 5'-CGAGCGTTGC-3', sequence b (SEQ ID NO:35): 5'-GCTTCGCCG-3', sequence c (SEQ ID NO:36): 5'-CGGCCATAGCCG-3'; (11) sequence a (SEQ ID NO:37): 5'-GGAGCGTTGG-3', sequence b (SEQ ID NO:38): 5'-CCTTCGGGG-3', sequence c (SEQ ID NO:39): 5'-CCCCCATAGCCC-3'; (12) sequence a (SEQ ID NO:40): 5'-GCAGCGTTCG-3', sequence b (SEQ ID NO:41): 5'-CGTTCGGCG-3', sequence c (SEQ ID NO:42): 5'-CGCCCATAGCGC-3'; (13) sequence a (SEQ ID NO:43): 5'-GCAGCGTTCG-3', sequence b (SEQ ID NO:44): 5'-CGTTCGGCC-3', sequence c (SEQ ID NO:45): 5'-GGCCCATAGCGC-3'; (14) sequence a (SEQ ID NO:46): 5'-CGAGCGTTGC-3', sequence b (SEQ ID NO:47): 5'-GCTTCGGCG-3', sequence c (SEQ ID NO:48): 5'-CGCCCATAGCCG-3'; and (15) sequence a (SEQ ID NO: 175): 5'-CGAGCGTTCC -3', sequence b (SEQ ID NO: 176): 5'-GGTTCGCCG -3', sequence c (SEQ ID NO: 177): 5'-CGGCCATAGCCG-3'.

The nucleic acid nanoparticles formed by the self-assembly of fifteen groups of the above sequences not only have the higher stability, but also have the higher self-assembly efficiency.

The nucleic acid nanoparticles mentioned above may not only be self-assembled for forming, but also have the ability to carry or load the drugs. According to the different positions of G-C or C-G base pairs in the above nucleic acid nanoparticles and differences of types or natures of the drugs to be loaded, the amounts of the loaded drugs are also different.

In order to enable the above nucleic acid domain to load more drugs and other bioactive substances (the introduction of the bioactive substance is described below), in a preferred embodiment, the above nucleic acid domain further includes a first extension fragment, the first extension fragment is an extension fragment of Watson-Crick pairing, and the first extension fragment is located at the 5'-end and/or 3'-end of any one of the sequences a, b, and c. A certain matching relation is required between the carrier and the loaded substance. While a molecular weight of the carrier is too small and a molecular weight of the loaded substance is too large, in view of mechanics, carrying or delivering capacity of the carrier to the loaded substance is relatively reduced. Therefore, based on the above nucleic acid nanostructure, through adding the first extension fragment at the 5'-end and/or 3'-end of any one sequence of the sequence a, the sequence b and the sequence c, the carrier matched with the size of the loaded substance may be acquired.

A specific length of the above first extension fragment may be determined according to the size of the substance to be loaded. In a preferred embodiment, the first extension fragment is selected from any one of the following groups: (1): a-strand 5'-end: 5'-CCCA-3', c-strand 3'-end: 5'-UGGG-3'; (2): a-strand 3'-end: 5'-GGG-3', b-strand 5'-end: 5'-CCC-3'; (3): b-strand 3'-end: 5'-CCA-3', c-strand 5'-end: 5'-UGG-3'; (4): a-strand 5'-end: 5'-CCCG-3', c-strand 3'-end: 5'-CGGG-3'; (5): a-strand 5'-end: 5'-CCCC-3', c-strand 3'-end: 5'-GGGG-3'; (6): b-strand 3'-end: 5'-CCC-3', c-strand 5'-end: 5'-GGG-3'; (7): b-strand 3'-end: 5'-CCG-3', c-strand 5'-end: 5'-CGG-3'; (8): a-strand 5'-end: 5'-CCCA-3', c-strand 3'-end: 5'-TGGG-3'; (9): b-strand 3'-end: 5'-CCA-3', c-strand 5'-end: 5'-TGG-3'; (10): a-strand 5'-end: 5'-GCGGCGAGCGGCGA-3' (SEQ ID NO:162), c-strand 3'-end: 5'-UCGCCGCUCGCCGC-3' (SEQ ID NO:163); (11): a-strand 3'-end: 5'-GGCCGGAGGCCGG-3' (SEQ ID NO:164), b-strand 5'-end: 5'-CCGGCCUCCGGCC-3' (SEQ ID NO:165); (12): b-strand 3'-end: 5'-CCAGCCGCC-3' (SEQ ID NO:166), c-strand 5'-end: 5'-GGCGGCAGG-3' (SEQ ID NO:167); (13): a-strand 5'-end: 5'-GCGGCGAGCGGCGA-3' (SEQ ID NO:168), c-strand 3'-end: 5'-TCGCCGCTCGCCGC-3' (SEQ ID NO:169); and (14): a-strand 3'-end: 5'-GGCCGGAGGCCGG-3' (SEQ ID NO:170), b-strand 5'-end: 5'-CCGGCCTCCGGCC-3' (SEQ ID NO:171).

The above first extension fragment not only increases a length of any one or more of the three sequences for

forming the nucleic acid nanostructure, but also the first extension fragment formed by the GC base further improves the stability of the formed nanoparticles. In addition, the first extension fragment formed by the above sequences also enables the sequence a, the sequence b and the sequence c to maintain the higher self-assembly activity and efficiency.

In view of the size of the formed nucleic acid nanoparticles and the stability thereof when delivered as a drug delivery carrier in vivo, it is necessary not to be filtered out by kidneys before reaching the target cells when the drugs may be delivered. In a preferred embodiment, the nucleic acid domain further includes a second extension fragment, the second extension fragment is positioned at the 5'-end and/or the 3'-end of any one sequence of the sequence a, the sequence b, or the sequence c, and the second extension fragment is an extension fragment of Watson-Crick pairing; more preferably, the second extension fragment is an extension sequence of a CG base pair; and further preferably, the second extension fragment is an extension sequence of 1-10 CG base pairs. The second extension fragment is the extension fragment further added on the basis of the first extension fragment.

In a preferred embodiment, the above nucleic acid domain further includes at least one group of the following second extension fragments: first group: a-strand 5'-end: 5'-CGCGCG-3', c-strand 3'-end: 5'-CGCGCG-3'; second group: a-strand 3'-end: 5'-CGCCGC-3', b-strand 5'-end: 5'-GCGGCG-3'; and third group: b-strand 3'-end: 5'-GGCGGC-3', c-strand 5'-end: 5'-GCCGCC-3'. Such a second extension fragment enables the nanoparticles not to have immunogenicity, and there is not a situation of the two-level structure in which each strand is folded and linked by itself.

It is to be noted that the above first extension fragment and/or second extension fragment may also be separated by an unpaired base pair.

In order to enable the above nucleic acid nanoparticles to load the bioactive substance (the introduction of the bioactive substance is described below) with the larger molecular weight, increase the drug loading amount and maintain the necessary stability, in a preferred embodiment, the second extension fragment is an extension sequence containing both CG base pair and AT/AU base pair, and preferably the second extension fragment is an extension sequence of 2-50 base pairs. Here, "/" in the "AT/AU base" is a relation of or, specifically, the second extension fragment is an extension sequence containing both CG base pair and AT base pair, or the second extension fragment is an extension sequence containing both CG base pair and AU base pair.

More specifically, the sequences a, b and c after the above second extension fragment is added may be the following sequences respectively:

The sequence a is (SEQ ID NO:49):

5'-CGCGCGAAAAAACGCGCGAAAAAACGCGCGCCCACCAGCGMMCCGGGCGCGCGAAAAAACGCGCGAAAAAACGCGCG-3'.

The sequence b is (SEQ ID NO:50):

5'-CGCGCGMMMMMMCGCGCGMMMMMMCGCGCGCCCGGMCGCCGCCAGCCGCCMMMMMMGCCGCCMMMMMMGCCGCC-3'.

The sequence c is (SEQ ID NO:51):

5'-GGCGGCAAAAAAGGCGGCAAAAAAGGCGGCAGGCGGCAMAGCGGMGGGCGCGCGMMMMMMCGCGCGMMMMMMCGCGCG-3'.

M in the above sequence a, sequence b and sequence c is U or T, when the M is the T, a synthetic cost of the above sequences is greatly reduced.

In practical applications, specific setting positions of the extension sequences of the above CG base pair and AT/AU base pair may be rationally adjusted according to actual needs. In a more preferred embodiment, the second

extension fragment is an extension sequence in which sequences of 2-8 continuous CG base pairs and sequences of 2-8 continuous AT/AU base pairs are alternately arranged; or the second extension fragment is an extension sequence in which a sequence of 1 CG base pair and a sequence of 1 AT/AU base pair are alternately arranged.

Specifically, positions of the extension fragment CGCGCG and the extension fragment CGCCGC in the sequence a as shown in the above SEQ ID NO:49 are interchanged with a position of the extension fragment AAAAAA, positions of the extension fragment GCGGCG and the extension fragment GGCGGC in the sequence b shown in the above SEQ ID NO:50 are interchanged with a position of the extension fragment TTTTTT, the extension fragment GCCGCC in the sequence c shown in the above SEQ ID NO:51 is interchanged with the extension fragment AAAAAA, and the extension fragment CGCCGC is interchanged with the extension fragment TTTTTT at the same time. The nucleic acid nanoparticles formed by the self-assembly of the above sequences are suitable for loading bioactive substences in indole molecular structures (indole drug molecules are preferably linked with A).

In the past few years, three major challenges of the RNA as a widely used construction material includes: 1) sensitivity to RNA enzymatic degradation; 2) sensitivity to dissociation after systemic injection; and 3) toxicity and adverse immune response. At present, the three challenges are overcome to a large extent already: 1) 2'-fluoro (2'-F) or 2'-O-methyl (2'-OMe) modification of a ribose-OH group may make the RNA chemically stable in serum; 2) some natural existing linking sequence motifs are thermodynamically stable, and may keep the overall RNA nanoparticles to be integral at an ultra-low concentration; and 3) the immunogenicity of the RNA nanoparticles is sequence and shape-dependent, and may be adjusted, so that the RNA nanoparticles stimulate generation of inflammatory cytokines, or the RNA nanoparticles have non-immunogenicity and non-toxicity when administered by repeated intravenous injection of 30 mg/kg.

Therefore, in order to further reduce the sensitivity of the above nucleic acid nanoparticles to the RNA enzymatic degradation, and improve the stability in the delivery process, in a preferred embodiment, a base, a ribose and a phosphate in the sequence a, the sequence b and the sequence c have at least one modifiable site, and any one of the modifiable sites is modified by any one of the following modification adapters: -F, a methyl, an amino, a disulfide, a carbonyl, a carboxyl, a sulfhydryl and a formyl; and preferably, the base C or U in the sequence a, the sequence b and the sequence c has 2'-F modification. When the modification linker is the sulfhydryl, it belongs to a thio modification, modification strength is weaker, and a cost is low.

The above drug (the drug includes one or more of the paclitaxel, lenalidomide, cytarabine, docetaxel, idarubicin and mitoxantrone) may be loaded in the modes of the physical linkage and/or the covalent linkage. When the drug is linked with the nucleic acid domain by using two modes of the physical insertion and the covalent linkage simultaneously, the physical insertion is usually inserted between the GC base pairs, the number of the preferred insertion sites is based on the different numbers of the GC base pairs on the nucleic acid domain, and the insertion is performed according to the ratio of 1 to 100:1. When the covalent linkage mode is used for linkage, the above drug usually chemically reacts with a G-exocyclic amino to form the covalent linkage. More preferably, a molar ratio between the drug and the nucleic acid nanoparticles is 2 to 300:1, preferably 2 to 290:1, more preferably 2 to 29:1, further preferably 10 to 50:1, and most preferably 15 to 25:1.

In the nucleic acid nanocarrier drug provided in the present application, the nucleic acid nanoparticles are served as a drug delivery carrier. In addition, according to different drug purposes, in a preferred embodiment, the above nucleic acid nanoparticles also include the bioactive substance, and the bioactive substance is linked with the nucleic acid domain. The bioactive substance is one of more of a target head, a fluorescein, an interfering nucleic acid siRNA, an miRNA, a ribozyme, a riboswitch, an aptamer, a RNA antibody, a protein, a peptide, a flavonoid, a glucose, a natural salicylic acid, a monoclonal antibody, a vitamin, phenol, a lecithin and a small molecular drug except the paclitaxel, lenalidomide, cytarabine, docetaxel, idarubicin and mitoxantrone.

In order to improve loading efficiency and delivering efficiency of the nucleic acid nanoparticles to the loaded bioactive substance, a relative molecular weight of the nucleic acid domain and a total relative molecular weight of the drug and the bioactive substance should preferably have a certain matching relation. In a preferred embodiment, the relative molecular weight of the nucleic acid domain is marked as N1, and the total relative molecular weight of the drug and the bioactive substance is marked as N2, $N_1/N_2 \geq 1:1$; preferably, the bioactive substance is one or more of the target head, the fluorescein, the interfering nucleic acid siRNA, the miRNA, the ribozyme, the riboswitch, the aptamer, the RNA antibody, the drug (usually interpreted as the small molecular drug, namely a chemosynthetic drug), the protein, the peptide, the flavonoid, the glucose, the natural salicylic acid, the monoclonal antibody, the vitamin, the phenols, and the lecithin.

According to the different types of the bioactive substances loaded specifically, performance optimization effects thereof on the nucleic acid nanoparticles of the present application are not the same. For example, when the bioactive substance is a biotin or a folic acid, a function thereof is to make the nucleic acid nanoparticles have a targeting property, for example, specifically targeted to cancer cells. When the bioactive substance is the fluorescein, a function thereof is to make the nucleic acid nanoparticles have a luminous tracking effect, for example, it may be one or more of FAM, CY3, CY5 or Quasar670 and the like. When the bioactive substance is some siRNA, miRNA, protein, peptide, RNA antibody and small molecular drug except the paclitaxel, lenalidomide, cytarabine, docetaxel, idarubicin and mitoxantrone, according to the different biological functions, the nucleic acid nanocarrier drug may be made into a new product with a specific therapeutic effect, such as a drug with more excellent performance. In addition, according to the different types of the bioactive substances loaded specifically, the DNA nanoparticles and the RNA nanoparticles are specifically preferably used, and may be rationally selected according to the actual needs. For example, when the bioactive substance is the drug, the DNA nanoparticles and the RNA nanoparticles are preferably used for loading, and there is no special requirement for a length of the single strand of the nanoparticles formed by assembly.

In a preferred embodiment, the bioactive substance is the target head, the fluorescein and the miRNA, herein, the target head is positioned on any one sequence of the sequences a, b and c, preferably the 5'-end or the 3'-end of any one sequence of the sequences a, b and c, or inserted between GC bonds of the nucleic acid domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and preferably, the target head is the folic acid or the biotin, the fluorescein is any one or more of FAM, CY5 and CY3, and the anti-miRNA is anti-miR-21.

The above target head may be linked on any one sequence of the sequences a, b and c in a mode of linker covalent linkage, the available linker is selected from a disulfide bond, a p-phenylazide, bromopropyne or PEG. Here, the "on any one sequence" refers to on a base in any one position of any one sequence of the sequences a, b and c, and it is more convenient to be linked at the 5'-end or 3'-end, the application is wider. Folic acid modification may be physical insertion mode linkage or physical insertion + covalent linkage.

The above fluorescein may be a commonly used fluorescein, and preferably any one of more of FAM, CY5 and CY3.

The above miRNA may be a miRNA with a cancer suppression effect, or may be an anti-miRNA which may suppress a corresponding disease, and it may be rationally selected in practical applications according to medical needs. The above anti-miRNA may be synthesized at any one or more positions of the 3'-end of the above sequence a, the 5'-end and 3'-end of the sequence c. When the anti-miRNA is synthesized in the above three positions, the anti-miRNA has a relatively stronger suppression effect on the corresponding miRNA.

It is anti-miR-21 preferably, the miR-21 participates in initiation and progression of multiple types of the cancers, and is a main oncogene for invasion and metastasis. The anti-miR-21 may effectively regulate a wide range of target genes at the same time, and is beneficial to solve a problem of cancer heterogeneity. Therefore, in the above preferred nucleic acid nanoparticles, the target head, such as the folic acid or the biotin, may be specifically targeted to the cancer cells, and after being linked and internalized with the cancer cells, the anti-miR-21 is complemented with a miR-21 base in very high affinity and specificity, thereby the expression of the oncogenic miR-21 is effectively reduced. Therefore, according to the actual needs, the above anti-miR-21 may be synthesized at any one or more positions of the 3'-end of the above sequence a, the 5'-end and the 3'-end of the sequence c. When the anti-miR-21 is synthesized in the above three positions, the anti-miR-21 has a relatively stronger suppression effect on the miR-21.

When the above bioactive substance capable of loading is other small molecular drugs except the paclitaxel, lenalidomide, cytarabine, docetaxel, idarubicin and mitoxantrone, the drug, according to types of diseases which may be treated by the different drugs, includes but not limited to drugs for treating liver cancer, gastric cancer, lung cancer, breast cancer, head and neck cancer, uterine cancer, ovarian cancer, melanoma, leukemia, Alzheimer's disease, ankylosing spondylitis, malignant lymphoma, bronchial cancer, rheumatoid arthritis, HBV hepatitis B, multiple myeloma, pancreatic cancer, non-small cell lung cancer, prostate cancer, nasopharyngeal cancer, esophageal cancer, oral cancer, lupus erythematosus; and preferably, the head and neck cancer is brain cancer, neuroblastoma or glioblastoma.

When the above bioactive substance capable of loading is other small molecular drugs except the paclitaxel, lenalidomide, cytarabine, docetaxel, idarubicin and mitoxantrone, according to a difference of molecular structures of the drugs or a difference of characteristic groups which it has, the drug includes but not limited to a drug containing any one or more of the following groups: an amino group, a hydroxyl group, a carboxyl group, a mercapto group, a benzene ring group and an acetamido group.

In a preferred embodiment, the above protein is one or more of antibodies or aptamers of superoxide dismutase (SOD), survivin, human telomerase reverse transcriptase (hTERT), epidermal growth factor receptor (EGFR) and prostate-specific membrane antigen (PSMA); the above vitamin is L-Vc and/or esterified Vc; and the above phenol is a tea polyphenol and/or a grape polyphenol.

In a preferred embodiment, a particle size of the nucleic acid nanoparticles is 1-100 nm, preferably 5-50 nm; more preferably 10-30 nm; and further preferably 10-15 nm. The size is appropriate within this range, it may not only enter a cell membrane through cell phagocytosis mediated by a cell surface receptor, but also avoid non-specific cell penetration so as to be filtered and removed by the kidneys. Therefore, the favorable particle size is helpful to improve pharmacokinetics, pharmacodynamics, biological distribution and toxicological distribution.

According to a second aspect of the present application, a preparation method for the above nucleic acid nanocarrier drug is further provided, and the preparation method includes the following steps: any one of the above nucleic acid nanoparticles is provided; a drug is loaded on the nucleic acid nanoparticle in modes of physical linkage

and/or covalent linkage, to obtain the nucleic acid nanocarrier drug.

When the physical linkage mode is used, the drug may be usually formed and inserted between GC base pairs in a physical insertion form. When the covalent linkage mode is used for linkage, the drug usually chemically reacts with a G-exocyclic amino to form the covalent linkage. The nucleic acid nanocarrier drug prepared by using the above method may have the better targeting property after it is modified by the target head, the drug may be stably delivered, and the reliability is very high.

In a preferred embodiment, the step of loading the drug in the physical linkage mode includes: enabling the drug, the nucleic acid nanoparticles and a first solvent to be mixed and stirring, to obtain a premixed system; and precipitating the premixed system, to obtain the nucleic acid nanocarrier drug. Specific dosages of the drug and the nucleic acid nanoparticles may be adjusted according to a change of the loading amount, this may be understood by those skilled in the art, and it is not repeatedly described here.

In order to improve the efficiency and the stability of the physical linkage, an amount of the drug added per liter of the first solvent is preferably 0.1 to 1 g. Preferably, the first solvent is selected from one or more of DCM, DCC, DMAP, Py, DMSO, PBS and glacial acetic acid. Preferably, the step of precipitating the premixed system, to obtain the nucleic acid nanocarrier drug includes: precipitating the premixed system, to obtain a precipitation; and washing the precipitation to remove impurities, to obtain the nucleic acid nanocarrier drug. More preferably, the premixed system is mixed with absolute ethyl alcohol, and precipitated at a temperature condition lower than 10 DEG C, to obtain the precipitation, further preferably, the precipitation is obtained by precipitating at a temperature condition of 0-5 DEG C. More preferably, the precipitation is washed to remove the impurities with 6-12 times of the absolute ethyl alcohol in volume, as to obtain the nucleic acid nanocarrier drug.

In a preferred embodiment, the step of loading the drug in the covalent linkage mode includes: drug solution is prepared; the drug solution reacts with the G-exocyclic amino of the nucleic acid nanoparticles under a mediating effect of the formaldehyde, to obtain a reaction system; and the reaction system is purified, to obtain the nucleic acid nanocarrier drug.

Preferably, the above reaction step includes: enabling paclitaxel solution to be mixed with the paraformaldehyde solution and the nucleic acid nanoparticles, and reacting in a dark condition, to obtain a reaction system. The paraformaldehyde solution may release small formaldehyde molecules to participate in the above chemical reaction. In order to improve the reaction efficiency, the concentration of the paraformaldehyde solution is preferably 3.7~4wt%, and the paraformaldehyde solution is preferably solution formed by mixing paraformaldehyde and a second solvent, and the second solvent is one or more of DCM, DCC, DMAP, Py, DMSO, PBS and glacial acetic acid.

In a preferred embodiment, the following reaction may happen:

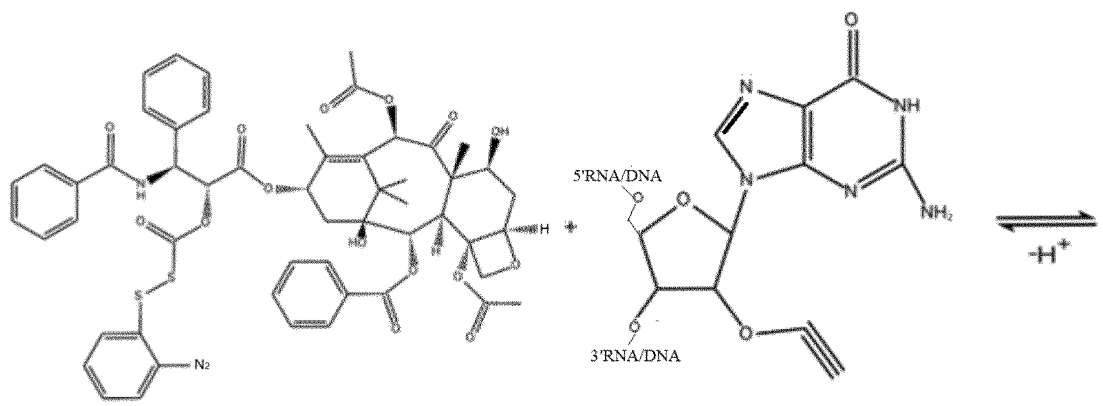

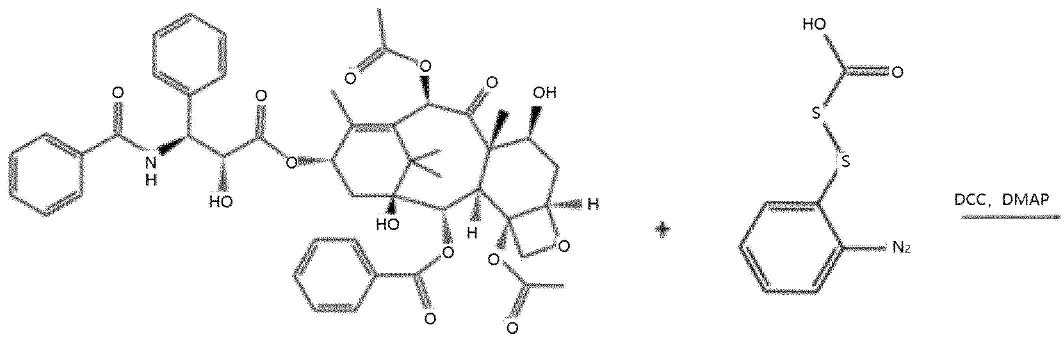


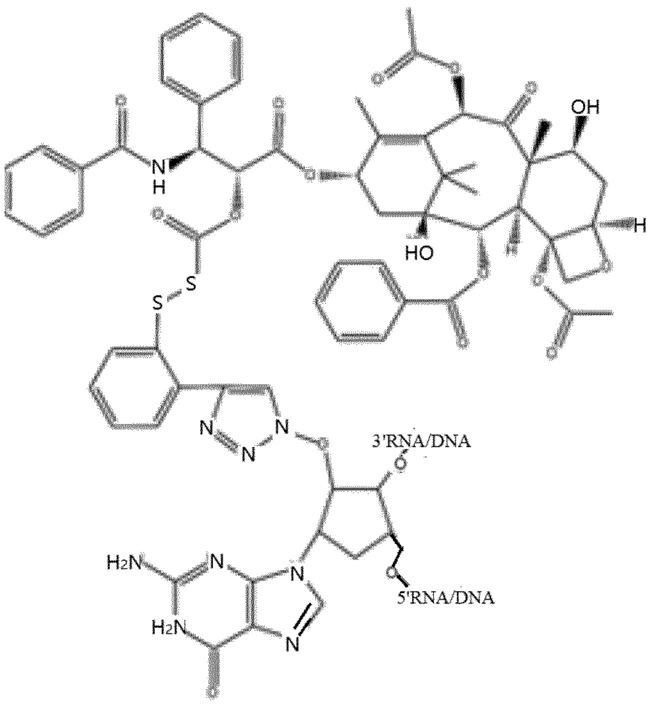

In the above preparation method, the nucleic acid nanoparticles may be prepared in a mode of self-assembly, for example: (1) enabling the RNA or DNA single strands a, b and c to be simultaneously mixed and dissolved in DEPC treated water or TMS buffer solution; (2) heating mixed solution to 80/95 DEG C (herein a RNA assembly temperature is 80 DEG C, and a DNA assembly temperature is 95 DEG C), after keeping for 5 min, slowly cooling to a room temperature at a rate of 2 DEG C/min; (3) enabling a product to be loaded on 8% (m/v) of non-denaturing PAGE gel and placed in TBM buffer solution under a condition of 4 DEG C, purifying a complex by 100 v of electrophoresis; and (4) cutting a target band and eluting in RNA/DNAelution buffer solution at 37 DEG C, after that, precipitating overnight in ethanol, volatilizing under reduced pressure and low temperature, to obtain a self-assembly product, namely the nucleic acid domain, and then acquiring the nucleic acid nanoparticles.

In a preferred embodiment, the step of loading the lenalidomide in the covalent linkage mode includes: preparing lenalidomide solution; enabling the lenalidomide solution to react with the G-exocyclic amino of the nucleic acid nanoparticles under a mediating effect of the formaldehyde, to obtain a reaction system; and purifying the reaction system, to obtain a drug containing the lenalidomide.

Through a formaldehyde-mediated form, the following reaction may happen:

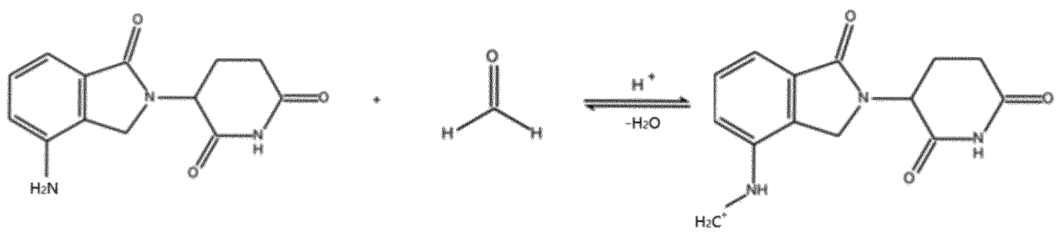


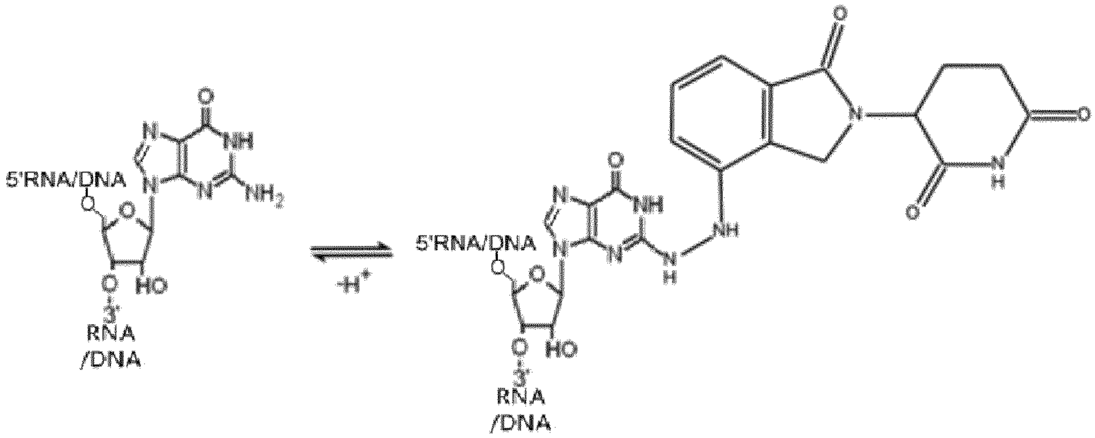


In a preferred embodiment, the step of loading the cytarabine in the covalent linkage mode includes: preparing cytarabine solution; enabling the cytarabine solution to react with the G-exocyclic amino of the nucleic acid nanoparticles under the mediating effect of the formaldehyde, to obtain a reaction system; and purifying the reaction system, to obtain a drug containing the cytarabine.

Through the formaldehyde-mediated form, the following reaction may happen:

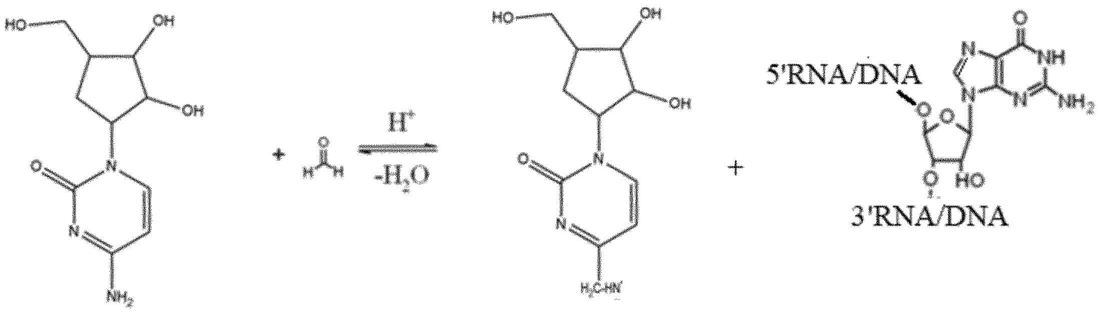


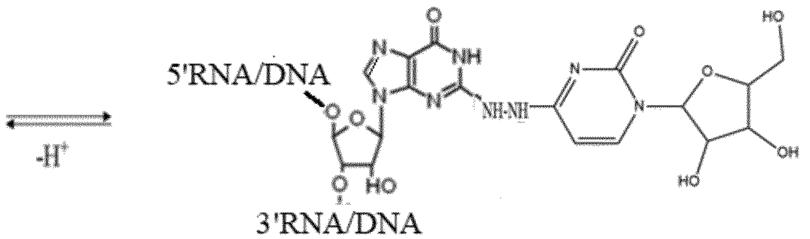


In a preferred embodiment, the following reaction may happen:

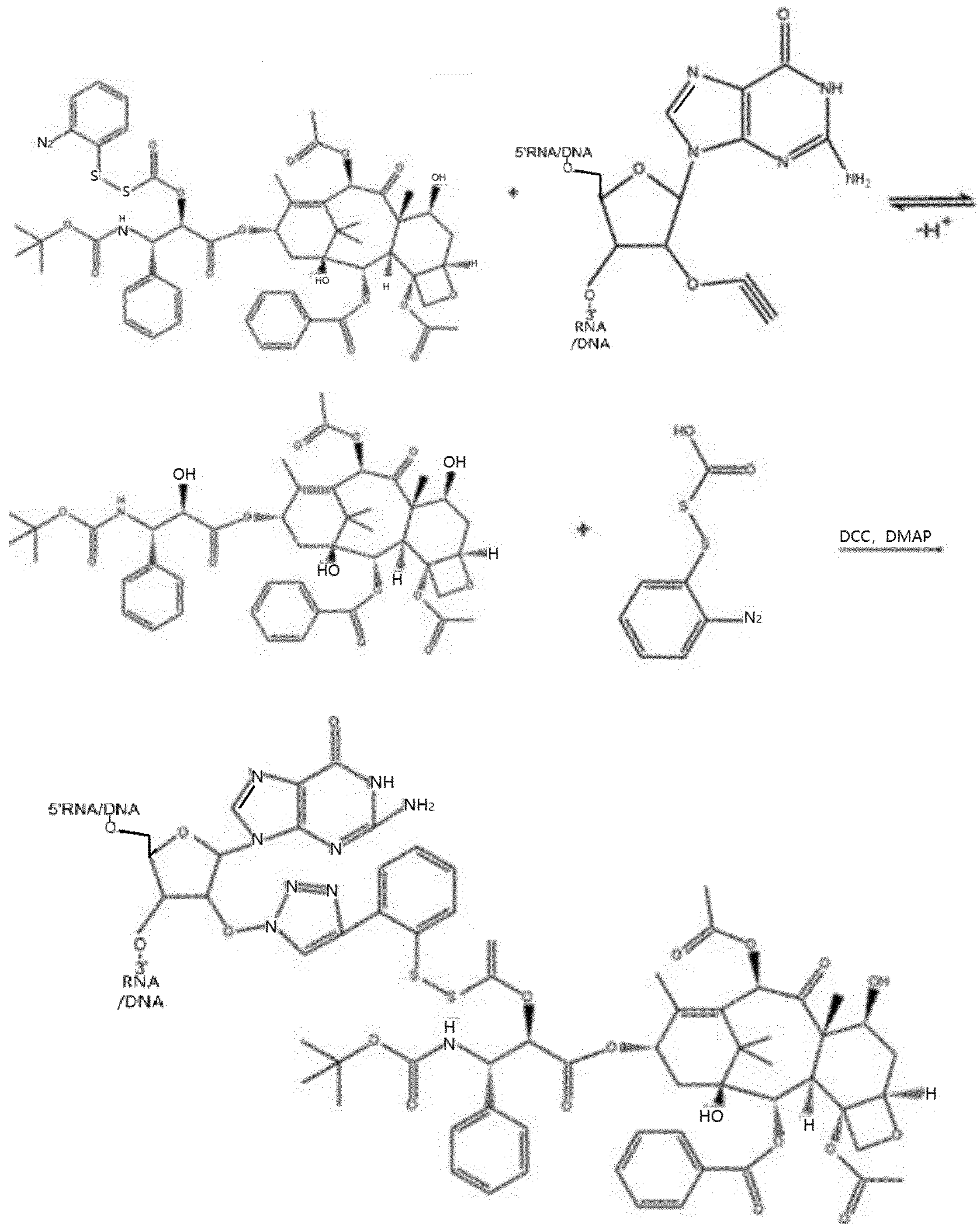


In a preferred embodiment, the step of loading the idarubicin in the covalent linkage mode includes: preparing idarubicin solution; enabling the idarubicin solution to react with the G-exocyclic amino of the nucleic acid nanoparticles under the mediating effect of the formaldehyde, to obtain a reaction system; and purifying the reaction system, to obtain a drug containing the idarubicin.

Through the formaldehyde-mediated form, the following reaction may happen:

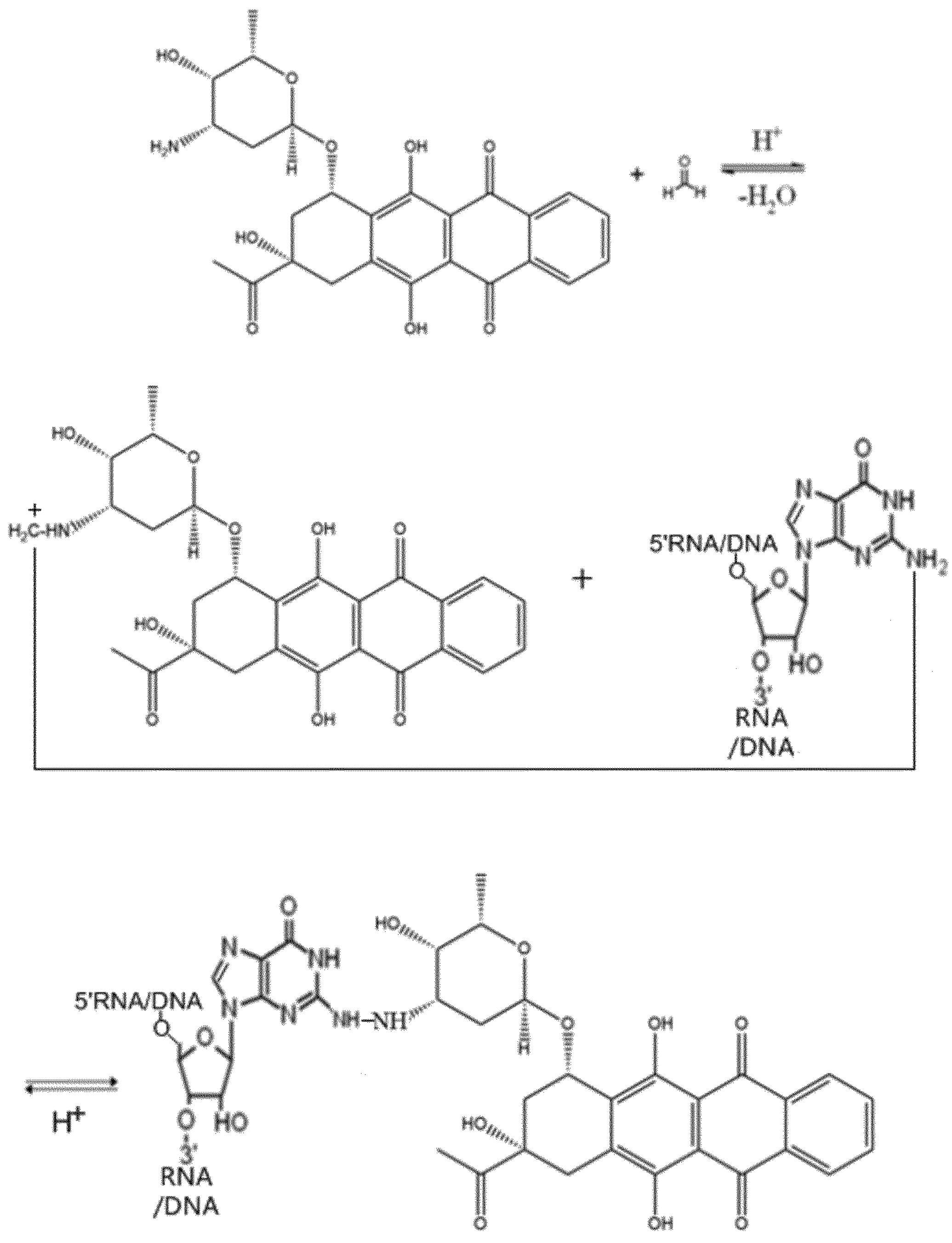


In a preferred embodiment, the step of loading the mitoxantrone in the covalent linkage mode includes: preparing mitoxantrone solution; enabling the mitoxantrone solution to react with the G-exocyclic amino of the nucleic acid nanoparticles under the mediating effect of the formaldehyde, to obtain a reaction system; and purifying the reaction system, to obtain a drug containing the mitoxantrone.

Through the formaldehyde-mediated form, the following reaction may happen:

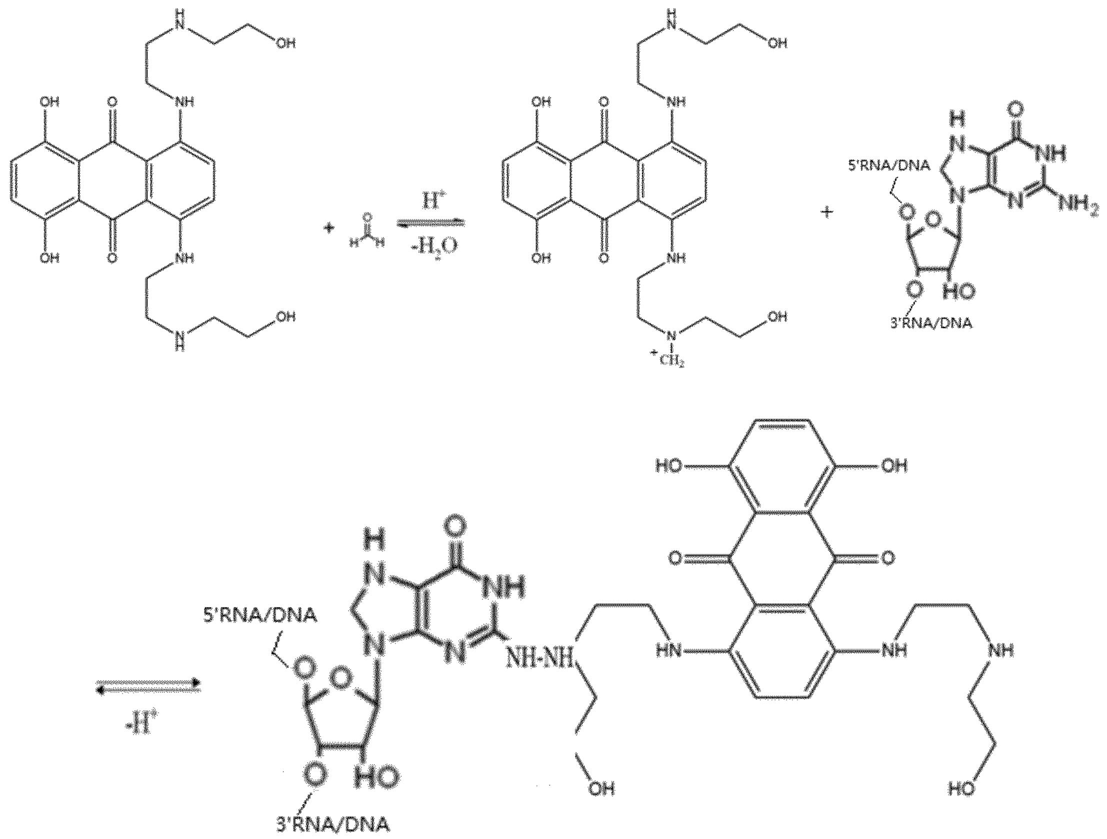


According to actual application needs, in order to make the above nucleic acid nanocarrier drug have other functions, in a preferred embodiment, after the nucleic acid domain is obtained, the preparation method further includes: enabling the bioactive substance as mentioned above to be loaded on the nucleic acid domain in the modes of physical linkage and/or covalent linkage, to obtain the nucleic acid nanoparticles. The loading mode of the bioactive substance may also be the physical linkage and/or the covalent linkage. The covalent linkage mode includes but not limited to the solvent covalent linkage, the linker covalent linkage or the click-linkage; preferably, a third solvent used in the solvent covalent linkage is served as a linkage medium, and the third solvent is selected from one or more of paraformaldehyde, DCM, DCC, DMAP, Py, DMSO, PBS and glacial acetic acid; preferably, the linker is selected from a disulfide bond, a p-phenylazide, bromopropyne or PEG; and preferably, the click-linkage is that alkynyl or azide modification is simultaneously performed on a bioactive substance precursor and the nucleic acid domain, and then they are linked through the click-linkage.

It is to be noted that the above classification does not mean that there is only one linkage mode to link a certain bioactive substance with the nucleic acid nanocarrier. However, some bioactive substances may be linked with the nucleic acid nanocarrier in the physical insertion mode, or may be linked with the nucleic acid nanocarrier in the physical insertion and covalent linkage modes, or may be linked by using the click-linkage mode at the same time. However, for a certain specific bioactive substance, there may be only one linkage mode, or there may be multiple linkage modes, but it may be that the efficiency of certain linkage has an advantageous practical value.

In the above linkage modes, when the different drugs are linked with the nucleic acid domain in the physical insertion mode, insertion linkage sites and numbers are also different slightly. For example, when anthracycline and acridine drugs are inserted, they are usually inserted between the GC base pairs, the preferred number of the insertion sites is based on the different numbers of the GC base pairs in the nucleic acid domain, and the insertion is performed in a ratio of 1 to 100:1. When a naphthylamide drug is inserted, it is usually inserted between the AA base pairs, the preferred number of the insertion sites is based on the different numbers of the AA base pairs in the nucleic acid domain, and pyridocarbazoles are inserted according to the different numbers of the AA base pairs in a ratio of 1 to 200:1.

Specifically, according to the different types of the bioactive substances, the lengths of the sequences a, b and c for forming the nucleic acid domain in the nucleic acid nanoparticles and the number of GC complementary base pairs thereof, the physical insertion may be performed by rationally selecting a molar ratio of the bioactive substance and the nucleic acid domain.

In a preferred embodiment, when the bioactive substance is linked with the nucleic acid domain in the physical insertion mode and the covalent linkage mode, a molar ratio of the bioactive substance linked in the physical insertion

mode and the drug linked in the covalent linkage mode is 1 to 200:1. The linkage mode is suitable for the anthracycline and acridine drugs. A proportion of the drugs linked in the above different linkage modes is not limited to the above range, as long as it may meet a requirement of high-efficient loading, there is a non-toxic effect on the cells, and the effective release of the drug is achieved after reaching a target.

When a bioactive substance precursor and the nucleic acid domain are simultaneously modified by an alkynyl or an azide, and linked in the click-linkage mode, the different clicklinkages are selected according to changes of the different structures of the drugs. In addition, along with the different structures of the bioactive substances, the linkage positions may also be changed correspondingly, this may be understood by those skilled in the art.

In a preferred embodiment, when the bioactive substance is linked with the nucleic acid domain in the click-linkage mode, a site, for performing the alkynyl or azide modification, of the bioactive substance precursor is selected from a hydroxyl, a carboxyl, a mercapto or an amino, and a site, for performing the alkynyl or azide modification, of the nucleic acid domain is selected from the amino, an imino or the hydroxyl.

It is to be noted that when the above nucleic acid domain is linked with the drug, the nucleic acid domain is water-soluble, and most of the drugs have poor water solubility. After it is linked with the nucleic acid domain, the water solubility is improved. When the above drugs are anthracyclines, these drugs are covalent-linked with the nucleic acid domain through a -NH bond (under a suitable pH value condition, the activity of the -NH group is hundreds of times greater than the activity of other groups which may be covalent-linked with the drugs) on a nucleotide guanosine, thereby the nucleic acid domain for loading the drugs is formed. Therefore, according to a size of a specific drug molecule and the number of the GC base pairs on the sequence a, the sequence b and the sequence c in the specifically designed nucleic acid domain, when linked, a linking reaction is performed theoretically according to 1.1-1.3 times of a supersaturated linking amount, and at most 35 to 45 drugs may be linked in one nucleic acid domain. When the above drugs are other structures, the loading amount is related to an occupation situation (including but not limited to a molecular structure, a morphology, a shape and a molecular weight) of the specific drug. Therefore, a linkage condition of an activity site of the drug and the -NH bond on the nucleotide guanosine of the nucleic acid domain is relatively harsh, and it may also be loaded but it is relatively difficult to cause a situation of excessive linkage.

In a typical implementation mode, a pharmaceutical composition is further provided, the pharmaceutical composition includes any one of the above nucleic acid nanoparticles. In the drug containing the above nucleic acid nanoparticles provided and stipulated by the present application, the nucleic acid domain may have the good targeting property after being modified by the target head targeted to a target cell, and may also be load with corresponding therapeutic drugs and/or tracer molecules, thereby the therapeutic drugs and/or tracer molecules may be stably delivered, and the reliability is very high.

According to a third aspect of the present application, a pharmaceutical composition is further provided, the pharmaceutical composition includes any one of the above nucleic acid nanocarrier drugs. Specifically, a suitable combination drug or auxiliaries may be selected according to actual needs to form the pharmaceutical composition which has the combined efficacy or may improve a certain aspect of the drug properties (such as stability).

According to a fourth aspect of the present application, an application of any one of the above nucleic acid nanocarrier drugs in preparing a drug for treating a tumor is further provided. When the drug is the paclitaxel, the tumor is breast cancer or ovarian cancer; when the drug is the lenalidomide, the tumor is acute leukemia or multiple myeloma; when the drug is the cytarabine, the tumor is any one or more of acute leukemia, malignant lymphoma, lung cancer, digestive tract cancer, colorectal cancer, and head and neck cancer; when the drug is the docetaxel, the tumor is any one or more of breast cancer, ovarian cancer, non-small cell lung cancer, head and neck cancer, pancreatic cancer, small cell lung cancer, gastric cancer, melanoma, and soft tissue sarcoma; when the drug is the idarubicin, the tumor is any one or more of acute pulmonary lymphocytic leukemia, advanced breast cancer and non-Hodgkin's lymphoma; and when the drug is the mitoxantrone, the tumor is any one or more of breast cancer, malignant lymphoma, gastric cancer, bowel cancer, leukemia, bladder cancer, liver cancer, multiple myeloma, malignant mesothelioma, and ovarian cancer. Further, an application of the nucleic acid nanocarrier drug in preparing a drug for treating viral keratitis and epidemic conjunctivitis is provided, herein the drug contained in the nucleic acid nanocarrier drug is the cytarabine. Further, an application of the nucleic acid nanocarrier drug in preparing a drug for treating myelodysplastic syndrome is provided, herein the drug contained in the nucleic acid nanocarrier drug is the idarubicin. For specific applications, a new drug may be obtained by improving the drug itself on the basis of the drug of the present application, or the drug of the present application is served as a main active ingredient and prepared into a preparation in a suitable dosage form and the like.

According to a fifth aspect of the present application, a method for preventing and/or treating a tumor is further provided, the method includes: providing any one of the above nucleic acid nanocarrier drugs or pharmaceutical compositions; and administering an effective dose of the above nucleic acid nanocarrier drug or pharmaceutical composition to a tumor patient. Further, the tumor is the breast cancer or the ovarian cancer.

The effective dosage herein includes a prophylactically effective dosage and/or a therapeutically effective dosage. The therapeutically effective dosage refers to a dosage that is effective to achieve a desired therapeutic result,

such as a reduction of the breast cancer or the ovarian cancer, within a necessary dosage and time period. In a specific implementation mode, the dosage may be adjusted to provide the optimal therapeutic response dosage, and the therapeutically effective dosage may be varied according to the following factors: a disease state, an age, a gender, and a weight of an individual and an ability of a preparation which causes a desired response in the individual. The meaning of the therapeutically effective dosage also includes a dosage of which beneficial effects of treatment exceed its toxic or harmful effects. The prophylactically effective dosage refers to a dosage that is effective to achieve the desired preventive result, such as preventing or inhibiting the occurrence of the breast cancer or the ovarian cancer, within the necessary dosage and time period. The prophylactically effective dosage may be determined according to the above description of the therapeutically effective dosage. For any specific subjects, the specific dosage may be adjusted along with time according to individual needs and the professional judgment of an administering person.

It is to be noted that the nucleic acid nanoparticles formed by the self-assembly of the sequences or sequence variations provided by the present application may also be used as basic structural units, and may be further polymerized to form multimers, such as a dimer, a trimer, a tetramer, a pentamer, a hexamer or a heptamer, according to the actual application needs.

The beneficial effects of the present application are further described below in combination with specific embodiments.

**Assembly of nucleic acid nanoparticles**

Embodiment 1

I. RNAand DNA nanoparticle carrier:

(1) Three polynucleotide base sequences for assembling RNA nanoparticles are specifically shown below in Table 1:

Table 1:

| Name | Sequence sense (5'-3') | Base number | Molecular weight | Chemical modification | Fluorescent label | Special modification | Total molecular weight |
|---|---|---|---|---|---|---|---|
| a-stran d (SEQ ID NO: 52) | cGcGcGcccAccAG cGuuccGGGcGcc Gc | 29 | 9678.7 | C/U base 2'Fmodification | / | 5'Biotin | 29524.9 |
| b-stran d (SEQ ID NO: 53) | GcGGcGcccGGuu cGccGccAGGcGG c | 27 | 9116.8 | C/U base 2'F modification | / | 5'Biotin | |
| c-stran d (SEQ ID NO: 54) | GccGccAGGcGGc cAuAGcGGuGGG cGcGcG | 31 | 10729.4 | C/U base 2'F modification | 5'CY3 | | |

(2) Three polynucleotide base sequences of DNA nanoparticles

DNA uses the same sequence as the above RNA, except that U is replaced by T. Herein, a molecular weight of the a-strand is 8802.66, a molecular weight of the b-strand is 8280.33, and a molecular weight of the c-strand is 9605.2.

The strands a, b and c of the above RNA nanoparticles and DNA nanoparticles are all commissioned to be synthesized by Sangon Bioengineering (Shanghai) Co., Ltd.

II. Self-assembly experiment steps:

(1) according to a molar ratio of 1:1:1, enabling the RNA or DNA single strands a, b and c to be simultaneously mixed and dissolved in DEPC treated water or TMS buffer solution;

(2) heating mixed solution to 80/95 DEG C (herein a RNA assembly temperature is 80 DEG C, and a DNA assembly temperature is 95 DEG C), after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100V of electrophoresis;

(4) cutting a target band and eluting in RNA/DNA elution buffer solution at 37 DEG C, after that, precipitating overnight in ethanol, volatilizing under reduced pressure and low temperature, to obtain a self-assembly product; and

(5) electrophoresis analysis detection and laser scanning observation.

III. Self-assembly experiment result

Electrophoresis detection result

An electrophoresis detection result of the RNA self-assembly product is shown in Fig. 1. In Fig. 1, lanes 1 to 3 from left to right are successively: the a-strand, the b-strand, and the RNA self-assembly product. It may be seen from the figure that although the RNA self-assembly product is slightly diffused, it may be apparently seen that it is a single band. In addition, because the molecular weight is a molecular weight after assembly, it is larger than a single-stranded molecular weight, a band position is behind the a-strand and the b-strand, and the actual situation is consistent with a theory, it is proved that a stable composite structure is formed between the above RNA single strands by the self-assembly, and RNA nanoparticles are formed.

An electrophoresis detection result of the DNA self-assembly product is shown in Fig. 2. In Fig. 2, lanes 1 to 3 from left to right are successively: the a-strand, the b-strand, and the DNA self-assembly product. It may be seen from the figure that a band of the DNA self-assembly product is bright and clear, and is a single band, it is proved that a stable composite structure is formed between the above DNA single strands by the self-assembly, and DNA nanoparticles are formed.

In the embodiment, it is verified by gel electrophoresis that the sequences a, b and c including the RNA core sequences SEQ ID NO:1, SEQ ID NO:3 and SEQ ID NO:5 may be successfully self-assembled into the RNA nanoparticles. The sequences a, b and c including the DNA core sequences SEQ ID NO:2, SEQ ID NO:4 and SEQ ID NO:6 may also be successfully self-assembled into the DNA nanoparticles.

In addition to the core sequences which form a nucleic acid domain, the sequences a, b and c of the above RNA nanoparticles and DNA nanoparticles also have various extension sequences (including a drug loading linking sequence) which promote a loading function of the nucleic acid domain and a target head or a fluorescein which is linked with the nucleic acid domain. It may be seen that the presence of substances other than these core sequences does not affect the formation of the nucleic acid domain and the successful self-assembly of the nucleic acid nanoparticles. The self-assembled nucleic acid nanoparticles may have a targeting property under the guidance of the target head, and the fluorescein may make the nucleic acid nanoparticles have visibility and traceability.

Embodiment 2

I. 7 groups of short-sequence RNA nanoparticle carriers:

(1) 7 groups of three polynucleotide base sequences for forming RNA nanoparticles are shown below in Table 2 to Table 8:

Table 2: R-1:

| Name | Sequence sense (5'-3') | Base numbe r | Chemical modification | Molecul ar weight | Total molecul ar weight |
|---|---|---|---|---|---|
| a-strand (SEQ ID NO: 55) | **GG**AGcGuu**GG** | 10 | c/u base 2'F modification | 3262.9 | 9828.7 |
| b-strand (SEQ ID NO: 56) | **cc**uuc**GCCG** | 9 | c/u base 2'F modification | 2780.6 | |
| c-strand (SEQ ID NO: 57) | c**GG**ccAuAGc**cc** | 12 | c/u base 2'F modification | 3785.2 | |

Table 3: R-2:

| Name | Sequence sense (5'-3') | Base number | Chemical modification | Molecul ar weight | Total molecul ar weight |
|---|---|---|---|---|---|
| a-strand (SEQ ID NO: 58) | **Gc**AGcGuuc**G** | 10 | c/u base 2'F modification | 3186.7 | 9829.4 |
| b-strand (SEQ ID NO: 59) | **cG**uuc**GccG** | 9 | c/u base 2'F modification | 2820.2 | |
| c-strand (SEQ ID NO: 60) | **cGGc**cAuAGc**G** **c** | 12 | c/u base 2'F modification | 3822.5 | |

Table 4: R-3:

| Name | Sequence sense (5'-3') | Base number | Chemical modification | Molecul ar weight | Total molecul ar weight |
|---|---|---|---|---|---|
| a-strand (SEQ ID NO: 61) | **cG**AGcGuu**Gc** | 10 | c/u base 2'F modification | 3187.5 | 9829.9 |
| b-strand (SEQ ID NO: 62) | **Gc**uuc**GccG** | 9 | c/u base 2'F modification | 2819.2 | |
| c-strand (SEQ ID NO: 63) | c**GG**ccAuAGc**cG** | 12 | c/u base 2'F modification | 3823.2 | |

Table 5: R-4:

| Name | Sequence sense (5'-3') | Base numbe r | Chemical modification | Molecul ar weight | Total molecul ar weight |
|---|---|---|---|---|---|
| a-strand (SEQ ID NO: 64) | **GG**AGcGuu**GG** | 10 | c/u base 2'F modification | 3263.7 | 9830.9 |
| b-strand (SEQ ID NO: 65) | **cc**uuc**GGGG** | 9 | c/u base 2'F modification | 2858.2 | |
| c-strand (SEQ ID NO: 66) | **cccc**cAuAGc**cc** | 12 | c/u base 2'F modification | 3709.0 | |

Table 6: R-5:

| Name | Sequence sense (5'-3') | Base number | Chemical modification | Molecul ar weight | Total molecul ar weight |
|---|---|---|---|---|---|
| a-strand (SEQ ID NO: 67) | **Gc**AGcGuu**cG** | 10 | c/u base 2'F modification | 3187.5 | |
| b-strand (SEQ ID NO: 68) | **cG**uuc**GGcG** | 9 | c/u base 2'F modification | 2857.5 | 9830.2 |
| c-strand (SEQ ID NO: 69) | **cG**cccAuAGc**Gc** | 12 | c/u base 2'F modification | 3785.2 | |

Table 7: R-6:

| Name | Sequence sense (5'-3') | Base number | Chemical modification | Molecul ar weight | Total molecul ar weight |
|---|---|---|---|---|---|
| a-strand (SEQ ID NO: 70) | GcAGcGuucG | 10 | c/u base 2'F modification | 3187.2 | |
| b-strand (SEQ ID NO: 71) | cGuucGGcc | 9 | c/u base 2'F modification | 2820.4 | 9830.5 |
| c-strand (SEQ ID NO: 72) | **GGcc**cAuAGc**Gc** | 12 | c/u base 2'F modification | 3822.9 | |

Table 8: R-7:

| Name | Sequence sense (5'-3') | Base number | Chemical modification | Molecul ar weight | Total molecul ar weight |
|---|---|---|---|---|---|
| a-strand (SEQ ID NO: 73) | cGAGcGuuGc | 10 | c/u base 2'F modification | 3187.6 | 9830.7 |
| b-strand (SEQ ID NO: 74) | GcuucGGcG | 9 | c/u base 2'F modification | 2857.8 | |
| c-strand (SEQ ID NO: 75) | **cGcc**cAuAGc**cG** | 12 | c/u base 2'F modification | 3785.3 | |

The single strands of the above 7 groups of the short-sequence RNA nanoparticle carriers are all commissioned to be synthesized by Sangon Bioengineering (Shanghai) Co., Ltd.

II. Self-assembly experiment steps:

(1) according to a molar ratio of 1:1:1, enabling the RNA single strands a, b and c to be simultaneously mixed and dissolved in DEPC treated water or TMS buffer solution;
(2) heating mixed solution to 80 DEG C, after keeping for 5 min, slowly cooling to a room temperature at a rate of 2 DEG C/min;
(3) enabling a product to be loaded on 8% (m/v) of non-denaturing PAGE gel and placed in TBM buffer solution under a condition of 4 DEG C, purifying a complex by 100V of electrophoresis;
(4) cutting a target band and eluting in RNA elution buffer solution at 37 DEG C, after that, precipitating overnight in ethanol, volatilizing under reduced pressure and low temperature, to obtain a short-sequence RNA self-assembly product;
(5) electrophoresis analysis detection and laser scanning observation; and
(6) Determination of molecular weight.

III. Self-assembly experiment result

(1) Electrophoresis detection result

A 2% agarose gel electrophoresis diagram of the 7 groups of the short-sequence RNA self-assembly products is shown in Fig. 3. From left to right, lanes 1 to 7 in Fig. 3 are successively: short-sequences R-1, R-2, R-3, R-4, R-5, R-6 and R-7.

A 4% agarose gel electrophoresis diagram of the 7 groups of the short-sequence RNA self-assembly products is shown in Fig. 4. From left to right, lanes 1 to 7 in Fig. 4 are successively: short-sequences R-1, R-2, R-3, R-4, R-5, R-6 and R-7.

It may be clearly seen from results of Fig. 3 and Fig. 4 that bands of the R-2, R-3, R-5 and R-7 in the 7 groups of the short-sequence self-assembly products are bright and clear, although the R-1, R-4 and R-6 are relatively diffused, it may still be seen as a single band, it is indicated that the 7 groups of the short-sequences may be self-assembled better into a RNA nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;

opening software, clicking a menu MeasUre€ManUal, so a manual measurement parameter setting dialog box appears;

setting a software detection parameter;

and then clicking Ok to complete the settings, after a measurement dialog box appears, clicking Start to start.

Measurement result: potential detection results of the 7 groups of the short-sequence RNA nanoparticles are shown in Table 9 to Table 15:

Table 9:

| Sample name | Detection times | Potential ZP (mV) |
|---|---|---|
| R-1 | 1 | -33.7 |
| | 2 | -35.6 |
| | 3 | -34.6 |
| Experim ent result | -34.65 mV. (±0.95mV) | |

Table 10:

| Sample name | Detection times | Potential ZP (mV) |
|---|---|---|
| R-2 | 1 | -37.9 |
| | 2 | -37.3 |
| | 3 | -36.8 |
| Experime nt result | -37.35 mV. (±0.55mV) | |

Table 11:

| Sample name | Detection times | Potential ZP (mV) |
|---|---|---|
| R-3 | 1 | -31.5 |
| | 2 | -33 |
| | 3 | -32.7 |
| Experime nt result | -32.425 mV. (±0.75mV) | |

Table 12:

| Sample name | Detection times | Potential ZP (mV) |
|---|---|---|
| R-4 | 1 | -35.40 |
| | 2 | -36.00 |
| | 3 | -35.50 |
| Experime nt result | -35.7 mV. (±0.3mV) | |

Table 13:

| Sample name | Detection times | Potential ZP (mV) |
|---|---|---|
| R-5 | 1 | -34.00 |
| | 2 | -33.30 |
| | 3 | -34.90 |
| Experime nt result | -34.1 mV. (±0.8mV) | |

Table 14:

| Sample name | Detection times | Potential ZP (mV) |
|---|---|---|
| R-6 | 1 | -33.10 |
| | 2 | -36.10 |
| | 3 | -35.60 |
| Experime nt result | -34.6 mV. (±1.5mV) | |

Table 15:

| Sample name | Detection times | Potential ZP (mV) |
|---|---|---|
| R-7 | 1 | -35.60 |
| | 2 | -34.80 |
| | 3 | -34.00 |
| Experime nt result | -34.80 mV. ± 0.8mV | |

It may be seen from the above potential detection data that the 7 groups of the short-sequence RNA self-assembly products all have good stability, and it is further indicated that the nanoparticles formed by the self-assembly of each short-sequence RNA have a relatively stable self-assembly structure.

It is indicated from the embodiment that: different combinations of the core sequences a, b and c may form the RNA nanoparticles with the nucleic acid domain through the self-assembly, and the structure is stable. It may be seen

on the basis of Embodiment 1 that the RNA nanoparticles may also be successfully assembled by adding various functional extension fragments or a linkage target head, a fluorescein and the like on the basis of these different core sequence combinations, and have functions such as drug loading, cell targeting, visibility and traceability.

In order to further verify these performances, the extension fragment is added on the basis of Embodiment 2, and it is specifically described in Embodiment 3. On the basis of the DNA core sequence corresponding to the RNA core sequence of Embodiment 2, the extension fragment is added, and the target head is connected or unconnected at the same time, it is specifically described in Embodiment 4.

Embodiment 3

I. 7 groups of conventional-sequence RNA nanoparticle carriers:

(1) 7 groups of three polynucleotide base sequences for forming RNA nanoparticles are shown below in Table 16 to Table 22:

Table 16: R-8

| Name | Sequence sense (5'-3') | Base numbe r | Chemical modification | Molecula r weight | Total molecul ar weight |
|---|---|---|---|---|---|
| a (SEQ ID NO: 76) | cGcGcGcccA**GG**AGcG uu**GG**cGGGcGGcG | 29 | C/U 2'F modification | 9462.78 | 28084.1 3 |
| b (SEQ ID NO: 77) | cGccGcccG**cc**uuc**Gcc** **G**ccAGccGcc | 27 | C/U 2'F modification | 8522.18 | |
| c (SEQ ID NO: 78) | GGcGGcAGG**cGGc**cA uAGc**cc**uGGGcGcGc G | 31 | C/U 2'F modification | 10099.1 7 | |

Table 17: R-9

| Name | Sequence sense (5'-3') | Base numbe r | Chemical modification | Molecula r weight | Total molecul ar weight |
|---|---|---|---|---|---|
| a (SEQ ID NO: 79) | cGcGcGcccA**Gc**AGcG uu**cG**cGGGcGGcG | 29 | C/U 2'F modification | 9386.73 | 28084.1 3 |
| b (SEQ ID NO: 80) | cGccGcccG**cG**uuc**Gcc** **G**ccAGccGcc | 27 | C/U 2'F modification | 8560.20 | |
| c (SEQ ID NO: 81) | GGcGGcAGG**cGGc**cA uAGc**Gc**uGGGcGcGc G | 31 | C/U 2'F modification | 10137.2 0 | |

Table 18: R-10

| Name | Sequence sense (5'-3') | Base numbe r | Chemical modification | Molecula r weight | Total molecul ar weight |
|---|---|---|---|---|---|
| a (SEQ ID NO: 82) | cGcGcGcccA**cG**AGcG uu**Gc**GGGGcGGcG | 29 | C/U 2'F modification | 9424.75 | 28084.1 3 |
| b (SEQ ID NO: 83) | cGccGcccc**Gc**uuc**Gcc** **G**ccAGccGcc | 27 | C/U 2'F modification | 8522.18 | |
| c (SEQ ID NO: 84) | GGcGGcAGG**cGGc**cA uAGc**cG**uGGGcGcGc G | 31 | C/U 2'F modification | 10137.2 0 | |

Table 19: R-11

| Name | Sequence sense (5'-3') | Base numbe r | Chemical modification | Molecula r weight | Total molecul ar weight |
|---|---|---|---|---|---|
| a (SEQ ID NO: 85) | cGcGcGcccA**GG**AGC Guu**GG**cccGcGGcG | 29 | C/U 2'F modification | 9386.73 | 28084.1 3 |
| b (SEQ ID NO: 86) | cGccGcGGG**cc**uuc**GG** **GG**ccAGccGcc | 27 | C/U 2'F modification | 8674.28 | |

(continued)

| Name | Sequence sense (5'-3') | Base numbe r | Chemical modification | Molecula r weight | Total molecul ar weight |
|---|---|---|---|---|---|
| c (SEQ ID NO: 87) | GGcGGcAGG**cccc**cAu<br>AGc**cc**uGGGcGcGcG | 31 | C/U 2'F modification | 10023.1 2 | |

Table 20: R-12

| Name | Sequence sense (5'-3') | Base numbe r | Chemical modification | Molecula r weight | Total molecul ar weight |
|---|---|---|---|---|---|
| a (SEQ ID NO: 88) | cGcGcGcccA**Gc**AGcG<br>uu**cG**ccccGccGc | 29 | C/U 2'F modification | 9234.62 | 28084.1 8 |
| b (SEQ ID NO: 89) | GcGGcGGG**cG**uuc**G**<br>**GcG**GcAGGcGGc | 27 | C/U 2'F modification | 8864.41 | |
| c (SEQ ID NO: 90) | GccGccAGc**cGcc**cAu<br>AGc**Gc**uGGGcGcGcG | 31 | C/U 2'F modification | 9985.10 | |

Table 21: R-13

| Name | Sequence sense (5'-3') | Base numbe r | Chemical modification | Molecula r weight | Total molecul ar weight |
|---|---|---|---|---|---|
| a (SEQ ID NO: 91) | cGcGcGcccA**Gc**AGcG<br>uu**cG**GGGcGccGc | 29 | C/U 2'F modification | 9348.70 | 28736.5 3 |
| b (SEQ ID NO: 92) | GcGGcGccc**cG**uuc**GG**<br>**cc**GGcAGGcGGc | 28 | C/U 2'F modification | 9057.52 | |
| c (SEQ ID NO: 93) | GccGccAGcc**GGcc**cA<br>uAGc**Gc**uGGGcGcGc<br>G | 32 | C/U 2'F modification | 10330.3 1 | |

Table 22: R-14 (uGAcAGAuAAGGAAccuGcudTdT in the following a-strand is survivin siRNA)

| Name | Sequence sense (5'-3') | Base numbe r | Chemical modification | Molecula r weight | Total molecul ar weight |
|---|---|---|---|---|---|
| a (SEQ ID NO: 94) | cGcGcG**cG**AGcGuu**G** **c**AAuGAcAGAuAAGG AAccuGcudTdT | 39 | C/U 2'F modification | 12901.9 1 | 31832.4 2 |
| b (SEQ ID NO: 95) | GGcAGGuuccuuAucu GucAAA**Gc**uuc**GGcG** GcAGc | 36 | C/U 2'F modification | 11555.9 7 | |
| c (SEQ ID NO: 96) | GcAGc**cGcc**cAuAGc**c** **G**cGcGcG | 23 | C/U 2'F modification | 7374.54 | |

The single strands of the above 7 groups of conventional-sequence RNA nanoparticle carriers are all commissioned to be synthesized by Suzhou Gima Company, herein the sequence a, the sequence b and the sequence c in R-8 to R-14 are respectively extended RNA oligonucleotide sequences formed by adding the extension fragments on the basis of the sequence a, the sequence b and the sequence c in R-1 to R-7, a targeting module fragment is not extended, and C/U base 2'F modification (resistance to enzyme digestion and stability are enhanced) is performed. In addition, a siRNA nucleic acid interference therapeutic fragment of a survivin is modified in the above RNA nanoparticle R-14, specifically a sense strand (see an underlined part of the a-strand) of Survivin siRNA is extended at a-strand 3'-end, and an antisense strand (see an underlined part of the b-strand) is extended and connected at b-strand 5'-end, so base pair complementary is formed.

II. Self-assembly experiment steps:

(1) according to a molar ratio of 1:1:1, enabling the RNA single strands a, b and c to be simultaneously mixed and dissolved in DEPC treated water or TMS buffer solution;

(2) heating mixed solution to 80 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100V of electrophoresis;

(4) cutting a target band and eluting in RNA elution buffer solution at 37 DEG C, after that, precipitating overnight in ethanol, volatilizing under reduced pressure and low temperature;

(5) electrophoresis analysis detection and laser scanning observation;

(6) electric potential detection.

III. Self-assembly experiment result

(1) Electrophoresis detection result

A 2% agarose gel electrophoresis diagram of the 7 groups of the conventional-sequence RNA self-assembly products is shown in Fig. 5. From left to right, lanes 1 to 7 in Fig. 5 are successively: conventional-sequence RNA self-assembly products R-8, R-9, R-10, R-11, R-12, R-13 and R-14.

A 4% agarose gel electrophoresis diagram of the 7 groups of the conventional-sequence RNA self-assembly

products is shown in Fig. 6. From left to right, lanes 1 to 7 in Fig. 6 are successively: conventional-sequence RNA self-assembly products R-8, R-9, R-10, R-11, R-12, R-13 and R-14.

It may be clearly seen from results of Fig. 5 and Fig. 6 that bands of the 7 groups of the conventional-sequence self-assembly products are bright and clear single bands, it is indicated that the 7 groups of the conventional-sequences may be self-assembled into a nanostructure. Herein after a Survivin siRNA nucleic acid interference therapeutic fragment is modified in the conventional-sequence RNA self-assembly product R-14, it still has the stable self-assembly structure, it is also indicated that the nucleic acid nanoparticles of the disclosure may load a nucleic acid drug, and have a delivery carrier function of the nucleic acid drug.

(2) Electric potential measurement

Measurement method: preparing a potential sample (the self-assembly product is dissolved in ultrapure water) and putting into a sample pool, opening a sample pool cover of an instrument, and placing the instrument;

opening software, clicking a menu MeasUre€ManUal, so a manual measurement parameter setting dialog box appears;

setting a software detection parameter;

and then clicking Ok to complete the settings, after a measurement dialog box appears, clicking Start to start.

Measurement result: potential detection results of the 7 groups of the conventional-sequence RNA nanoparticles are shown below in Table 23 to Table 29:

Table 23:

| Sample name | Detection times | Potential ZP (mV) |
|---|---|---|
| R-8 | 1 | -18.00 |
| | 2 | -16.10 |
| | 3 | -18.20 |
| Experim ent result | -17.43 mV (±1.33mV) | |

Table 24:

| Sample name | Detection times | Potential ZP (mV) |
|---|---|---|
| R-9 | 1 | -16.90 |
| | 2 | -17.50 |
| | 3 | -20.20 |
| Experime nt result | -18.20 mV (±1.3mV) | |

Table 25:

| Sample name | Detection times | Potential ZP (mV) |
|---|---|---|
| R-10 | 1 | -10.40 |
| | 2 | -11.80 |
| | 3 | -10.40 |
| Experime nt result | -10.87 mV (±0.47mV) | |

Table 26:

| Sample name | Detection times | Potential ZP (mV) |
|---|---|---|
| R-11 | 1 | -28.30 |
| | 2 | -26.00 |
| | 3 | -33.10 |
| Experime nt result | -29.13mV (±3.13mV) | |

Table 27:

| Sample name | Detection times | Potential ZP (mV) |
|---|---|---|
| R-12 | 1 | -7.59 |
| | 2 | -7.80 |
| | 3 | -17.20 |
| Experime nt result | -10.86 mV (±3.27mV) | |

Table 28:

| Sample name | Detection times | Potential ZP (mV) |
|---|---|---|
| R-13 | 1 | -9.64 |
| | 2 | -15.60 |
| | 3 | -21.10 |
| Experime nt result | -15.45 mV (±5.81 mV) | |

Table 29:

| Sample name | Detection times | Potential ZP (mV) |
|---|---|---|
| R-14 | 1 | -21.40 |
| | 2 | -21.20 |
| | 3 | -28.00 |
| Experime nt result | -23.53 mV. ± 2.33mV | |

It may be seen from the above potential detection data that the 7 groups of the conventional-sequence RNA self-assembly products all have good stability, and it is further indicated that the nanoparticles formed by the self-assembly of each conventional-sequence RNA have a relatively stable self-assembly structure.

It is indicated from the embodiment that: on the basis of different combinations of the RNA core sequences, the RNA nanoparticles with the stable structure may also be successfully self-assembled by adding the extension fragments. At the same time, the added extension fragments make the RNA nanoparticles have superior drug loading performance (specifically described in Embodiment 5).

Embodiment 4

I. 7 groups of conventional-sequence DNA nanoparticle carriers:

(1) 9 groups of three polynucleotide base sequences for forming DNA nanoparticles are respectively shown in Table 30 to Table 36:

An EGFRapt target head or a PSMAapt (A9L) target head is extended in a part of a-strands in the table:

EGFRapt (SEQ ID NO:97): GCCTTAGTAACGTGCTTTGATGTCGATTCGACAGGAGGC;

PSMAapt (A9L, SEQ ID NO: 98): GGGCCGAAAAAGACCTGACTTCTATACTAAGTCTACGTCCC.

Table 30: D-1

| Name | Sequence sense (5'-3') | Base number | Chemical modification | Molecular weight | Total molecular weight |
|---|---|---|---|---|---|
| a (SEQ ID NO: 99) | CGCGCGCCCAGGAGCGTTG GCGGGCGGCGGCCTTAGTAA CGTGCTTTGATGTCGATTCGA CAGGAGGC | 68 | 3 bases before 5'-end and 3'-end, thio modification | 21214.63 | 39092.09 |
| b (SEQ ID NO: 100) | CGCCGCCCGCCTTCGCCGC CAGCCGCC | 27 | 3 bases before 5'-end and 3'-end, thio modification | 8176.24 | |
| c (SEQ ID NO: 101) | GGCGGCAGGCGGCCATAGC CCTGGGCGCGCG | 31 | 3 bases before 5'-end and 3'-end, thio modification | 9701.22 | |

Table 31: D-2

| Name | Sequence sense (5'-3') | Base number | Chemical modification | Molecular weight | Total molecular weight |
|---|---|---|---|---|---|
| a (SEQ ID NO: 102) | CGCGCGCCCAGCAGCGTTC GCGGGCGGCGGCCTTAGTAA CGTGCTTTGATGTCGATTCGA CAGGAGGC | 68 | 3 bases before 5'-end and 3'-end, thio modification | 21134.59 | 39092.11 |
| b (SEQ ID NO: 103) | CGCCGCCCGCGTTCGCCGC CAGCCGCC | 27 | 3 bases before 5'-end and 3'-end, thio modification | 8216.27 | |
| c (SEQ ID NO: 104) | GGCGGCAGGCGGCCATAGC GCTGGGCGCGCG | 31 | 3 bases before 5'-end and 3'-end, thio modification | 9741.25 | |

Table 32: D-3

| Name | Sequence sense (5'-3') | Base number | Chemical modification | Molecular weight | Total molecular weight |
|---|---|---|---|---|---|
| a (SEQ ID NO: 105) | CGCGCGCCCACGAGCGTTGCGGGGCGGCGGCCTTAGTAACGTGCTTTGATGTCGATTCGACAGGAGGC | 68 | 3 bases before 5'-end and 3'-end, thio modification | 21174.60 | 39092.09 |
| b (SEQ ID NO: 106) | CGCCGCCCCGCTTCGCCGCCAGCCGCC | 27 | 3 bases before 5'-end and 3'-end, thio modification | 8176.24 | |
| c (SEQ ID NO: 107) | GGCGGCAGGCGGCCATAGCCGTGGGCGCGCG | 31 | 3 bases before 5'-end and 3'-end, thio modification | 9741.25 | |

Table 33: D-4

| Name | Sequence sense (5'-3') | Base number | Chemical modification | Molecular weight | Total molecular weight |
|---|---|---|---|---|---|
| a (SEQ ID NO: 108) | CGCGCGCCCAGGAGCGTTGGCCCGCGGCGTGGGCCGAAAAAGACCTGACTTCTATACTAAGTCTACGTCCC | 71 | 3 bases before 5'-end and 3'-end, thio modification | 21923.12 | 39780.63 |
| b (SEQ ID NO: 109) | CGCCGCGGGCCTTCGGGGCCAGCCGCC | 27 | 3 bases before 5'-end and 3'-end, thio modification | 8236.34 | |
| c (SEQ ID NO: 110) | GGCGGCAGGCCCCCATAGCCCTGGGCGCGCG | 31 | 3 bases before 5'-end and 3'-end, thio modification | 9621.17 | |

Table 34: D-5

| Name | Sequence sense (5'-3') | Base number | Chemical modification | Molecular weight | Total molecular weight |
|---|---|---|---|---|---|
| a (SEQ ID NO: 111) | CGCGCGCCCAGCAGCGTTCGCCCCGCCGCTGGGCCGAAAAAGACCTGACTTCTATACTAAGTCTACGTCCC | 71 | 53 bases before 5'-end and 3'-end, thio modificatio n | 21763.03 | 39880.64 |
| b (SEQ ID NO: 112) | GCGGCGGGGCGTTCGGCGGCAGGCGGC | 27 | 3 bases before 5'-end and 3'-end, thio modification | 8536.46 | |
| c (SEQ ID NO: 113) | GCCGCCAGCCGCCCATAGCGCTGGGCGCGCG | 31 | 3 bases before 5'-end and 3'-end, thio modification | 9581.15 | |

Table 35: D-6

| Name | Sequence sense (5'-3') | Base number | Chemical modification | Molecular weight | Total molecular weight |
|---|---|---|---|---|---|
| a (SEQ ID NO: 114) | CGCGCGCCCAGCAGCGTTCGGGGCGCCGC | 29 | 3 bases before 5'-end and 3'-end, thio modification | 8978.76 | 27594.69 |
| b (SEQ ID NO: 115) | GCGGCGCCCCGTTCGGCCGGCAGGCGGC | 28 | 3 bases before 5'-end and 3'-end, thio modification | 8705.57 | |
| c (SEQ ID NO: 116) | GCCGCCAGCCGGCCCATAGCGCTGGGCGCGCG | 32 | 3 bases before 5'-end and 3'-end, thio modification | 9910.36 | |

Table 36: D-7

| Name | Sequence sense (5'-3') | Base number | Chemical modification | Molecular weight | Total molecular weight |
|---|---|---|---|---|---|
| a (SEQ ID NO: 117) | CGCGCGCCCACGAGCGT TGCGGGCGCCGC | 29 | 3 bases before 5'-end and 3'-end, thio modification | 8978.76 | 26976.30 |
| b (SEQ ID NO: 118) | GCGGCGCCCGCTTCGG CGGCAGGCGGC | 27 | 3 bases before 5'-end and 3'-end, thio modification | 8416.39 | |
| c (SEQ ID NO: 119) | GCCGCCAGCCGCCCATA GCCGTGGGCGCGCG | 31 | 3 bases before 5'-end and 3'-end, thio modification | 9581.15 | |

Single strands of the above 7 groups of the conventional-sequence DNA nanoparticles are all commissioned to be synthesized by Suzhou Hongxun Company, herein:

D-1 is the conventional-sequence DNA nanoparticles formed after adding an extension sequence containing the EGFRapt target head (see an underlined part) on the basis of the above core sequences (8) (sequence a: 5'-GGAGCGTTGG-3', sequence b: 5'-CCTTCGCCG-3', and sequence c: 5'-CGGCCATAGCCC-3');
D-2 is the conventional-sequence DNA nanoparticles formed after adding an extension sequence containing the EGFRapt target head (see an underlined part) on the basis of the above core sequences (9) (sequence a: 5'-GCAGCGTTCG-3', sequence b: 5'-CGTTCGCCG-3', and sequence c: 5'-CGGCCATAGCGC-3');
D-3 is the conventional-sequence DNA nanoparticles formed after adding an extension sequence containing the EGFRapt target head (see an underlined part) on the basis of the above core sequences (10) (sequence a: 5'-CGAGCGTTGC-3', sequence b: 5'-GCTTCGCCG-3', and sequence c: 5'-CGGCCATAGCCG-3');
D-4 is the conventional-sequence DNA nanoparticles formed after adding an extension sequence containing the PSMAapt target head (see an underlined part) on the basis of the above core sequences (11) (sequence a: 5'-GGAGCGTTGG-3', sequence b: 5'-CCTTCGGGG-3', and sequence c: 5'-CCCCCATAGCCC-3');
D-5 is the conventional-sequence DNA nanoparticles formed after adding an extension sequence containing the PSMAapt target head (see an underlined part) on the basis of the above core sequences (12) (sequence a: 5'-GCAGCGTTCG-3', sequence b: 5'-CGTTCGGCG-3', and sequence c: 5'-CGCCCATAGCGC-3');
D-6 is the conventional-sequence DNA nanoparticles formed after adding an extension sequence without containing a target head structure on the basis of the above core sequences (13) (sequence a: 5'-GCAGCGTTCG-3', sequence b: 5'-CGTTCGGCC-3', and sequence c: 5'-GGCCCATAGCGC-3'); and
D-7 is the conventional-sequence DNA nanoparticles formed after adding an extension sequence without containing a target head structure on the basis of the above core sequences (14) (sequence a: 5'-CGAGCGTTGC-3', sequence b: 5'-GCTTCGGCG-3', and sequence c: 5'-CGCCCATAGCCG-3').

In addition, a single-stranded sequence used to form the 8-th group of the DNA nanoparticles and a single-stranded sequence used to form the 9-th group of the DNA nanoparticles are also synthesized.

Herein, the 8-th group is the conventional sequence DNA nanoparticles formed after the extension sequence containing the EGFRapt target head (see a bolded part) is added on the basis of the core sequence (15) described above. A specific sequence is as follows:

a-strand (SEQ ID NO:172): 5'-CGCGCGCCCACGAGCGTTCCGGGCGC**GCCTTAGTAACGTGCTTTGATGTCGATTCG ACAGGAGGC-**3', thio modification is respectively performed on the first three bases at the 5'-end and the last three bases at the 3'-end, and the 5'-end is linked to Biotin, the bolded part is the EGFRapt sequence;

b-strand (SEQ ID NO:173): 5'-GCGCCCGGTTCGCCGCCAGCCGCCGC-3', the thio modification is respectively performed on the first three bases at the 5'-end and the last three bases at the 3'-end; and

c-strand (SEQ ID NO:174): 5'-GCGGCGGCAGGCGGCCATAGCCGTGGGCGCGCG-3', the thio modification is respectively performed on the first three bases at the 5'-end and the last three bases at the 3'-end, and the 3'-end is linked with a Cy5 fluorescent label.

Herein, the 9-th group is the DNA nanoparticles formed after the extension sequence is added on the basis of the core sequence (5) described above. A specific sequence is as follows:

a-strand (SEQ ID NO:178): 5'-**CGC**GCGCGCCCACGAGCGTTCCGGGCGCCGC**CGC**-3', the thio modification is respectively performed on the first three bases at the 5'-end and the last three bases at the 3'-end, and the 5'-end is linked with a Biotin;

b-strand (SEQ ID NO:179): 5'-**GCG**GCGGCGCCCGGTTCGCCGCCAGCCGCC**GCC**-3', the thio modification is respectively performed on the first three bases at the 5'-end and the last three bases at the 3'-end; and

c-strand (SEQ ID NO:180): 5'-**GGC**GGCGGCAGGCGGCCATAGCCGTGGGCGCGC**GCG-**3', the thio modification is respectively performed on the first three bases at the 5'-end and the last three bases at the 3'-end, and the 5'-end is linked with a Cy5 fluorescent label.

II. Self-assembly experiment steps:

(1) According to a molar ratio of 1:1:1, enabling the DNA single strands a, b and c to be simultaneously mixed and dissolved in DEPC treated water or TMS buffer solution;

(2) heating mixed solution to 95 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in DNA elution buffer solution at 37 DEG C, after that, precipitating overnight in ethanol, volatilizing under reduced pressure and low temperature, to obtain a conventional-sequence DNA self-assembly product;

(5) electrophoresis analysis detection and laser scanning observation;

(6) electric potential detection;

(7) particle size measurement; and

(8) observing by a transmission electron microscope.

III. Self-assembly experiment result

(1) Electrophoresis detection result

A 2% agarose gel electrophoresis diagram of the 7 groups of the conventional-sequence DNA self-assembly products is shown in Fig. 7. From left to right, lanes 1 to 7 in Fig. 7 are successively: conventional-sequence DNA self-assembly products D-1, D-2, D-3, D-4, D-5, D-6 and D-7.

A 4% agarose gel electrophoresis diagram of the 7 groups of the conventional-sequence DNA self-assembly products is shown in Fig. 8. From left to right, lanes 1 to 7 in Fig. 8 are successively: conventional-sequence DNA self-assembly products D-1, D-2, D-3, D-4, D-5, D-6 and D-7.

A 2% agarose gel electrophoresis diagram of the 8-th and 9-th groups of the sequence DNA self-assembly products is shown in Fig. 9. From right to left, lanes in Fig. 9 are successively: a a-strand single-strand in the 8-th group, and DNA self-assembly products D-8 and D-9.

It may be clearly seen from results of Fig. 7, Fig. 8 and Fig. 9 that bands of the 7 groups of the conventional-sequence DNA self-assembly products are bright and clear, it is indicated that the 7 groups of the conventional-sequence DNA strands are all self-assembled to form stable nanoparticle structures. Herein two groups of the self-assembly structures D-6 and D-7 carry the EGFRapt or PSMAapt target head, and the molecular weights are slightly low, positions of bands thereof are apparently higher than other bands, actual and theoretical conditions are completely consistent, so the stability of the self-assembly structures is further proved.

It is indicated from the embodiment: on the base of combinations of these different DNA core sequences, when various functional extension fragments are added or the target head is linked at the same time, the DNA nanoparticles

may also be successfully assembled, and also have functions such as drug loading, cell targeting, visibility and traceability.

(2) Electric potential measurement

Measurement method: preparing a potential sample (the self-assembly product is dissolved in ultrapure water) and putting into a sample pool, opening a sample pool cover of an instrument, and placing the instrument;

opening software, clicking a menu MeasUre€ManUal, so a manual measurement parameter setting dialog box appears;

setting a software detection parameter;

and then clicking Ok to complete the settings, after a measurement dialog box appears, clicking Start to start.

Measurement result: potential detection results of the 3 groups of the conventional-sequence DNA nanoparticles are shown below in Table 37 to 39:

Table 37:

| Sample name | Detection times | Potential ZP (mV) |
|---|---|---|
| D-2 | 1 | -36.00 |
| | 2 | -35.80 |
| | 3 | -37.60 |
| Experiment result | -36.47 mV (±0.67mV) | |

Table 38:

| Sample name | Detection times | Potential ZP (mV) |
|---|---|---|
| D-6 | 1 | -31.70 |
| | 2 | -32.10 |
| | 3 | -31.90 |
| Experiment result | -31.90 mV (±0.2mV) | |

Table 39:

| Sample name | Detection times | Potential ZP (mV) |
|---|---|---|
| D-7 | 1 | -32.10 |
| | 2 | -31.70 |
| | 3 | -32.80 |
| Experiment result | -32.20 mV (±0.0.5mV) | |

It may be seen from the above potential detection data that the 3 groups of the conventional-sequence DNA self-assembly products have good stability, it is further indicated that the nanoparticles formed by the self-assembly of each conventional-sequence DNA have a relatively stable self-assembly structure.

(3) Particle size measurement

1. Preparing a potential sample (conventional-sequence DNA self-assembly product D-7) and putting into a sample pool, opening a sample pool cover of an instrument, and placing the instrument;

2. opening software, and clicking a menu, so a manual measurement parameter setting dialog box appears;

3. setting a software detection parameter; and then

4. clicking Ok to complete the settings, when a measurement dialog box appears, clicking Start to start, a DLS measurement value result of a hydrodynamic size of the self-assembly product D-7 is shown below in Table 40:

Table 40:

| Sample name | Detection times | Particle size Z-Ave (d.nm) |
|---|---|---|
| 1 | 1 | 10.76 |
| | 2 | 13.9 |
| | 3 | 10.36 |
| Experiment result | 12.33 d.nm (±1.57 d.nm) | |

(4) Observed result of transmission electron microscope

The above conventional-sequence DNA self-assembly product D-7 is irradiated by a transmission electron microscope, and steps are as follows:

1. taking a drop of a sample and suspending on 400 meshes of a carbon-coated film copper grid, keeping for 1 min at a room temperature;

2. absorbing liquid by filter paper;

3. staining for 1 min by 2% uranyl acetate;

4. absorbing by the filter paper, and drying at the room temperature; and

5. observing and taking a picture by a transmission electron microscope JEM-1400 at 120 kv.

The result is as shown in Fig. 10, it may be apparently seen from the figure that the above conventional-sequence DNA self-assembly product D-7 is an integral structure, and it may be clearly seen that it has a T-type structure.

Embodiment 5

**Paclitaxel loading experiment**

(I) Chemical loading:

I. Experiment material and experiment method

1. Experiment materials and reagents:

(1) Nucleic acid nanoparticles: it is similar to the RNA nanoparticles in Embodiment 1, a difference is that a fluorescent label on the c-strand is Cy5.

(2) DEPC treated water: Biyuntian Company.

(3) PBS buffer solution: cellgro.

(4) 4% paraformaldehyde.

(5) Paclitaxel (Epirarunixon).

(6) Chloroform: Beijing Beihua Fine Chemicals Co., Ltd.

(7) Anhydrous ethanol: Beijing Beihua Fine Chemicals Co., Ltd.

2. Experiment method:

(1) Accurately weighing the paclitaxel (1.354μmol) and dissolving in the DEPC treated water (1.0 mL) and the PBS buffer solution (1.25 mL), adding 4% paraformaldehyde aqueous solution (0.25 mL) under cooling of an ice-water bath and uniformly mixing, enabling the mixed solution to be totally uniformly mixed with the RNA nanoparticles (33.84 nmol), and reacting for 72 hours at 4 DEG C under a dark condition.

(2) Taking 10 μL of reaction solution and diluting for 10 times, using 50 μM of the paclitaxel aqueous solution and 310 ng/μL of the RNA nanoparticles as controls, and performing HPLC analysis according to equal volume injection. According to a peak area ratio of each component, it may be adjusted that reaction conversion is basically completed.

(3) Extracting the reaction solution by the chloroform (10mLx3), and then adding 25 mL of the anhydrous ethanol, after uniformly mixing, keeping at 4 DEG C in the dark and enabling a product to be adequately precipitated (4 hours). Centrifuging (4000/min), transferring supernatant, and washing the solid product again by the ethanol (50 mL), and evaporating a solvent under reduced pressure and lower temperature, to obtain a dark red solid product.

(4) Loading rate calculation:

1. preparing a known concentration of paclitaxel-anhydrous ethanol standard solution: 2 μM, 4 μM,6 μM,8 μM,and 10 μM,100 μL each;

2. enabling paclitaxel-RNAh particles to be dissolved in 100 μL of the PBS;

3. putting the standard solution and the paclitaxel-RNAh particles into a PCR plate, heating at 85 DEG C for 5 min, and then cooling to the room temperature;

4. measuring an absorbance of the paclitaxel at 233 nm by using a microplate reader, drawing a standard curve, and calculating the molar concentration of the paclitaxel in the loaded product;

5. measuring an absorbance of RNA at 260 nm by using a spectrophotometer, to obtain the mass concentration of the RNAh particles contained in each sample; and

6. according to the paclitaxel molar concentration and the mass concentration of the RNAh particles obtained by measuring, calculating a loading rate.

The standard curve of the RNA nucleic acid nanoparticles loading the paclitaxel is shown in Fig. 11a, and a specific calculation process is as follows:

$$C_{RNAh}\text{-1} = 32.9\ \text{ug/ml},\ M_{RNAh} \approx 30000,\ 100\text{ul};\ C\ \text{paclitaxel -1} = 12.8\ \mu M,\ 100\text{ul};$$

$$C_{RNAh}\text{-2} = 58.5\ \text{ug/ml},\ M_{RNAh} \approx 30000,\ 100\text{ul};\ C\ \text{paclitaxel -2} = 24.03\ \mu M,\ 100\text{ul};$$

$$\text{N-1} = \frac{12.8 \times 10\text{-}6 \times 100 \times 10\text{-}6}{0.0329 \times 100 \times 10\text{-}6/30000} = 11.6 \qquad \text{N-2} = \frac{24.03 \times 10\text{-}6 \times 100 \times 10\text{-}6}{0.0585 \times 100 \times 10\text{-}6/30000} = 12.3$$

An average value thereof is taken, and it is obtained that the loading rate of the paclitaxel-RNAh nucleic acid

nanoparticles is about 12, it represents that about 12 paclitaxel molecules may be loaded on each nucleic acid nanoparticle carrier.

(II) Loading experiment of DNA nucleic acid nanoparticles

The loading method and the calculation mode of the loading rate are the same as the above RNA nucleic acid nanoparticles. The specific nucleic acid nanoparticles used are: DNAh-Bio-EFGRapt-Cy5, herein, the three strands of DNAh are respectively:

a-strand (SEQ ID NO:172): 5'-CGCGCGCCCACGAGCGTTCCGGGCGC**GCCTTAGTAACGTGCTTTGATGTCGATTCG ACAGGA**GGC-3', thio modification is respectively performed on the front three bases of the 5'-end and the last three bases (italic part) of the 3'-end, the 5'-end is linked with Biotin, and a bolded part is a sequence siRNA of EGFR;

b-strand (SEQ ID NO:173): 5'-GCGCCCGGTTCGCCGCCAGCCGCCGC-3', the thio modification is respectively performed on the front three bases of the 5'-end and the last three bases (italic part) of the 3'-end; and

c-strand (SEQ ID NO:174): 5'-GCGGCGGCAGGCGGCCATAGCCGTGGGCGCGCG-3', the thio modification is respectively performed on the front three bases of the 5'-end and the last three bases (italic part) of the 3'-end, and the 3'-end is linked with the fluorescent label Cy5.

The standard curve of the DNA nucleic acid nanoparticles loading the paclitaxel is shown in Fig. 11b, and a specific calculation process is as follows:

$$C_{DNAh}\text{-}1 = 32.9\ ug/ml,\ M_{DNAh} \approx 39500,\ 100ul;\ C\ paclitaxel\ \text{-}1 = 12.8\ \mu M,\ 100ul;$$

$$C_{DNAh}\text{-}2 = 58.5\ ug/ml,\ M_{DNAh} \approx 39500,\ 100ul;\ C\ paclitaxel\ \text{-}2 = 24.03\ \mu M,\ 100ul;$$

$$N\text{-}1 = \frac{10.48\times 10\text{-}6\times 100\times 10\text{-}6}{0.3585\times 100\times 10\text{-}6/39500} \approx 11.55 \qquad N\text{-}2 = \frac{19.35\times 10\text{-}6\times 100\times 10\text{-}6}{0.5794\times 100\times 10\text{-}6/39500} \approx 13.19$$

An average value thereof is taken, and it is obtained that the loading rate of the paclitaxel-DNAh nucleic acid nanoparticles is about 12, it represents that about 12 paclitaxel may be loaded on each DNA nanoparticle carrier.

It is indicated from Embodiment 5 that the RNA nanoparticles (in Embodiment 1) and DNA nanoparticles with the extension fragment, the target head and the fluorescein both have a function of loading drugs, and the small molecular drug paclitaxel may achieve the loading in a mode of covalent linkage (paraformaldehyde-solvent covalence).

Embodiment 6

**Cell binding ability of drug-loaded DNA nanoparticles detected by flow cytometry experiment**

I. Cell information

SK-OV-3 (source: ATCC, and article number: HTB-77), a medium is MEM + 10% FBS, and culture conditions are 37°C, 5%$CO_2$, and saturated humidity.

II. Substances to be tested

Targeted drug: DNAh-Bio-EGFRapt-Cy5-paclitaxel (loaded product of the DNA nanoparticles in Embodiment 5). Fluorescent carrier: DNAh-Bio-EGFRapt-Cy5

III. Devices and consumables (see Table 41)

Table 41:

| Name | Brand | Article number / Model |
|---|---|---|
| 24-well plate | Corning | 3526 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |
| Flow cytometer | ACEA | Novo Cyte |

IV. Reagents (see Table 42)

Table 42:

| Name | Brand | Article number |
|---|---|---|
| MEM medium (without folic acid and biotin) | provided by Baiyao Zhida Nanobiotechnology Co., Ltd | YS4150-500 |
| Fetal bovine serum FBS | Cegrogen | A0500-3018 |
| GlutaMax | Gibco | 35050-061 |
| Trypsin-EDTA digestive juice (0.25%) | Solarbio | T1320-100ml |
| Penicillin-streptomycin (100X) | Solarbio | P1400 |
| CellTiter-Glo® 2.0 | Promega | G9243 |

V. Experiment method:

1) Adjusting a cell state to a logarithmic growth phase, changing the medium to a medium without biotin and folic acid, and incubating overnight in the incubator at 37 DEG C;

2) dissolving a substance to be tested and preparing stock solution of the substance to be tested;

3) digesting and collecting single-cell suspension and counting, adjusting a cell density to $2\times10^5$/mL, and planting 1 mL/well into the 24-well plate;

4) respectively adding the substance to be tested to the corresponding cell wells, herein a final concentration is 0.1 μM, 0.2 μM, and 0.4 μM, shaking and mixing uniformly;

5) placing the cell plate in the incubator at 37 DEG C and incubating for 2 hours;

6) after the incubation, trypsinizing and collecting the cell suspension;

7) collect a cell precipitation by centrifugation, and washing twice with the PBS;

8) finally, resuspending the cell precipitation with 300 μL of the PBS, and performing a flow cytometry on-machine detection;

9) fluorescent carrier or paclitaxel detection channels: excitation light wavelength: 488 nm, and emission light wavelength: 560 nm; and

10) data analysis.

VI. Experiment result (see Table 43)

Table 43:

| Cell line | Test sample | Binding rate (%) |
|---|---|---|
| SK-OV-3 | DNAh-Bio-EFGRapt-Cy5-paclitaxel | 99.99 |
| | DNAh-Bio-EFGRapt-Cy5 | 100 |
| | Blank control (cell-only medium) | 0.11 |

It may be seen from Table 43 that the paclitaxel targeted drug DNAh-Bio-EGFRapt-Cy5-paclitaxel may bind to the SK-OV-3 cells, and the binding rate is almost 100%; and the fluorescent carrier DNAh-Bio-EGFRapt-Cy5 may also bind to the SK-OV-3 cells, the binding rate is 100% too.

Embodiment 7

**Stability detection of DNAh-Bio-EGFRapt-Cy5-paclitaxel nanoparticles in serum**

I. Experiment material, reagent and device

1. Experiment material:
DNAh-Bio-EGFRapt-Cy5-paclitaxel (same as Embodiment 6), herein a concentration is 1000.0 μg/ml.

2. Experiment reagent:
6×DNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).

PowerPac Basic (Bio-rad), Mini PROTEAN Tetra Cell (Bio-rad), orbital shaker (TS-3D), and Tanon (Tanon 3500).

II. Experiment method

(1) Taking 2 μL of the DNAh-Bio-EGFRapt-Cy5-paclitaxel nanoparticles, diluting with the medium containing 2 μL of 50% FBS 1640 + 6μL of RPMI 1640, herein the concentration may reach 200 μg/ml after dilution, diluting by 5 tubes respectively, and placing the diluted sample at 37 DEG C of a water bath for different times (0, 10 min, 1 h, 12 h, and 36 h).

(2) Taking 10 μl of the treated sample and uniformly mixing with 2 μl of the 6×DNA Loading Buffer, operating on ice, and making a label.

(3) Taking 8% Native PAGE, applying a piece of the gel to the nanoparticle samples with the different incubation times, herein a loading amount is 12 μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 12, herein 1 represents 0 min, 2 represents 10 min, 3 represents 1 h, 4 represents 12 h, and 5 represents 36 h. The target band of the DNAh-Bio-EGFRapt-Cy5-paclitaxel nanoparticles is about 200 bp. It may be seen from Fig. 12 that the DNAh-Bio-EGFRapt-Cy5-paclitaxel nanoparticles are basically stable after being incubated at 37 DEG C.

Embodiment 8

**Cytotoxicity research of RNAh-Bio-670-paclitaxel nanoparticles in U87MG cells**

I. Experiment material and experiment method

1. Experiment material:
Samples to be tested: small molecular drug paclitaxel and RNAh-Bio-670-paclitaxel nanoparticles (note: RNAh-Bio-670-paclitaxel nanoparticles are nanoparticles prepared according to the self-assembly method in Embodiment 1 and formed by performing Biotin modification at the 5'-end of the a-strand and b-strand, performing quasar670 fluorescein modification at the 3'-end of the c-strand, and further loading the paclitaxel (loaded according to the chemical method in Embodiment 5)).

Drug concentration preparation:
Preparing a freshly prepared reagent into a corresponding volume container, and adding PBS to be quantified to 10 μM.
2. Experiment reagent:
EMEM medium (Gibco); Fetal bovine serum (FBS) (ExCell Bio, FNA500-500 mL); Penicillin/Streptomycin (PS) (Gibco, 15140-122-100 mL); PBS buffer solution (Gibco, C20012500BT-500 mL); Trypsin-EDTA (Stemcell, 07901-500 mL); DMSO (Sigma, D5879-1 L); and CellTiter-Glo Luminescent Cell Viability Assay kit (CTG) (Promega, G7572-100 mL).
3. Experiment instrument:
Inverted Microscope (Olympus IX71, No.112A-1); 96-well Plate Reader (Molecular Devices, Flexstation 3); and Perkin Elmer Envision 2104 Multilabel Reader (No. 01-094- 0002).
4. Experiment method:

1) Cell culturing and plating

U87MG cells are cultured at 37 DEG C and 5%$CO_2$ in the EMEM basal medium in which 10%FBS and 1%PS are respectively added. The cell density used in the experiment is above 80%. The cells are collected, and centrifuged at 1000 rpm for 4 minutes, the medium is resuspended, the cell concentration is adjusted, and it is added to the 96-well plate in a volume of 5000 cells per 90μL, and each group has 4 replicate wells.

2) Gradient drug concentration preparation and administration

After 24 hours, the compound solution is transferred to each well, 200 nM of each sample per well, and 4 replication wells.

Solvent control = DMSO

Medium (untreated) control: only cells without compound treatment

Blank control: without cells, used for instrument zero calibration

3) Cell culture after administration

The above cells after administration are cultured for 72 hours under a condition of 37 DEG C and 5% $CO_2$.

4) Cells treated by detection kit

The well plate is brought to a room temperature in advance and stands for 30 minutes. 100μL of a CellTiter-Glo® reagent is added to each well of the well plate and mixed for 2 minutes on a shaker to promote cell lysis. The Perkin Elmer Envision 2104 Multilabel Reader is used to read values and the values are recorded.

5) Experiment data acquisition and processing

The acquired experiment data is analyzed and processed by using excel software, and GraphPad Prism 5 software is used for curve fitting analysis.

II. Experiment result:

Table 44: cell survival rate (%)

| Cell line | Treatment time | Paclitaxel | RNAh-Bio-670-paclitaxel |
|---|---|---|---|
| U87MG | 72h | 56.74 | 76.23 |

Experimental results are shown in Table 44 and Fig. 13, it may be seen from Table 44 and Fig. 13 that the paclitaxel and RNAh-Bio-670-paclitaxel nanoparticles both have a significant inhibitory effect on the proliferation of U87MG cells, and it is unpredictable that: when the drug concentration is 10 $\mu$M, the inhibition rates of the two drugs to the cells are 23.77% and 43.26% respectively. It may be seen that RNAh-Bio-670-paclitaxel nanoparticles have the stronger inhibitory activity to the cell proliferation, so the dosage of the drugs may be significantly reduced, and the toxic side effects are reduced.

Furthermore, in order to determine that the targeted fluorescent carrier itself is not significantly toxic to the U87MG cells, the present application further designs a toxicity experiment of the RNAh-Bio-FAM targeted fluorescent carrier to the U87MG cells, and the small molecular chemical drug Cisplatin is used as a control of the toxicity of the U87MG cells, and a result shows that the fluorescent carrier itself has no apparent toxicity to the U87MG cells (data is not shown).

Embodiment 9

**Cytotoxicity of DNAh-Bio-EGFRapt-Cy5-paclitaxel nanoparticles in SKOV3 cells**

I. Experiment material

1. Cell information (see Table 45)

Table 45:

| Name | Source | Medium | Culture condition |
|---|---|---|---|
| SKOV3 | ATCC | MEM, 10% FBS | 37°C, 5%$CO_2$, Saturated humidity |

2. Samples to be tested (see Table 46)

Table 46:

| Name | Carrier mass (mg) | Carrier molecular weight | Drug loaded mass (mg) | Drug molecular weight | Character | Preservation condition |
|---|---|---|---|---|---|---|
| DNAh-Bio-EGFRapt-Cy5-paclitaxel (same as Embodiment 6) | 0.5 | 39493 | 0.097 | 853.9 | Blue solid | -20°C |
| Paclitaxel | - | - | 1.500 | 853.91 | White powder | -20°C |

(continued)

| Name | Carrier mass (mg) | Carrier molecular weight | Drug loaded mass (mg) | Drug molecular weight | Character | Preservation condition |
|---|---|---|---|---|---|---|
| DNAh-Bio-EGFRapt-Cy5 (DNAh3) | 1.25* 3 | 39485. 9 | — | — | Blue solid | -20°C |

3. Consumables and devices (see Table 47):

Table 47:

| Name | Brand | Article number / Model |
|---|---|---|
| 96-well plate | Corning | 3599 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |

4. Reagents (see Table 48):

Table 48:

| Name | Brand | Article number |
|---|---|---|
| RPMI 1640 medium (without folic acid and biotin) | provided by Baiyao Zhida Nano-biotechnology Co., Ltd | YS3160-500 |
| MEM medium (without folic acid and biotin) | provided by Baiyao Zhida Nano-biotechnology Co., Ltd | YS4150-500 |
| DMEM medium (without folic acid and biotin) | provided by Baiyao Zhida Nano-biotechnology Co., Ltd | YS1200-500 |
| FBS | Cegrogen | A0500-3018 |
| GlutaMax | Gibco | 35050-061 |
| PBS | Gibco | C14190500BT |
| DMSO | Solarbio | D8371 |
| Trypsin-EDTA digestive juice (0.25%) | Solarbio | T1320-100ml |
| CellTiter 96® AQueous One Solution | Promega | G3581 |

II. Experiment method:

1) Harvesting cells in a logarithmic growth phase, taking a small amount and staining with trypan blue for counting to ensure that the cell viability reaches more than 98%;
2) adjusting the cell density to $2.22 \times 10^4$ /mL with a growth medium;
3) planting 90 μL/well of cell suspension into the 96-well plate, herein the number of cells per well in the plate is 2000;
4) placing the planted cell plate in the incubator at 37 DEG C and incubating overnight;

5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action concentration is shown in Table 49 below;

Table 49:

| Test sample | Concentration gradient |
|---|---|
| Paclitaxel | 100μM, 31.6μM, 10μM, 3.16μM, 1μM, 316nM, 100nM, 31.6nM, 10nM, 0 (10%PBS) |
| DNAh-Bio-EGFRapt-Cy5-paclitaxel | 100μM, 31.6μM, 10μM, 3.16μM, 1μM, 316nM, 100nM, 31.6nM, 10nM, 0 (10%PBS) |
| DNAh-Bio-EGFRapt-Cy5 | 10μM, 3.16μM, 1μM, 316nM, 100nM, 31.6nM, 10nM, 3.16nM, 1nM, 0 (10%PBS) |
| DMSO | 1%, 0.5%, 0.25%, 0.125%, 0.06%, 0.03%, 0 |

7) placing the cell culture plate in the incubator and continuously incubating for 96 hours;
8) placing the CellTiter 96® AQueous One Solution reagent at the room temperature and thawing for 90 minutes or thawing in a water bath at 37 DEG C, and placing at the room temperature and equilibrating for 30 minutes;
9) adding 20 μL/well of the CellTiter 96® AQueous One Solution reagent to the cell culture plate;
10) placing the cell culture plate in the incubator at 37 DEG C and continuously incubating for 3 hours;
11) using a microplate reader to read a $OD_{490}$ value of each well in the cell plate; and
12) data processing and analysis.

GraphPad Prism 5.0 software is used to process data graphically. In order to calculate the IC50, "S"-shaped nonlinear regression analysis is performed on the data to match an appropriate dosage-effect curve. A calculation formula of the survival rate is as follows, the IC50 may be automatically calculated in GraphPad Prism 5.0.

$$\text{Cell survival rate (\%)} = (OD_{\text{test well}} - OD_{\text{blank control}}) / (OD_{\text{negative control}} - OD_{\text{blank control}}) \times 100\%.$$

III. Experiment result (see Table 50, Fig. 14a to 14d)

Table 50:

| Cell line | Test sample | IC50 (μM) |
|---|---|---|
| SKOV3 | Paclitaxel | <0.001 |
| | DNAh-Bio-EGFRapt-Cy5-paclitaxel | 16.05 |
| | DNAh-Bio-EGFRapt-Cy5 | 1.201 |
| | DMSO | >1% |

It may be seen from Table 50 and Fig. 14a, 14b, 14c, and 14d that for the SKOV3 cell line, compared with the simple DNAh targeted fluorescent carrier, the small molecular drug paclitaxel and DNAh drug-loaded particles DNAh-Bio-EGFRapt-Cy5-paclitaxel are both toxic to the SKOV3 cells. The IC50 of the paclitaxel and paclitaxel-Bio-EGFRapt-DNAh acting on the SKOV3 cells is <0.001 μM and 16.05 μM, respectively. The IC50 of the DNAh-Bio-EGFRapt-Cy5 and DMSO acting on the SKOV3 cells is > 1μM and >1%.

**Assembly of nucleic acid nanoparticles**

Embodiment 10

I. 7 groups of extension fragment deformation + core short-sequence RNA nanoparticle carriers:

(1) 7 groups of three polynucleotide base sequences for forming extension fragment deformation + core short-sequence RNA nanoparticles:

Table 51: R-15

| Name | Sequence sense (5'-3') | Base number | Chemical modification | Molecular weight | Total molecular weight |
|---|---|---|---|---|---|
| a (SEQ ID NO: 120) | GCGGCGAGCGGCGAGGAGC<br>GUUGGGGCCGGAGGCCGG | 37 | 3'-end is linked with a biotin (Bio); bases C and U, and 2'F modification | 12668.8 | 33713 |
| b (SEQ ID NO: 121) | CCGGCCUCCGGCC**CCUUCG**<br>**GGG**CCAGCCGCC | 31 | bases C and U, and 2'F modification | 9866.8 | |
| c (SEQ ID NO: 122) | GGCGGCAGG**CCCCCAUAGC**<br>**CC**UCGCCGCUCGCCGC | 35 | bases C and U, and 2'F modification | 11177.4 | |

Table 52: R-16:

| Name | Sequence sense (5'-3') | Base number | Chemical modification | Molecular weight | Total molecular weight |
|---|---|---|---|---|---|
| a (SEQ ID NO: 123) | GCGGCGAGCGGCGAGCAGC<br>GUUCGGGCCGGAGGCCGG | 37 | 3'-end is linked with a biotin (Bio); bases C and U, and 2'F modification | 12591.6 | 33709.8 |
| b (SEQ ID NO: 124) | CCGGCCUCCGGCCCGUUCG<br>CCGCCAGCCGCC | 31 | bases C and U, and 2'F modification | 9827.6 | |
| c (SEQ ID NO: 125) | GGCGGCAGGCGGCCAUAGC<br>GCUCGCCGCUCGCCGC | 35 | bases C and U, and 2'F modification | 11290.6 | |

Table 53: R-17:

| Name | Sequence sense (5'-3') | Base number | Chemical modification | Molecular weight | Total molecular weight |
|---|---|---|---|---|---|
| a (SEQ ID NO: 126) | GCGGCGAGCGGCGA**GGAGCGUUGG**GGCCGGAGGCCGG | 37 | 3'-end is linked with a biotin (Bio); bases C and U, and 2'F modification | 12668.9 | 33713 |
| b (SEQ ID NO: 127) | CCGGCCUCCGGCC**CCUUCGCCG**CCAGCCGCC | 31 | bases C and U, and 2'F modification | 9790.6 | |
| c (SEQ ID NO: 128) | GGCGGCAGG**CGGCCAUAGCCC**UCGCCGCUCGCCGC | 35 | bases C and U, and 2'F modification | 11253.5 | |

Table 54: R-18:

| Name | Sequence sense (5'-3') | Base number | Chemical modification | Molecular weight | Total molecular weight |
|---|---|---|---|---|---|
| a (SEQ ID NO: 129) | GCGGCGAGCGGCGAGCAGCGUUCGGGCCGGAGGCCGG | 37 | 3'-end is linked with a biotin (Bio); bases C and U, and 2'F modification | 12591.6 | 33709.8 |
| b (SEQ ID NO: 130) | CCGGCCUCCGGCCCGUUCGGCGCCAGCCGCC | 31 | bases C and U, and 2'F modification | 9865.7 | |
| c (SEQ ID NO: 131) | GGCGGCAGGCGCCCAUAGCGCUCGCCGCUCGCCGC | 35 | bases C and U, and 2'F modification | 11252.5 | |

Table 55: R-19:

| Name | Sequence sense (5'-3') | Base number | Chemical modification | Molecular weight | Total molecular weight |
|---|---|---|---|---|---|
| a (SEQ ID NO: 132) | GCGGCGAGCGGCGAGCAGCGUUCGGGCCGGAGGCCGG | 37 | 3'-end is linked with a biotin (Bio); bases C and U, and 2'F modification | 12591.6 | 33709.8 |

(continued)

| Name | Sequence sense (5'-3') | Base number | Chemical modification | Molecular weight | Total molecular weight |
|---|---|---|---|---|---|
| b (SEQ ID NO: 133) | CCGGCCUCCGGCCCGUUCG GCCCCAGCCGCC | 31 | bases C and U, and 2'F modification | 9827.6 | |
| c (SEQ ID NO: 134) | GGCGGCAGGGGCCCAUAGC GCUCGCCGCUCGCCGC | 35 | bases C and U, and 2'F modification | 11290.6 | |

Table 56: R-20:

| Name | Sequence sense (5'-3') | Base number | Chemical modification | Molecular weight | Total molecular weight |
|---|---|---|---|---|---|
| a (SEQ ID NO: 135) | GCGGCGAGCGGCGACGAGC GUUGCGGCCGGAGGCCGG | 37 | 3'-end is linked with a biotin (Bio); bases C and U, and 2'F modification | 12591.6 | 33709.8 |
| b (SEQ ID NO: 136) | CCGGCCUCCGGCCGCUUCG CCGCCAGCCGCC | 31 | bases C and U, and 2'F modification | 9827.6 | |
| c (SEQ ID NO: 137) | GGCGGCAGGCGGCCAUAGC CGUCGCCGCUCGCCGC | 35 | bases C and U, and 2'F modification | 11290.6 | |

Table 57: R-21:

| Name | Sequence sense (5'-3') | Base number | Chemical modification | Molecular weight | Total molecular weight |
|---|---|---|---|---|---|
| a (SEQ ID NO: 138) | GCGGCGAGCGGCGACGAGC GUUGCGGCCGGAGGCCGG | 37 | 3'-end is linked with a biotin (Bio); bases C and U, and 2'F modification | 11290.6 | 32408.8 |
| b (SEQ ID NO: 139) | CCGGCCUCCGGCCGCUUCG GCGCCAGCCGCC | 31 | bases C and U, and 2'F modification | 9865.7 | |
| c (SEQ ID NO: 140) | GGCGGCAGGCGCCCAUAGC CGUCGCCGCUCGCCGC | 35 | bases C and U, and 2'F modification | 11252.5 | |

II. Self-assembly experiment steps:

(1) according to a molar ratio of 1:1:1, enabling the RNA single strands a, b and c to be simultaneously mixed and dissolved in DEPC treated water or TMS buffer solution;

(2) heating mixed solution to 80 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100V of electrophoresis;

(4) cutting a target band and eluting in RNA elution buffer solution at 37 DEG C, after that, precipitating overnight in ethanol, and volatilizing under reduced pressure and low temperature; and

(5) electrophoresis analysis detection and laser scanning observation.

III. Self-assembly experiment result

(1) Electrophoresis detection

Main reagents and instruments are as follows:

Table 58:

| Reagent name | Article number | Manufacturer |
|---|---|---|
| 6×DNA Loading buffer | TSJ010 | Qingke Biotechnology Co.,Ltd |
| 20bp DNA Ladder | 3420A | TAKARA |
| 10000*SolarGelRed nucleic acid dye | E1020 | solarbio |
| 8% non-denaturing PAGE gel | / | Self-made |
| 1× TBE Buffer (RNA enzyme-free) | / | Self-made |

Table 59:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |
| Gel imager | Tanon 3500 | Tanon |

Steps:

① The RNA nanoparticles are diluted with ultrapure water by using a method shown in Table 60 below.

Table 60:

| Serial number | Measured concentration (μg/mL) |
|---|---|
| R-15 | 165.937 |
| R-16 | 131.706 |
| R-17 | 144.649 |

(continued)

| Serial number | Measured concentration (μg/mL) |
|---|---|
| R-18 | 164.743 |
| R-19 | 126.377 |
| R-20 | 172.686 |
| R-21 | 169.455 |

②10μL (500ng) of the processed sample is taken and uniformly mixed with 2μL of 6×DNA Loading Buffer, it is operated on ice, and a label is made.
③A 8% non-denaturing PAGE gel is taken, a piece of the gel is applied to samples with different incubation times, 12 μL of the processed samples are all loaded, and a program is set to run the gel at 100V for 40 minutes.
④ After running the gel, dyeing is performed, it is placed on a horizontal shaker for 30 minutes, and pictures are taken for imaging.

Detection result:
Non-denaturing PAGE gel running results of the 7 groups of extension fragment deformation + core short-sequence RNA self-assembly products are shown in Fig. 15. Lanes 1 to 7 in Fig. 15 from left to right are successively: the 7 groups of extension fragment deformation + core short-sequence RNA self-assembly products R-15, R-16, R-17, R-18, R-19, R-20 and R-21.

It may be clearly seen from the results in Fig. 15 that the bands of the 7 groups of extension fragment deformation + core short-sequence RNA self-assembly products are bright and clear, it is indicated that the 7 groups of extension fragment deformation + core short-sequence RNA strands are all self-assembled completely, to form a stable nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;

opening software, clicking a menu MeasUre€ManUal, so a manual measurement parameter setting dialog box appears;

setting a software detection parameter;

and then clicking Ok to complete the settings, after a measurement dialog box appears, clicking Start to start.

Measurement result: potential detection results of the 7 groups of the extension fragment deformation + core short-sequence RNA nanoparticles at 25 DEG C are as follows:

Table 61:

| Sample name | Detection times | Potential ZP (mV) |
|---|---|---|
| R-15 | 1 | -22.50 |
| | 2 | -23.90 |
| | 3 | -23.30 |
| Experim ent result | -23.23 mV | |

Table 62:

| Sample name | Detection times | Potential ZP (mV) |
|---|---|---|
| R-16 | 1 | -21.10 |
| | 2 | -19.80 |

(continued)

| Sample name | Detection times | Potential ZP (mV) |
|---|---|---|
| | 3 | -21.90 |
| Experim ent result | -20.93 mV | |

Table 63:

| Sample name | Detection times | Potential ZP (mV) |
|---|---|---|
| R-17 | 1 | -24.90 |
| | 2 | -20.90 |
| | 3 | -24.70 |
| Experim ent result | -23.50 mV | |

Table 64:

| Sample name | Detection times | Potential ZP (mV) |
|---|---|---|
| R-18 | 1 | -20.80 |
| | 2 | -21.30 |
| | 3 | -21.70 |
| Experim ent result | -21.27 mV | |

Table 65:

| Sample name | Detection times | Potential ZP (mV) |
|---|---|---|
| R-19 | 1 | -16.80 |
| | 2 | -16.90 |
| | 3 | -21.20 |
| Experim ent result | -18.30 mV | |

Table 66:

| Sample name | Detection times | Potential ZP (mV) |
|---|---|---|
| R-20 | 1 | -16.00 |
| | 2 | -16.90 |
| | 3 | -21.20 |
| Experim ent result | -17.90 mV | |

Table 67:

| Sample name | Detection times | Potential ZP (mV) |
|---|---|---|
| R-21 | 1 | -15.20 |
| | 2 | -16.70 |
| | 3 | -16.40 |

(continued)

| Sample name | Detection times | Potential ZP (mV) |
|---|---|---|
| Experim ent result | -16.10 mV | |

It may be seen from the above potential detection data that: the 7 groups of extension fragment deformation + core short-sequence RNA nanoparticles all have the good stability, and it is further indicated that the nanoparticles formed by the self-assembly of each extension fragment deformation + core short-sequence RNA have a relative stable self-assembly structure.

(3) Particle size measurement

1. Preparing a potential sample (7 groups of extension fragment deformation + core short-sequence RNA) and putting into a sample pool, opening a sample pool cover of an instrument, and placing the instrument;
2. opening software, and clicking a menu, so a manual measurement parameter setting dialog box appears;
3. setting a software detection parameter; and then
4. clicking Ok to complete the settings, when a measurement dialog box appears, clicking Start to start, a DLS measurement value result of a hydrodynamic size of the 7 groups of extension fragment deformation + core short-sequence RNA is as follows:

Table 68:

| Serial number | Mean particle size (nm) |
|---|---|
| R-15 | 6.808 |
| R-16 | 6.978 |
| R-17 | 7.592 |
| R-18 | 7.520 |
| R-19 | 6.936 |
| R-20 | 7.110 |
| R-21 | 6.720 |

(4) TM value detection

A solubility curve method is used to detect TM values of the 7 groups of the extension fragment deformation + core short-sequence RNA nanoparticles, and the samples are consistent with the potential samples.

Reagents and instruments are as follows:

Table 69:

| Reagent name | Article number | Manufacturer |
|---|---|---|
| AE buffer | / | Takara |
| SYBR Green I Dye | / | Self-made |

Table 70:

| Name | Model | Manufacturer |
|---|---|---|
| Real-Time System | CFX Connect | Bio-rad |
| Clean bench | HDL | Beijing Donglian Haer Instrument Manufacturing Co., Ltd. |

Steps:

①After the sample is diluted with ultrapure water, 5μg of the diluted sample is mixed with 2μL of SYBR Green I dye (diluted by 1:200), a final volume is 20μL, and the dilution concentration is as follows:

Table 71:

| Serial number | Measured concentration (μg/mL) |
|---|---|
| R-15 | 773.009 |
| R-16 | 782.098 |
| R-17 | 740.607 |
| R-18 | 806.163 |
| R-19 | 829.996 |
| R-20 | 723.082 |
| R-21 | 721.674 |

②It is incubated for 30 min in the dark at a room temperature; and
③On-machine detection is performed, a program is set to start at 20 DEG C, the temperature rises per second from 0.1 DEG C to 95 DEGC, and reading is performed every 5 s.

Detection result:
The TM values of the 7 groups of extension fragment deformation + core short-sequence RNA nanoparticles are as follows, a solubility curve diagram of the R-15 is shown in Fig. 16, a solubility curve diagram of the R-16 is shown in Fig. 17, a solubility curve diagram of the R-17 is shown in Fig. 18, a solubility curve diagram of the R-18 is shown in Fig. 19, a solubility curve diagram of the R-19 is shown in Fig. 20, a solubility curve diagram of the R-20 is shown in Fig. 21, and a solubility curve diagram of the R-21 is shown in Fig. 22. Due to the particularity of the RNA samples, a temperature corresponding to 1/2 RFUmax in a range of 20 to 90 DEG C is used as a sample Tm value in this test.

Table 72:

| | TM value (°C) |
|---|---|
| R-15 | 57.5 °C |
| R-16 | 53.8 °C |
| R-17 | 55.2 °C |
| R-18 | 54.5 °C |
| R-19 | 54.0 °C |
| R-20 | 59.5 °C |
| R-21 | 65.0 °C |

The TM values of the 7 groups of extension fragment deformation + core short-sequence RNA nanoparticles are all high, it is indicated that the self-assembly products have the good structural stability.

Embodiment 11

I. 7 groups of extension fragment deformation + core short-sequence DNA nanoparticle carriers:

(1) 7 groups of three polynucleotide base sequences for forming extension fragment deformation + core short-sequence DNA nanoparticles:

Table 73: D-8:

| Name | Sequence sense (5'-3') | Base number | Chemical modification | Molecular weight | Total molecular weight |
|---|---|---|---|---|---|
| a (SEQ ID NO: 141) | GCGGCGAGCGGCGA**GGAGC GTTGG**GGCCGGAGGCCGG | 37 | 3'-end is linked with a biotin (Bio) | 12087.3 | 32081.8 |
| b (SEQ ID NO: 142) | CCGGCCTCCGGCC**CCTTCGG GG**CCAGCCGCC | 31 | | 9375.1 | |
| c (SEQ ID NO: 143) | GGCGGCAGG**CCCCCATAGC CC**TCGCCGCTCGCCGC | 35 | | 10619.4 | |

Table 74: D-9:

| Name | Sequence sense (5'-3') | Base number | Chemical modification | Molecular weight | Total molecular weight |
|---|---|---|---|---|---|
| a (SEQ ID NO: 144) | GCGGCGAGCGGCGAGCAGC GTTCGGGCCGGAGGCCGG | 37 | 3'-end is linked with a biotin (Bio) | 12000.4 | 32071.6 |
| b (SEQ ID NO: 145) | CCGGCCTCCGGCCCGTTCGC CGCCAGCCGCC | 31 | | 9333.2 | |
| c (SEQ ID NO: 146) | GGCGGCAGGCGGCCATAGC GCTCGCCGCTCGCCGC | 35 | | 10738 | |

Table 75: D-10:

| Name | Sequence sense (5'-3') | Base number | Chemical modification | Molecular weight | Total molecular weight |
|---|---|---|---|---|---|
| a (SEQ ID NO: 147) | GCGGCGAGCGGCGA**GGAGC GTTGG**GGCCGGAGGCCGG | 37 | 3'-end is linked with a biotin (Bio) | 12087.7 | 32081.6 |
| b (SEQ ID NO: 148) | CCGGCCTCCGGCC**CCTTCGC CG**CCAGCCGCC | 31 | | 9294.3 | |
| c (SEQ ID NO: 149) | GGCGGCAGG**CGGCCATAGC CC**TCGCCGCTCGCCGC | 35 | | 10699.6 | |

Table 76: D-11:

| Name | Sequence sense (5'-3') | Base number | Chemical modification | Molecular weight | Total molecular weight |
|---|---|---|---|---|---|
| a (SEQ ID NO: 150) | GCGGCGAGCGGCGAGCAGC GTTCGGGCCGGAGGCCGG | 37 | 3'-end is linked with a biotin (Bio) | 12000.4 | 32071.6 |
| b (SEQ ID NO: 151) | CCGGCCTCCGGCCCGTTCG GCGCCAGCCGCC | 31 | | 9333.2 | |
| c (SEQ ID NO: 152) | GGCGGCAGGCGCCCATAGC GCTCGCCGCTCGCCGC | 35 | | 10738 | |

Table 77: D-12:

| Name | Sequence sense (5'-3') | Base number | Chemical modification | Molecular weight | Total molecular weight |
|---|---|---|---|---|---|
| a (SEQ ID NO: 153) | GCGGCGAGCGGCGAGCAGC GTTCGGGCCGGAGGCCGG | 37 | 3'-end is linked with a biotin (Bio) | 12000.4 | 32071.6 |
| b (SEQ ID NO: 154) | CCGGCCTCCGGCCCGTTCG GCCCCAGCCGCC | 31 | | 9333.2 | |
| c (SEQ ID NO: 155) | GGCGGCAGGGGCCCATAGC GCTCGCCGCTCGCCGC | 35 | | 10738 | |

Table 78: D-13:

| Name | Sequence sense (5'-3') | Base number | Chemical modification | Molecular weight | Total molecular weight |
|---|---|---|---|---|---|
| a (SEQ ID NO: 156) | GCGGCGAGCGGCGACGAGC GTTGCGGCCGGAGGCCGG | 37 | 3'-end is linked with a biotin (Bio) | 12000.4 | 32071.6 |
| b (SEQ ID NO: 157) | CCGGCCTCCGGCCGCTTCGC CGCCAGCCGCC | 31 | | 9333.2 | |
| c (SEQ ID NO: 158) | GGCGGCAGGCGGCCATAGC CGTCGCCGCTCGCCGC | 35 | | 10738 | |

Table 79: D-14:

| Name | Sequence sense (5'-3') | Base number | Chemical modification | Molecular weight | Total molecular weight |
|---|---|---|---|---|---|
| a (SEQ ID NO: 159) | GCGGCGAGCGGCGACGAGC GTTGCGGCCGGAGGCCGG | 37 | 3'-end is linked with a biotin (Bio) | 12000.4 | 32071.6 |
| b (SEQ ID NO: 160) | CCGGCCTCCGGCCGCTTCG GCGCCAGCCGCC | 31 | | 9373.2 | |
| c (SEQ ID NO: 161) | GGCGGCAGGCGCCCATAGC CGTCGCCGCTCGCCGC | 35 | | 10698 | |

II. Self-assembly experiment steps:

(1) according to a molar ratio of 1:1:1, enabling the DNA single strands a, b and c to be simultaneously mixed and dissolved in DEPC treated water or TMS buffer solution;

(2) heating mixed solution to 95 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in DNA elution buffer solution at 37 DEG C, after that, precipitating overnight in ethanol, and volatilizing under reduced pressure and low temperature, to obtain the DNA self-assembly products;

(5) electrophoresis analysis detection and laser scanning observation;

(6) electric potential detection;

(7) particle size detection; and

(8) TM value detection.

III. Self-assembly experiment result

(1) Electrophoresis detection

Main reagents and instruments are as follows:

Table 80:

| Reagent name | Article number | Manufacturer |
|---|---|---|
| 6×DNA Loading buffer | TSJ010 | Qingke Biotechnology Co.,Ltd |
| 20bp DNA Ladder | 3420A | TAKARA |
| 10000*SolarGelRed nucleic acid dye | E1020 | solarbio |
| 8% non-denaturing PAGE gel | / | Self-made |
| 1× TBE Buffer (RNA enzyme-free) | / | Self-made |

Table 81:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |
| Gel imager | Tanon 3500 | Tanon |

Steps:

① The DNA nanoparticles are diluted with ultrapure water by using a method shown in Table 82 below.

Table 82:

| | Measured concentration (μg/mL) |
|---|---|
| D-8 | 2890.932 |
| D-9 | 2238.682 |
| D-10 | 2075.084 |
| D-11 | 3117.389 |
| D-12 | 2880.939 |
| D-13 | 2704.757 |
| D-14 | 3216.917 |

②10μL (500ng) of the processed sample is taken and uniformly mixed with 2μL of 6×DNA Loading Buffer, it is operated on ice, and a label is made.
③A 8% non-denaturing PAGE gel is taken, a piece of the gel is applied to samples with different incubation times, 12 μL of the processed samples are all loaded, and a program is set to run the gel at 100V for 40 minutes.
④ After running the gel, dyeing is performed, it is placed on a horizontal shaker for 30 minutes, and pictures are taken for imaging.
Detection result:
Non-denaturing PAGE gel running results of the 7 groups of extension fragment deformation + core short-sequence DNA self-assembly products are shown in Fig. 23. Lanes 1 to 7 in Fig. 23 from left to right are successively: the 7 groups of extension fragment deformation + core short-sequence DNA self-assembly products D-8, D-9, D-10, D-11, D-12, D-13 and D-14.
It may be clearly seen from the results in Fig. 38 that the bands of the 7 groups of extension fragment deformation + core short-sequence DNA self-assembly products are bright and clear, it is indicated that the 7 groups of extension fragment deformation + core short-sequence DNA strands are all self-assembled completely, to form a stable nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;

opening software, clicking a menu MeasUre€ManUal, so a manual measurement parameter setting dialog box appears;

setting a software detection parameter;

and then clicking Ok to complete the settings, after a measurement dialog box appears, clicking Start to start.

Measurement result: potential detection results of the 7 groups of the extension fragment deformation + core short-sequence DNA nanoparticles at 25 DEG C are as follows:

Table 83:

| Sample name | Detection times | Potential ZP (mV) |
|---|---|---|
| D-8 | 1 | -29.20 |
| | 2 | -32.90 |
| | 3 | -28.60 |
| Experim ent result | -30.23 mV | |

Table 84:

| Sample name | Detection times | Potential ZP (mV) |
|---|---|---|
| D-9 | 1 | -39.20 |
| | 2 | -34.20 |
| | 3 | -31.80 |
| Experim ent result | -35.07 mV | |

Table 85:

| Sample name | Detection times | Potential ZP (mV) |
|---|---|---|
| D-10 | 1 | -27.90 |
| | 2 | -28.30 |
| | 3 | -21.10 |
| Experim ent result | -25.77 mV | |

Table 86:

| Sample name | Detection times | Potential ZP (mV) |
|---|---|---|
| D-11 | 1 | -29.70 |
| | 2 | -24.80 |
| | 3 | -27.80 |
| Experim ent result | -27.43 mV | |

Table 87:

| Sample name | Detection times | Potential ZP (mV) |
|---|---|---|
| D-12 | 1 | -33.50 |
| | 2 | -29.80 |
| | 3 | -34.20 |
| Experim ent result | -32.50 mV | |

Table 88:

| Sample name | Detection times | Potential ZP (mV) |
|---|---|---|
| D-13 | 1 | -26.80 |
| | 2 | -27.80 |
| | 3 | -26.40 |
| Experim ent result | -27.00 mV | |

Table 89:

| Sample name | Detection times | Potential ZP (mV) |
|---|---|---|
| D-14 | 1 | -31.70 |
| | 2 | -32.10 |
| | 3 | -22.40 |
| Experim ent result | -28.73 mV | |

It may be seen from the above potential detection data that: the 7 groups of extension fragment deformation + core short-sequence DNA nanoparticles all have the good stability, and it is further indicated that the nanoparticles formed by the self-assembly of each extension fragment deformation + core short-sequence DNA have a relative stable self-assembly structure.

(3) Particle size measurement

①Preparing a potential sample (7 groups of extension fragment deformation + core short-sequence DNA) and putting into a sample pool, opening a sample pool cover of an instrument, and placing the instrument;

②opening software, and clicking a menu, so a manual measurement parameter setting dialog box appears;

③setting a software detection parameter; and then

④clicking Ok to complete the settings, when a measurement dialog box appears, clicking Start to start, a DLS measurement value result of a hydrodynamic size of the 7 groups of extension fragment deformation + core short-sequence DNA is as follows:

Table 90:

| Serial number | Mean particle size (nm) |
|---|---|
| D-8 | 7.460 |
| D-9 | 7.920 |
| D-10 | 7.220 |
| D-11 | 7.472 |
| D-12 | 6.968 |
| D-13 | 7.012 |
| D-14 | 6.896 |

(4) TM value detection

A solubility curve method is used to detect TM values of the 7 groups of the extension fragment deformation + core short-sequence DNA nanoparticles, and the samples are consistent with the potential samples.

Reagents and instruments are as follows:

Table 91:

| Reagent name | Article number | Manufacturer |
|---|---|---|
| AE buffer | / | Takara |
| SYBR Green I Dye | / | Self-made |

Table 92:

| Name | Model | Manufacturer |
|---|---|---|
| Real-Time System | CFX Connect | Bio-rad |
| Clean bench | HDL | Beijing Donglian Haer Instrument Manufacturing Co., Ltd. |

Steps:

①After the sample is diluted with ultrapure water, 5μg of the diluted sample is mixed with 2μL of SYBR Green I dye (diluted by 1:200), a final volume is 20μL, and the dilution concentration is as follows:

Table 93:

| Serial number | Measured concentration (μg/mL) |
|---|---|
| D-8 | 2890.932 |
| D-9 | 2238.682 |
| D-10 | 2075.084 |
| D-11 | 3117.389 |
| D-12 | 2880.939 |
| D-13 | 2704.757 |
| D-14 | 3216.917 |

②It is incubated for 30 min in the dark at a room temperature; and

③On-machine detection is performed, a program is set to start at 20 DEG C, the temperature rises per second from 0.1 DEG C to 95 DEGC, and reading is performed every 5 s.

Detection result:

The TM values of the 7 groups of extension fragment deformation + core short-sequence DNA nanoparticles are as follows, a solubility curve diagram of the D-8 is shown in Fig. 24, a solubility curve diagram of the D-9 is shown in Fig. 25, a solubility curve diagram of the D-10 is shown in Fig. 26, a solubility curve diagram of the D-11 is shown in Fig. 27, a solubility curve diagram of the D-12 is shown in Fig. 28, a solubility curve diagram of the D-13 is shown in Fig. 29, and a solubility curve diagram of the D-14 is shown in Fig. 30.

Table 94:

| Serial number | TM value (°C) |
|---|---|
| D-8 | 48.5 |
| D-9 | 52.5 |
| D-10 | 54.5-55.0 |

(continued)

| Serial number | TM value (°C) |
|---|---|
| D-11 | 48.7 |
| D-12 | 51.5 |
| D-13 | 51.0 |
| D-14 | 49.2 |

It may be seen from Figs. 26 to 30 that the TM values of the 7 groups of extension fragment deformation + core short-sequence DNA nanoparticles are all high, it is indicated that the sample is higher in purity and stable in self-assembly structure.

**Stability detection of nucleic acid nanoparticles in serum**

Embodiment 12

A non-denaturing PAGE method is used to characterize the stability of 7 groups of extension fragment deformation + core short-sequence RNA nanoparticles in serum.
Main reagents and instruments are as follows:

Table 95:

| Reagent name | Article number | Manufacturer |
|---|---|---|
| 6×DNA Loading buffer | TSJ010 | Qingke Biotechnology Co.,Ltd |
| 20bp DNA Ladder | 3420A | TAKARA |
| 10000*SolarGelRed nucleic acid dye | E1020 | solarbio |
| 8% non-denaturing PAGE gel | / | Self-made |
| 1× TBE Buffer (RNA enzyme-free) | / | Self-made |
| Serum (FBS) | / | Excel |
| RPMI 1640 | / | GBICO |

Table 96:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |
| Gel imager | GenoSens1880 | Shanghai Qinxiang Scientific Instrument Co., Ltd. |

Steps:

① The RNA nanoparticles are prepared to the concentration in the table below, and then the prepared sample is diluted according to a method in the table below. It is diluted by 5 tubes. After being diluted, the sample is placed in a water bath at 37 DEG C for different times (0, 10 min, 1 h, 12 h, and 36 h);

Table 97:

| Sample name | Sample concentration (μg/mL) | Sample volume | 50%FBS-1640 / μL | 1640/ μL | Diluted concentration (μg/mL) |
|---|---|---|---|---|---|
| R-15 | 773.009 | 1.3 | 4 | 14.7 | 50 μg/mL |
| R-16 | 782.098 | 1.3 | 4 | 14.7 | 50 μg/mL |
| R-17 | 740.607 | 1.4 | 4 | 14.6 | 50 μg/mL |
| R-18 | 806.163 | 1.2 | 4 | 14.8 | 50 μg/mL |
| R-19 | 829.996 | 1.2 | 4 | 14.8 | 50 μg/mL |
| R-20 | 723.082 | 1.4 | 4 | 14.6 | 50 μg/mL |
| R-21 | 721.674 | 1.4 | 4 | 14.6 | 50 μg/mL |

②10μL of the processed sample is taken and uniformly mixed with 2μL of 6×DNA Loading Buffer, it is operated on ice, and a label is made;

③a 8% non-denaturing PAGE gel is taken, a piece of the gel is applied on the samples with different incubation times, 12 μL of the processed samples are all loaded, and a program is set to run the gel at 100V for 40 min; and

④ after running the gel, dyeing is performed, it is placed on a horizontal shaker and shaken slowly for 30 min, pictures are taken for imaging.

An electrophoresis detection result of the R-15 is shown in Fig. 31, an electrophoresis detection result of the R-16 is shown in Fig. 32, an electrophoresis detection result of the R-17 is shown in Fig. 33, an electrophoresis detection result of the R-18 is shown in Fig. 34, an electrophoresis detection result of the R-19 is shown in Fig. 35, an electrophoresis detection result of the R-20 is shown in Fig. 36, and an electrophoresis detection result of the R-21 is shown in Fig. 37. In Fig. 31 to 37, lanes from left to right are respectively M: marker; 1: 36 h; 2: 12 h; 3: 1 h; 4: 10 min; 5: 0 min. It may be seen from the results of the serum stability test that: the results of the non-denaturing gel at 10 min, 1 h, 12 h and 36 h show that there is no significant difference between bands of RNA nanoparticle samples at different times, it is indicated that RNA nanoparticles R-15 to R-21 are relatively stable in a 1640 medium of 50% FBS without apparent degradation.

Embodiment 13

A non-denaturing PAGE method is used to characterize the stability of 7 groups of extension fragment deformation + core short-sequence DNA nanoparticles in serum.

Main reagents and instruments are as follows:

Table 98:

| Reagent name | Article number | Manufacturer |
|---|---|---|
| 6×DNA Loading buffer | TSJ010 | Qingke Biotechnology Co.,Ltd |
| 20bp DNA Ladder | 3420A | TAKARA |
| 10000*SolarGelRed nucleic acid dye | E1020 | solarbio |
| 8% non-denaturing PAGE gel | / | Self-made |
| 1× TBE Buffer (RNA enzyme-free) | / | Self-made |
| Serum (FBS) | / | Excel |
| RPMI 1640 | / | GBICO |

Table 99:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |

(continued)

| Name | Model | Manufacturer |
|---|---|---|
| Discoloration shaker | TS-3D | orbital shaker |
| Gel imager | GenoSens1880 | Shanghai Qinxiang Scientific Instrument Co., Ltd. |

Steps:

① The DNA nanoparticles are prepared to the concentration in the table below, and then the prepared sample is diluted according to a method in the table below. It is diluted by 5 tubes. After being diluted, the sample is placed in a water bath at 37 DEG C for different times (0, 10 min, 1 h, 12 h, and 36 h);

Table 100:

| Sample name | Sample concentration (μg/mL) | Sample volume | 50%FBS-1640 / μL | 1640/ μL | Diluted concentration (μg/mL) |
|---|---|---|---|---|---|
| D-8 | 2890.932 | 1.4 | 8 | 30.6 | 100 |
| D-9 | 2238.682 | 1.8 | 8 | 30.2 | 100 |
| D-10 | 2075.084 | 1.9 | 8 | 30.1 | 100 |
| D-11 | 3117.389 | 1.3 | 8 | 30.7 | 100 |
| D-12 | 2880.939 | 1.4 | 8 | 30.6 | 100 |
| D-13 | 2704.757 | 1.5 | 8 | 30.5 | 100 |
| D-14 | 3216.917 | 1.2 | 8 | 30.8 | 100 |

②10μL of the processed sample is taken and uniformly mixed with 2μL of 6×DNA Loading Buffer, it is operated on ice, and a label is made;
③a 8% non-denaturing PAGE gel is taken, a piece of the gel is applied on the samples with different incubation times, 6 μL of the processed samples are all loaded, and a program is set to run the gel at 100V for 40 min; and
④ after running the gel, dyeing is performed, it is placed on a horizontal shaker and shaken slowly for 30 min, pictures are taken for imaging.

An electrophoresis detection result of the D-8 is shown in Fig. 38, an electrophoresis detection result of the D-9 is shown in Fig. 39, an electrophoresis detection result of the D-10 is shown in Fig. 40, an electrophoresis detection result of the D-11 is shown in Fig. 41, an electrophoresis detection result of the D-12 is shown in Fig. 42, an electrophoresis detection result of the D-13 is shown in Fig. 43, and an electrophoresis detection result of the D-14 is shown in Fig. 44. In Fig. 38 to 44, lanes from left to right are respectively M: marker; 1: 36 h; 2: 12 h; 3: 1 h; 4: 10 min; 5: 0 min. It may be seen from the results of the serum stability test that: the results of the non-denaturing gel at 10 min, 1 h, 12 h and 36 h show that there is no significant difference between bands of DNA nanoparticle samples at different times, it is indicated that DNA nanoparticles D-8 to D-14 are relatively stable in a 1640 medium of 50% FBS without apparent degradation.

## Drug loading test of nucleic acid nanoparticle

Embodiment 14

Doxorubicin loading experiment:
According to the chemical loading method (unless otherwise specified, the method is the same as that of Embodiment 5) of Embodiment 5, the RNA nanoparticles formed by the self-assembly of the previous R-15, R-16, R-17, R-18, R-19, R-20 and R-21 in Embodiment 10, and the DNA nanoparticles formed by the self-assembly of the D-8, D-9, D-10, D-11, D-12, D-13 and D-14 in Embodiment 11 are respectively used as doxorubicin loading carriers, doxorubicin loading rates measured are respectively as follows:

The doxorubicin loading rate of RNA nanoparticles R-15 is 20.5.

The doxorubicin loading rate of RNA nanoparticles R-16 is 29.4.

The doxorubicin loading rate of RNA nanoparticles R-17 is 30.9.

The doxorubicin loading rate of RNA nanoparticles R-18 is 34.1.

The doxorubicin loading rate of RNA nanoparticles R-19 is 27.1.

The doxorubicin loading rate of RNA nanoparticles R-20 is 30.2.

The doxorubicin loading rate of RNA nanoparticles R-21 is 20.1.

The doxorubicin loading rate of DNA nanoparticles D-8 is 28.0.

The doxorubicin loading rate of DNA nanoparticles D-9 is 27.9.

The doxorubicin loading rate of DNA nanoparticles D-10 is 18.9.

The doxorubicin loading rate of DNA nanoparticles D-11 is 26.8.

The doxorubicin loading rate of DNA nanoparticles D-12 is 27.6.

The doxorubicin loading rate of DNA nanoparticles D-13 is 31.8.

The doxorubicin loading rate of DNA nanoparticles D-14 is 32.

**Cell binding ability of DNA nanoparticles and carrier drugs detected by Fluorescence Activated Cell Sorter (FACS) Experiment**

Embodiment 15

I. Cell information

HepG2 (from Concord Cell Bank), a medium is DMEM+10% FBS+1% double antibody (gibco, 15140-122), and culture conditions are 37 DEG C, 5% $CO_2$ and saturated humidity.

II. Substances to be tested

Blank carrier: DNA nanoparticle carriers formed by the self-assembly of the previous D-8, D-9, D-10, D-11, D-12, D-13 and D-14 in the above Embodiment 11.

Carrier drug: according to the chemical loading method (unless otherwise specified, the method is the same as that of Embodiment 5) of Embodiment 5, the DNA nanoparticles formed by the self-assembly of the previous D-8, D-9, D-10, D-11, D-12, D-13 and D-14 in Embodiment 11 are used to load a doxorubicin, they are respectively marked as D-8-doxorubicin, D-9-doxorubicin, D-10-doxorubicin, D-11-doxorubicin, D-12-doxorubicin, D-13-doxorubicin and D-14-doxorubicin.

III. Main devices and consumables

Table 101:

| | **Manufacturer** | **Model** |
|---|---|---|
| Biosafety cabinet | Beijing Donglian Har Instrument Manufacturing Co., Ltd. | BSC-1360 II A2 |
| Low-speed centrifuge | Zhongke Zhongjia Instrument Co., Ltd. | SC-3612 |
| $CO_2$ Incubator | Thermo | 3111 |

(continued)

| | Manufacturer | Model |
|---|---|---|
| Inverted microscope | UOP | DSZ2000X |
| Fluorescence activated cell sorter | BD | BD FACSCalibur™ |

IV. Main reagent

Table 102:

| Reagent name | Manufacturer | Article number | Remark |
|---|---|---|---|
| DMEM (biotin-free) | Provided by Baiyao Zhida Nano-biotechnology Co., Ltd. | YS3160 | |
| 1%BSA-PBS | Self-made | — | |

V. Experiment method

1. A cell state is adjusted to a logarithmic growth phase, the medium is changed into a medium without biotin and folic acid, and it is incubated overnight in an incubator at 37 DEG C.

2. After incubation, cell suspension is trypsinized and collected, and centrifuged at 1000 rpm for 5 min, after the concentration is adjusted, a $2\times10^5$-$5\times10^5$ cell/EP tube is taken and washed twice with 1 mL/tube of 1% BSA-PBS, and cells at the bottom of the tube is observed to prevent them from being absorbed.

3. A substance to be tested is dissolved, and the substance to be tested is diluted to a use concentration.

4. Cell supernatant is absorbed, 100μL of a corresponding sample is added to each tube in order, light is avoided, and it is incubated at 37 DEG C for 2 h.

5. It is washed twice with 1% BSA-PBS; and centrifuged at 1000 rpm for 5min.

6. Finally, it is precipitated with 300 μL of PBS cell resuspension, and flow cytometric on-machine detection (the blank carrier used in the present embodiment is labeled by Quasar670, and the doxorubicin in the carrier drug has its own fluorescence, so the detection may be performed through FL4-APC and FL2-PE respectively) is performed.

7. Data analysis.

VI. Experiment result

1. An experiment result is shown in the table below:

Table 103:

| Detected substance | | Experiment cell | Positive rate |
|---|---|---|---|
| PBS | | HepG2 | 2.11% |
| D-8-doxorubicin (carrier drug) | 1μM | HepG2 | 93.1% |
| | 2μM | HepG2 | 96.3% |
| D-8 (blank carrier) | 1μM | HepG2 | 96.9% |
| | 2μM | HepG2 | 98.4% |

(continued)

| Detected substance | | Experiment cell | Positive rate |
|---|---|---|---|
| D-9-doxorubicin (carrier drug) | 1μM | HepG2 | 88.6% |
| | 2μM | HepG2 | 95.4% |
| D-9 (blank carrier) | 1μM | HepG2 | 96.7% |
| | 2μM | HepG2 | 98.1% |
| D-10-doxorubicin (carrier drug) | 1μM | HepG2 | 89.4% |
| | 2μM | HepG2 | 95.5% |
| D-10 (blank carrier) | 1μM | HepG2 | 97.9% |
| | 2μM | HepG2 | 98.3% |
| D-11-doxorubicin (carrier drug) | 1μM | HepG2 | 89.3% |
| | 2μM | HepG2 | 95.5% |
| D-11 (blank carrier) | 1μM | HepG2 | 97.7% |
| | 2μM | HepG2 | 97.8% |
| D-12-doxorubicin (carrier drug) | 1μM | HepG2 | 94.6% |
| | 2μM | HepG2 | 95.9% |
| D-12 (blank carrier) | 1μM | HepG2 | 96.9% |
| | 2μM | HepG2 | 97.1% |
| D-13-doxorubicin (carrier drug) | 1μM | HepG2 | 89.6% |
| | 2μM | HepG2 | 94.0% |
| D-13 (blank carrier) | 1μM | HepG2 | 97.6% |
| | 2μM | HepG2 | 98.2% |
| D-14-doxorubicin (carrier drug) | 1μM | HepG2 | 90.3% |
| | 2μM | HepG2 | 96.1% |
| D-14 (blank carrier) | 1μM | HepG2 | 97.4% |
| | 2μM | HepG2 | 98.3% |

2. Conclusion

1. After the HepG2 cells are incubated with D-8-doxorubicin (carrier drug) and D-8 (blank carrier), the binding rates are very high (93.1%-98.4%).

2. After the HepG2 cells are incubated with D-9-doxorubicin (carrier drug) and D-9 (blank carrier), the binding rates are very high (88.6%-98.1%).

3. After the HepG2 cells are incubated with D-10-doxorubicin (carrier drug) and D-10 (blank carrier), the binding rates are very high (89.4%-98.3%).

4. After the HepG2 cells are incubated with D-11-doxorubicin (carrier drug) and D-11 (blank carrier), the binding rates are very high (89.3%-97.8%).

5. After the HepG2 cells are incubated with D-12-doxorubicin (carrier drug) and D-12 (blank carrier), the binding rates are very high (94.6%-97.1%).

6. After the HepG2 cells are incubated with D-13-doxorubicin (carrier drug) and D-13 (blank carrier), the binding rates are very high (89.6%-98.2%).

7. After the HepG2 cells are incubated with D-14-doxorubicin (carrier drug) and D-14 (blank carrier), the binding rates are very high (90.3%-98.3%).

**Cytotoxicity research of DNA nanoparticles and carrier drugs in HepG2 cells**

Embodiment 16

A CCK8 method is used to detect the toxicity of DNA naoparticles and carrier drugs to HepG2.

I. Main reagent

Table 104:

| Reagent name | Manufacturer | Article number |
|---|---|---|
| PBS | - | — |
| DMSO | SIGMA | D2650 |
| DMEM (biotin-free) | Provided by Baiyao Zhida Nano-biotechnology Co., Ltd. | YS3160 |
| FBS | Excell Bio | FSP500 |
| Double-antibody | gibco | 15140-122 |
| pancreatin | gibco | 25200-056 |
| CCK8 kit | Biyuntian Company | C0038 |

II. Main consumables and instruments

Table 105:

| **Name** | **Manufacturer** | **Model** |
|---|---|---|
| 96-well cell culture plate | NEST | 701001 |
| Biosafety cabinet | Beijing Donglian Har Instrument Manufacturing Co., Ltd. | BSC-1360 II A2 |
| Low-speed centrifuge | Zhongke Zhongjia Instrument Co., Ltd. | SC-3612 |
| $CO_2$ Incubator | Thermo | 3111 |
| Inverted microscope | UOP | DSZ2000X |
| Microplate reader | Shanghai Ouying Experimental Equipment Co., Ltd. | K3 |

III. Cell information

HepG2 (from Concord Cell Bank), a medium is DMEM+10% FBS+1% double antibody (gibco, 15140-122), and culture conditions are 37 DEG C, 5% $CO_2$ and saturated humidity.

IV. Experiment material

1. Samples to be tested

Blank carrier: DNA nanoparticle carriers formed by the self-assembly of the previous D-8, D-9, D-10, D-11, D-12, D-13 and D-14 in Embodiment 11, respectively marked as: D-8, D-9, D-10, D-11, D-12, D-13 and D-14.
Carrier drug: according to the chemical loading method (unless otherwise specified, the method is the same as that of Embodiment 5) of Embodiment 5, the DNA nanoparticles formed by the self-assembly of the previous D-8, D-9, D-10, D-11, D-12, D-13 and D-14 in Embodiment 11 are used to load a doxorubicin, they are respectively marked as D-8-doxorubicin, D-9-doxorubicin, D-10-doxorubicin, D-11-doxorubicin, D-12-doxorubicin, D-13-doxorubicin and D-14-doxorubicin.

Original drug doxorubicin.

DMSO.

V. Experiment procedure

1. HepG2 cells in a logarithmic growth phase are taken, trypan blue is used for staining and the cell viability is counted to be 98.3%, plating is performed with 5000 Cell/well, a volume is 100 μL/well, 8 96-well plates are paved with 57 wells per plate, and it is incubated overnight at 37 DEG C.

2. A sample to be tested is diluted according to the following table and added: the original culture medium is removed and 100 μL of a culture medium of the sample to be tested with the different concentration is added, and there are 3 replicate wells in each group.

Table 106:

| Well number | C9 | C8 | C7 | C6 | C5 | C4 | C3 | C2 | C1 |
|---|---|---|---|---|---|---|---|---|---|
| Final concentration of loaded drug | 10μM | 3.16μM | 1μM | 316nM | 100nM | 31.6nM | 10nM | 3.16nM | 1nM |
| Final concentration of blank carrier | 1μM | 316nM | 100nM | 31.6M | 10nM | 3.16nM | 1nM | 0.316n M | 0.1nM |
| Final concentration of original drug doxorubicin | 10μM | 3.16μM | 1μM | 316nM | 100nM | 31.6nM | 10nM | 3.16nM | 1nM |
| DMSO (%) | 0.1 | 0.0316 | 0.01 | 0.00316 | 0.001 | 0.00036 | 0.0001 | 0.000036 | 0.00001 |

In the present embodiment, the loaded drug and the blank carrier are firstly prepared into 100 μM of stock solution by using the PBS, and then diluted with the complete culture medium (biotin-free DMEM). The original drug doxorubicin is firstly prepared into 100 μM of stock solution by using the DMSO, and then diluted with the complete culture medium (biotin-free DMEM). The DMSO is directly diluted with the complete medium (biotin-free DMEM).
3. After the sample to be tested is added, the 96-well plate is placed in an incubator under 37 DEG C and 5% $CO_2$ and incubated for 72 hours.
4. A kit is taken out and melted at a room temperature, 10 μL of CCK-8 solution is added to each well, or the CCK8 solution is mixed with the culture medium at a ratio of 1:9, and then added to the well in an amount of 100 μL/well.
5. It is continuously incubated in the cell incubator for 4 hours, and a length of time depends on experimental conditions such as a cell type and a cell density.
6. A microplate reader is used to measure an absorbance at 450 nm.
7. Calculation: cell viability (%) = (OD experimental group-OD blank group) ×100%/ (OD control group-OD blank group), $IC_{50}$ is calculated by GraphPad Prism 5.0.

VI. Experiment result

Table 107:

| Cell line | Detected sample | $IC_{50}$ (μM) |
|---|---|---|
| HepG 2 | Original drug doxorubicin | 0.2725 |
| | D-8-doxorubicin (loaded drug) | 0.05087 |
| | D-8 (blank carrier) | >1 |
| | D-9-doxorubicin (loaded drug) | 0.0386 |
| HepG 2 | D-9 (blanking carrier) | >1 |
| | D-10-doxorubicin (loaded drug) | 0.03955 |
| | D-10 (blank carrier) | >1 |
| | D-11-doxorubicin (loaded drug) | 0.04271 |
| HepG 2 | D-11 (blank carrier) | >1 |
| | D-12-doxorubicin (loaded drug) | 0.02294 |
| | D-12 (blank carrier) | >1 |
| | D-13-doxorubicin (loaded drug) | 0.03017 |
| HepG 2 | D-13 (blank carrier) | >1 |
| | D-14-doxorubicin (loaded drug) | 0.03458 |
| | D-14 (blank carrier) | >1 |
| | DMSO | >0.1% |

Conclusion:
It may be seen from the above table and Fig. 45a, Fig. 45b, Fig. 45c, Fig. 45d, Fig. 45e, Fig. 45f. Fig. 45g and Fig. 45h that the $IC_{50}$ of the original drug doxorubicin and the loaded drugs D-8-doxorubicin, D-9-doxorubicin, D-10-doxorubicin, D-11-doxorubicin, D-12-doxorubicin, D-13-doxorubicin and D-14-doxorubicin acting on the HepG2 cells is 0.2725 μM, 0.05087 μM, 0.0386, 0.03955, 0.04271, 0.02294, 0.03017 and 0.03458 respectively; the $IC_{50}$ of the DMSO acting on the HepG2 cells is >0.1%; the $IC_{50}$ of the D-8 (blank carrier), D-9 (blank carrier), D-10 (blank carrier), D-11 (blank carrier), D-12 (blank carrier), D-13 (blank carrier) and D-14 (blank carrier) acting on the HepG2 cells is >1μM. It is indicated that for the HepG2 cell line, compared with the simple blank carriers D-8, D-9, D-10, D-11, D-12, D-13 and D-14, the original drug doxorubicin of small molecular drug and the loaded drugs D-8-doxorubicin, D-9-doxorubicin, D-10-doxorubicin, D-11-doxorubicin, D-12-doxorubicin, D-13-doxorubicin Both D-14-doxorubicin and D-14-doxorubicin are toxic to the HepG2 cells, and compared with the original drug doxorubicin, the loaded drugs D-8-doxorubicin, D-9-doxorubicin, D-10-doxorubicin, and D-11-doxorubicin, D-12-doxorubicin, D-13-doxorubicin and D-14-doxorubicin have an apparent synergistic effect.

Embodiment 17

According to the chemical loading method (unless otherwise specified, the method is the same as that of Embodiment 5) of Embodiment 5, the DNA nanoparticles formed by the self-assembly of the previous D-10 and D-14 in the above Embodiment 27 are used as a daunorubicin loading carrier. A microplate reader is used to measure an absorbance of the daunorubicin at 492 nm, and a standard curve (as shown in Fig.46) is drawn.
Measured loading rates of the daunorubicin are respectively as follows:

The daunorubicin loading rate of the DNA nanoparticles D-10 is 24.0.

The daunorubicin loading rate of the DNA nanoparticles D-14 is 25.1.

Embodiment 18

**Lenalidomide loading experiment**

(I) Chemical loading:

I. Experiment material and experiment method

1. Experiment materials and reagents:

(1) Nucleic acid nanoparticles: it is similar to the RNA nanoparticles in Embodiment 1, a difference is that a fluorescent label on the c-strand is Cy5.

(2) DEPC treated water: Biyuntian Company.

(3) PBS buffer solution: cellgro.

(4) 4% paraformaldehyde.

(5) Lenalidomide (Epirarunixon).

(6) Chloroform: Beijing Beihua Fine Chemicals Co., Ltd.

(7) Anhydrous ethanol: Beijing Beihua Fine Chemicals Co., Ltd.

2. Experiment method:

(1) Accurately weighing the lenalidomide (1.354μmol) and dissolving in the DEPC treated water (1.0 mL) and the PBS buffer solution (1.25 mL), adding 4% paraformaldehyde aqueous solution (0.25 mL) under cooling of an ice-water bath and uniformly mixing, enabling the mixed solution to be totally uniformly mixed with the RNA nanoparticles (33.84 nmol), and reacting for 72 hours at 4 DEG C under a dark condition.

(2) Taking 10 μL of reaction solution and diluting for 10 times, using 50 μM of the lenalidomide aqueous solution and 310 ng/μL of the RNA nanoparticles as controls, and performing HPLC analysis according to equal volume injection. According to a peak area ratio of each component, it may be adjusted that reaction conversion is basically completed.

(3) Extracting the reaction solution by the chloroform (10mLx3), and then adding 25 mL of the anhydrous ethanol, after uniformly mixing, keeping at 4 DEG C in the dark and enabling a product to be adequately precipitated (4 hours). Centrifuging (4000/min), transferring supernatant, and washing the solid product again by the ethanol (50 mL), and evaporating a solvent under reduced pressure and lower temperature, to obtain a dark red solid product.

(4) Loading rate calculation:

1. preparing a known concentration of lenalidomide-methanol standard solution: 2 μM, 4 μM, 6 μM, 8 μM, and 10 μM, 100 μL each;

2. enabling lenalidomide-RNAh particles to be dissolved in 100 μl of the PBS;

3. putting the standard solution and the lenalidomide-RNAh particles into a PCR plate, heating at 85 DEG C for 5 min, and then cooling to the room temperature;

4. measuring an absorbance of the lenalidomide at 210 nm by using a microplate reader, drawing a standard curve, and calculating the molar concentration of the paclitaxel in the loaded product;

5. measuring an absorbance of RNA at 260 nm by using a spectrophotometer, to obtain the mass concentration of the RNAh particles contained in each sample; and

6. according to the lenalidomide molar concentration and the mass concentration of the RNAh particles obtained by measuring, calculating a loading rate.

The standard curve of the RNA nucleic acid nanoparticles loading the lenalidomide is shown in Fig. 47a, and a specific calculation process is as follows:

$$C_{RNAh}\text{-}1 = 38.4\ \mu g/ml,\ M_{RNAh} \approx 30000,\ 100 \mu l;\ C_{lenalidomide}\text{-}1 = 12.2 \mu M,\ 100 \mu l;$$

$$C_{RNAh}\text{-}2 = 49.\ 3\ \mu g/ml,\ M_{RNAh} \approx 30000,\ 100 \mu l;\ C_{lenalidomide}\text{-}2 = 26.7\ \mu M,\ 100 \mu l;$$

$$N\text{-}2 = \frac{26.74 \times 10\text{-}6 \times 100 \times 10\text{-}6}{0.0493 \times 100 \times 10\text{-}6/30000} = 16.3 \quad N\text{-}1 = \frac{12.2 \times 10\text{-}6 \times 100 \times 10\text{-}6}{0.0384 \times 100 \times 10\text{-}6/30000} = 9.5$$

An average value thereof is taken, and it is obtained that the loading rate of the lenalidomide -RNAh nucleic acid nanoparticles is about 13, it represents that about 13 lenalidomide molecules may be loaded on each nucleic acid nanoparticle carrier.

(II) Loading experiment of DNA nucleic acid nanoparticles

The loading method and the calculation mode of the loading rate are the same as the above RNA nucleic acid nanoparticles. The specific nucleic acid nanoparticles used are: DNAh-Bio-EFGRapt-Cy5, herein, the three strands of DNAh are respectively:

a-strand (SEQ ID NO:172): 5'-CGCGCGCCCACGAGCGTTCCGGGCGC**GCCTTAGTAACGTGCTTTGATGTCGATTCG ACAGGA**GGC-3', thio modification is respectively performed on the front three bases of the 5'-end and the last three bases (italic part) of the 3'-end, the 5'-end is linked with Biotin, and a bolded part is a sequence siRNA of EGFR;

b-strand (SEQ ID NO:173): 5'-GCGCCCGGTTCGCCGCCAGCCGCCGC-3', the thio modification is respectively performed on the front three bases of the 5'-end and the last three bases of the 3'-end; and

c-strand (SEQ ID NO:174): 5'-GCGGCGGCAGGCGGCCATAGCCGTGGGCGCGCG-3', the thio modification is respectively performed on the front three bases of the 5'-end and the last three bases of the 3'-end, and the 3'-end is linked with the fluorescent label Cy5.

The standard curve of the DNA nucleic acid nanoparticles loading the lenalidomide is shown in Fig. 47b, and a specific calculation process is as follows:

$C_{DNAh}$-1 = 13.13 μg/ml, $M_{DNAh}$ ≈39500, 100μl; C $_{lenalidomide}$ -1 =1.99μM, 100μl;

$C_{DNAh}$-2 = 26.63 μg/ml, $M_{DNAh}$ ≈39500, 100μl; C $_{lenalidomide}$ -2 = 13.27 μM, 100μl;

$$N\text{-}1 = \frac{1.99\times 10\text{-}6\times 100\times 10\text{-}6}{0.1313\times 100\times 10\text{-}6/39500} \approx 6 \qquad N\text{-}2 = \frac{13.27\times 10\text{-}6\times 100\times 10\text{-}6}{0.2663\times 100\times 10\text{-}6/39500} \approx 19.7$$

.

An average value thereof is taken, and it is obtained that the loading rate of the lenalidomide -DNAh nucleic acid nanoparticles is about 13, it represents that about 13 lenalidomide may be loaded on each DNA nanoparticle carrier.

It is indicated from Embodiment 18 that the RNA nanoparticles (in Embodiment 1) and DNA nanoparticles with the extension fragment, the target head and the fluorescein both have a function of loading drugs, and the small molecular drug lenalidomide may achieve the loading in a mode of covalent linkage (paraformaldehyde-solvent covalence).

Embodiment 19

**Cell binding ability of drug-loaded DNA nanoparticles detected by flow cytometry experiment**

I. Cell information

RPMI 8226 (source: ATCC, and article number: CRM-CCL-155), a medium is RPMI1640 + 10% FBS, and culture conditions are 37°C, 5%$CO_2$, and saturated humidity.

II. Substances to be tested

Targeted drug: DNAh-Bio-EGFRapt-Cy5-lenalidomide (loaded product of the DNA nanoparticles in Embodiment 18).

Fluorescent carrier: DNAh-Bio-EGFRapt-Cy5 (DNA nanoparticles in Embodiment 18).

III. Devices and consumables (see Table 108)

Table 108:

| Name | Brand | Article number / Model |
|---|---|---|
| 24-well plate | Corning | 3526 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |
| Flow cytometer | ACEA | Novo Cyte |

IV. Reagents (see Table 109)

Table 109:

| Name | Brand | Article number |
|---|---|---|
| MEM medium (without folic acid and biotin) | provided by Baiyao Zhida Nano-biotechnology Co., Ltd | YS4150-500 |
| Fetal bovine serum FBS | Cegrogen | A0500-3018 |

(continued)

| Name | Brand | Article number |
|---|---|---|
| GlutaMax | Gibco | 35050-061 |
| Trypsin-EDTA digestive juice (0.25%) | Solarbio | T1320-100ml |
| Penicillin-streptomycin (100X) | Solarbio | P1400 |
| CellTiter-Glo® 2.0 | Promega | G9243 |

V. Experiment method:

1) Adjusting a cell state to a logarithmic growth phase, changing the medium to a medium without biotin and folic acid, and incubating overnight in the incubator at 37 DEG C;

2) dissolving a substance to be tested and preparing stock solution of the substance to be tested;

3) digesting and collecting single-cell suspension and counting, adjusting a cell density to $2\times10^5$/mL, and planting 1 mL/well into the 24-well plate;

4) respectively adding the substance to be tested to the corresponding cell wells, herein a final concentration is 0.1 μM, 0.2 μM, and 0.4 μM, shaking and mixing uniformly;

5) placing the cell plate in the incubator at 37 DEG C and incubating for 2 hours;

6) after the incubation, trypsinizing and collecting the cell suspension;

7) collect a cell precipitation by centrifugation, and washing twice with the PBS;

8) finally, resuspending the cell precipitation with 300 μL of the PBS, and performing a flow cytometry on-machine detection;

9) fluorescent carrier or lenalidomide detection channels: excitation light wavelength: 488 nm, and emission light wavelength: 560 nm; and

10) data analysis.

VI. Experiment result (see Table 110)

Table 110

| Cell line | Test sample | Binding rate (%) |
|---|---|---|
| RPMI 8226 | DNAh-Bio-EFGRapt-Cy5-lenalidomide | 100 |
| | DNAh-Bio-EFGRapt-Cy5 | 99.98 |
| | Blank control (cell-only medium) | 0.27 |

It may be seen from Table 110 that the lenalidomide targeted drug DNAh-Bio-EGFRapt-Cy5-lenalidomide may bind to the RPMI 8226 cells, and the binding rate is 100%; and the fluorescent carrier DNAh-Bio-EGFRapt-Cy5 may also bind to the RPMI 8226 cells, the binding rate is almost 100%.

Embodiment 20

**Stability detection of DNAh-Bio-EGFRapt-Cy5-lenalidomide nanoparticles in serum**

I. Experiment material, reagent and device

1. Experiment material:
DNAh-Bio-EGFRapt-Cy5-lenalidomide (same as Embodiment 19), herein a concentration is 1.4 mg/ml.
2. Experiment reagent:
6×DNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).

PowerPac Basic (Bio-rad), Mini PROTEAN Tetra Cell (Bio-rad), orbital shaker (TS-3D), and Tanon (Tanon 3500).

II. Experiment method

(1) Taking 3 μL of the DNAh-Bio-EGFRapt-Cy5-lenalidomide nanoparticles, diluting with 58.8μL of the RPMI 1640 medium containing 10% serum, herein the concentration may reach 200 μg/ml after dilution, diluting by 5 tubes respectively, and placing the diluted sample at 37 DEG C of a water bath for different times (0, 10 min, 1 h, 12 h, and 36 h).

(2) Taking the treated sample and uniformly mixing with the 6×DNA Loading Buffer, operating on ice, and making a label.

(3) Taking 8% Native PAGE, applying a piece of the gel to the nanoparticle samples with the different incubation times, herein a loading amount is 20 μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 48, herein 1 represents 0 min, 2 represents 10 min, 3 represents 1 h, 4 represents 12 h, and 5 represents 36 h. The target band of the DNAh-Bio-EGFRapt-Cy5-lenalidomide nanoparticles is about 200 bp. It may be seen from Fig. 48 that the DNAh-Bio-EGFRapt-Cy5-lenalidomide nanoparticles have slight drug release or degradation after being incubated at 37 DEG C.

Embodiment 21

**Cytotoxicity of DNAh-Bio-EGFRapt-Cy5-lenalidomide nanoparticles in RPMI 8226 cells**

I. Experiment material

1. Cell information (see Table 111):

Table 111:

| Name | Source | Medium | Culture condition |
|---|---|---|---|
| RPMI 8226 | ATCC | RPMI 1640, 10%FBS | 37°C, 5%$CO_2$, Saturated humidity |

2. Samples to be tested (see Table 112):

Table 112:

| Name | Carrier mass (mg) | Carrier molecular weight | Drug loaded mass (mg) | Drug molecular weight | Character | Preservation condition |
|---|---|---|---|---|---|---|
| DNAh-Bio-EGFRapt-Cy5-lenalidomide (same as Embodiment 19) | 0.5 | 39493 | 0.032 | 259.26 | Blue solid | -20 °C |
| Lenalidomide | — | — | 3.600 | 259.26 | Blue solid | -20 °C |
| DNAh-Bio-EGFRapt-Cy5 (same as Embodiment 19) | 1.25*3 | 39485.9 | — | — | Blue solid | -20 °C |

3. Consumables and devices (see Table 113):

Table 113:

| Name | Brand | Article number / Model |
|---|---|---|
| 96-well plate | Corning | 3599 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |

4. Reagents (see Table 114):

Table 114:

| Name | Brand | Article number |
|---|---|---|
| RPMI 1640 medium (without folic acid and biotin) | provided by Baiyao Zhida Nano-biotechnology Co., Ltd | YS3160-500 |
| MEM medium (without folic acid and biotin) | provided by Baiyao Zhida Nano-biotechnology Co., Ltd | YS4150-500 |
| DMEM medium (without folic acid and biotin) | provided by Baiyao Zhida Nano-biotechnology Co., Ltd | YS1200-500 |
| FBS | Cegrogen | A0500-3018 |
| GlutaMax | Gibco | 35050-061 |
| PBS | Gibco | C14190500BT |
| DMSO | Solarbio | D8371 |
| Trypsin-EDTA digestive juice (0.25%) | Solarbio | T1320-100ml |
| CellTiter 96® AQueous One Solution | Promega | G3581 |

II. Experiment method:

1) Harvesting cells in a logarithmic growth phase, taking a small amount and staining with trypan blue for counting to ensure that the cell viability reaches more than 98%;
2) adjusting the cell density to $1.11 \times 10^5$ /mL with a growth medium;
3) planting 90 μL/well of cell suspension into the 96-well plate, herein the number of cells per well in the plate is 10000;
4) placing the planted cell plate in the incubator at 37 DEG C and incubating overnight;
5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action concentration is shown in Table 115 below;

Table 115:

| Test sample | Concentration gradient |
|---|---|
| Lenalidomide | 100μM, 31.6μM, 10μM, 3.16μM, 1μM, 316nM, 100nM, 31.6nM, 10nM, 0 (10%PBS) |
| DNAh-Bio-EGFRapt-Cy5-lenalidomide | 100μM, 31.6μM, 10μM, 3.16μM, 1μM, 316nM, 100nM, 31.6nM, 10nM, 0 (10%PBS) |
| DNAh-Bio-EGFRapt-Cy5 | 10μM, 3.16μM, 1μM, 316nM, 100nM, 31.6nM, 10nM, 3.16nM, 1nM, 0 (10%PBS) |
| DMSO | 1%, 0.5%, 0.25%, 0.125%, 0.06%, 0.03%, 0 |

7) placing the cell culture plate in the incubator and continuously incubating for 96 hours;
8) placing the CellTiter 96® AQueous One Solution reagent at the room temperature and thawing for 90 minutes or thawing in a water bath at 37 DEG C, and placing at the room temperature and equilibrating for 30 minutes;
9) adding 20 μL/well of the CellTiter 96® AQueous One Solution reagent to the cell culture plate;
10) placing the cell culture plate in the incubator at 37 DEG C and continuously incubating for 3 hours;
11) using a microplate reader to read a $OD_{490}$ value of each well in the cell plate; and
12) data processing and analysis.

GraphPad Prism 5.0 software is used to process data graphically. In order to calculate the IC50, "S"-shaped nonlinear regression analysis is performed on the data to match an appropriate dosage-effect curve. A calculation formula of the survival rate is as follows, the IC50 may be automatically calculated in GraphPad Prism 5.0.

$$\text{Cell survival rate (\%)} = (OD_{\text{test well}} - OD_{\text{blank control}}) / (OD_{\text{negative control}} - OD_{\text{blank control}}) \times 100\%.$$

III. Experiment result (see Table 116, Fig. 49a to 49d)

Table 116:

| Cell line | Test sample | IC50 (μM) |
|---|---|---|
| RPMI 8226 | Lenalidomide | 72.33 |
| | DNAh-Bio-EGFRapt-Cy5-lenalidomide | 13.35 |
| | DNAh-Bio-EGFRapt-Cy5 | 0.1455 |
| | DMSO | >1% |

It may be seen from Table 116 and Fig. 49a, 49b, 49c, and 49d that for the RPMI 8226 cell line, compared with the simple DNAh targeted fluorescent carrier, the small molecular drug lenalidomide and DNAh drug-loaded particles DNAh-Bio-EGFRapt-Cy5-lenalidomide are both toxic to the RPMI 8226 cells, and the IC50 drug concentration of the DNAh drug-loaded particles DNAh-Bio-EGFRapt-Cy5-lenalidomide is less than 1/5 of the IC50 drug concentration of the small molecular drug lenalidomide. The IC50 of the lenalidomide and DNAh-Bio-EGFRapt-Cy5-lenalidomide acting

on the RPMI 8226 cells is 72.33μM and 13.35μM, and the IC50 of the DNAh-Bio-EGFRapt-Cy5 and DMSO acting on the RPMI 8226 cells is > 0.1μM and >1%.

Embodiment 22

**Cytarabine loading experiment**

(I) Chemical loading:

I. Experiment material and experiment method

1. Experiment materials and reagents:

(1) Nucleic acid nanoparticles are: DNAh-Bio-EFGRapt-Cy5, herein, the three strands of DNAh are respectively:

a-strand (SEQ ID NO:172): 5'-CGCGCGCCCACGAGCGTTCCGGGCGC**GCCTTAGTAACGTGCTTTGATGTCGATTCG ACAGGA**GGC-3', thio modification is respectively performed on the front three bases of the 5'-end and the last three bases of the 3'-end, the 5'-end is linked with Biotin, and a bolded part is a sequence siRNA of EGFR;

b-strand (SEQ ID NO:173): 5'-GCGCCCGGTTCGCCGCCAGCCGCCGC-3', the thio modification is respectively performed on the front three bases of the 5'-end and the last three bases of the 3'-end; and

c-strand (SEQ ID NO:174): 5'-GCGGCGGCAGGCGGCCATAGCCGTGGGCGCGCG-3', the thio modification is respectively performed on the front three bases of the 5'-end and the last three bases of the 3'-end, and the 3'-end is linked with the fluorescent label Cy5.

(2) DEPC treated water: Biyuntian Company.

(3) PBS buffer solution: cellgro.

(4) 4% paraformaldehyde

(5) Cytarabine (Epirarunixon).

(6) Chloroform: Beijing Beihua Fine Chemicals Co., Ltd.

(7) Anhydrous ethanol: Beijing Beihua Fine Chemicals Co., Ltd.

2. Experiment method:

(1) Accurately weighing the cytarabine (1.354μmol) and dissolving in the DEPC treated water (1.0 mL) and the PBS buffer solution (1.25 mL), adding 4% paraformaldehyde aqueous solution (0.25 mL) under cooling of an ice-water bath and uniformly mixing, enabling the mixed solution to be totally uniformly mixed with the RNA nanoparticles (33.84 nmol), and reacting for 72 hours at 4 DEG C under a dark condition.

(2) Taking 10 μL of reaction solution and diluting for 10 times, using 50 μM of the cytarabine aqueous solution and 310 ng/μL of the RNA nanoparticles as controls, and performing HPLC analysis according to equal volume injection. According to a peak area ratio of each component, it may be adjusted that reaction conversion is basically completed.

(3) Extracting the reaction solution by the chloroform (10mLx3), and then adding 25 mL of the anhydrous ethanol, after uniformly mixing, keeping at 4 DEG C in the dark and enabling a product to be adequately

precipitated (4 hours). Centrifuging (4000/min), transferring supernatant, and washing the solid product again by the ethanol (50 mL), and evaporating a solvent under reduced pressure and lower temperature, to obtain a dark red solid product.

(4) Loading rate calculation:

1. preparing a known concentration of cytarabine-PBS standard solution: 2 μM, 4 μM, 6 μM, 8 μM, and 10 μM, 100 μl each;

2. enabling cytarabine-DNAh particles to be dissolved in 100 μL of the PBS;

3. putting the standard solution and the cytarabine-DNAh particles into a PCR plate, heating at 85 DEG C for 5 min, and then cooling to the room temperature;

4. measuring an absorbance of the cytarabine at 272 nm by using a microplate reader, drawing a standard curve, and calculating the molar concentration of the cytarabine in the loaded product;

5. measuring an absorbance of DNAat 260 nm by using a spectrophotometer, to obtain the mass concentration of the DNAh particles contained in each sample; and

6. according to the cytarabine molar concentration and the mass concentration of the DNAh particles obtained by measuring, calculating a loading rate.

The standard curve diagram of the DNA nanoparticles loading the cytarabine is shown in Fig. 50.

$$C_{DNAh}\text{-}1 = 32.4\ \mu g/ml,\ M_{DNAh} \approx 39500,\ 100\mu l;\ C_{cytarabine}\text{-}1 = 9.8\ \mu M,\ 100\mu l;$$

$$C_{DNAh}\text{-}2 = 43.8\ \mu g/ml,\ M_{DNAh} \approx 39500,\ 100\mu l;\ C_{cytarabine}\text{-}2 = 12.32\ \mu M,\ 100\mu l;$$

$$N\text{-}1 = \frac{66.30\times 10\text{-}6\times 100\times 10\text{-}6}{0.3233\times 100\times 10\text{-}6/39500} \approx 81.01 \qquad N\text{-}2 = \frac{147.4\times 10\text{-}6\times 100\times 10\text{-}6}{0.6058\times 100\times 10\text{-}6/39500} \approx 96.11$$

An average value thereof is taken, and it is obtained that the loading rate of the cytarabine-DNAh nucleic acid nanoparticles is about 89, it represents that about 89 cytarabine molecules may be loaded on each nucleic acid nanoparticle carrier.

It is indicated from Embodiment 22 that the DNA nanoparticles with the extension fragment, the target head and the fluorescein both have a function of loading drugs, and the small molecular drug cytarabine may achieve the loading in a mode of covalent linkage.

Embodiment 23

**Cell binding ability of drug-loaded DNA nanoparticles detected by flow cytometry experiment**

I. Cell information

MV4-11 (source: ATCC, and article number: CRL-9591), a medium is RPMI1640 + 10% FBS, and culture conditions are 37 °C, 5%$CO_2$, and saturated humidity.

II. Substances to be tested

Targeted drug: DNAh-Bio-EGFRapt-Cy5-cytarabine (loaded product of the DNA nanoparticles in Embodiment 22).

Fluorescent carrier: DNAh-Bio-EGFRapt-Cy5 (DNA nanoparticles in Embodiment 22)

III. Devices and consumables (see Table 117)

Table 117:

| Name | Brand | Article number / Model |
|---|---|---|
| 24-well plate | Corning | 3526 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |
| Flow cytometer | ACEA | Novo Cyte |

IV. Reagents (see Table 118)

Table 118:

| Name | Brand | Article number |
|---|---|---|
| MEM medium (without folic acid and biotin) | provided by Baiyao Zhida Nano-biotechnology Co., Ltd | YS4150-500 |
| Fetal bovine serum FBS | Cegrogen | A0500-3018 |
| GlutaMax | Gibco | 35050-061 |
| Trypsin-EDTA digestive juice (0.25%) | Solarbio | T1320-100ml |
| Penicillin-streptomycin (100X) | Solarbio | P1400 |
| CellTiter-Glo® 2.0 | Promega | G9243 |

V. Experiment method:

1) Adjusting a cell state to a logarithmic growth phase, changing the medium to a medium without biotin and folic acid, and incubating overnight in the incubator at 37 DEG C;

2) dissolving a substance to be tested and preparing stock solution of the substance to be tested;

3) digesting and collecting single-cell suspension and counting, adjusting a cell density to $2\times10^5$/mL, and planting 1 mL/well into the 24-well plate;

4) respectively adding the substance to be tested to the corresponding cell wells, herein a final concentration is 0.1 μM, 0.2 μM, and 0.4 μM, shaking and mixing uniformly;

5) placing the cell plate in the incubator at 37 DEG C and incubating for 2 hours;

6) after the incubation, trypsinizing and collecting the cell suspension;

7) collect a cell precipitation by centrifugation, and washing twice with the PBS;

8) finally, resuspending the cell precipitation with 300 μL of the PBS, and performing a flow cytometry on-machine detection;

9) fluorescent carrier or cytarabine detection channels: excitation light wavelength: 488 nm, and emission light

wavelength: 560 nm; and

10) data analysis.

VI. Experiment result (see Table 119)

Table 119:

| Cell line | Test sample | Binding rate (%) |
|---|---|---|
| MV4-11 | DNAh-Bio-EFGRapt-Cy5-cytarabine | 99.85 |
| | DNAh-Bio-EFGRapt-Cy5 | 99.95 |
| | Blank control (cell-only medium) | 0.49 |

It may be seen from Table 119 that the cytarabine targeted drug DNAh-Bio-EGFRapt-Cy5-cytarabine may bind to the MV4-11 cells, and the binding rate is almost 100%; and the fluorescent carrier DNAh-Bio-EGFRapt-Cy5 may also bind to the MV4-11 cells, the binding rate is also almost 100%.

Embodiment 24

**Stability detection of DNAh-Bio-EGFRapt-Cy5-**cytarabine **nanoparticles in serum**

I. Experiment material, reagent and device

1. Experiment material:
DNAh-Bio-EGFRapt-Cy5-cytarabine (same as Embodiment 23), herein a concentration is 1321.8 μg/ml.

2. Experiment reagent:
6×DNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).

PowerPac Basic (Bio-rad), Mini PROTEAN Tetra Cell (Bio-rad), orbital shaker (TS-3D), and Tanon (Tanon 3500).

II. Experiment method

(1) Taking 4 μL of the DNAh-Bio-EGFRapt-Cy5-cytarabine nanoparticles, diluting with 21.8μL of the RPMI 1640 medium containing 10% serum, herein the concentration may reach 197.5 μg/ml after dilution, diluting by 5 tubes respectively, and placing the diluted sample at 37 DEG C of a water bath for different times (0, 10 min, 1 h, 12 h, and 36 h).

(2) Taking 20 μl of the treated sample and uniformly mixing with 4 μl of the 6×DNA Loading Buffer, operating on ice, and making a label.

(3) Taking 8% Native PAGE, applying a piece of the gel to the nanoparticle samples with the different incubation times, herein a loading amount is 20 μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 51, herein 1 represents 0 min, 2 represents 10 min, 3 represents 1 h, 4 represents 12 h, and 5 represents 36 h. The target band of the DNAh-Bio-EGFRapt-Cy5-cytarabine nanoparticles is about 200 bp. It may be seen from Fig. 51 that the DNAh-Bio-EGFRapt-Cy5-cytarabine nanoparticles are basically stable after being incubated at 37 DEG C, and slight drug release or degradation occurs after being incubated for 12 h and 36 h.

Embodiment 25

**Cytotoxicity of DNAh-Bio-EGFRapt-Cy5-cytarabine nanoparticles in MV4-11 cells**

I. Experiment material

1. Cell information (see Table 120)

Table 120:

| Name | Source | Medium | Culture condition |
|---|---|---|---|
| MV4-11 | ATCC | RPMI 1640, 10%FBS | 37°C, 5%$CO_2$, Saturated humidity |

2. Samples to be tested (see Table 121)

Table 121:

| Name | Carrier mass (mg) | Carrier molecular weight | Drug loaded mass (mg) | Drug molecular weight | Character | Preservation condition |
|---|---|---|---|---|---|---|
| DNAh-Bio-EGFRapt-Cy5-cytarabine (same as Embodiment 23) | 0.5 | 39493 | 0.092 | 243.22 | Blue solid | -20 °C |
| Cytarabine | — | — | 5.000 | 279.68 | White powder | -20 °C |
| DNAh-Bio-EGFRapt-Cy5 (same as Embodiment 23) | 1.25*3 | 39485.9 | — | — | Blue solid | -20°C |

3. Consumables and devices (see Table 122):

Table 122:

| Name | Brand | Article number / Model |
|---|---|---|
| 96-well plate | Corning | 3599 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |

4. Reagents (see Table 123):

Table 123:

| Name | Brand | Article number |
|---|---|---|
| RPMI 1640 medium (without folic acid and biotin) | provided by Baiyao Zhida Nano-biotechnology Co., Ltd | YS3160-500 |
| MEM medium (without folic acid and biotin) | provided by Baiyao Zhida Nano-biotechnology Co., Ltd | YS4150-500 |
| DMEM medium (without folic acid and biotin) | provided by Baiyao Zhida Nano-biotechnology Co., Ltd | YS1200-500 |
| FBS | Cegrogen | A0500-3018 |
| GlutaMax | Gibco | 35050-061 |
| PBS | Gibco | C14190500BT |
| DMSO | Solarbio | D8371 |
| Trypsin-EDTA digestive juice (0.25%) | Solarbio | T1320-100ml |
| CellTiter 96® AQueous One Solution | Promega | G3581 |

II. Experiment method:

1) Harvesting cells in a logarithmic growth phase, taking a small amount and staining with trypan blue for counting to ensure that the cell viability reaches more than 98%;
2) adjusting the cell density to $1.11 \times 10^5$ /mL with a growth medium;
3) planting 90 μL/well of cell suspension into the 96-well plate, herein the number of cells per well in the plate is 10000;
4) placing the planted cell plate in the incubator at 37 DEG C and incubating overnight;
5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action concentration is shown in Table 124 below;

Table 124:

| Test sample | Concentration gradient |
|---|---|
| Cytarabine | 100μM, 31.6μM, 10μM, 3.16μM, 1μM, 316nM, 100nM, 31.6nM, 10nM, 0 (10%PBS) |
| DNAh-Bio-EGFRapt-Cy5-cytarabine | 100μM, 31.6μM, 10μM, 3.16μM, 1μM, 316nM, 100nM, 31.6nM, 10nM, 0 (10%PBS) |
| DNAh-Bio-EGFRapt-Cy5 | 10μM, 3.16μM, 1μM, 316nM, 100nM, 31.6nM, 10nM, 3.16nM, 1nM, 0 (10%PBS) |
| DMSO | 1%, 0.5%, 0.25%, 0.125%, 0.06%, 0.03%, 0 |

7) placing the cell culture plate in the incubator and continuously incubating for 96 hours;
8) placing the CellTiter 96® AQueous One Solution reagent at the room temperature and thawing for 90 minutes or thawing in a water bath at 37 DEG C, and placing at the room temperature and equilibrating for 30 minutes;
9) adding 20 μL/well of the CellTiter 96® AQueous One Solution reagent to the cell culture plate;
10) placing the cell culture plate in the incubator at 37 DEG C and continuously incubating for 3 hours;
11) using a microplate reader to read a $OD_{490}$ value of each well in the cell plate; and
12) data processing and analysis.

GraphPad Prism 5.0 software is used to process data graphically. In order to calculate the IC50, "S"-shaped nonlinear regression analysis is performed on the data to match an appropriate dosage-effect curve. A calculation formula

of the survival rate is as follows, the IC50 may be automatically calculated in GraphPad Prism 5.0.

$$\text{Cell survival rate (\%)} = (\text{OD}_{\text{test well}} - \text{OD}_{\text{blank control}}) / (\text{OD}_{\text{negative control}} - \text{OD}_{\text{blank control}}) \times 100\%.$$

III. Experiment result (see Table 125, Fig. 52a to 52d)

Table 125:

| Cell line | Test sample | IC50 (μM) |
|---|---|---|
| MV4-11 | Cytarabine | 1.252 |
| | DNAh-Bio-EGFRapt-Cy5-cytarabine | 16.62 |
| | DNAh-Bio-EGFRapt-Cy5 | >0.1 |
| | DMSO | >1% |

It may be seen from Table 125 and Fig. 52a, 52b, 52c, and 52d that for the MV4-11 cell line, compared with the simple DNAh targeted fluorescent carrier, the small molecular drug cytarabine and DNAh drug-loaded particles DNAh-Bio-EGFRapt-Cy5- cytarabine are both toxic to the MV4-11 cells.

Embodiment 26

**Docetaxel loading experiment**

(I) Chemical loading:

I. Experiment material and experiment method

1. Experiment materials and reagents:

(1) Nucleic acid nanoparticles: it is similar to the RNA nanoparticles in Embodiment 1, a difference is that a fluorescent label on the c-strand is Cy5.

(2) Docetaxel: molecular weight: 850

(3) Solvent solution, such as DEPC treated water, insertion buffer solution (0.1 M solidum acetate, 0.005 M NaCl, and 0.01 M $MgCl_2$) and ethanol, is provided by North China Harvey Industries Co., Ltd.

2. Experiment method:

(1) Accurately weighing CX16113 (0.144 mg, 40 μmol) and dissolving in RNAh nanoparticle (0.25 mg, 2 μm) solution (1 mL) of the insertion buffer solution (0.1 M solidum acetate, 0.05 M NaCl, and 0.001 M $MgCl_2$);

(2) incubating in the dark at 37 °C for 1.5 hours;

(3) extracting mixed solution after incubation with chloroform (3 mL, 2-3 times) until extract liquor is clear;

(4) extracting upper mixed solution, adding absolute ethanol (10 mL) to it, and keeping in the dark at 4 °C to precipitate a product;

(5) centrifuging after 2 h and transferring supernatant, eluting a solid with 70% ethanol (1 mL), and washing with absolute ethanol (1 mL × 2), and evaporating the solvent under low temperature and reduced pressure, to obtain the docetaxel-RNAh nanoparticles; and

(6) calculating a loading rate:

1. preparing a known concentration of docetaxel-anhydrous ethanol standard solution: 2 μM, 4 μM, 6 μM, 8 μM, and 10 μM, 100 μL each;

2. enabling docetaxel-RNAh particles to be dissolved in 100 μL of the PBS;

3. putting the standard solution and the docetaxel-RNAh particles into a PCR plate, heating at 85 DEG C for 5 min, and then cooling to the room temperature;

4. measuring an absorbance of the docetaxel at 233 nm by using a microplate reader, drawing a standard curve, and calculating the molar concentration of the docetaxel in the loaded product;

5. measuring an absorbance of RNA at 260 nm by using a spectrophotometer, to obtain the mass concentration of the RNAh particles contained in each sample; and

6. according to the docetaxel molar concentration and the mass concentration of the RNAh particles obtained by measuring, calculating the loading rate.

The standard curve of the DNA nucleic acid nanoparticles loading the docetaxel is shown in Fig. 53a, and a specific calculation process is as follows:

$$C_{RNAh}\text{-}1 = 46.1\ \mu g/ml,\ M_{RNAh} \approx 30000,\ 100 \mu l;\ C_{docetaxel}\ \text{-}1 = 23.0\ \mu M,\ 100 \mu l;$$

$$C_{RNAh}\text{-}2 = 49.4\ \mu g/ml,\ M_{RNAh} \approx 30000,\ 100 \mu l;\ C_{docetaxel}\ \text{-}2 = 24.9\ \mu M,\ 100 \mu l;$$

$$N\text{-}2 = \frac{24.9 \times 10\text{-}6 \times 100 \times 10\text{-}6}{0.0494 \times 100 \times 10\text{-}6/30000} = 14.96 \quad N\text{-}1 = \frac{23.0 \times 10\text{-}6 \times 100 \times 10\text{-}6}{0.0461 \times 100 \times 10\text{-}6/30000} = 15.1$$

An average value thereof is taken, and it is obtained that the loading rate of the docetaxel-RNAh nucleic acid nanoparticles is about 15, it represents that about 15 docetaxel molecules may be loaded on each nucleic acid nanoparticle carrier.

(II) Loading experiment of DNA nucleic acid nanoparticles

The loading method and the calculation mode of the loading rate are the same as the above RNA nucleic acid nanoparticles. The specific nucleic acid nanoparticles used are: DNAh-Bio-EFGRapt-Cy5, herein, the three strands of DNAh are respectively:

a-strand (SEQ ID NO:120): 5'-CGCGCGCCCACGAGCGTTCCGGGCGC**GCCTTAGTAACGTGCTTTGATGTCGATTCG ACAGGA**GGC-3', thio modification is respectively performed on the front three bases of the 5'-end and the last three bases of the 3'-end, the 5'-end is linked with Biotin, and a bolded part is a sequence siRNA of EGFR;

b-strand (SEQ ID NO:121): 5'-GCGCCCGGTTCGCCGCCAGCCGCCGC-3', the thio modification is respectively performed on the front three bases of the 5'-end and the last three bases of the 3'-end; and

c-strand (SEQ ID NO:122): 5'-GCGGCGGCAGGCGGCCATAGCCGTGGGCGCGCG-3', the thio modification is respectively performed on the front three bases of the 5'-end and the last three bases of the 3'-end, and the 3'-end is linked with the fluorescent label Cy5.

The standard curve of the DNA nucleic acid nanoparticles loading the docetaxel is shown in Fig. 53b, and a specific calculation process is as follows:

$$C_{DNAh}\text{-}1 = 46.1\ \mu g/ml,\ M_{DNAh} \approx 39500,\ 100 \mu l;\ C_{docetaxel}\text{-}1 = 23.0\ \mu M,\ 100 \mu l;$$

$C_{DNAh}$-2 = 49.4 μg/ml, $M_{DNAh}$ ≈39500, 100μl; C $_{docetaxel}$-2 = 24.9 μM, 100μl;

$$N\text{-}1 = \frac{19.68\times10\text{-}6\times100\times10\text{-}6}{0.2862\times100\times10\text{-}6/39500} \approx 27.16 \qquad N\text{-}2 = \frac{35.20\times10\text{-}6\times100\times10\text{-}6}{0.4705\times100\times10\text{-}6/39500} \approx 29.55$$

An average value thereof is taken, and it is obtained that the loading rate of the docetaxel-DNAh nucleic acid nanoparticles is about 28, it represents that about 28 docetaxel may be loaded on each DNA nanoparticle carrier.

In addition, on the basis of loading the docetaxel on the RNA or DNA nanoparticles, other small molecular drugs may be loaded for the second time in the same method as that of loading the docetaxel. For example, the present application also further loads a folic acid, to obtain the RNA or DNA nanoparticles co-loaded with two small molecular drugs of the docetaxel and folic acid, and the loading rates of the two drugs may be detected by referring to the above method (values are not shown).

It is indicated from Embodiment 26 that the RNA nanoparticles (in Embodiment 1) and DNA nanoparticles with the extension fragment, the target head and the fluorescein both have a function of loading drugs, and the small molecular drug docetaxel may achieve the loading in a mode of covalent linkage (paraformaldehyde-solvent covalence).

Embodiment 27

**Cell binding ability of drug-loaded DNA nanoparticles detected by flow cytometry experiment**

I. Cell information

MCF-7 (source: ATCC, and article number: HTB-22), and SK-OV-3 (source: ATCC, and article number: HTB-77); a medium is MEM + 10% FBS, and culture conditions are 37 °C, 5%$CO_2$, and saturated humidity.

II. Substances to be tested

Targeted drug: DNAh-Bio-EGFRapt-Cy5-docetaxel (loaded product of the DNA nanoparticles in Embodiment 26).
Fluorescent carrier: DNAh-Bio-EGFRapt-Cy5

III. Devices and consumables (see Table 126)

Table 126:

| Name | Brand | Article number / Model |
|---|---|---|
| 24-well plate | Corning | 3526 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |
| Flow cytometer | ACEA | Novo Cyte |

IV. Reagents (see Table 127)

Table 127:

| Name | Brand | Article number |
|---|---|---|
| MEM medium (without folic acid and biotin) | Provided by Baiyao Zhida Nano-biotechnology Co., Ltd | YS4150-500 |

(continued)

| Name | Brand | Article number |
|---|---|---|
| Fetal bovine serum FBS | Cegrogen | A0500-3018 |
| GlutaMax | Gibco | 35050-061 |
| Trypsin-EDTA digestive juice (0.25%) | Solarbio | T1320-100ml |
| Penicillin-streptomycin (100X) | Solarbio | P1400 |
| CellTiter-Glo® 2.0 | Promega | G9243 |

V. Experiment method:

1) Adjusting a cell state to a logarithmic growth phase, changing the medium to a medium without biotin and folic acid, and incubating overnight in the incubator at 37 DEG C;

2) dissolving a substance to be tested and preparing stock solution of the substance to be tested;

3) digesting and collecting single-cell suspension and counting, adjusting a cell density to $2\times10^5$/mL, and planting 1 mL/well into the 24-well plate;

4) respectively adding the substance to be tested to the corresponding cell wells, herein a final concentration is 0.1 μM, 0.2 μM, and 0.4 μM, shaking and mixing uniformly;

5) placing the cell plate in the incubator at 37 DEG C and incubating for 2 hours;

6) after the incubation, trypsinizing and collecting the cell suspension;

7) collect a cell precipitation by centrifugation, and washing twice with the PBS;

8) finally, resuspending the cell precipitation with 300 μL of the PBS, and performing a flow cytometry on-machine detection;

9) fluorescent carrier or docetaxel detection channels: excitation light wavelength: 488 nm, and emission light wavelength: 560 nm; and

10) data analysis.

VI. Experiment result (see Table 128)

Table 128:

| Cell line | Test sample | Binding rate (%) |
|---|---|---|
| MCF-7 | DNAh-Bio-EFGRapt-Cy5-docetaxel | 99.60 |
| | DNAh-Bio-EFGRapt-Cy5 | 99.97 |
| | Blank control (cell-only medium) | 0.16 |
| SK-OV-3 | DNAh-Bio-EFGRapt-Cy5-docetaxel | 99.98 |
| | DNAh-Bio-EFGRapt-Cy5 | 100 |
| | Blank control (cell-only medium) | 0.11 |

It may be seen from Table 128 that the docetaxel targeted drug DNAh-Bio-EGFRapt-Cy5-docetaxel may bind to the MCF-7 cells and the SK-OV-3 cells, and the binding rates are both almost 100%; and the fluorescent carrier DNAh-Bio-EGFRapt-Cy5 may also bind to the MCF-7 cells and the SK-OV-3 cells, the binding rate is 100% too.

Embodiment 28

**Stability detection of DNAh-Bio-EGFRapt-Cy5-docetaxel nanoparticles in serum**

I. Experiment material, reagent and device

1. Experiment material:
DNAh-Bio-EGFRapt-Cy5-docetaxel (loaded product of DNA nanoparticles in Embodiment 26), herein a concentration is 1795.4 μg/ml.
2. Experiment reagent:
6×DNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).
PowerPac Basic (Bio-rad), Mini PROTEAN Tetra Cell (Bio-rad), orbital shaker (TS-3D), and Tanon (Tanon 3500).

II. Experiment method

(1) Taking 3 μL of the DNAh-Bio-EGFRapt-Cy5-docetaxel nanoparticles, diluting with 22.3μL of the RPMI 1640 medium containing 10% serum, herein the concentration may reach 197.5 μg/ml after dilution, diluting by 5 tubes respectively, and placing the diluted sample at 37 DEG C of a water bath for different times (0,10 min, 1 h, 12 h, and 36 h).

(2) Taking 20 μl of the treated sample and uniformly mixing with 4 μl of the 6×DNA Loading Buffer, operating on ice, and making a label.

(3) Taking 8% Native PAGE, applying a piece of the gel to the nanoparticle samples with the different incubation times, herein a loading amount is 20 μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 54, herein 1 represents 0 min, 2 represents 10 min, 3 represents 1 h, 4 represents 12 h, and 5 represents 36 h. The target band of the DNAh-Bio-EGFRapt-Cy5-docetaxel nanoparticles is about 200 bp. It may be seen from Fig. 54 that the DNAh-Bio-EGFRapt-Cy5-docetaxel nanoparticles are basically stable after being incubated at 37 DEG C, and slight drug release or degradation occurs after being incubated for 12 h and 36 h.

Embodiment 29

**Cytotoxicity research of RNAh-Bio-670-docetaxel nanoparticles in** HCT116 **cells**

I. Experiment material and experiment method

1. Experiment material:
Samples to be tested: small molecular drug docetaxel and RNAh-Bio-670-docetaxel nanoparticles (loaded product of DNA nanoparticles in Embodiment 26).
Drug concentration preparation:
Preparing a freshly prepared reagent into a corresponding volume container, and adding PBS to be quantified to 10 μM.
Preparing serial dilution solvents with the medium, from 10 μM to 3.33 μM, 1.11 μM, 0.370 μM, 0.124 μM, 0.041 μM, 0.014 μM, 0.0046 μM, and 0.0015 μM successively.
Transfering 50 μl of the solution to each well to obtain final concentrations of 5 μM, 1.667 μM, 0.556 μM, 0.185

μM, 0.062 μM, 0.021 μM, 0.0069 μM, and 0.0023 μM, respectively.

2. Experiment reagent:

CellTiter-Glo Luminescent Cell Viability Assay kit (CTG) (Promega, G7572-100 mL); DMEM (Gibco); Fetal bovine serum (FBS) (ExCell Bio, FNA500-500 mL); Trypsin-EDTA (Stemcell, 07901-500 mL); DMSO (Sigma, D5879-1 L); DMSO (Sigma, D5879-1 L); Penicillin/Streptomycin (Invitrogen); and PBS buffer solution (Gibco, C20012500BT-500 mL).

3. Experiment instrument:

Inverted Microscope (Olympus IX71, No.112A-1); 96-well Plate Reader (Molecular Devices, Flexstation 3); and Perkin Elmer Envision 2104 Multilabel Reader (No. 01-094- 0002).

4. Experiment method:

1) Cell culturing and plating

HCT116 cells are cultured at 37 DEG C and 5%$CO_2$ in the DMEM basal medium in which 10%FBS and 1%PS are respectively added. The cell density used in the experiment is above 80%. The cells are collected, and centrifuged at 1000 rpm for 4 minutes, the medium is resuspended, the cell concentration is adjusted, and it is added to the 96-well plate in a volume of 5000 cells per 90μL, and each group has 4 replicate wells.

2) Gradient drug concentration preparation and administration

After 24 hours, the compound solution is transferred to each well, 200 nM of each sample per well, and 4 replication wells.

Solvent control = DMSO

Medium (untreated) control: only cells without compound treatment

Blank control: without cells, used for instrument zero calibration

3) Cell culture after administration

The above cells after administration are cultured for 72 hours under a condition of 37 DEG C and 5% $CO_2$.

4) Cells treated by detection kit

The well plate is brought to a room temperature in advance and stands for 30 minutes. 100μL of a CellTiter-Glo® reagent is added to each well of the well plate and mixed for 2 minutes on a shaker to promote cell lysis. The Perkin Elmer Envision 2104 Multilabel Reader is used to read values and the values are recorded.

5) Experiment data acquisition and processing

The acquired experiment data is analyzed and processed by using excel software, and GraphPad Prism 5 software is used for curve fitting analysis.

II. Experiment result:

Table 129: cell survival rate (%)

| Cell line | Treatment time | Docetaxel | RNAh-Bio-670-docetaxel |
|---|---|---|---|
| HCT116 | 72h | 10.68% | 49.49% |

Experimental results are shown in Table 129 and Fig. 55, it may be seen from Table 129 and Fig. 55 that the docetaxel and RNAh-Bio-670-docetaxel nanoparticles both have a significant inhibitory effect on the proliferation of HCT116 cells, and when the drug concentration is 200 nM, the docetaxel chemical drug group had a significant inhibitory effect on the HCT116 cells, and the cell inhibition rates are 50.51% and 89.32% respectively.

Furthermore, in order to determine that the targeted fluorescent carrier itself is not significantly toxic to the

HCT116 cells, the present application further designs a toxicity experiment of the RNAh-Bio-FAM targeted fluorescent carrier to the HCT116 cells, and the small molecular chemical drug Cisplatin is used as a control of the toxicity of the HL60 cells, and a result shows that the fluorescent carrier itself has no apparent toxicity to the HCT116 cells (data is not shown).

Embodiment 30

**Cytotoxicity of DNAh-Bio-EGFRapt-Cy5-docetaxel nanoparticles in MCF-7 and SKOV3 cells**

I. Experiment material

1. Cell information (see Table 130)

Table 130:

| Name | Source | Medium | Culture condition |
|---|---|---|---|
| **SKOV3** | ATCC | MEM, 10%FBS | 37°C, 5%$CO_2$, Saturated humidity |
| MCF-7 | ATCC | MEM, 10% FBS | 37°C, 5%$CO_2$, Saturated humidity |

2. Samples to be tested (see Table 131)

Table 131:

| Name | Carrier mass (mg) | Carrier molecular weight | Drug loaded mass (mg) | Drug molecular weight | Character | Preservation condition |
|---|---|---|---|---|---|---|
| DNAh-Bio-EGFRapt-Cy5-docetaxel | 0.5 | 39493 | 0.225 | 807.88 | Blue solid | -20 °C |
| Eocetaxel | — | — | 2.300 | 807.88 | White powder | -20 °C |
| DNAh-Bio-EGFRapt-Cy5 | 1.25*3 | 39485.9 | — | — | Blue solid | -20 °C |
| Note: DNAh-Bio-EGFRapt-Cy5-docetaxel (loaded product of DNA nanoparticles in Embodiment 26). | | | | | | |

3. Consumables and devices (see Table 132):

Table 132:

| Name | Brand | Article number / Model |
|---|---|---|
| 96-well plate | Corning | 3599 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |

4. Reagents (see Table 133):

Table 133:

| Name | Brand | Article number |
|---|---|---|
| RPMI 1640 medium (without folic acid and biotin) | provided by Baiyao Zhida Nano-biotechnology Co., Ltd | YS3160-500 |
| MEM medium (without folic acid and biotin) | provided by Baiyao Zhida Nano-biotechnology Co., Ltd | YS4150-500 |
| DMEM medium (without folic acid and biotin) | provided by Baiyao Zhida Nano-biotechnology Co., Ltd | YS1200-500 |
| FBS | Cegrogen | A0500-3018 |
| GlutaMax | Gibco | 35050-061 |
| PBS | Gibco | C14190500BT |
| DMSO | Solarbio | D8371 |
| Trypsin-EDTA digestive juice (0.25%) | Solarbio | T1320-100ml |
| CellTiter 96® AQueous One Solution | Promega | G3581 |

II. Experiment method:

1) Harvesting cells in a logarithmic growth phase, taking a small amount and staining with trypan blue for counting to ensure that the cell viability reaches more than 98%;
2) adjusting the cell density to $2.22 \times 10^4$ /mL with a growth medium;
3) planting 90 μL/well of cell suspension into the 96-well plate, herein the number of cells per well in the plate is 2000;
4) placing the planted cell plate in the incubator at 37 DEG C and incubating overnight;
5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action concentration is shown in Table 134 below;

Table 134:

| Test sample | Concentration gradient |
|---|---|
| Docetaxel | 100μM, 31.6μM, 10μM, 3.16μM, 1μM, 316nM, 100nM, 31.6nM, 10nM, 0 (10%PBS) |
| DNAh-Bio-EGFRapt-Cy5-docetaxel | 100μM, 31.6μM, 10μM, 3.16μM, 1μM, 316nM, 100nM, 31.6nM, 10nM, 0 (10%PBS) |
| DNAh-Bio-EGFRapt-Cy5 | 10μM, 3.16μM, 1μM, 316nM, 100nM, 31.6nM, 10nM, 3.16nM, 1nM, 0 (10%PBS) |
| DMSO | 1%, 0.5%, 0.25%, 0.125%, 0.06%, 0.03%, 0 |

7) placing the cell culture plate in the incubator and continuously incubating for 96 hours;
8) placing the CellTiter 96® AQueous One Solution reagent at the room temperature and thawing for 90 minutes or thawing in a water bath at 37 DEG C, and placing at the room temperature and equilibrating for 30 minutes;
9) adding 20 μL/well of the CellTiter 96® AQueous One Solution reagent to the cell culture plate;
10) placing the cell culture plate in the incubator at 37 DEG C and continuously incubating for 3 hours;
11) using a microplate reader to read a $OD_{490}$ value of each well in the cell plate; and
12) data processing and analysis.

GraphPad Prism 5.0 software is used to process data graphically. In order to calculate the IC50, "S"-shaped nonlinear regression analysis is performed on the data to match an appropriate dosage-effect curve. A calculation formula

of the survival rate is as follows, the IC50 may be automatically calculated in GraphPad Prism 5.0.

$$\text{Cell survival rate (\%)} = (OD_{\text{test well}} - OD_{\text{blank control}}) / (OD_{\text{negative control}} - OD_{\text{blank control}}) \times 100\%.$$

III. Experiment result (see Table 135, Fig. 56a to 56d and Fig. 57a to 57d)

Table 135:

| Cell line | Test sample | IC50 (μM) |
|---|---|---|
| MCF-7 | Docetaxel | <0.001 |
| | DNAh-Bio-EGFRapt-Cy5-docetaxel | 5.909 |
| | DNAh-Bio-EGFRapt-Cy5 | >1 |
| | DMSO | >1% |
| SKOV3 | Docetaxel | <0.001 |
| | DNAh-Bio-EGFRapt-Cy5-docetaxel | 36.59 |
| | DNAh-Bio-EGFRapt-Cy5 | >1 |
| | DMSO | >1% |

It may be seen from Table 135 and Fig. 56a, 56b, 56c, and 56d that for the MCF-7 cell line, the docetaxel and DNAh-Bio-EGFRapt-Cy5-docetaxel are both cytotoxic. The IC50 acting on the MCF-7 cells is <0.001μM and 5.909 μM, respectively. The IC50 of the DNAh-Bio-EGFRapt-Cy5 and DMSO acting on the SKOV3 cells is >1 μM and >1%. Similarly, it may be seen from Table 135 and Fig.57a, 57b, 57c, and 57d that for the SKOV3 cell line, the docetaxel and DNAh-Bio-EGFRapt-Cy5-docetaxel are both cytotoxic, the IC50 acting on the SKOV3 cells is <0.001 μM and 36.59μM, and the IC50 of the DNAh-Bio-EGFRapt-Cy5 and DMSO acting on the SKOV3 cells is >1μM and >1%, respectively.

Embodiment 31

**Mitoxantrone loading experiment**

(I) Chemical loading:

I. Experiment material and experiment method

1. Experiment materials and reagents:

(1) The DNA nucleic acid nanoparticles D-8 (hereinafter referred to as DNAh nanoparticles) formed by the self-assembly of the 8-th group of the DNA sequences in Embodiment 4 are used for loading. Specific information is shown in Embodiment 4.

(2) DEPC treated water: Biyuntian Company.

(3) PBS buffer solution: cellgro.

(4) 4% paraformaldehyde

(5) Mitoxantrone.

(6) Chloroform: Beijing Beihua Fine Chemicals Co., Ltd.

(7) Anhydrous ethanol: Beijing Beihua Fine Chemicals Co., Ltd.

2. Experiment method:

(1) Accurately weighing the mitoxantrone (1.354μmol) and dissolving in the DEPC treated water (1.0 mL) and the PBS buffer solution (1.25 mL), adding 4% paraformaldehyde aqueous solution (0.25 mL) under cooling of an ice-water bath and uniformly mixing, enabling the mixed solution to be totally uniformly mixed with the DNAh nanoparticles (1 mg, 33.84 nmol), and reacting for 72 hours at 4 DEG C under a dark condition.

(2) Taking 10 μL of reaction solution and diluting for 10 times, using 50 μM of the mitoxantrone aqueous solution and 310 ng/μL of the DNAh nanoparticles as controls, and performing HPLC analysis according to equal volume injection. According to a peak area ratio of each component, it may be adjusted that reaction conversion is basically completed.

(3) Extracting the reaction solution by the chloroform (10mLx3), and then adding 25 mL of the anhydrous ethanol, after uniformly mixing, keeping at 4 DEG C in the dark and enabling a product to be adequately precipitated (4 hours). Centrifuging (4000/min), transferring supernatant, and washing the solid product again by the ethanol (50 mL), evaporating a solvent under reduced pressure and lower temperature, to obtain a blue solid product of mitoxantrone-DNAh particles

(4) Loading rate calculation:

1. preparing a known concentration of mitoxantrone-PBS standard solution: 2 μM, 4 μM, 6 μM, 8 μM, and 10 μM, 100 μL each;

2. enabling mitoxantrone-DNAh particles to be dissolved in 100 μL of the PBS;

3. putting the standard solution and the mitoxantrone-DNAh particles into a PCR plate, heating at 85 DEG C for 5 min, and then cooling to the room temperature;

4. measuring an absorbance of the mitoxantrone at 272 nm by using a microplate reader, drawing a standard curve (as shown in Fig. 58), and calculating the molar concentration of the mitoxantrone in the loaded product;

5. measuring an absorbance of DNA at 260 nm by using a spectrophotometer, to obtain the mass concentration of the DNAh particles contained in each sample; and

6. according to the mitoxantrone molar concentration and the mass concentration of the oxaliplatin-DNAh particles obtained by measuring, calculating a loading rate.

A calculation process is specifically as follows:

$$C_{DNAh}\text{-1} = 32.4\ \text{ug/ml},\ M_{DNAh} \approx 39500,\ 100\text{ul};\ C_{\text{mitoxantrone}}\text{-1} = 9.8\ \mu M,\ 100\text{ul};$$

$$C_{DNAh}\text{-2} = 43.8\ \text{ug/ml},\ M_{DNAh} \approx 39500,\ 100\text{ul};\ C_{\text{mitoxantrone}}\text{-2} = 12.32\ \mu M,\ 100\text{ul};$$

$$\text{N-1}= \frac{33.62\times 10\text{-}6\times 100\times 10\text{-}6}{0.1796\times 100\times 10\text{-}6/39500} \approx 73.95,\quad \text{N-2}= \frac{71.69\times 10\text{-}6\times 100\times 10\text{-}6}{0.3687\times 100\times 10\text{-}6/39500} \approx 76.80.$$

An average value thereof is taken, it is obtained that the loading rate of the mitoxantrone-DNAh particles is about 11.5, it represents that about 75 mitoxantrone molecules may be loaded on each DNAh nanoparticle carrier.

In addition, on the basis of the above DNAh nanoparticles loading the mitoxantrone, other small molecular drugs may be further loaded for the second time according to the same method as that of loading the mitoxantrone. For

example, a folic acid is further loaded in the present application, to obtain DNA nanoparticles co-loaded with two small molecular drugs of the mitoxantrone and folic acid, and the loading rates of the two drugs may be detected by referring to the above method (values are not shown).

It is indicated from Embodiment 31 that the DNA nanoparticles with the extension fragment, the target head and the fluorescein have a function of loading drugs, the small molecular drug mitoxantrone may achieve the loading in a mode of covalent linkage (paraformaldehyde-solvent covalence), and may also achieve the co-loading with other small molecular drugs.

Embodiment 32

**Cell binding ability of drug-loaded DNA nanoparticles detected by flow cytometry**

I. Cell information (see Table 136 below)

Table 136:

| Cell line | Source | Product model or article number | Medium | Culture condition |
|---|---|---|---|---|
| MCF-7 | ATCC | HTB-22 | MEM + 10% FBS | Saturated humidity |

II. Samples to be tested

Mitoxantrone targeted drug: DNAh-Biotin- EGFRapt-Cy5-Mit (loaded according to the loading method of the DNA nanoparticles in Embodiment 31).

Targeted fluorescent carrier: DNAh-Bio-EGFRapt-Cy5 (DNA nanoparticles provided in Embodiment 31).

III. Instrument, device and related reagent information (see Table 137 and Table 138)

Table 137:

| Name | Brand | Article number / Model |
|---|---|---|
| 24-well plate | Corning | 3526 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |
| Flow cytometer | ACEA | Novo Cyte |

Table 138:

| Name | Brand | Article number |
|---|---|---|
| Fetal bovine serum (FBS) | Cegrogen | A0500-3018 |
| Trypsin-EDTA digestive juice (0.25%) | Solarbio | T1320-100ml |
| CellTiter-Glo® 2.0 | Promega | G9243 |

IV. Experiment method:

1) Adjusting a cell state to a logarithmic growth phase, changing the medium to a medium without biotin and folic acid, and incubating overnight in the incubator at 37 DEG C;

2) dissolving a substance to be tested and preparing stock solution of the substance to be tested;

3) digesting and collecting single-cell suspension and counting, adjusting a cell density to $2\times10^5$/mL, and planting 1 mL/well into the 24-well plate;

4) respectively adding the substance to be tested to the corresponding cell wells, herein a final concentration is 2 μM, shaking and mixing uniformly;

5) placing the cell plate in the incubator at 37 DEG C and incubating for 16 hours;

6) after the incubation, trypsinizing and collecting the cell suspension;

7) collect a cell precipitation by centrifugation, and washing twice with the PBS;

8) finally, resuspending the cell precipitation with 300 μL of the PBS, and performing a flow cytometry on-machine detection, herein, fluorescent carrier or mitoxantrone targed drug detection channels: excitation light wavelength: 488 nm, and emission light wavelength: 560 nm; and

9) data analysis, an analysis result is shown in Table 139.

Table 139:

| Cell line | Test sample | Binding rate (%) |
|---|---|---|
| MCF-7 | DNAh-Biotin- EGFRapt - Cy5-Mit | 100 |
| | DNAh-Bio-EFGRapt-Cy5 | 99.97 |
| | Blank control (medium only) | 0.16 |

It may be seen from Table 139 that the DNAh nanoparticles carrying the target head and the small molecular drug mitoxantrone have a high binding rate to the cells, and it may be apparently seen that it may efficiently bind and internalize with the human breast cancer cells MCF-7. It may be seen that the mitoxantrone targeted drug DNAh-Biotin-EGFRapt-Cy5-Mit has an application prospect for the treatment of the breast cancer.

Embodiment 32

**Stability detection of DNAh-Bio-EGFRapt-Cy5-Mit nanoparticles in serum**

I. Experiment material, reagent and device

1. Experiment material:
Sample to be tested: DNAh-Bio-EGFRapt-Cy5-Mit, herein a concentration is 1.8 mg/ml.
2. Experiment reagent:
6×DNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).
PowerPac Basic (Bio-rad), Mini PROTEAN Tetra Cell (Bio-rad), orbital shaker (TS-3D), and Tanon (Tanon 3500).

II. Experiment method

(1) Taking 6 μL of the DNAh-Biotin- EGFRapt -Cy5-Mit nanoparticles, diluting with 6 μL of the RPMI 1640 medium

containing 10% of serum, herein the concentration may reach 900 μg/ml after dilution, diluting by 5 tubes respectively, and placing the diluted sample at 37 DEG C of a water bath for different times (0, 10 min, 1 h, 12 h, and 36 h).

(2) Taking the treated sample and uniformly mixing with the 6×DNA Loading Buffer, operating on ice, and making a label.

(3) Taking 8% Native PAGE, applying a piece of the gel to the nanoparticle samples with the different incubation times, herein a loading amount is 20 μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 59, herein 1 represents 0 min, 2 represents 10 min, 3 represents 1 h, 4 represents 12 h, and 5 represents 36 h. The target band of the DNAh-Bio-EGFRapt-Cy5-Mit nanoparticles is about 200 bp. It may be seen from Fig. 59 that the DNAh-Bio-EGFRapt-Cy5-Mit nanoparticles are basically stable after being incubated at 37 DEG C for 36 h.

Embodiment 33

**Cytotoxicity of DNAh-Biotin-EGFRapt-Cy5-Mit nanoparticles in MCF-7 cells**

I. Experiment material and method

1. Cell information (see Table 140)

Table 140:

| Name | Source | Serial number | Medium | Culture condition |
|---|---|---|---|---|
| MCF-7 | ATCC | HTB-22 | MEM, 10%FBS | 37°C, 5%$CO_2$, Saturated humidity |

2. Samples to be tested (see Table 141)

Table 141:

| Name | Carrier mass (mg) | Carrier molecular weight | Drug loaded mass (mg) | Drug molecular weight | Character | Preservation condition |
|---|---|---|---|---|---|---|
| DNAh-Biotin-EGFRapt -Cy5-Mit | 0.5 | 39493 | 0.169 | 444.48 | Blue solid | -20 °C |
| Mit (mitoxantrone ) | — | — | 0.800 | 444.48 | Blue solid | -20 °C |
| DNAh-Biotin-EGFRapt-Cy5 | 1.25*3 | 39485.9 | — | — | Blue solid | -20 °C |

3. Consumables and devices (see Table 142):

Table 142:

| Name | Brand | Article number / Model |
|---|---|---|
| 96-well plate | Corning | 3599 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |

4. Reagents (see Table 143):

Table 143:

| Name | Brand | Article number |
|---|---|---|
| RPMI 1640 medium (without folic acid and biotin) | provided by Baiyao Zhida Nano-biotechnology Co., Ltd | YS3160-500 |
| MEM medium (without folic acid and biotin) | provided by Baiyao Zhida Nano-biotechnology Co., Ltd | YS4150-500 |
| DMEM medium (without folic acid and biotin) | provided by Baiyao Zhida Nano-biotechnology Co., Ltd | YS1200-500 |
| Fetal Bovine Serum (FBS) | Cegrogen | A0500-3018 |
| GlutaMax | Gibco | 35050-061 |
| PBS | Gibco | C14190500BT |
| DMSO | Solarbio | D8371 |
| Trypsin-EDTA digestive juice (0.25%) | Solarbio | T1320-100ml |
| CellTiter 96® AQueous One Solution | Promega | G3581 |

II. Experiment method:

1) Harvesting cells in a logarithmic growth phase, taking a small amount and staining with trypan blue for counting to ensure that the cell viability reaches more than 98%;
2) adjusting the cell density to $2.22 \times 10^4$ /mL with a growth medium;
3) planting 90 μL/well of cell suspension into the 96-well plate, herein the number of cells per well in the plate is 2000;
4) placing the planted cell plate in the incubator at 37 DEG C and incubating overnight;
5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action concentration is shown in Table 144 below;

Table 144:

| Test sample | Concentration gradient |
|---|---|
| Mix (mitoxantrone) | 10μM, 3.16μM, 1μM, 316nM, 100nM, 31.6nM, 10nM, 3.16nM, 1nM, 0 (10%PBS) |
| DNAh-Biotin-EGFRapt-Cy5-Mit | 100μM, 31.6μM, 10μM, 3.16μM, 1μM, 316nM, 100nM, 31.6nM, 10nM, 0 (10%PBS) |
| DNAh-Bio-EGFRapt-Cy5 | 10μM, 3.16μM, 1μM, 316nM, 100nM, 31.6nM, 10nM, 3.16nM, 1nM, 0 (10%PBS) |

(continued)

| Test sample | Concentration gradient |
| --- | --- |
| DMSO | 1%, 0.5%, 0.25%, 0.125%, 0.06%, 0.03%, 0 |

7) placing the cell culture plate in the incubator and continuously incubating for 96 hours;
8) placing the CellTiter 96® AQueous One Solution reagent at the room temperature and thawing for 90 minutes or thawing in a water bath at 37 DEG C, and placing at the room temperature and equilibrating for 30 minutes;
9) adding 20 μL/well of the CellTiter 96® AQueous One Solution reagent to the cell culture plate;
10) placing the cell culture plate in the incubator at 37 DEG C and continuously incubating for 3 hours;
11) using a microplate reader to read a $OD_{490}$ value of each well in the cell plate; and
12) data processing and analysis.

GraphPad Prism 5.0 software is used to process data graphically. In order to calculate the IC50, "S"-shaped nonlinear regression analysis is performed on the data to match an appropriate dosage-effect curve. A calculation formula of the survival rate is as follows, the IC50 may be automatically calculated in GraphPad Prism 5.0.

$$\text{Cell survival rate (\%)} = (OD_{\text{test well}} - OD_{\text{blank control}}) / (OD_{\text{negative control}} - OD_{\text{blank control}}) \times 100\%.$$

III. Experiment result (see Table 145, Fig. 60 to 63)

Table 145:

| Cell line | Test sample | IC50 (μM) |
| --- | --- | --- |
| MCF-7 | Mit (mitoxantrone) | 0.5383 |
| | DNAh-Bio-EGFRapt-Cy5-Mit | 0.4589 |
| | DNAh-Bio-EGFRapt-Cy5 | >1 |
| | DMSO | >1% |

It may be seen from Table 145 and Fig. 60, 61, 62 and 63 that for the MCF-7 cell line, compared with the simple DNAh targeted fluorescent carrier, the small molecular drug mitoxantrone (Mit) and DNAh drug-loaded particles DNAh-Bio -EGFRapt-Cy5-Mit are both toxic to the MCF-7 cells.

Embodiment 34

**Idarubicin loading experiment**

(I) Chemical loading:

I. Experiment material and experiment method

1. Experiment materials and reagents:

(1) The DNA nucleic acid nanoparticles D-8 formed by the self-assembly of the 8-th group of the DNA sequences in Embodiment 4 are used for loading. Specific information is shown in Embodiment 4.

(2) DEPC treated water: Biyuntian Company.

(3) PBS buffer solution: cellgro.

(4) 4% paraformaldehyde

(5) Idarubicin.

(6) Chloroform: Beijing Beihua Fine Chemicals Co., Ltd.

(7) Anhydrous ethanol: Beijing Beihua Fine Chemicals Co., Ltd.

2. Experiment method:

(1) Accurately weighing the idarubicin (1.354μmol) and dissolving in the DEPC treated water (1.0 mL) and the PBS buffer solution (1.25 mL), adding 4% paraformaldehyde aqueous solution (0.25 mL) under cooling of an ice-water bath and uniformly mixing, enabling the mixed solution to be totally uniformly mixed with the nucleic acid nanoparticles D-8 (33.84 nmol), and reacting for 72 hours at 4 DEG C under a dark condition.

(2) Taking 10 μL of reaction solution and diluting for 10 times, using 50 μM of the idarubicin aqueous solution and 310 ng/μL of the nucleic acid nanoparticles D-8 as controls, and performing HPLC analysis according to equal volume injection. According to a peak area ratio of each component, it may be adjusted that reaction conversion is basically completed.

(3) Extracting the reaction solution by the chloroform (10mLx3), and then adding 25 mL of the anhydrous ethanol, after uniformly mixing, keeping at 4 DEG C in the dark and enabling a product to be adequately precipitated (4 hours). Centrifuging (4000/min), transferring supernatant, and washing the solid product again by the ethanol (50 mL), evaporating a solvent under reduced pressure and lower temperature, to obtain a solid product of idarubicin-DNAh particles

(4) Idarubicin-DNAh loading rate calculation:

1. preparing a known concentration of idarubicin-methyl alcohol standard solution: 2 μM, 4 μM, 6 μM, 8 μM, and 10 μM, 100 μL each;

2. enabling idarubicin-DNAh particles to be dissolved in 100 μL of the PBS;

3. putting the standard solution and the idarubicin-DNAh particles into a PCR plate, heating at 85 DEG C for 5 min, and then cooling to the room temperature;

4. measuring an absorbance of the idarubicin at 485 nm by using a microplate reader, drawing a standard curve (as shown in Fig. 64), and calculating the molar concentration of the idarubicin in the loaded product;

5. measuring an absorbance of DNA at 260 nm by using a spectrophotometer, to obtain the mass concentration of the DNAh particles contained in each sample; and

6. according to the idarubicin molar concentration and the mass concentration of the idarubicin-DNAh particles obtained by measuring, calculating a loading rate.

A calculation process is specifically as follows:

$$C_{DNAh}\text{-1} = 23.32\ \text{ug/ml},\ M_{DNAh} \approx 39500,\ 100\text{ul};\ C_{idarubicin}\text{-1} = 18.37\ \mu\text{M},\ 100\text{ul};$$

$$C_{DNAh}\text{-2} = 45.07\ \text{ug/ml},\ M_{DNAh} \approx 39500,\ 100\text{ul};\ C_{idarubicin}\text{-2} = 32.52\ \mu\text{M},\ 100\text{ul};$$

$$\text{N-1}=\frac{18.37\times 10\text{-}6\times 100\times 10\text{-}6}{0.2332\times 100\times 10\text{-}6/39500}\approx 31.11 \qquad \text{N-2}=\frac{32.52\times 10\text{-}6\times 100\times 10\text{-}6}{0.4507\times 100\times 10\text{-}6/39500}\approx 28.50$$

An average value thereof is taken, it is obtained that the loading rate of the idarubicin-DNAh particles is about 30, it represents that about 30 oxaliplatin may be loaded on each DNA nanoparticle carrier.

In addition, on the basis of the DNA nanoparticles loading the idarubicin, other small molecular drugs may be

further loaded for the second time according to the same method as that of loading the idarubicin. For example, a folic acid is further loaded in the present application, to obtain DNA nanoparticles co-loaded with two small molecular drugs of the idarubicin and folic acid, and the loading rates of the two drugs may be detected by referring to the above method (values are not shown).

It is indicated from Embodiment 34 that the DNA nanoparticles with the extension fragment, the target head and the fluorescein have a function of loading drugs, the small molecular drug idarubicin may achieve the loading in a mode of covalent linkage (paraformaldehyde-solvent covalence), and may also achieve the co-loading with other small molecular drugs.

Embodiment 35

**Cell binding ability of drug-loaded DNA nanoparticles detected by flow cytometry**

I. Cell information (see Table 146 below)

Table 146:

| Cell line | Source | Product model or article number | Medium | Culture condition |
|---|---|---|---|---|
| MCF-7 | ATCC | HTB-22 | MEM + 10% FBS | 37°C, 5%$CO_2$, saturated humidity |
| MV4-11 | ATCC | CRL-9591 | RPMI 1640, 10%FBS | |

II. Samples to be tested

Idarubicin targeted drug: DNAh-Biotin- EGFRapt-Cy5-idarubicin (loaded product of the DNA nanoparticles in Embodiment 34).

Targeted fluorescent carrier: DNAh-Bio-EGFRapt-Cy5.

III. Instrument, device and related reagent information (see Table 147 and Table 148)

Table 147:

| Name | Brand | Article number / Model |
|---|---|---|
| 24-well plate | Corning | 3526 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |
| Flow cytometer | ACEA | Novo Cyte |

Table 148:

| Name | Brand | 货号 |
|---|---|---|
| RPMI 1640 medium (without folic acid and biotin) | provided by Baiyao Zhida Nano-biotechnology Co., Ltd | YS3160-500 |
| MEM medium (without folic acid and biotin) | provided by Baiyao Zhida Nano-biotechnology Co., Ltd | YS4150-500 |

(continued)

| Name | Brand | 货号 |
|---|---|---|
| Fetal bovine serum FBS | Cegrogen | A0500-3018 |
| GlutaMax | Gibco | 35050-061 |
| Trypsin-EDTA digestive juice (0.25%) | Solarbio | T1320-100ml |
| Penicillin-streptomycin (100X) | Solarbio | P1400 |
| CellTiter-Glo® 2.0 | Promega | G9243 |

IV. Experiment method:

1) Adjusting a cell state to a logarithmic growth phase, changing the medium to a medium without biotin and folic acid, and incubating overnight in the incubator at 37 DEG C;

2) digesting and collecting single-cell suspension and counting, adjusting a cell density to $2\times10^5$/mL, and planting 1 mL/well into the 24-well plate;

3) dissolving a substance to be tested and preparing stock solution of the substance to be tested;

4) respectively adding the substance to be tested to the corresponding cell wells, herein a final concentration is 2 μM, shaking and mixing uniformly;

5) placing the cell plate in the incubator at 37 DEG C and incubating for 16 hours;

6) after the incubation, trypsinizing and collecting the cell suspension;

7) collect a cell precipitation by centrifugation, and washing twice with the PBS;

8) finally, resuspending the cell precipitation with 300 μL of the PBS, and performing a flow cytometry on-machine detection; and

9) data analysis, an analysis result is shown in Table 149.

Table 149:

| Cell line | Test sample | Binding rate (%) |
|---|---|---|
| MCF-7 | DNAh-Bio-EFGRapt-Cy5-idarubicin | 98.33 |
| | DNAh-Bio-EFGRapt-Cy5 | 99.97 |
| | Blank control (medium only) | 0.16 |
| MV4-11 | DNAh-Bio-EFGRapt-Cy5-idarubicin | 98.50 |
| | DNAh-Bio-EFGRapt-Cy5 | 99.95 |
| | Blank control (medium only) | 0.49 |

It may be seen from Table 149 that the DNA nucleic acid nanoparticles carrying the target head and the small molecular drug idarubicin have a high binding rate to the cells, and it may be apparently seen that it may bind and internalize with the cells in the corresponding tumor cell line. In addition, it may also be seen from the above Table 149 that the DNAh-Bio-EFGRapt-Cy5-idarubicin may not only efficiently bind and internalize with the MCF-7 cells in the human breast cancer cell line, but also may bind and internalize with the MV4 -11 cells of the acute monocytic leukemia. It may be seen that the idarubicin targeted drug DNAh-Bio-EFGRapt-Cy5-idarubicin has an application prospect for the

treatment of the breast cancer, and has an application prospect for the treatment of the leukemia.

Embodiment 36

**Stability detection of DNAh-Bio-EGFRapt-Cy5-idarubicin nanoparticles in serum**

I. Experiment material, reagent and device

1. Experiment material:
Sample to be tested: DNAh-Bio-EGFRapt-Cy5-idarubicin, herein a concentration is 1.32 mg/ml.

2. Experiment reagent:
6×DNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).

PowerPac Basic (Bio-rad), Mini PROTEAN Tetra Cell (Bio-rad), orbital shaker (TS-3D), and Tanon (Tanon 3500).

II. Experiment method

(1) Taking 4 μL of the DNAh drug-loaded nanoparticles, diluting with 21.7 μL of the RPMI 1640 medium containing 10% of serum, herein the concentration may reach 197.5 μg/ml after dilution, diluting by 5 tubes respectively, and placing the diluted sample at 37 DEG C of a water bath for different times (0, 10 min, 1 h, 12 h, and 36 h).

(2) Taking 20 μl of the treated sample and uniformly mixing with the 6×DNA Loading Buffer, operating on ice, and making a label.

(3) Taking 8% Native PAGE, applying a piece of the gel to the nanoparticle samples with the different incubation times, herein a loading amount is 20 μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 65, herein 1 represents 0 min, 2 represents 10 min, 3 represents 1 h, 4 represents 12 h, and 5 represents 36 h. The target band of the DNAh-Bio-EGFRapt-Cy5-idarubicin nanoparticles is about 200 bp. It may be seen from Fig. 65 that the DNAh-Bio-EGFRapt-Cy5-idarubicin nanoparticles are basically stable after being incubated at 37 DEG C for 36 h.

Embodiment 37

**Cytotoxicity of DNAh-Biotin-EGFRapt-Cy5-idarubicin nanoparticles in MCF-7 and MV4-11 cells**

I. Experiment material and method

1. Cell information (see Table 150)

Table 150:

| Name | Source | Serial number | Medium | Medium |
|---|---|---|---|---|
| MCF-7 | ATCC | HTB-22 | MEM, 10%FBS | 37 °C, 5%$CO_2$, Saturated humidity |
| MV4-11 | ATCC | CRL-9591 | RPMI1640, 10%FBS | 37 °C, 5%$CO_2$, Saturated humidity |

2. Samples to be tested (see Table 151)

Table 151:

| Name | Carrier mass (mg) | Carrier molecular weight | Drug loaded mass (mg) | Drug molecular weight | Character | Preservation condition |
|---|---|---|---|---|---|---|
| DNAh-Biotin-EGFRapt-Cy5-idarubicin | 0.5 | 39493 | 0.202 | 497.49 | Blue solid | -20 °C |
| Idarubicin | — | — | 1.300 | 497.49 | Blue solid | -20 °C |
| DNAh-Biotin-EGFRapt-Cy5 | 1.25*3 | 39485.9 | — | — | Blue solid | -20 °C |

3. Consumables and devices (see Table 152):

Table 152:

| Name | Brand | Article number / Model |
|---|---|---|
| 96-well plate | Corning | 3599 |
| Centrifuge | Gineek | LD5-2B |
| $CO_2$ incubator | Thermo | 3111 |
| Microplate shaker | QILINBEIER | QB-9001 |
| Microscope | Olympus | IX53 |
| Multifunctional microplate reader | Bio Tek | Synergy H1 |

4. Reagents (see Table 153):

Table 153:

| Name | Brand | Article number |
|---|---|---|
| RPMI 1640 medium (without folic acid and biotin) | Provided by Baiyao Zhida Nano-biotechnology Co., Ltd | YS3160-500 |
| MEM medium (without folic acid and biotin) | Provided by Baiyao Zhida Nano-biotechnology Co., Ltd | YS4150-500 |
| FBS | Cegrogen | A0500-3018 |
| GlutaMax | Gibco | 35050-061 |
| PBS | Gibco | C14190500BT |
| DMSO | Solarbio | D8371 |
| Trypsin-EDTA digestive juice (0.25%) | Solarbio | T1320-100ml |

(continued)

| Name | Brand | Article number |
|---|---|---|
| CellTiter 96® AQueous One Solution | Promega | G3581 |

II. Experiment method:

1) Harvesting cells in a logarithmic growth phase, taking a small amount and staining with trypan blue for counting to ensure that the cell viability reaches more than 98%;
2) adjusting the cell density to $2.22 \times 10^4$ /mL with a growth medium;
3) planting 90 μL/well of cell suspension into the 96-well plate, herein the number of cells per well in the plate is 2000;
4) placing the planted cell plate in the incubator at 37 DEG C and incubating overnight;
5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action concentration is shown in Table 154 below;

Table 154:

| Test sample | Concentration gradient |
|---|---|
| Small molecular drug | 100μM, 31.6μM, 10μM, 3.16μM, 1μM, 316nM, 100nM, 31.6nM, 10nM, 0 (10%PBS) |
| DNAh-Bio-EGFRapt-Cy5-small molecular drug | 100μM, 31.6μM, 10μM, 3.16μM, 1μM, 316nM, 100nM, 31.6nM, 10nM, 0 (10%PBS) |
| DNAh-Bio-EGFRapt-Cy5 | 10μM, 3.16μM, 1μM, 316nM, 100nM, 31.6nM, 10nM, 3.16nM, 1nM, 0 (10%PBS) |
| DMSO | 1%, 0.5%, 0.25%, 0.125%, 0.06%, 0.03%, 0 |

7) placing the cell culture plate in the incubator and continuously incubating for 96 hours;
8) placing the CellTiter 96® AQueous One Solution reagent at the room temperature and thawing for 90 minutes or thawing in a water bath at 37 DEG C, and placing at the room temperature and equilibrating for 30 minutes;
9) adding 20 μL/well of the CellTiter 96® AQueous One Solution reagent to the cell culture plate;
10) placing the cell culture plate in the incubator at 37 DEG C and continuously incubating for 3 hours;
11) using a microplate reader to read a $OD_{490}$ value of each well in the cell plate; and
12) data processing and analysis.

GraphPad Prism 5.0 software is used to process data graphically. In order to calculate the $IC_{50}$, "S"-shaped nonlinear regression analysis is performed on the data to match an appropriate dosage-effect curve. A calculation formula of the survival rate is as follows, the $IC_{50}$ may be automatically calculated in GraphPad Prism 5.0.

$$\text{Cell survival rate (\%)} = (OD_{\text{test well}} - OD_{\text{blank control}}) / (OD_{\text{negative control}} - OD_{\text{blank control}}) \times 100\%.$$

III. Experiment result (see Table 155, Fig. 66a to 66d and Fig. 67a to 67d)

Table 155:

| Cell line | Test sample | $IC_{50}$ (μM) |
|---|---|---|
| MCF-7 | Idarubicin | 0.07248 |
| | DNAh-Bio-EGFRapt-Cy5-idarubicin | 0.02530 |
| | DNAh-Bio-EGFRapt-Cy5 | >1 |
| | DMSO | >1% |

(continued)

| Cell line | Test sample | $IC_{50}$ (μM) |
|---|---|---|
| MV4-11 | Idarubicin | <0.001 |
| | DNAh-Bio-EGFRapt-Cy5-idarubicin | <0.001 |
| | DNAh-Bio-EGFRapt-Cy5 | >0.1 |
| | DMSO | >1% |

It may be seen from Table 155 and Fig. 66a, 66b, 66c, and 66d that for the MCF-7 cell line, compared with the simple DNAh targeted fluorescent carrier, the small molecular drug idarubicin and DNAh drug-loaded particles DNAh-Bio -EGFRapt-Cy5-idarubicin are both toxic to the MCF-7 cells. Similarly, it may be seen from Table 155 and Fig. 67a, 67b, 67c, and 67d that for the MV4-11 cell line, compared with the simple DNAh targeted fluorescent carrier, the small molecular drug idarubicin and DNAh drug-loaded particles DNAh-Bio-EGFRapt-Cy5-idarubicin are both toxic to the MV4-11 cells.

It may be seen from the above description that the above embodiments of the present application achieve the following technical effects: the present application provides a series of nucleic acid nanoparticle carriers with thermodynamic stability, chemical stability, high loading rate and multivalent combination modules. A unique modular design of this type of the carriers is performed to obtain a core modular structure which not only maintains a naturally compatible affinity, but also has high stability and diverse combination characteristics. This structure may flexibly and efficiently integrate various functional modules, including a targeting module, an imaging and probe module, a treatment module and other composite intelligent modules, so that it may be used for targeted delivery in vivo to achieve precise diagnosis and treatment.

Through loading the small molecular drug paclitaxel on the nucleic acid nanoparticle carrier provided by the present application to form the nucleic acid nanocarrier drug, not only the delivery stability of the paclitaxel may be improved, but also in the case that the nucleic acid nanoparticles carry the target head, on the one hand, the paclitaxel is targed and delivered to the target cells, and the bioavailability of the drug is imporved; and on the other hand, the toxic and side effects to the non-target cells or tissues are reduced due to the targeted delivery, and the local drug concentration is reduced, thus the toxic and side effects caused by the high drug concentration are further reduced.

The above are only the preferred embodiments of the disclosure, and are not used to limit the disclosure, and various modifications and changes may be made to the disclosure by those skilled in the art. Any modifications, equivalent replacements, improvements and the like made within spirit and principle of the disclosure should be included in a scope of protection of the disclosure.

## Claims

1. A nucleic acid nanocarrier drug, wherein the nucleic acid nanocarrier drug comprises a nucleic acid nanoparticle carrier and a drug, the drug is loaded on the nucleic acid nanoparticle, and the drug comprises one or more of paclitaxel, lenalidomide, cytarabine, docetaxel, idarubicin and mitoxantrone; and

   the nucleic acid nanoparticle comprises a nucleic acid domain, the nucleic acid domain comprises a sequence a, a sequence b and a sequence c, the sequence a comprises a sequence a1 or a sequence obtained by insertion, deletion or substitution of at least one base in the sequence a1, the sequence b comprises a sequence b1 or a sequence obtained by insertion, deletion or substitution of at least one base in the sequence b1, and the sequence c comprises a sequence c1 or a sequence obtained by insertion, deletion or substitution of at least one base in the sequence c1,
   wherein, the sequence a1 is SEQ ID NO:1: 5'-CCAGCGUUCC-3'or SEQ ID NO:2: 5'-CCAGCGTTCC-3';
   the sequence b1 is SEQ ID NO:3: 5'-GGUUCGCCG-3'or SEQ ID NO:4: 5'-GGTTCGCCG-3'; and
   the sequence c1 is SEQ ID NO:5: 5'-CGGCCAUAGCGG-3'or SEQ ID NO:6: 5'-CGGCCATAGCGG-3'.

2. The nucleic acid nanocarrier drug according to claim 1, wherein when the sequence a1 is the SEQ ID NO:1, the sequence b1 is the SEQ ID NO:3, and the sequence c1 is the SEQ ID NO:5, at least one sequence of the sequence a, the second b and the sequence c comprises a sequence obtained by insertion, deletion or substitution of at least one base.

3. The nucleic acid nanocarrier drug according to claim 1 or 2, wherein the insertion, deletion or substitution of at least one base is occurred:

   (1) on 1, 2, 4 or 5-th base starting from a 5 '-end of the sequence shown in the SEQ ID NO:1 or the SEQ ID NO:2; and/or
   (2) between 8-th and 10-th bases starting from the 5 '-end of the sequence shown in the SEQ ID NO:1 or the SEQ ID NO:2; and/or
   (3) between 1-th and 3-th bases starting from a 5 '-end of the sequence shown in the SEQ ID NO:3 or the SEQ ID NO:4; and/or
   (4) between 6-th and 9-th bases starting from the 5 '-end of the sequence shown in the SEQ ID NO:3 or the SEQ ID NO:4; and/or
   (5) between 1-th and 4-th bases starting from a 5 '-end of the sequence shown in the SEQ ID NO:5 or the SEQ ID NO:6; and/or
   (6) between 9-th and 12-th bases starting from the 5 '-end of the sequence shown in the SEQ ID NO:5 or the SEQ ID NO:6.

4. The nucleic acid nanocarrier drug according to claim 1 or 2, wherein the sequence a, the sequence b and the sequence c are self-assembled into a structure shown in Formula (1):

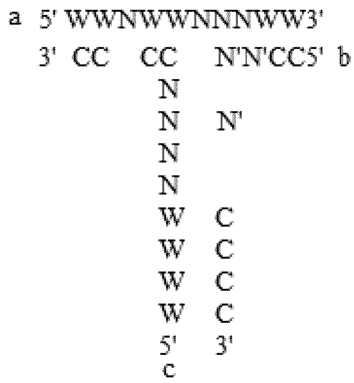

Formula (1),

wherein, W-C represents Watson-Crick pairing, N and N' represent non-Watson-Crick pairing, the W-C in any one position is independently selected from C-G or G-C;
in the sequence a, the first N from the 5'-end is A, the second N is G, the third N is U or T, and the fourth N is any one of U, T, A, C or G;
in the sequence b, the first N' from the 5'-end is any one of U, T, A, C or G, the second N' is U or T, and the third N' is C; and
in the sequence c, a sequence NNNN along a direction from the 5'-end to the 3'-end is CAUA or CATA.

**5.** The nucleic acid nanocarrier drug according to claim 4, wherein the sequence a, the sequence b and the sequence c are any one of the following groups:

(1) sequence a: 5'-GGAGCGUUGG-3',

sequence b: 5'-CCUUCGCCG-3',
sequence c: 5'-CGGCCAUAGCCC-3';

(2) sequence a: 5'-GCAGCGUUCG-3',

sequence b: 5'-CGUUCGCCG-3',
sequence c: 5'-CGGCCAUAGCGC-3';

(3) sequence a: 5'-CGAGCGUUGC-3',

sequence b: 5'-GCUUCGCCG-3',
sequence c: 5'-CGGCCAUAGCCG-3';

(4) sequence a: 5'-GGAGCGUUGG-3',

sequence b: 5'-CCUUCGGGG-3',
sequence c: 5'-CCCCCAUAGCCC-3';

(5) sequence a: 5'-GCAGCGUUCG-3',

sequence b: 5'-CGUUCGGCG-3',
sequence c: 5'-CGCCCAUAGCGC-3';

(6) sequence a: 5'-GCAGCGUUCG-3',

sequence b: 5'-CGUUCGGCC-3',
sequence c: 5'-GGCCCAUAGCGC-3';

(7) sequence a: 5'-CGAGCGUUGC-3',

sequence b: 5'-GCUUCGGCG-3',

sequence c: 5'-CGCCCAUAGCCG-3';

(8) sequence a: 5'-GGAGCGTTGG-3',

sequence b: 5'-CCTTCGCCG-3',
sequence c: 5'-CGGCCATAGCCC-3';

(9) sequence a: 5'-GCAGCGTTCG-3',

sequence b: 5'-CGTTCGCCG-3',
sequence c: 5'-CGGCCATAGCGC-3';

(10) sequence a: 5'-CGAGCGTTGC-3',

sequence b: 5'-GCTTCGCCG-3', sequence c: 5'-CGGCCATAGCCG-3';

(11) sequence a: 5'-GGAGCGTTGG-3',

sequence b: 5'-CCTTCGGGG-3',
sequence c: 5'-CCCCCATAGCCC-3';

(12) sequence a: 5'-GCAGCGTTCG-3',

sequence b: 5'-CGTTCGGCG-3',
sequence c: 5'-CGCCCATAGCGC-3';

(13) sequence a: 5'-GCAGCGTTCG-3',

sequence b: 5'-CGTTCGGCC-3',
sequence c: 5'-GGCCCATAGCGC-3';

(14) sequence a: 5'-CGAGCGTTGC-3',

sequence b: 5'-GCTTCGGCG-3',
sequence c: 5'-CGCCCATAGCCG-3';

(15) sequence a: 5'-CGAGCGTTCC -3',

sequence b: 5'-GGTTCGCCG -3',
sequence c: 5'-CGGCCATAGCCG-3'.

**6.** The nucleic acid nanocarrier drug according to claim 4, wherein the nucleic acid domain further comprises a first extension fragment, the first extension fragment is an extension fragment of Watson-Crick pairing, and the first extension fragment is positioned at the 5'-end and/or the 3'-end of any one sequence of the sequence a, the sequence b or the sequence c;
preferably, the first extension fragment is selected from any one of the following groups:

(1): a-strand 5'-end: 5'-CCCA-3', c-strand 3'-end: 5'-UGGG-3';
(2): a-strand 3'-end: 5'-GGG-3', b-strand 5'-end: 5'-CCC-3';
(3): b-strand 3'-end: 5'-CCA-3', c-strand 5'-end: 5'-UGG-3';
(4): a-strand 5'-end: 5'-CCCG-3', c-strand 3'-end: 5'-CGGG-3';
(5): a-strand 5'-end: 5'-CCCC-3', c-strand 3'-end: 5'-GGGG-3';
(6): b-strand 3'-end: 5'-CCC-3', c-strand 5'-end: 5'-GGG-3';
(7): b-strand 3'-end: 5'-CCG-3', c-strand 5'-end: 5'-CGG-3';
(8): a-strand 5'-end: 5'-CCCA-3', c-strand 3'-end: 5'-TGGG-3'; and
(9): b-strand 3'-end: 5'-CCA-3', c-strand 5'-end: 5'-TGG-3'.

**7.** The nucleic acid nanocarrier drug according to any one of claims 1 to 6, wherein the nucleic acid domain further

comprises a second extension fragment, the second extension fragment is positioned at the 5'-end and/or the 3'-end of any one sequence of the sequence a, the sequence b or the sequence c, and the second extension fragment is an extension fragment of Watson-Crick pairing;

preferably, the second extension fragment is an extension sequence of a CG base pair;
more preferably, the second extension fragment is an extension sequence of 1-10 CG base pairs.

8. The nucleic acid nanocarrier drug according to claim 7, wherein the nucleic acid domain further comprises at least one group of the following second extension fragments:

first group: a-strand 5'-end: 5'-CGCGCG-3', c-strand 3'-end: 5'-CGCGCG-3';
second group: a-strand 3'-end: 5'-CGCCGC-3', b-strand 5'-end: 5'-GCGGCG-3'; and
third group: b-strand 3'-end: 5'-GGCGGC-3', c-strand 5'-end: 5'-GCCGCC-3'.

9. The nucleic acid nanocarrier drug according to claim 7, wherein the second extension fragment is an extension sequence containing both CG base pair and AT/AU base pair, and preferably the second extension fragment is an extension sequence of 2-50 base pairs.

10. The nucleic acid nanocarrier drug according to claim 9, wherein,

the second extension fragment is an extension sequence in which sequences of 2-8 continuous CG base pairs and sequences of 2-8 continuous AT/AU base pairs are alternately arranged; or
the second extension fragment is an extension sequence in which a sequence of 1 CG base pair and a sequence of 1 AT/AU base pair are alternately arranged.

11. The nucleic acid nanocarrier drug according to any one of claims 1 to 10, wherein a base, a ribose and a phosphate in the sequence a, the sequence b and the sequence c have at least one modifiable site, and any one of the modifiable sites is modified by any one of the following modification adapters: -F, a methyl, an amino, a disulfide, a carbonyl, a carboxyl, a sulfhydryl and a formyl; and
preferably, the base C or U in the sequence a, the sequence b and the sequence c has 2'-F modification.

12. The nucleic acid nanocarrier drug according to any one of claims 1 to 11, wherein the drug is loaded on the nucleic acid nanoparticle in modes of physical linkage and/or covalent linkage, and a molar ratio between the drug and the nucleic acid nanoparticle is 2-300:1, preferably 10-50:1, and more preferably 15-25:1.

13. The nucleic acid nanocarrier drug according to any one of claims 1 to 12, wherein the nucleic acid nanoparticle further comprise a bioactive substance, the bioactive substance is linked with the nucleic acid domain, and the bioactive substance is one or more of a target head, a fluorescein, an interfering nucleic acid siRNA, a miRNA, a ribozyme, a riboswitch, an aptamer, a RNA antibody, a protein, a polypeptide, a flavonoid, a glucose, a natural salicylic acid, a monoclonal antibody, a vitamin, phenol, a lecithin, and a small molecular drug except the paclitaxel, lenalidomide, cytarabine, docetaxel, idarubicin and mitoxantrone.

14. The nucleic acid nanocarrier drug according to claim 13, wherein a relative molecular weight of the nucleic acid domain is marked as $N_1$, and a total relative molecular weight of the drug and the bioactive substance is marked as $N_2$, $N_1/N_2 \geq 1:1$.

15. The nucleic acid nanocarrier drug according to claim 13, wherein the bioactive substance is one or more of the target head, the fluorescein and the miRNA,

wherein the target head is positioned on any one sequence of the sequences a, b and c, preferably the 5'-end or the 3'-end of any one sequence of the sequences a, b and c, or inserted between GC bonds of the nucleic acid domain,
the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and
preferably, the target head is a folic acid or a biotin, the fluorescein is any one or more of FAM, CY5 and CY3, and the anti-miRNA is anti-miR-21.

**16.** The nucleic acid nanocarrier drug according to claim 13, wherein the small molecular drug except the paclitaxel, lenalidomide, cytarabine, docetaxel, idarubicin and mitoxantrone is a drug containing any one or more of the following groups: an amino group, a hydroxyl group, a carboxyl group, a mercapto group, a benzene ring group and an acetamido group.

**17.** The nucleic acid nanocarrier drug according to claim 13, wherein the protein is one or more of SOD, survivin, hTERT, EGFR and PSMA; the vitamin is L-Vc and/or esterified Vc; and the phenols are tea polyphenols and/or grape polyphenols.

**18.** The nucleic acid nanocarrier drug according to claim 1, wherein a particle size of the nucleic acid nanoparticle is 1-100 nm, preferably 5-50 nm; more preferably 10-30 nm; and further preferably 10-15 nm.

**19.** A method for preparing a nucleic acid nanocarrier drug, wherein the preparation method comprises the following steps:

providing nucleic acid nanoparticle in the nucleic acid nanocarrier drug according to any one of claims 1 to 18; and loading the drug on the nucleic acid nanoparticle in modes of physical linkage and/or covalent linkage, to obtain the nucleic acid nanocarrier drug.

**20.** The method according to claim 19, wherein the step of loading the drug in the physical linkage mode comprises:

mixing and stirring the drug, the nucleic acid nanoparticle and a first solvent to obtain a premixed system; and precipitating the premixed system, to obtain the nucleic acid nanocarrier drug;
preferably, the first solvent is selected from one or more of DCM, DCC, DMAP, Py, DMSO, PBS and glacial acetic acid;
preferably, the step of precipitating the premixed system, to obtain the nucleic acid nanocarrier drug comprises:

precipitating the premixed system, to obtain a precipitation; and
washing the precipitation to remove impurities, as to obtain the nucleic acid nanocarrier drug;
more preferably, mixing the premixed system with absolute ethyl alcohol, and precipitating at a temperature condition lower than 10 DEG C, to obtain the precipitation; and further preferably, precipitating at a temperature condition of 0-5 DEG C; and
more preferably, washing the precipitation to remove the impurities with 6-12 times of the absolute ethyl alcohol in volume, as to obtain the nucleic acid nanocarrier drug.

**21.** The method according to claim 19, wherein the step of loading the drug in the covalent linkage mode comprises:

preparing a drug solution;
enabling the drug solution to react with the G-exocyclic amino of the nucleic acid nanoparticle under a mediating effect of the formaldehyde, to obtain a reaction system; and purifying the reaction system, to obtain the nucleic acid nanocarrier drug;
preferably, the reaction step comprises:
mixing the drug solution with paraformaldehyde solution and the nucleic acid nanoparticle, and reacting in a dark condition, to obtain the reaction system; wherein the concentration of the paraformaldehyde solution is preferably 3.7-4wt%, and the paraformaldehyde solution is preferably a solution formed by mixing paraformaldehyde and a second solvent, and the second solvent is one or more of DCM, DCC, DMAP, Py, DMSO, PBS and glacial acetic acid.

**22.** The method according to any one of claims 19 to 21, wherein the preparation method further comprises a step of preparing the nucleic acid nanoparticle, the step comprises: self-assembling a single strand corresponding to the nucleic acid domain in the nucleic acid nanocarrier drug according to any one of claims 1 to 18, to obtain the nucleic acid domain;
preferably, after the nucleic acid domain is obtained, the preparation method further comprises: loading the bioactive substance in the drug according to any one of claims 13 to 17 on the nucleic acid domain in the modes of physical linkage and/or covalent linkage, to obtain the nucleic acid nanoparticle.

**23.** The method according to claim 22, wherein in a process of loading the bioactive substance in the covalent linkage mode, the loading is performed through solvent covalent linkage, linker covalent linkage or click-linkage;

preferably, a third solvent used in the solvent covalent linkage is served as a linkage medium, and the third solvent is selected from one or more of paraformaldehyde, DCM, DCC, DMAP, Py, DMSO, PBS and glacial acetic acid;
preferably, the linker is selected from a disulfide bond, a p-phenylazide, bromopropyne or PEG; and
preferably, the click-linkage is that a bioactive substance precursor and the nucleic acid domain are modified by alkynyl or azide modification simultaneously, and then linked through a click reactione.

**24.** The method according to claim 23, wherein the bioactive substance is linked with the nucleic acid domain in the click-linkage mode, a site, for performing the alkynyl or azide modification, of the bioactive substance precursor is selected from a 2'-hydroxyl, a carboxyl or an amino, and a site, for performing the alkynyl or azide modification, of the nucleic acid domain is selected from a G-exocyclic amino, a 2'-hydroxyl, an $\alpha$-amino or a 2'-hydroxyl.

**25.** A nucleic acid nanocarrier pharmaceutical composition, wherein the nucleic acid nanocarrier pharmaceutical composition comprises the nucleic acid nanocarrier drug according to any one of claims 1 to 18.

**26.** Use of the nucleic acid nanocarrier drug according to any one of claims 1 to 18 in preparing a drug for treating a tumor.

**27.** The use according to claim 26, wherein when the drug is the paclitaxel, the tumor is breast cancer or ovarian cancer;

when the drug is the lenalidomide, the tumor is acute leukemia or multiple myeloma;
when the drug is the cytarabine, the tumor is any one or more of acute leukemia, malignant lymphoma, lung cancer, digestive tract cancer, colorectal cancer, and head and neck cancer;
when the drug is the docetaxel, the tumor is any one or more of breast cancer, ovarian cancer, non-small cell lung cancer, head and neck cancer, pancreatic cancer, small cell lung cancer, gastric cancer, melanoma, and soft tissue sarcoma;
when the drug is the idarubicin, the tumor is any one or more of acute pulmonary lymphocytic leukemia, advanced breast cancer and non-Hodgkin's lymphoma; and
when the drug is the mitoxantrone, the tumor is any one or more of breast cancer, malignant lymphoma, gastric cancer, bowel cancer, leukemia, bladder cancer, liver cancer, multiple myeloma, malignant mesothelioma, and ovarian cancer.

**28.** Use of the nucleic acid nanocarrier drug according to any one of claims 1 to 18 in preparing a drug for treating viral keratitis and epidemic conjunctivitis, wherein the drug comprised in the nucleic acid nanocarrier drug is the cytarabine.

**29.** Use of the nucleic acid nanocarrier drug according to any one of claims 1 to 18 in preparing a drug for treating myelodysplastic syndrome, wherein the drug comprised in the nucleic acid nanocarrier drug is the idarubicin.

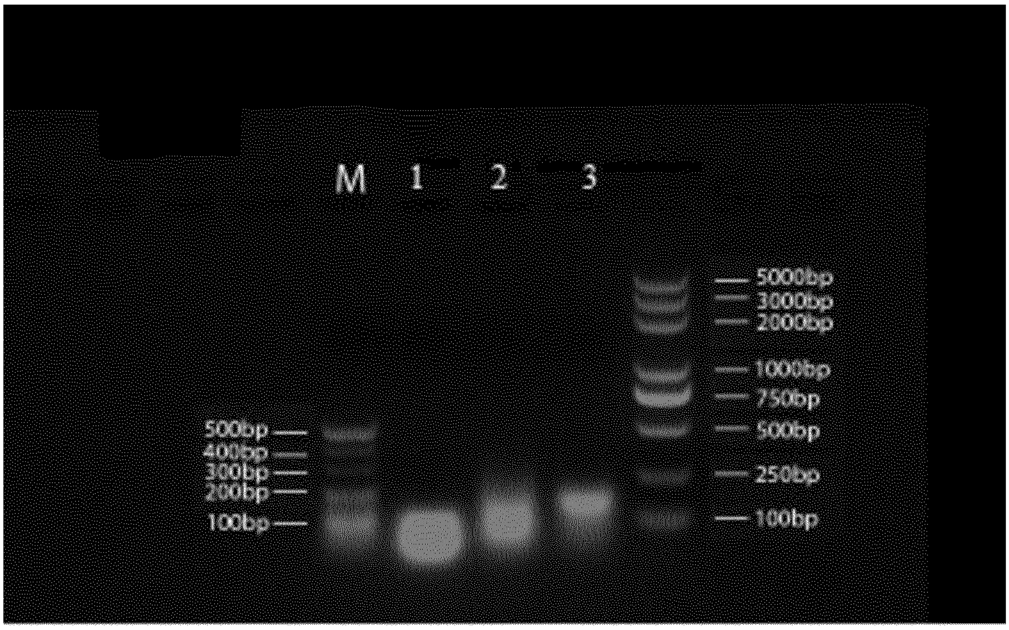
M
1
2
3
500bp
400bp
300bp
200bp
100bp
5000bp
3000bp
2000bp
1000bp
750bp
500bp
250bp
100bp

Fig. 1

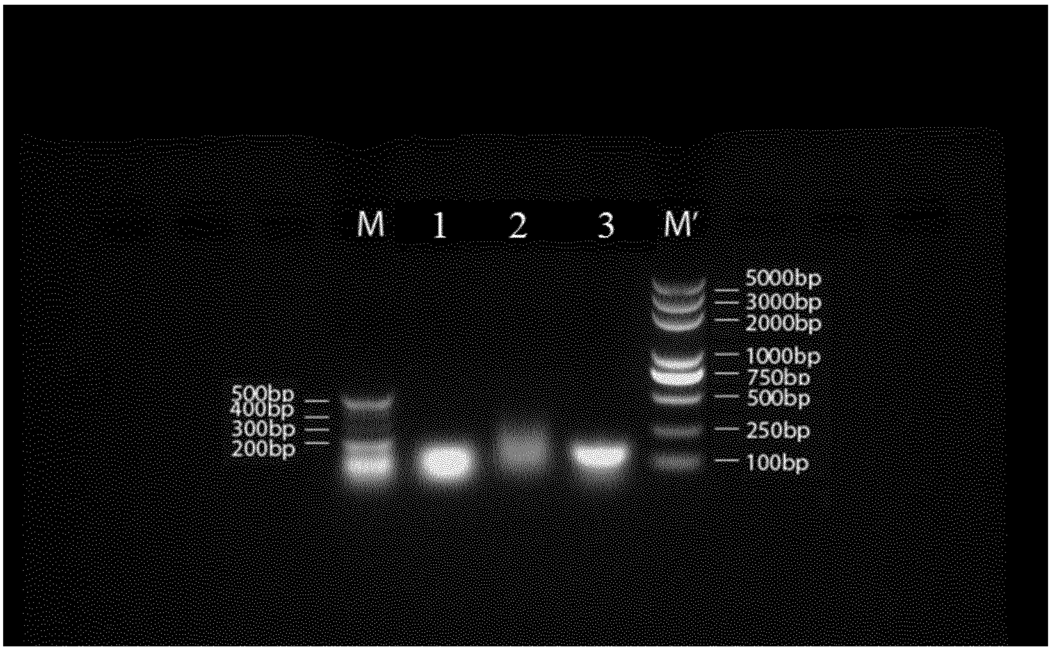
M
1
2
3
M'
500bp
400bp
300bp
200bp
5000bp
3000bp
2000bp
1000bp
750bp
500bp
250bp
100bp

Fig. 2

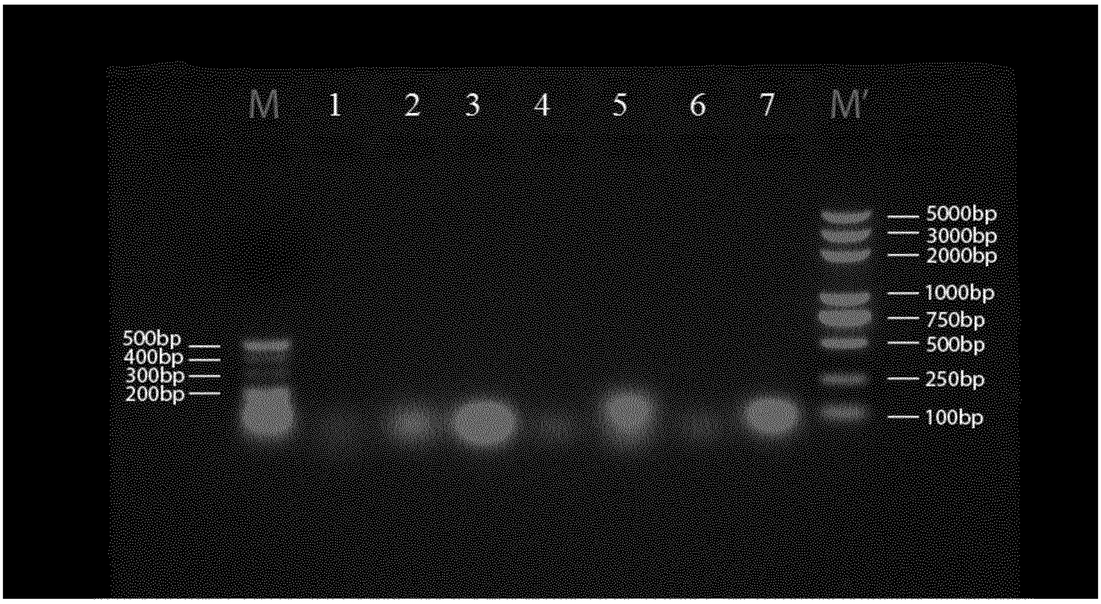
M
1
2
3
4
5
6
7
M′
500bp
400bp
300bp
200bp
5000bp
3000bp
2000bp
1000bp
750bp
500bp
250bp
100bp

Fig. 3

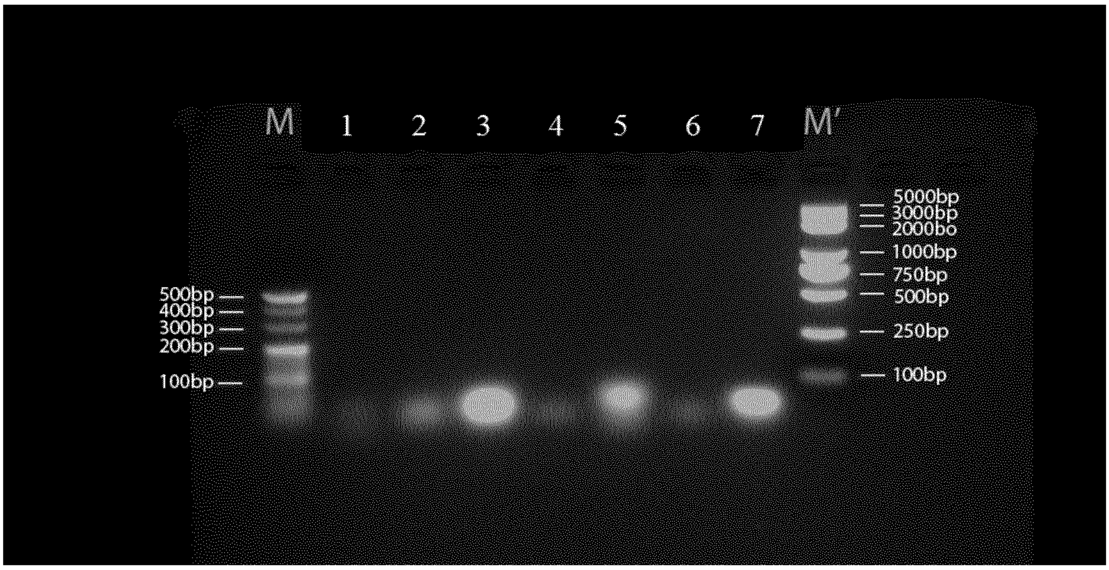
M
1
2
3
4
5
6
7
M′
500bp
400bp
300bp
200bp
100bp
5000bp
3000bp
2000bo
1000bp
750bp
500bp
250bp
100bp

Fig. 4

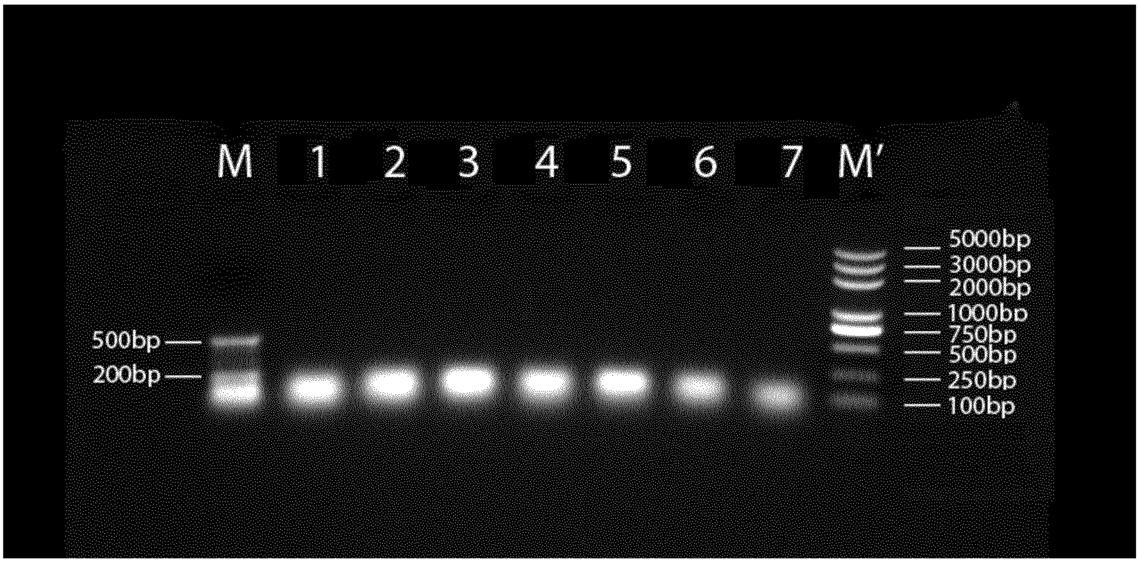
M 1 2 3 4 5 6 7 M'
500bp
200bp
5000bp
3000bp
2000bp
1000bp
750bp
500bp
250bp
100bp

Fig. 5

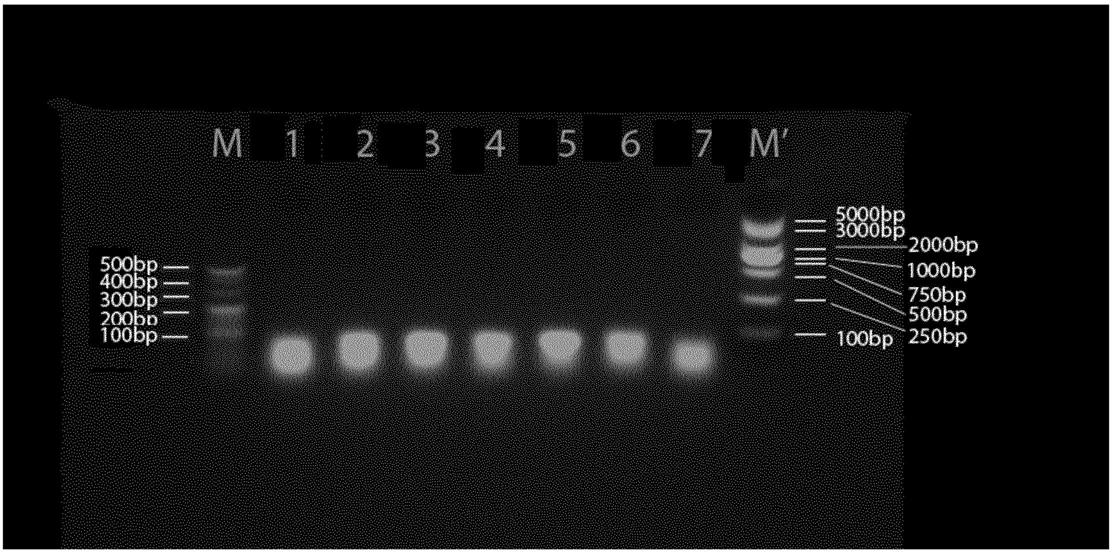
M 1 2 3 4 5 6 7 M'
500bp
400bp
300bp
200bp
100bp
5000bp
3000bp
2000bp
1000bp
750bp
500bp
250bp
100bp

Fig. 6

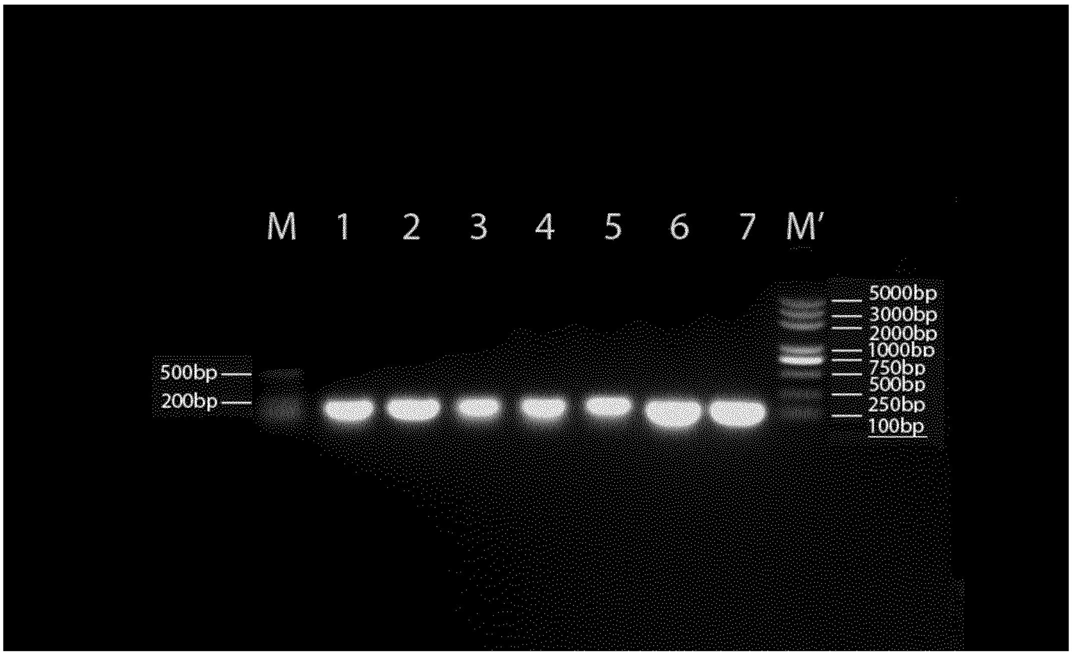
M 1 2 3 4 5 6 7 M'
500bp
200bp
5000bp
3000bp
2000bp
1000bp
750bp
500bp
250bp
100bp

Fig. 7

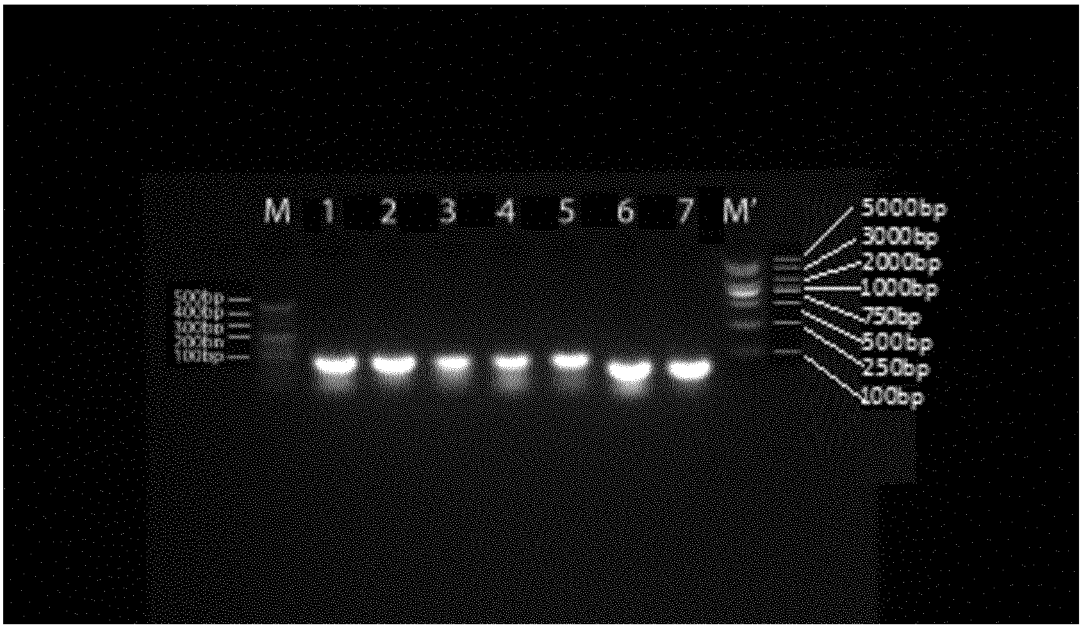
M 1 2 3 4 5 6 7 M'
5000bp
3000bp
2000bp
1000bp
750bp
500bp
250bp
100bp

Fig. 8

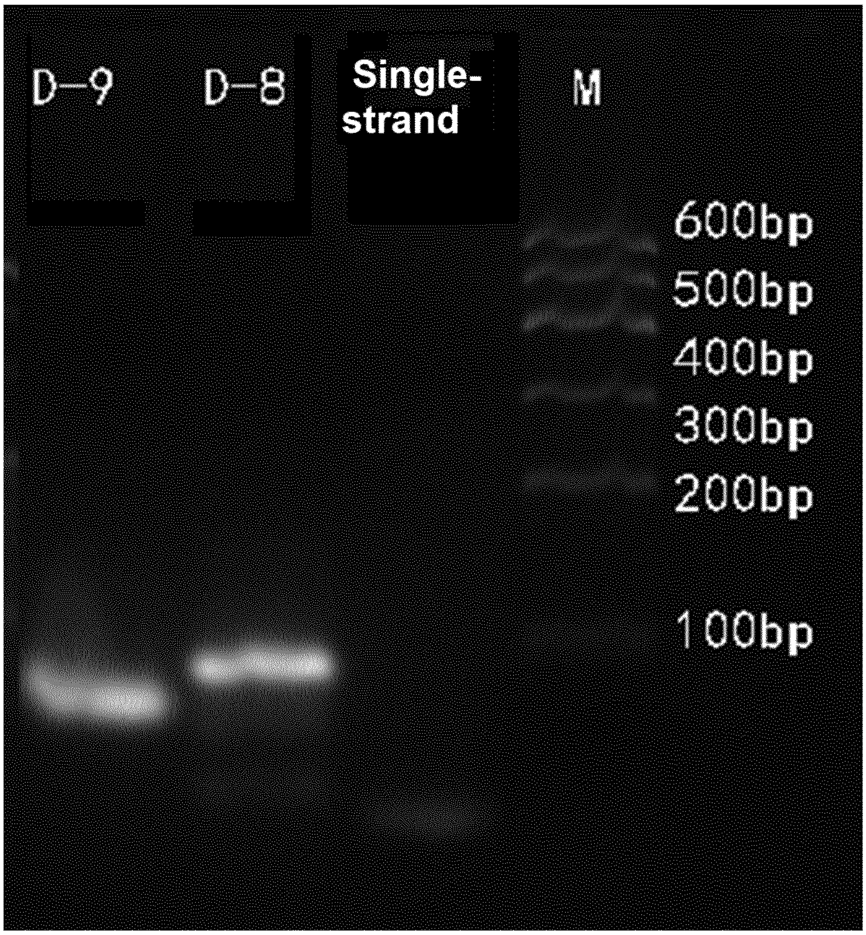


Fig. 9

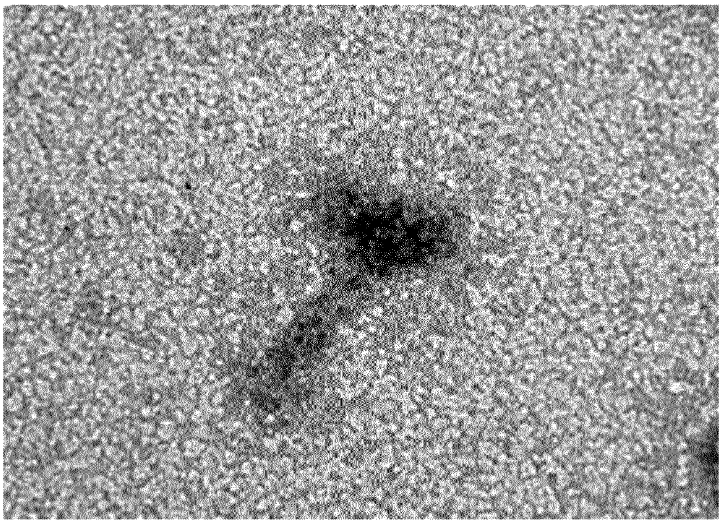

Fig. 10

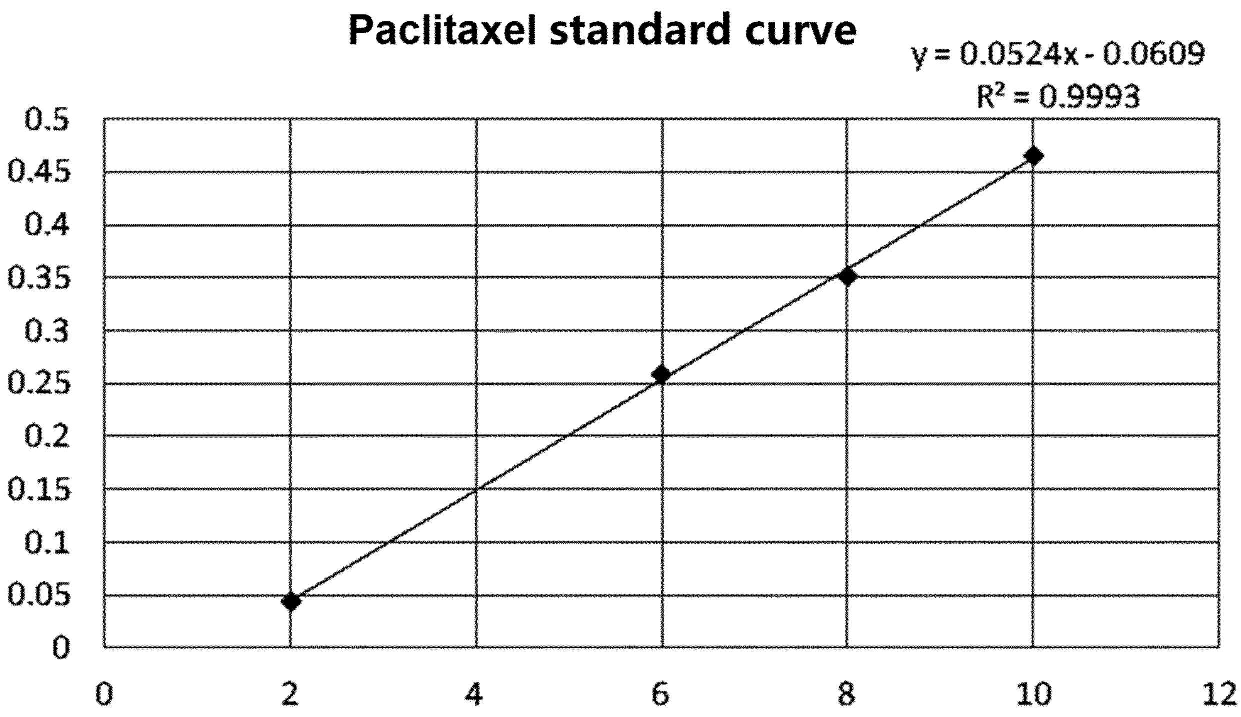


Fig. 11a

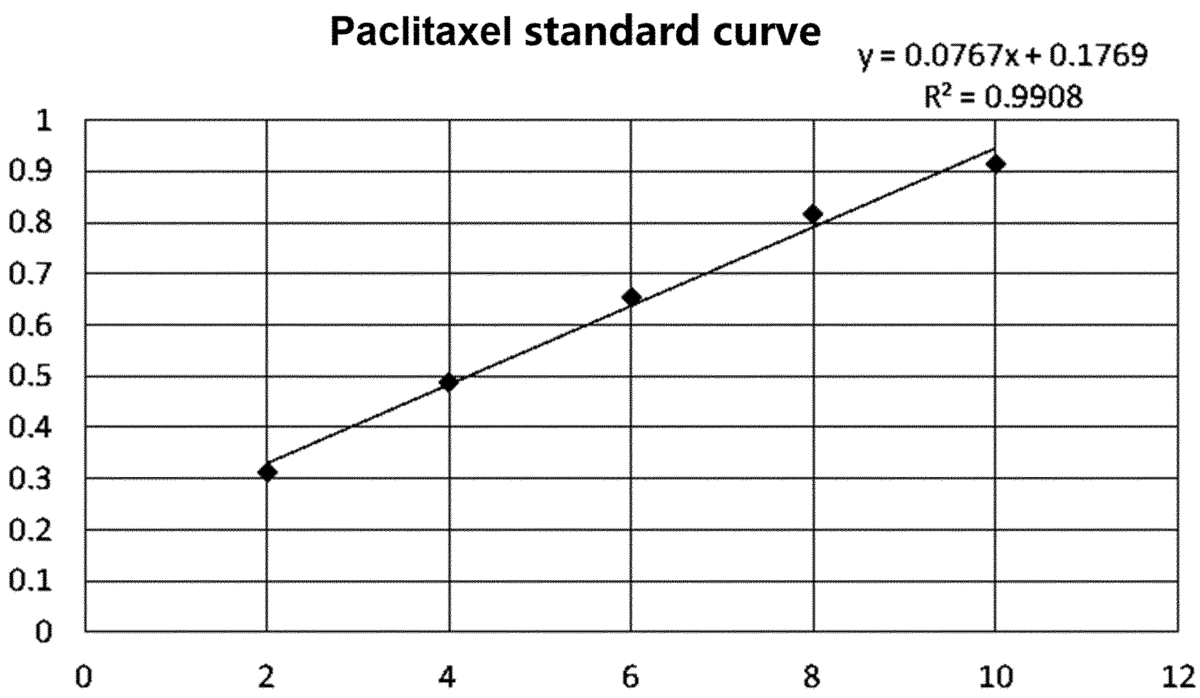


Fig. 11b

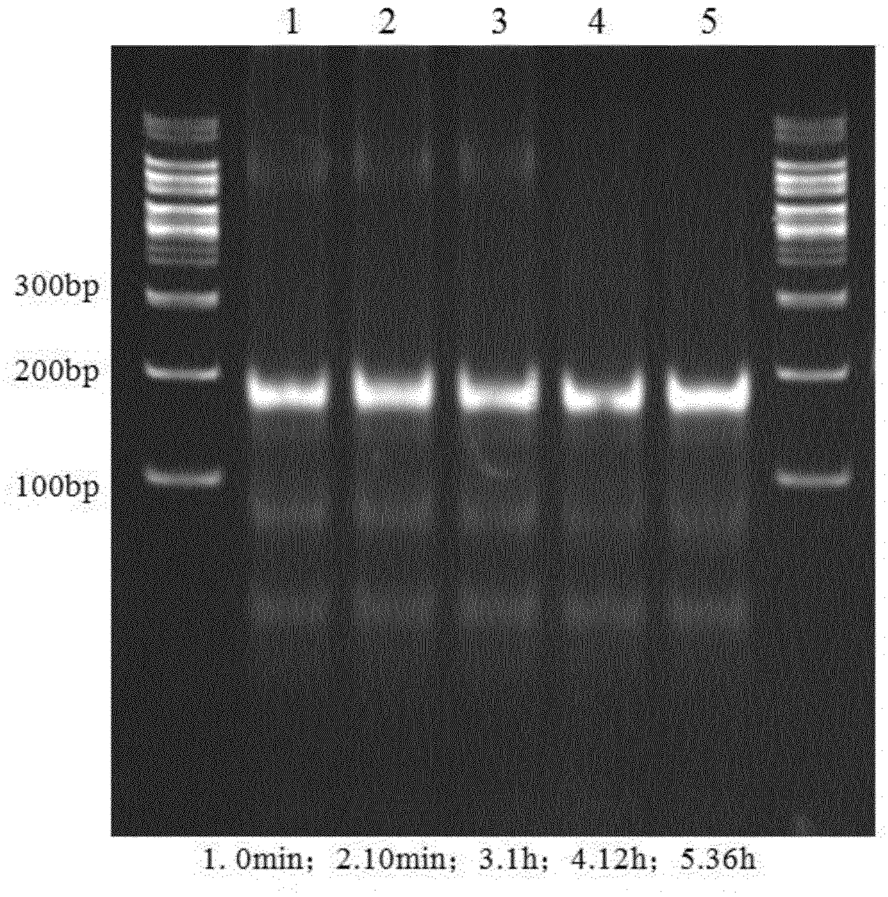
1
2
3
4
5
300bp
200bp
100bp
1. 0min; 2.10min; 3.1h; 4.12h; 5.36h

Fig. 12

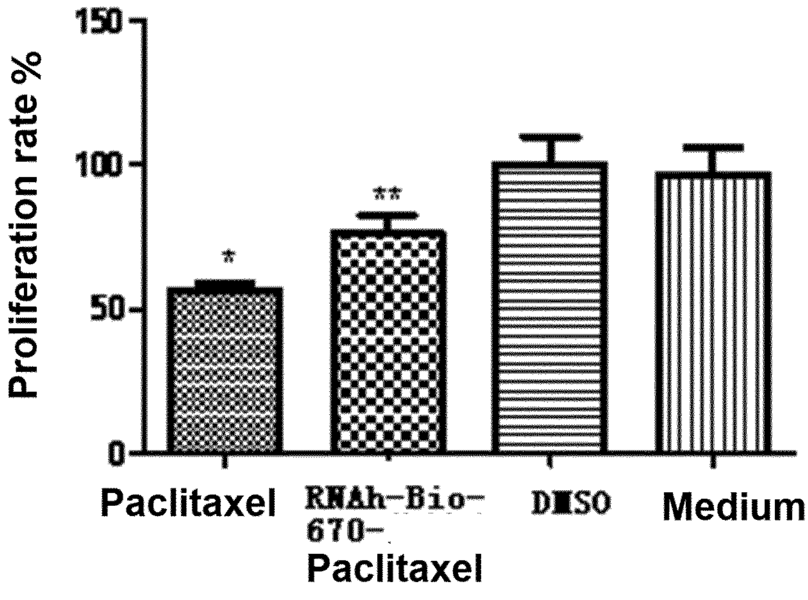
150
100
50
0
Proliferation rate %
*
**
Paclitaxel
RNAh-Bio-670-Paclitaxel
DMSO
Medium

Fig. 13

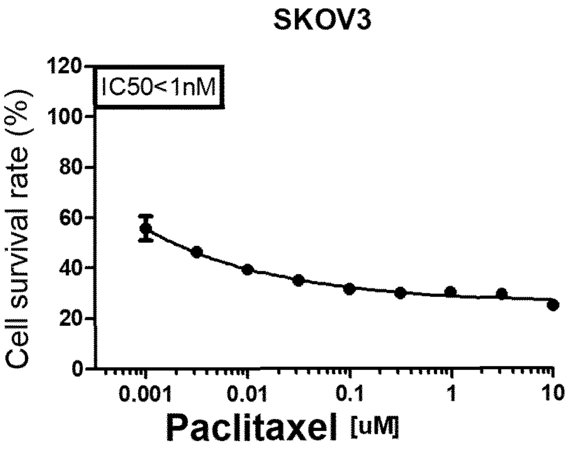


Fig. 14a

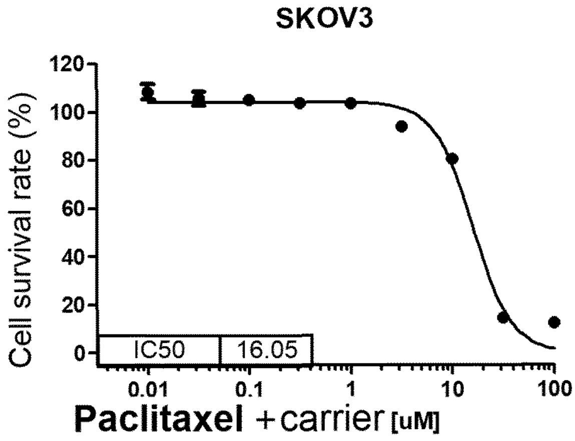


Fig. 14b

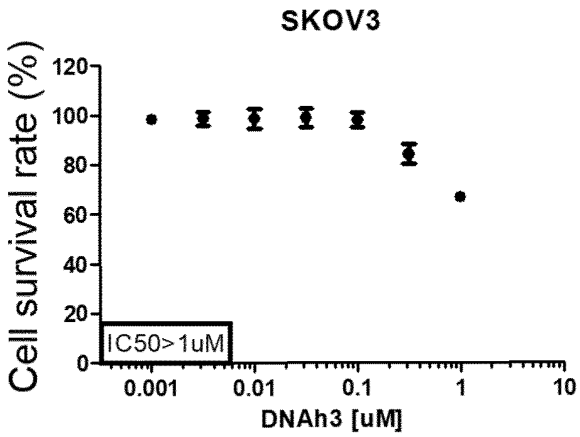


Fig. 14c

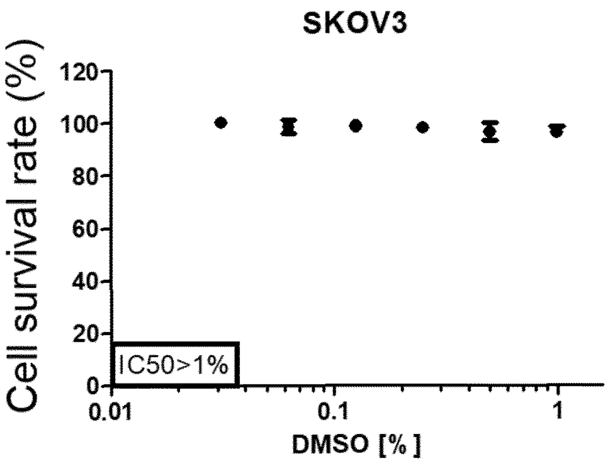


Fig. 14d

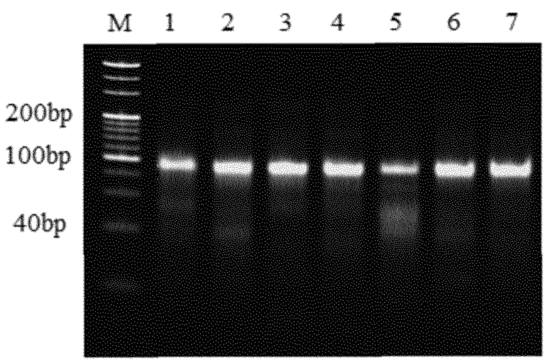

Fig. 15

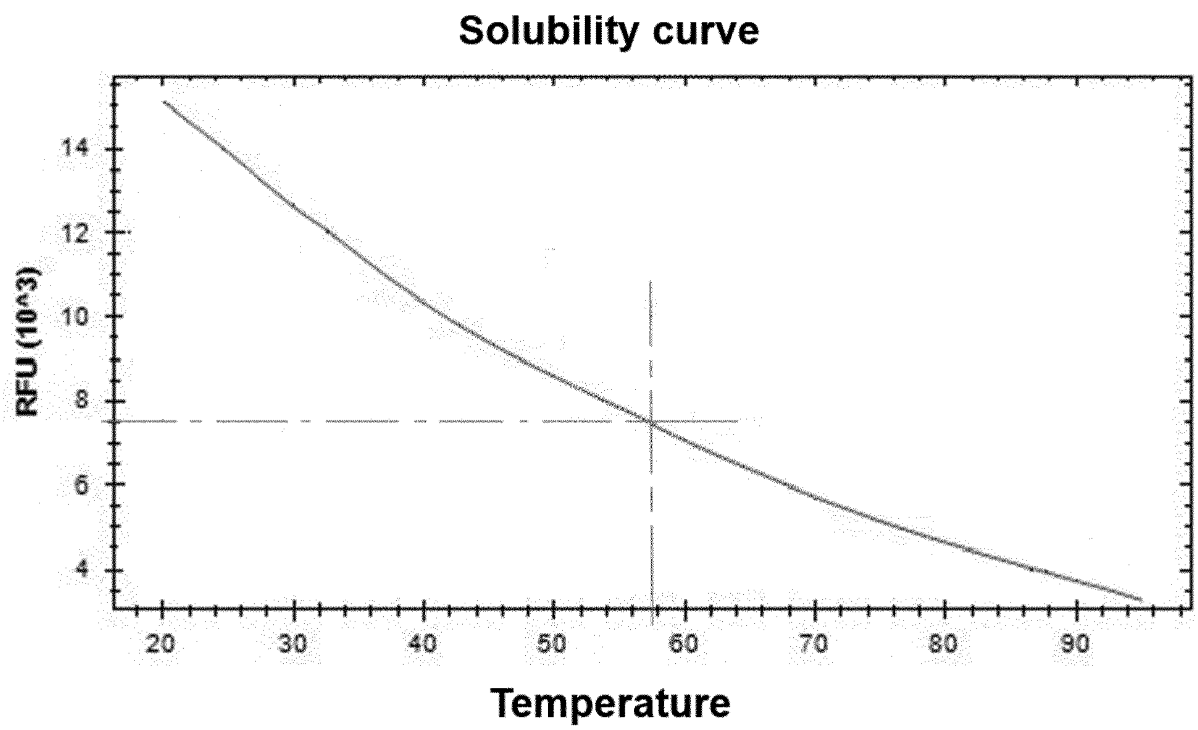
Solubility curve
RFU (10^3)
14
12
10
8
6
4
20
30
40
50
60
70
80
90
Temperature

Fig. 16

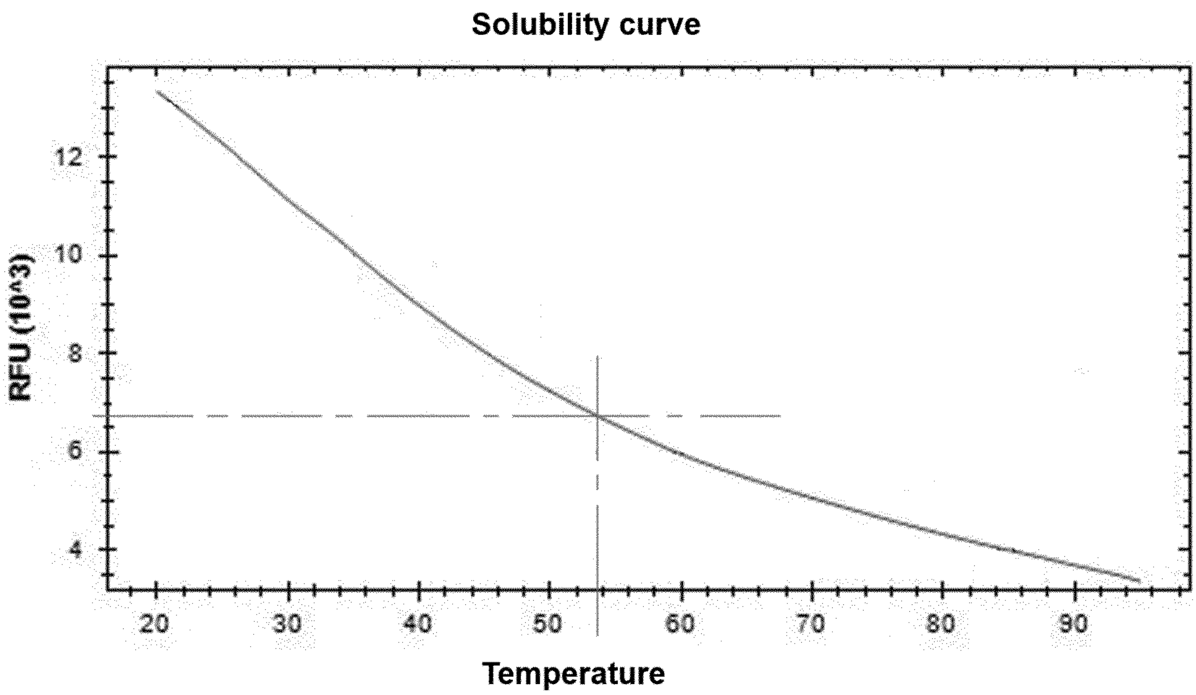
Solubility curve
RFU (10^3)
12
10
8
6
4
20
30
40
50
60
70
80
90
Temperature

Fig. 17

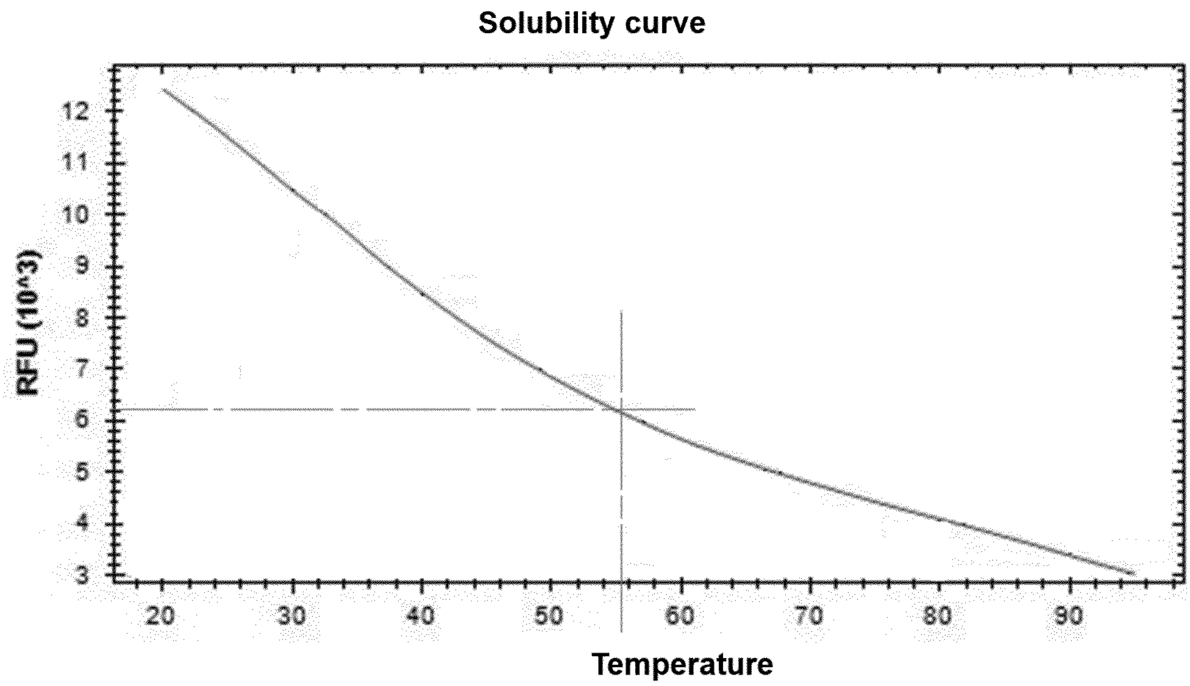


Fig. 18

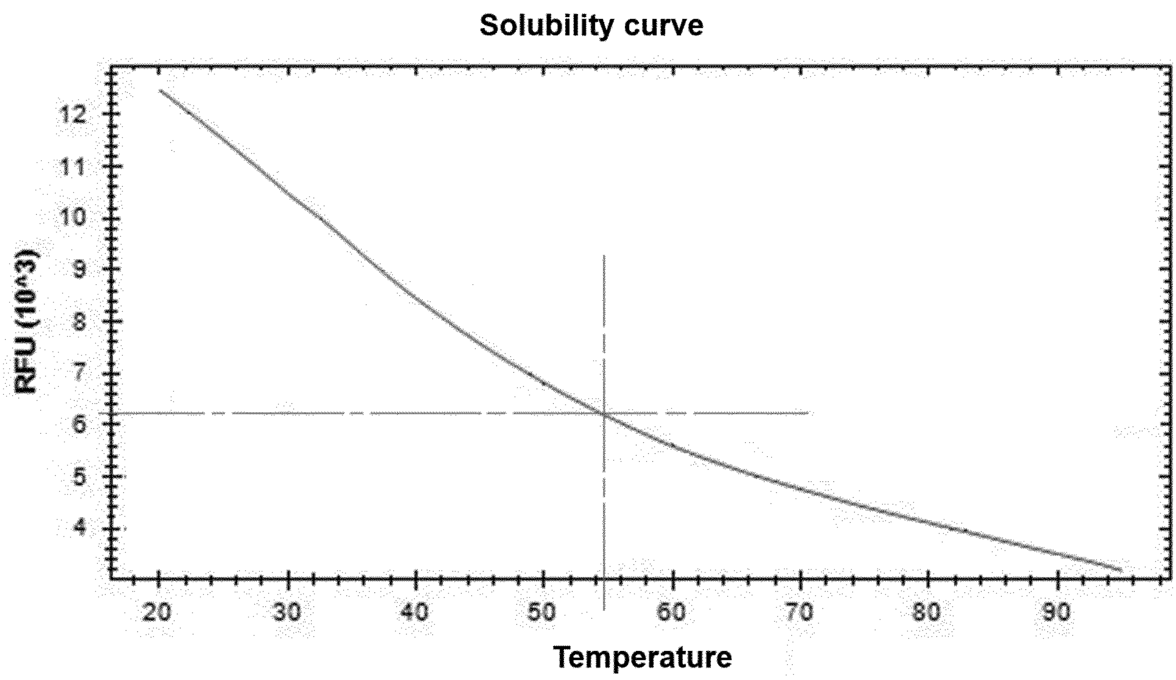


Fig. 19

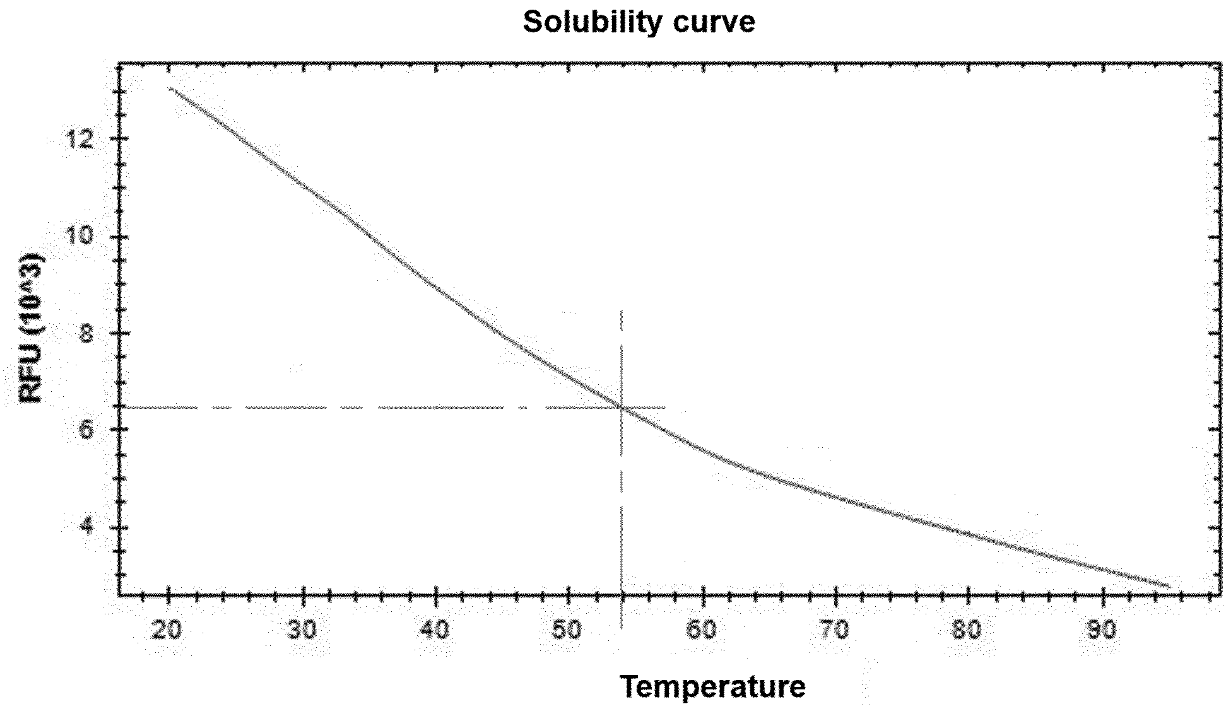
Solubility curve
RFU (10^3)
12
10
8
6
4
20
30
40
50
60
70
80
90
Temperature

Fig. 20

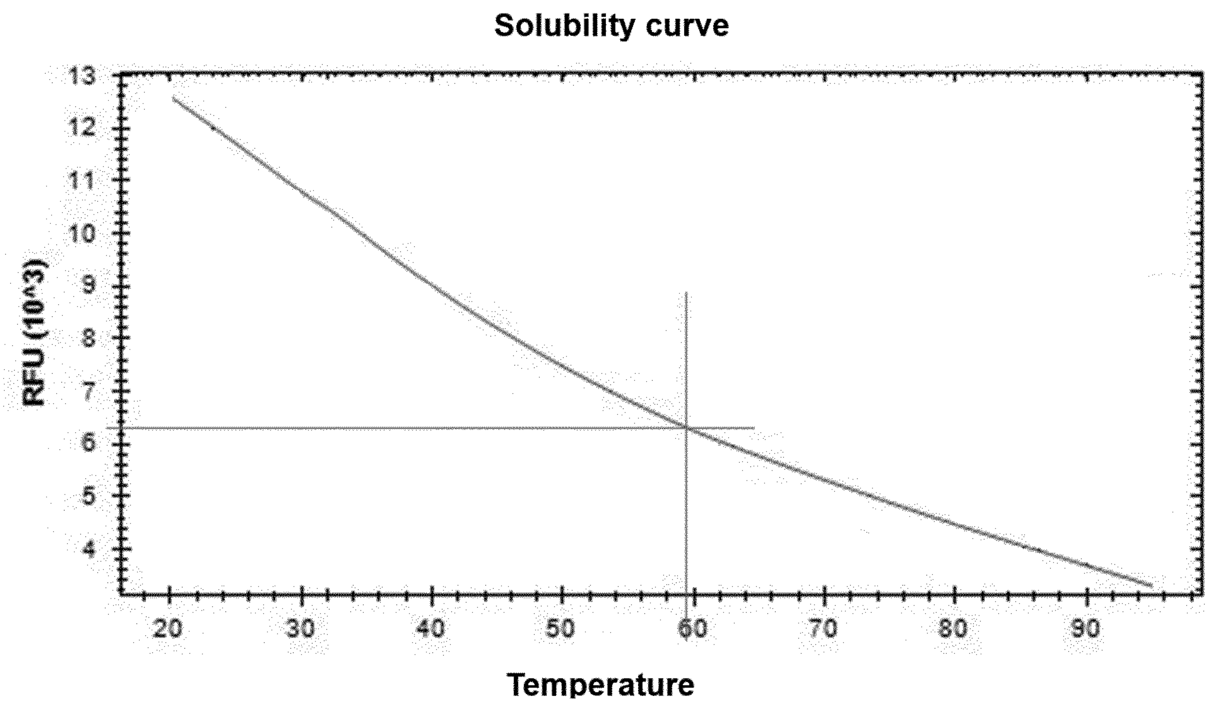
Solubility curve
RFU (10^3)
13
12
11
10
9
8
7
6
5
4
20
30
40
50
60
70
80
90
Temperature

Fig. 21

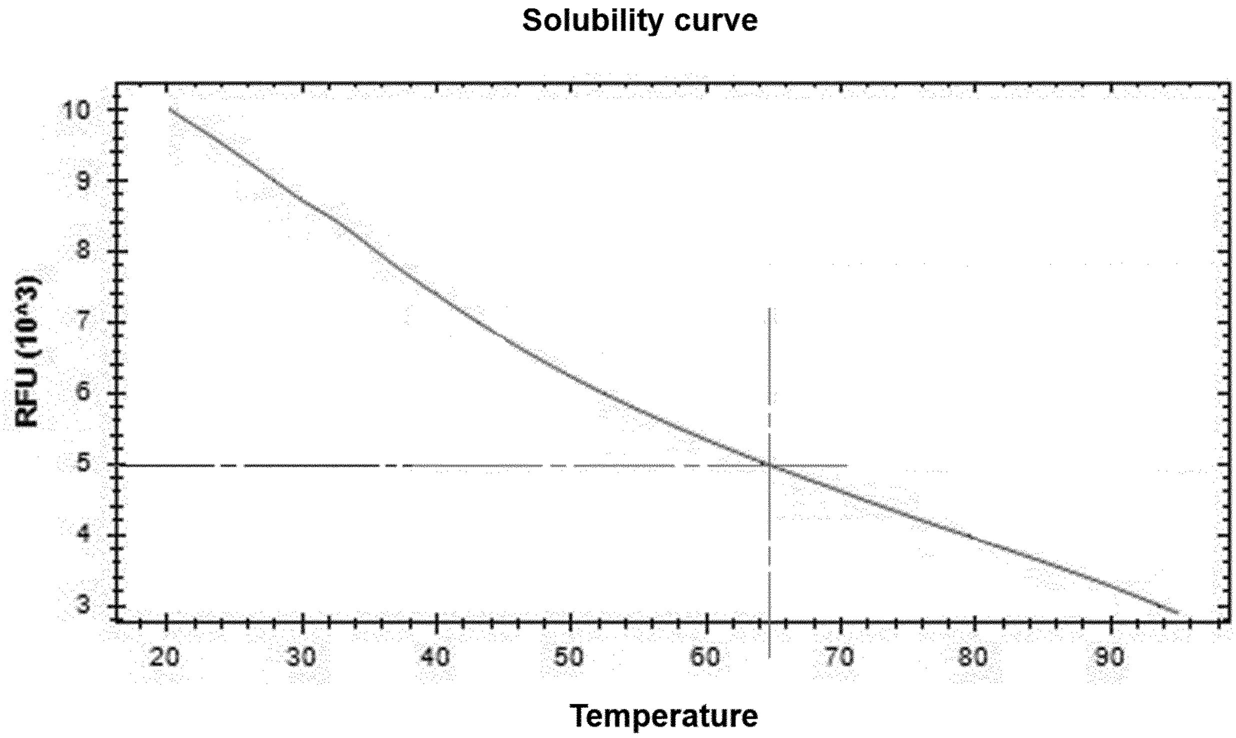
Solubility curve
RFU (10^3)
10
9
8
7
6
5
4
3
20
30
40
50
60
70
80
90
Temperature

Fig. 22

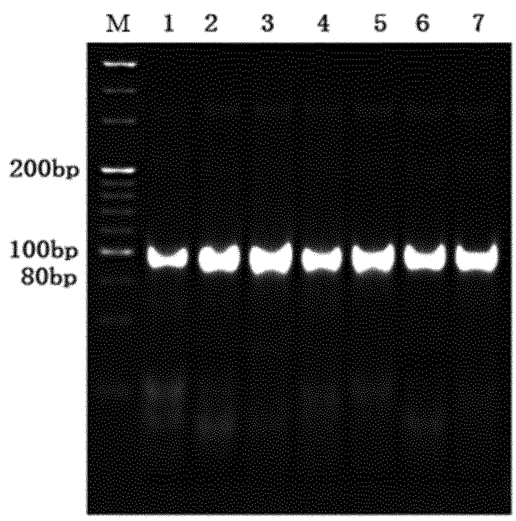
M 1 2 3 4 5 6 7
200bp
100bp
80bp

Fig. 23

Solubility curve

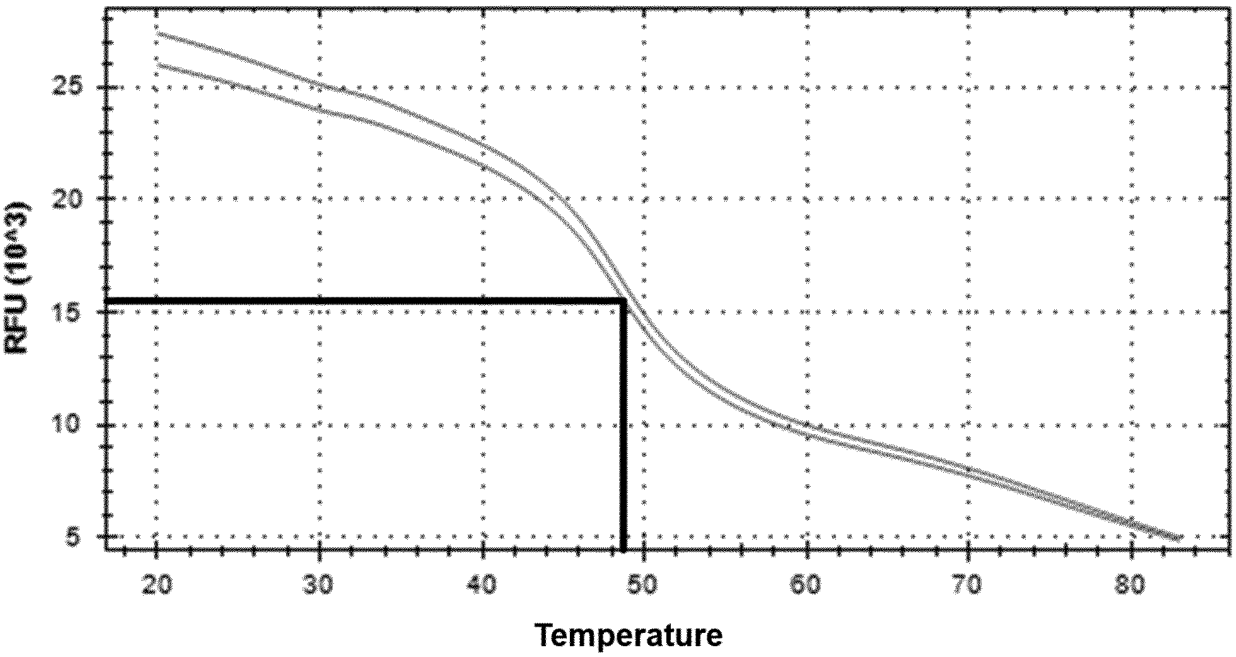


Fig. 24

Solubility curve

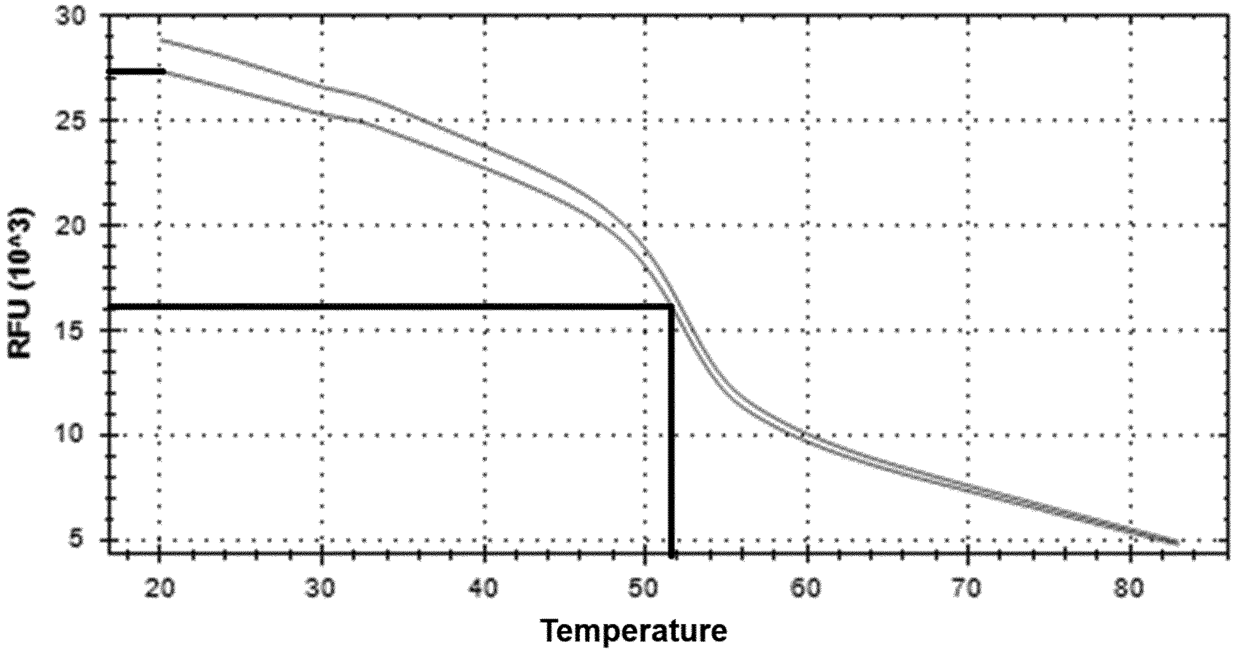


Fig. 25

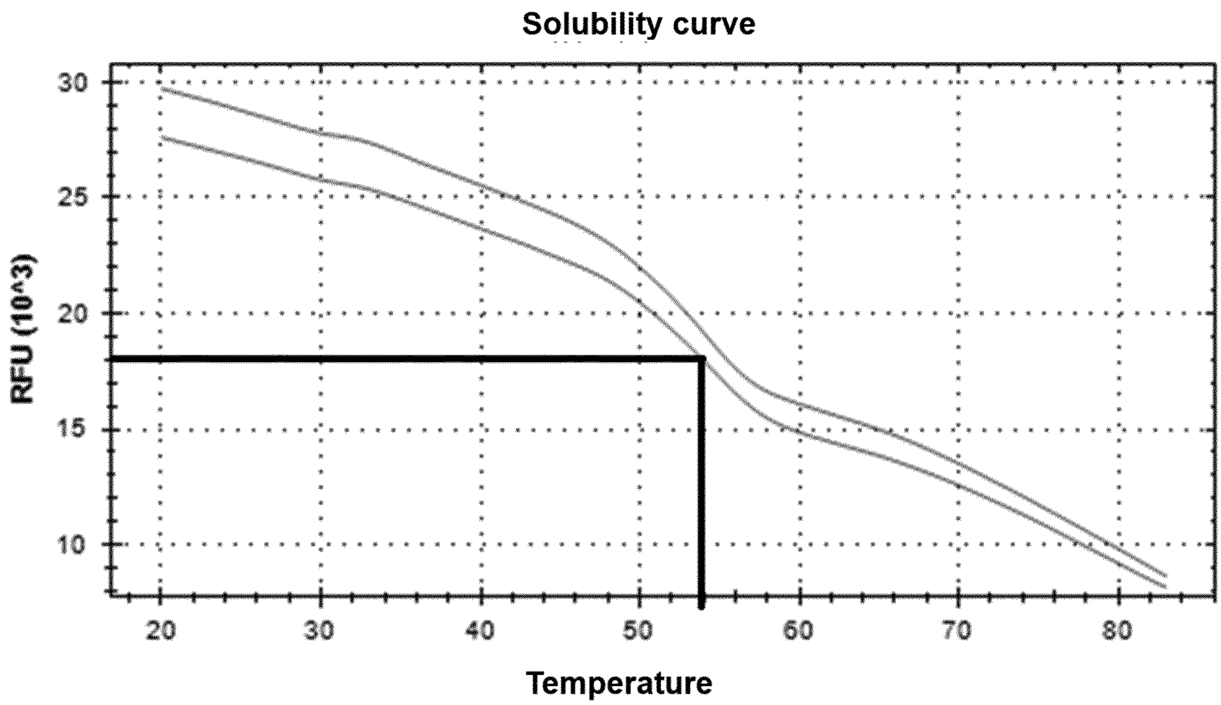
Solubility curve
RFU (10^3)
30
25
20
15
10
20
30
40
50
60
70
80
Temperature

Fig. 26

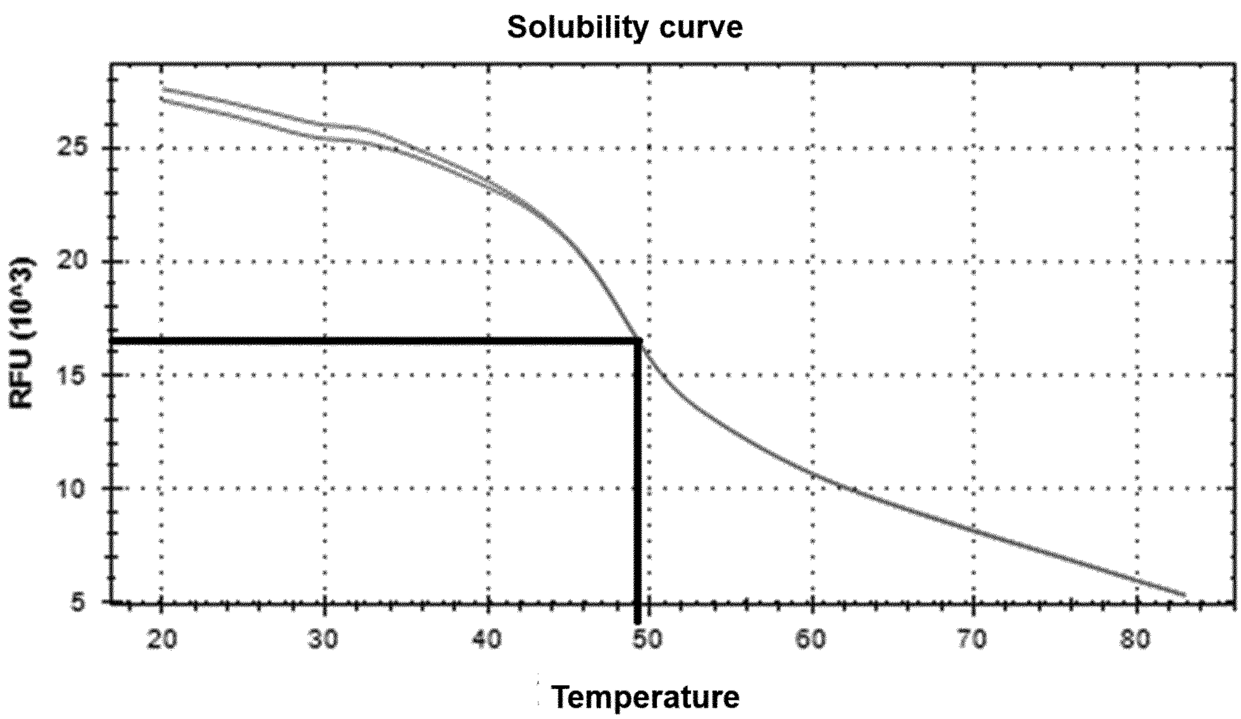
Solubility curve
RFU (10^3)
25
20
15
10
5
20
30
40
50
60
70
80
Temperature

Fig. 27

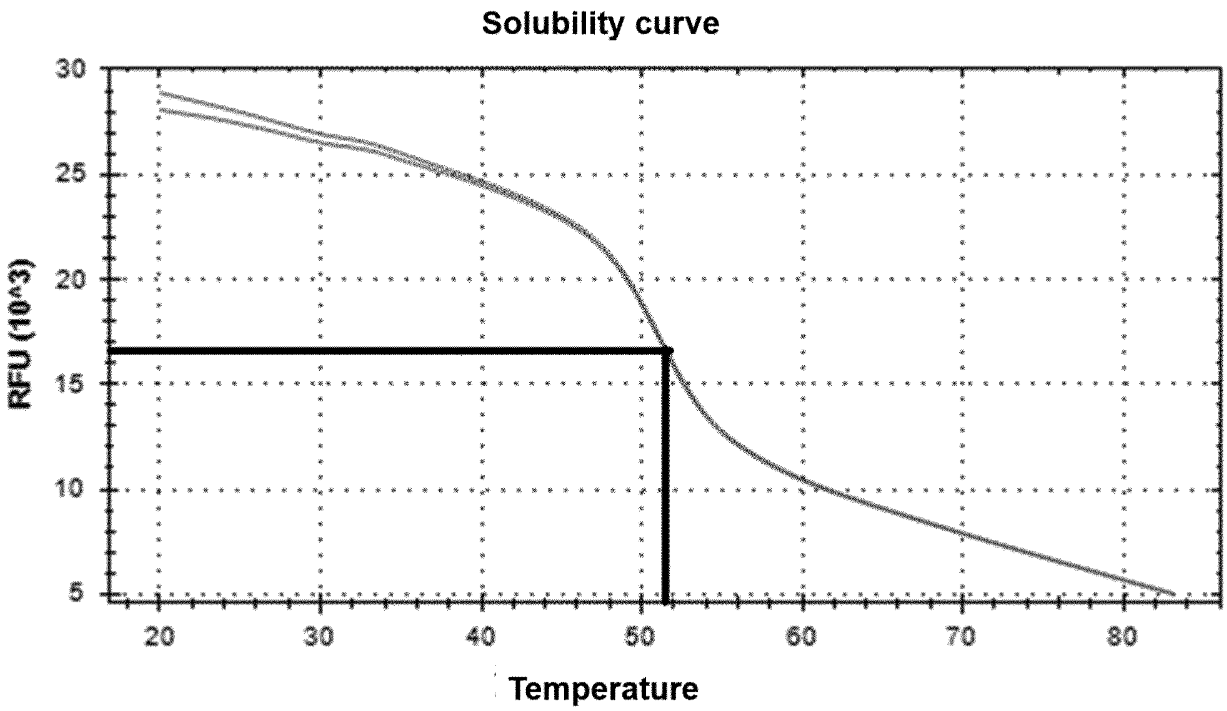
Solubility curve
30
25
20
15
10
5
RFU (10^3)
20
30
40
50
60
70
80
Temperature

Fig. 28

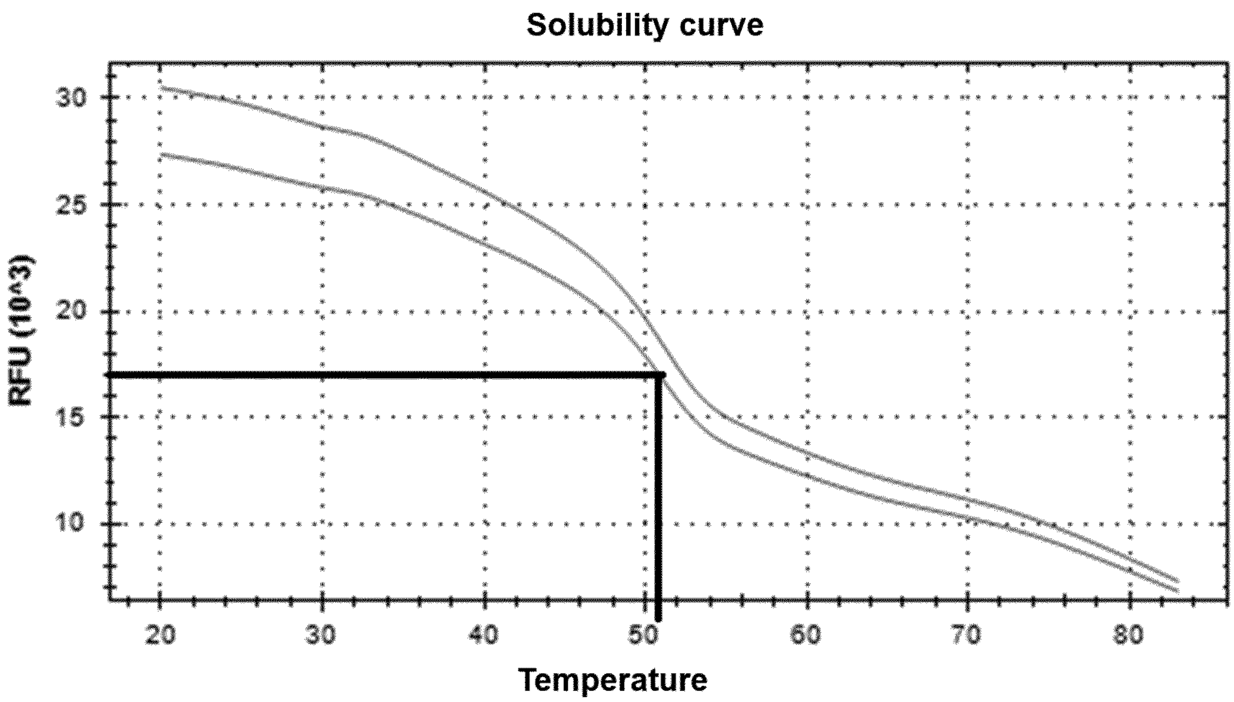
Solubility curve
30
25
20
15
10
RFU (10^3)
20
30
40
50
60
70
80
Temperature

Fig. 29

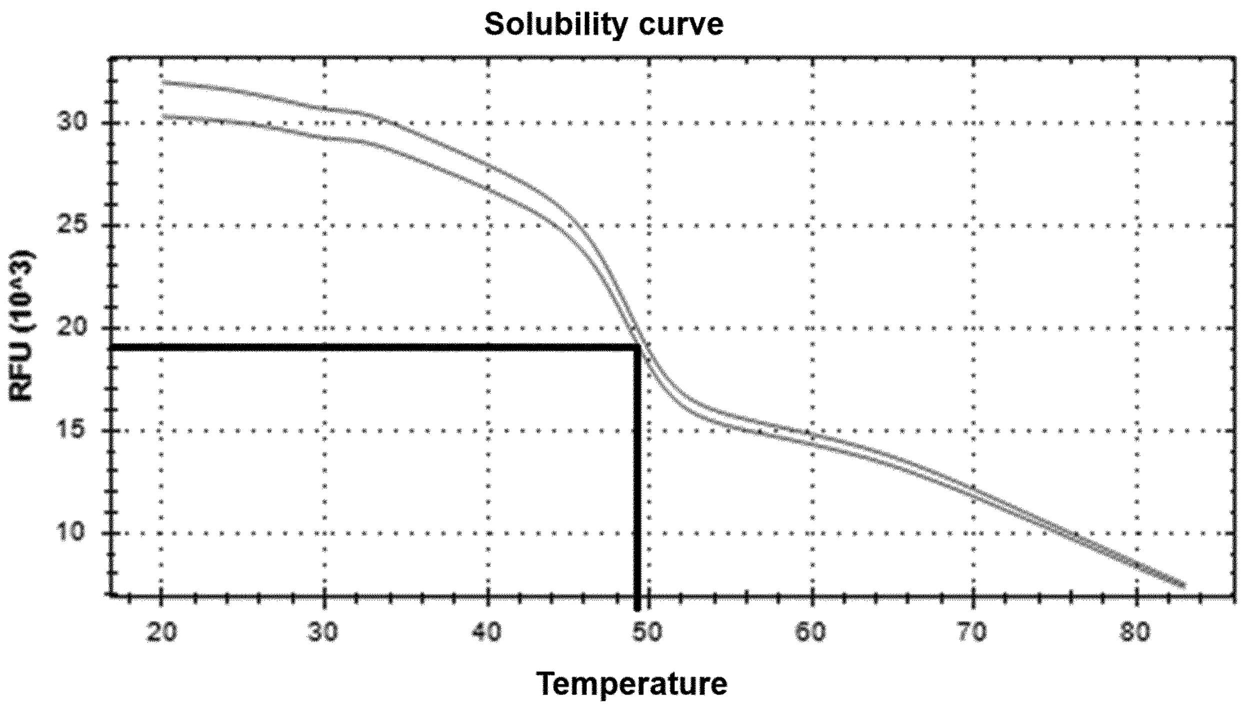
Solubility curve
RFU (10^3)
30
25
20
15
10
20
30
40
50
60
70
80
Temperature

Fig. 30

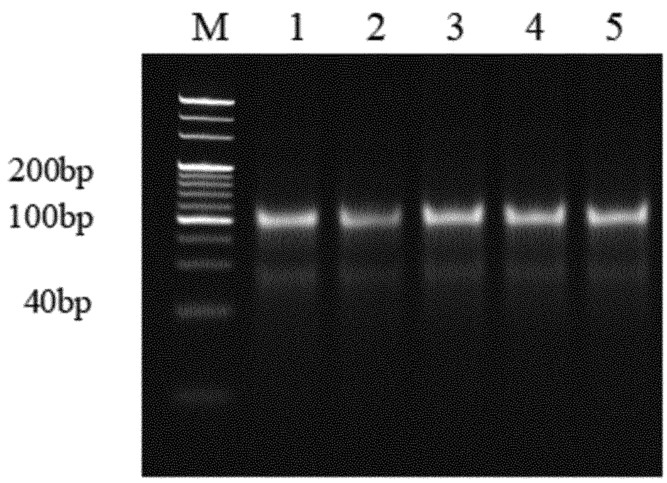
M 1 2 3 4 5
200bp
100bp
40bp

Fig. 31

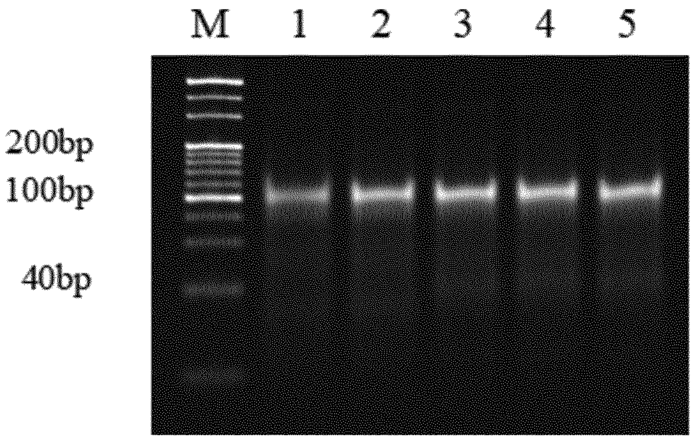


Fig. 32

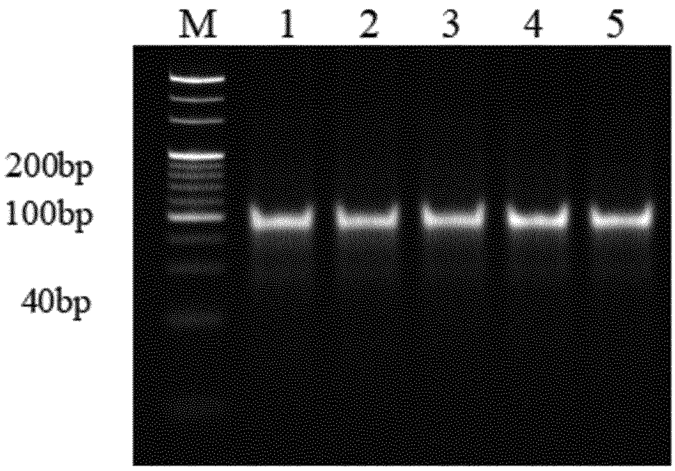


Fig. 33

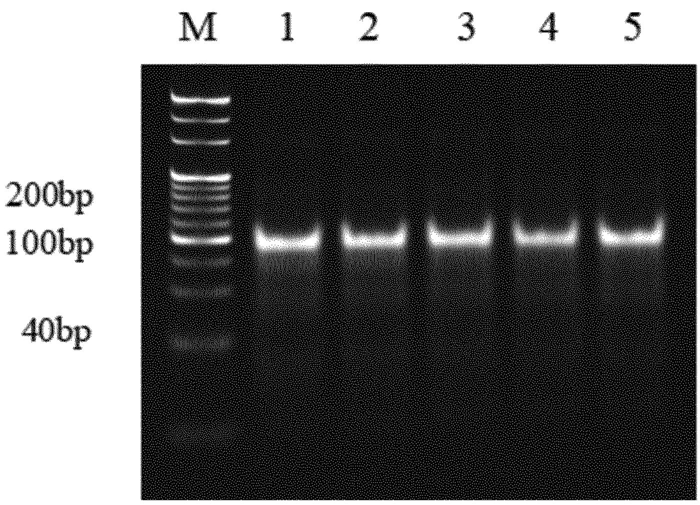


Fig. 34

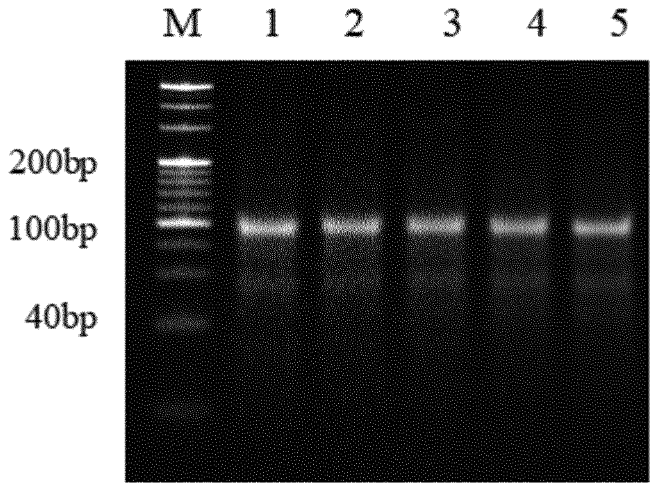


Fig. 35

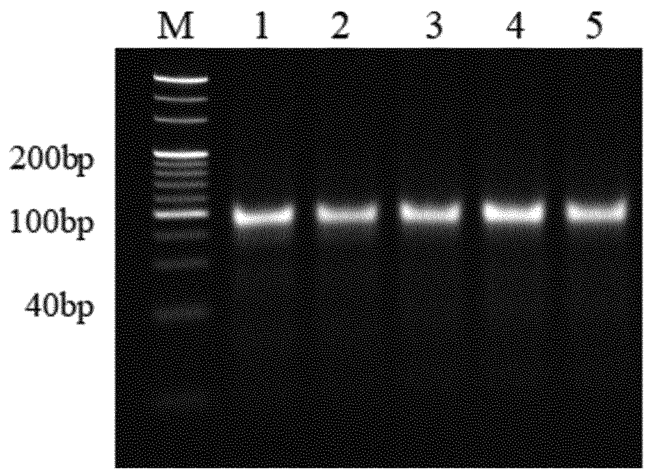


Fig. 36

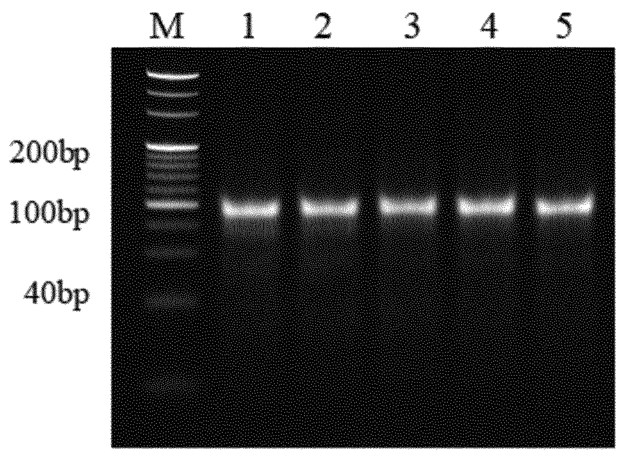


Fig. 37

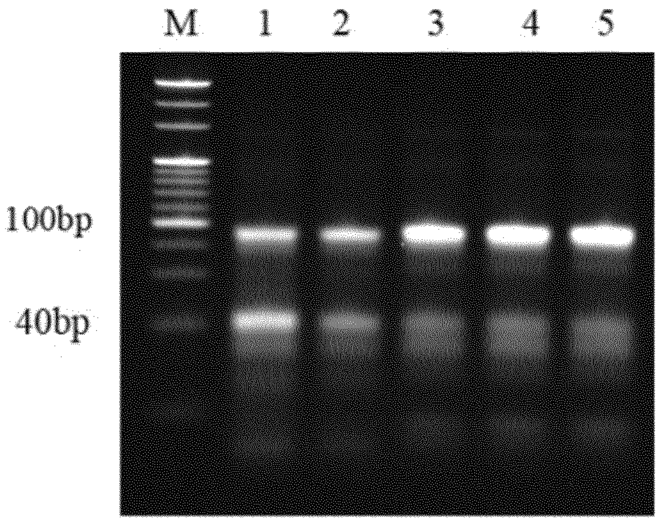


Fig. 38

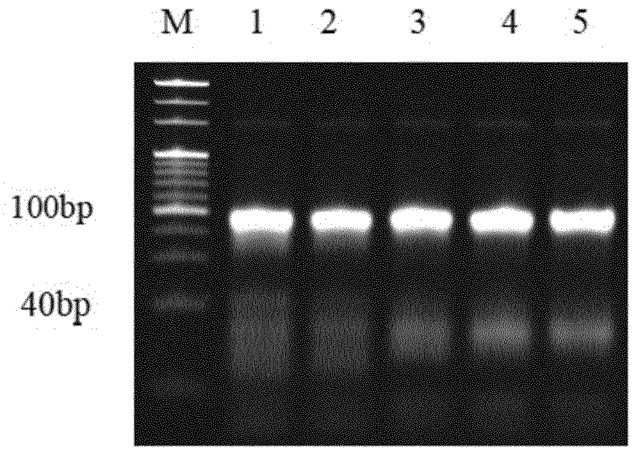


Fig. 39

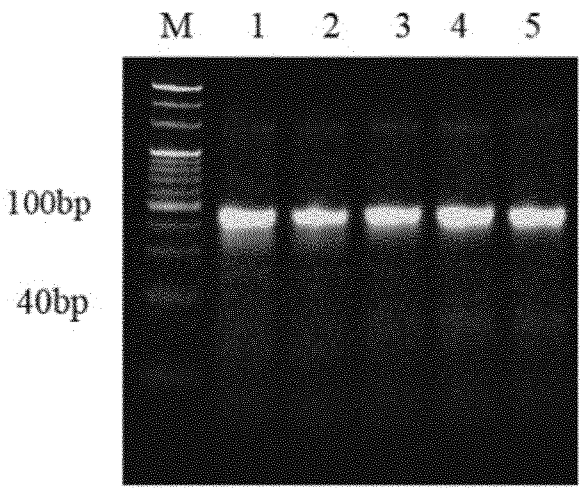


Fig. 40

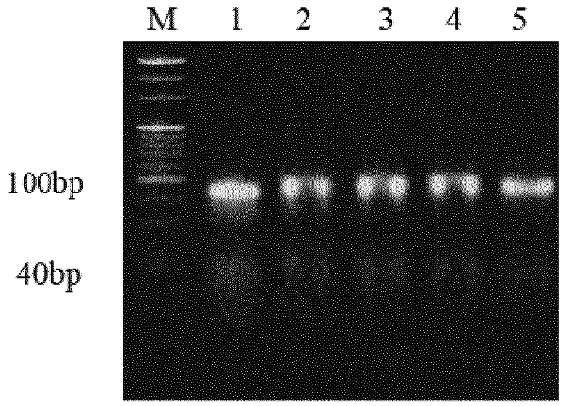


Fig. 41

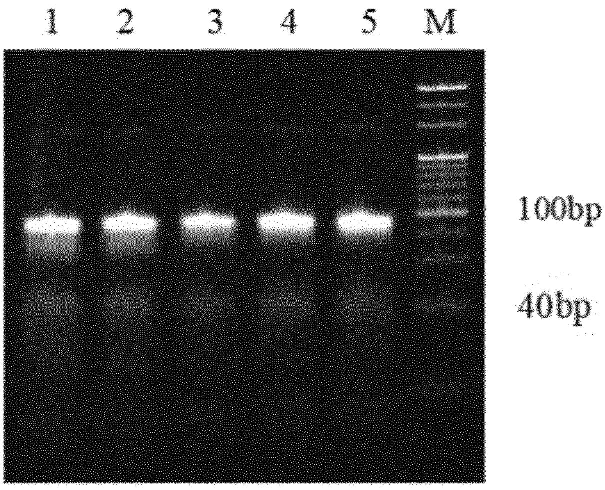


Fig. 42

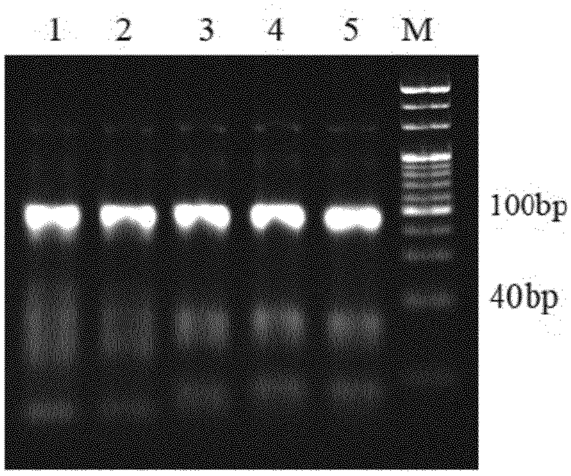


Fig. 43

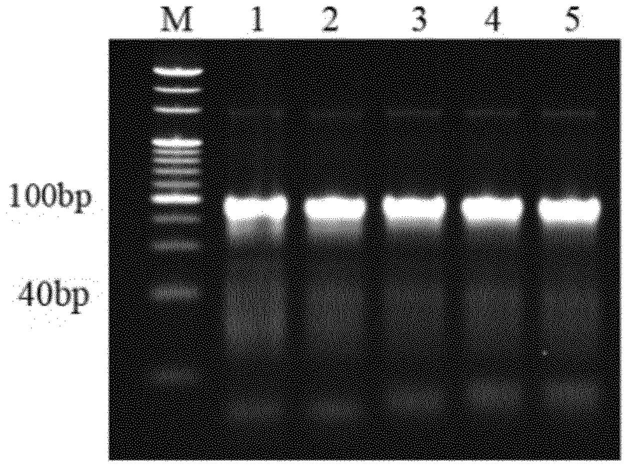


Fig. 44

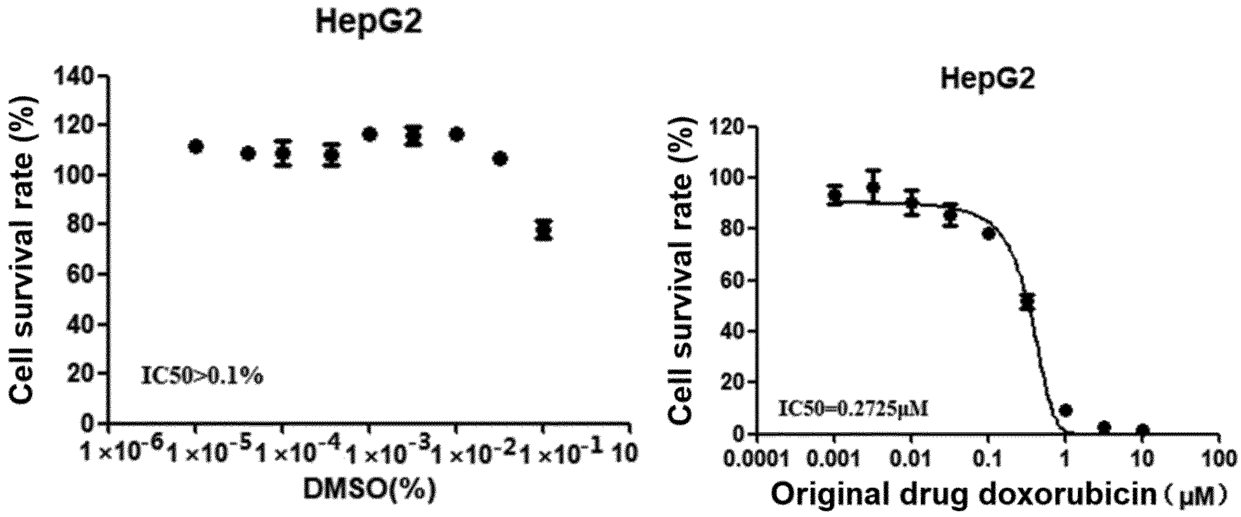


Fig. 45a

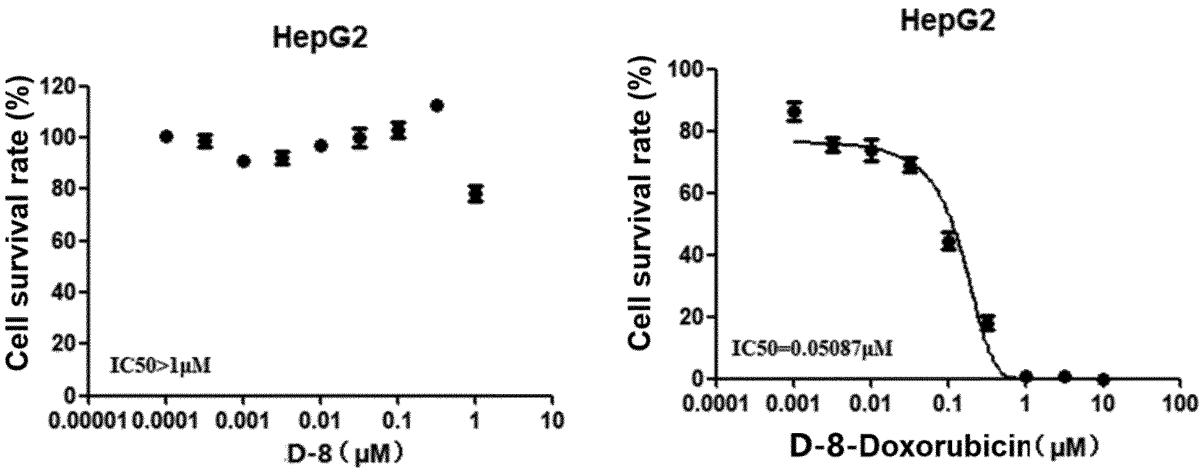


Fig. 45b

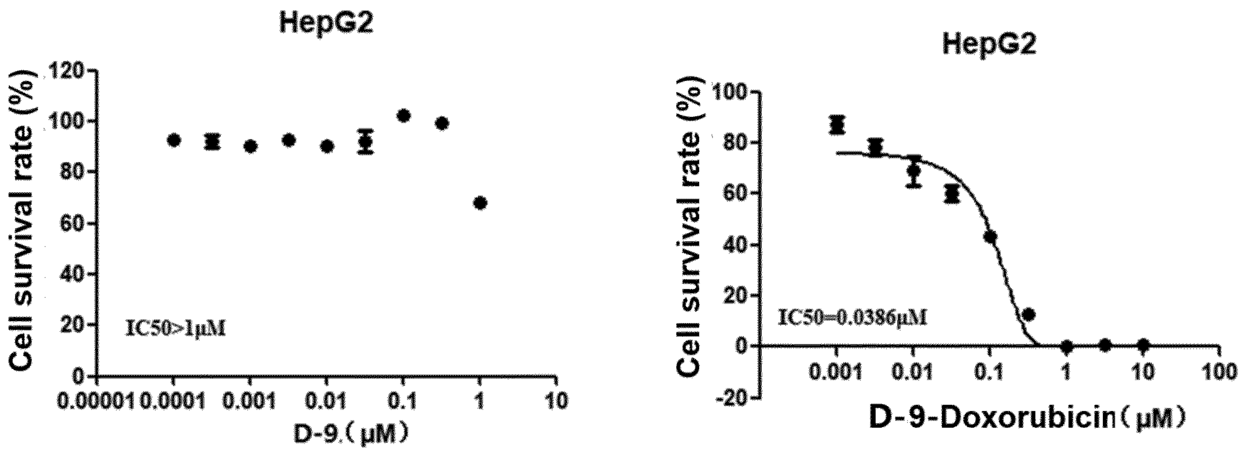


Fig. 45c

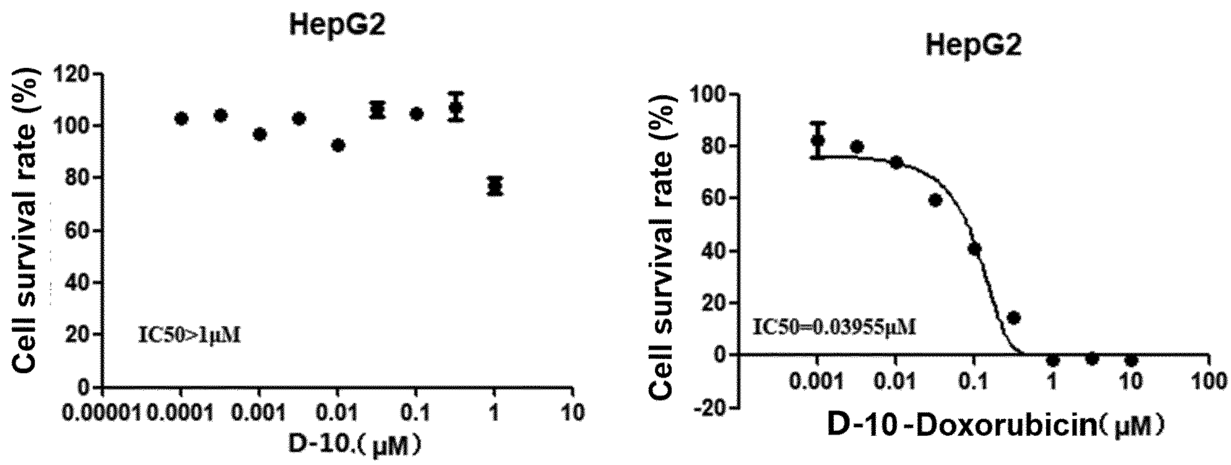


Fig. 45d

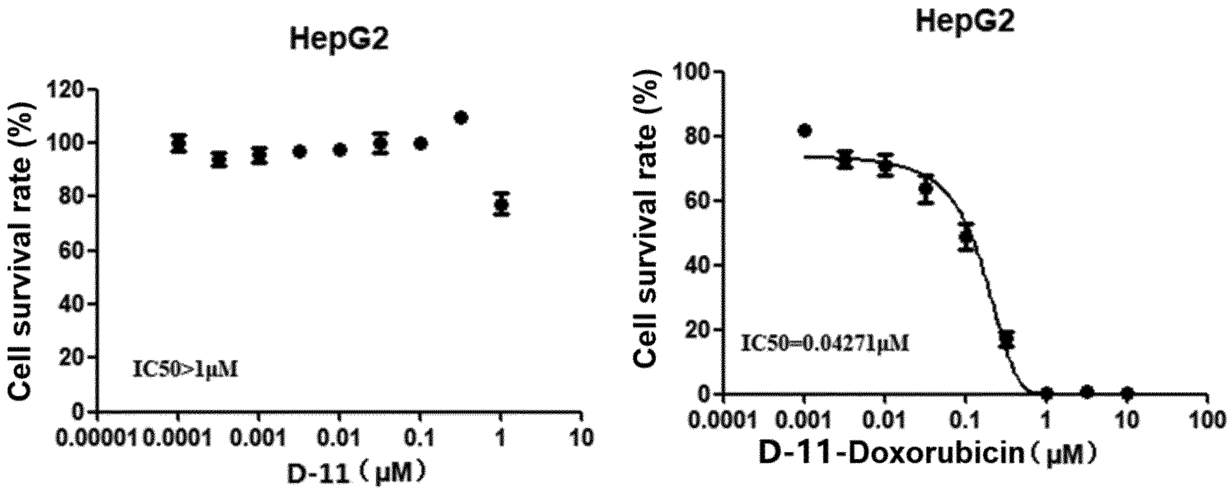


Fig. 45e

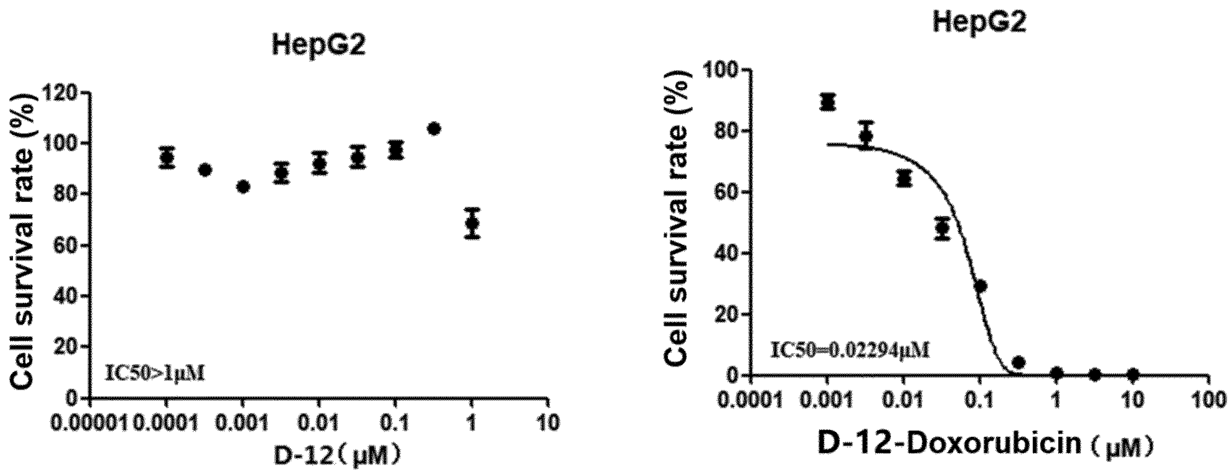


Fig. 45f

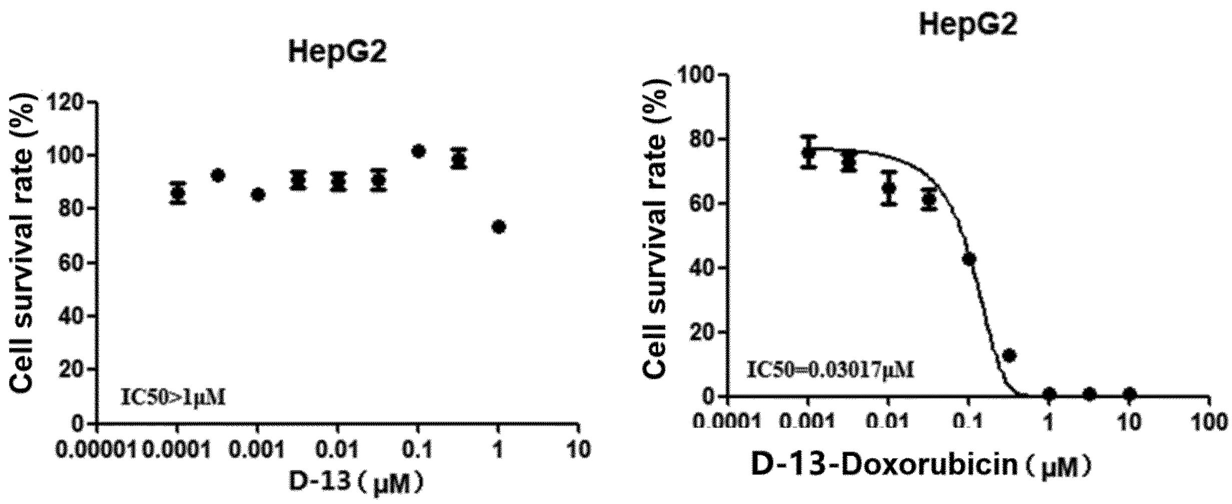


Fig. 45g

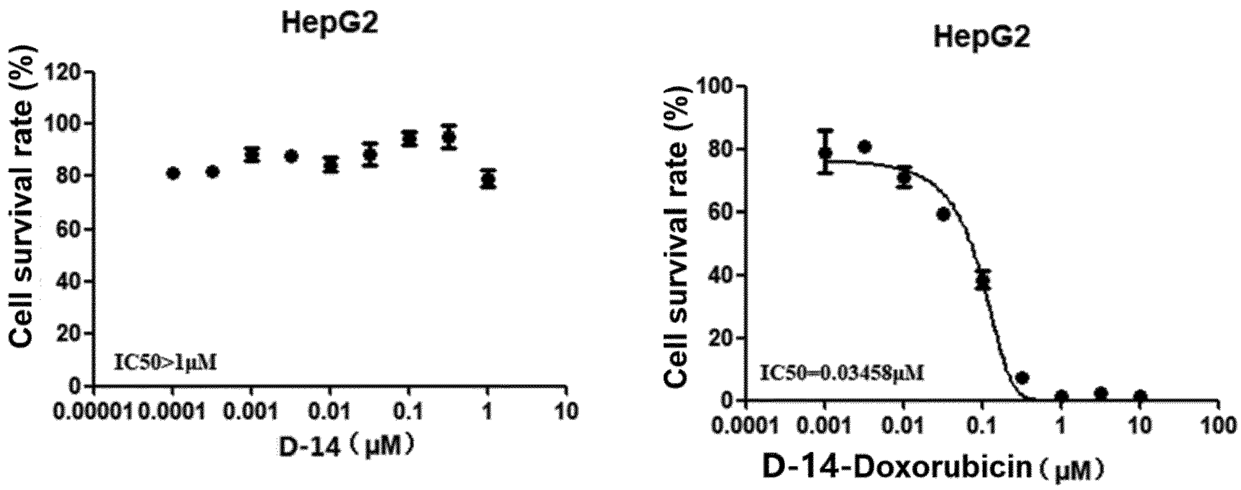


Fig. 45h

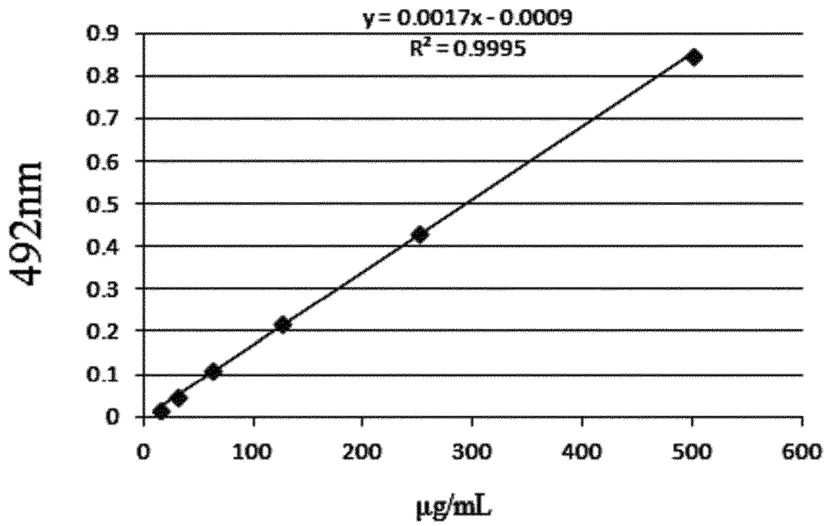


Fig. 46

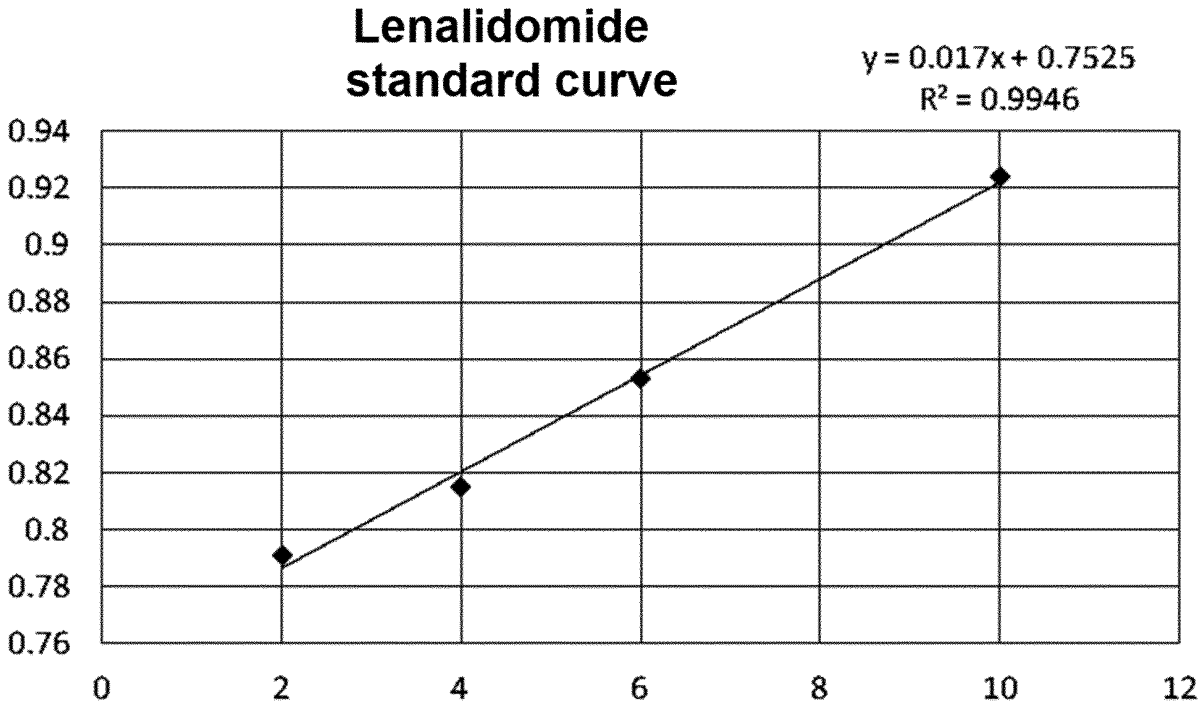


Fig. 47a

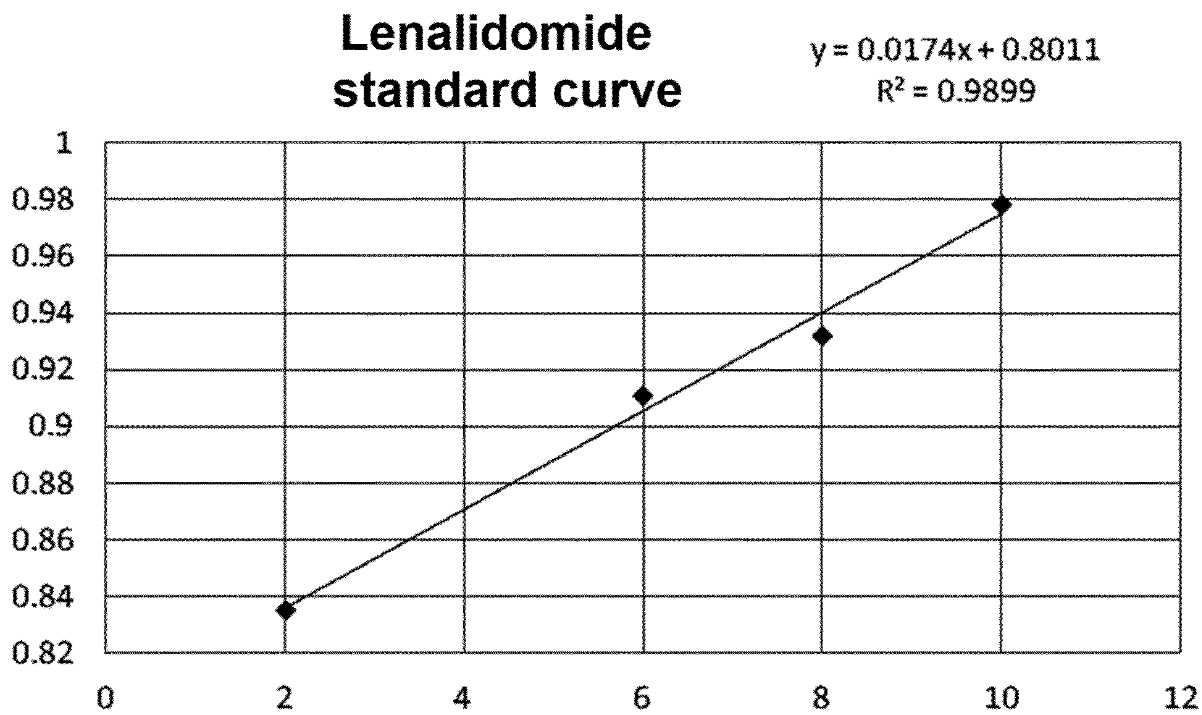


Fig. 47b

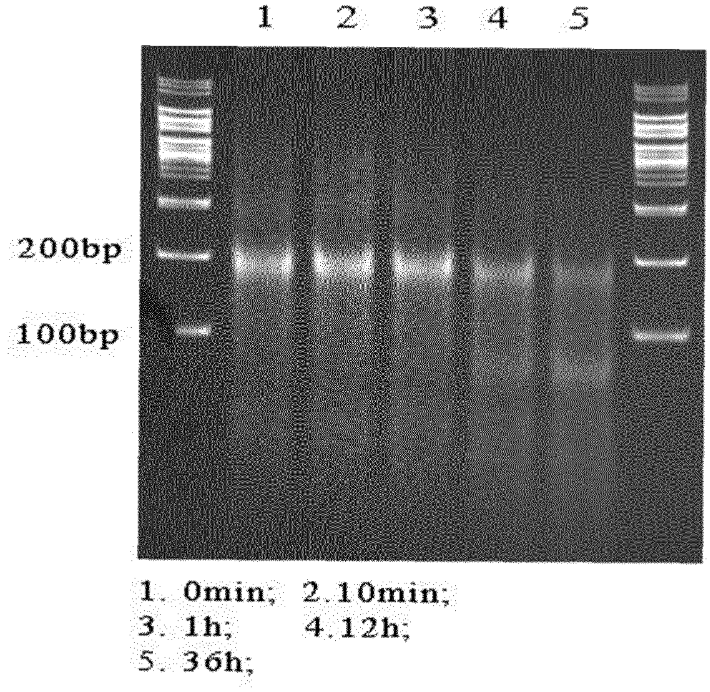


Fig. 48

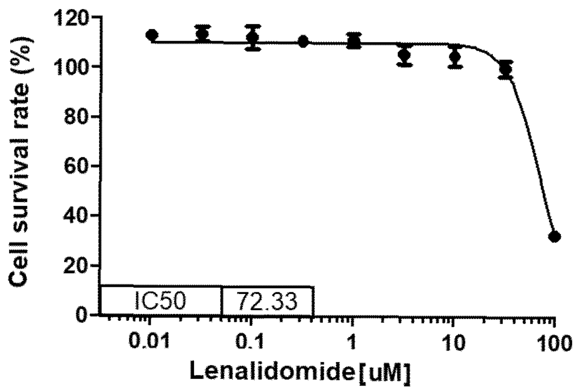


Fig. 49a

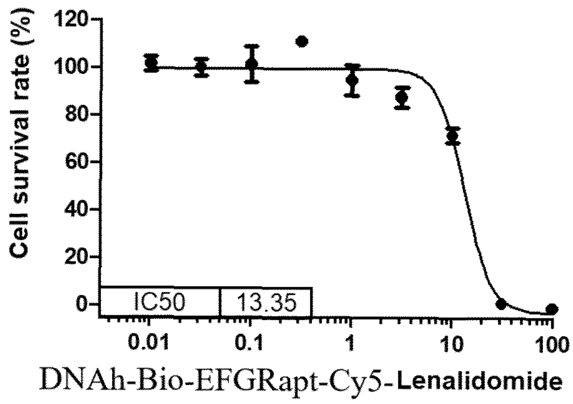


Fig. 49b

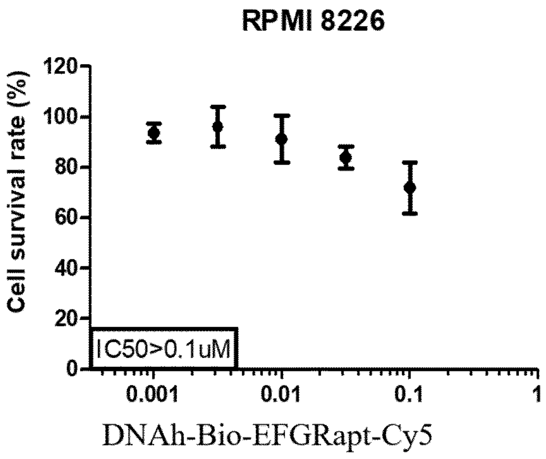


Fig. 49c

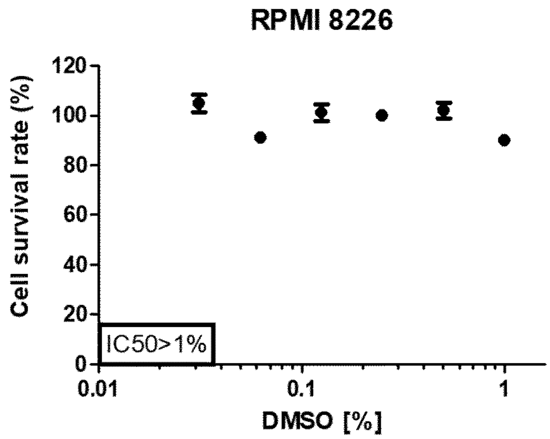


Fig. 49d

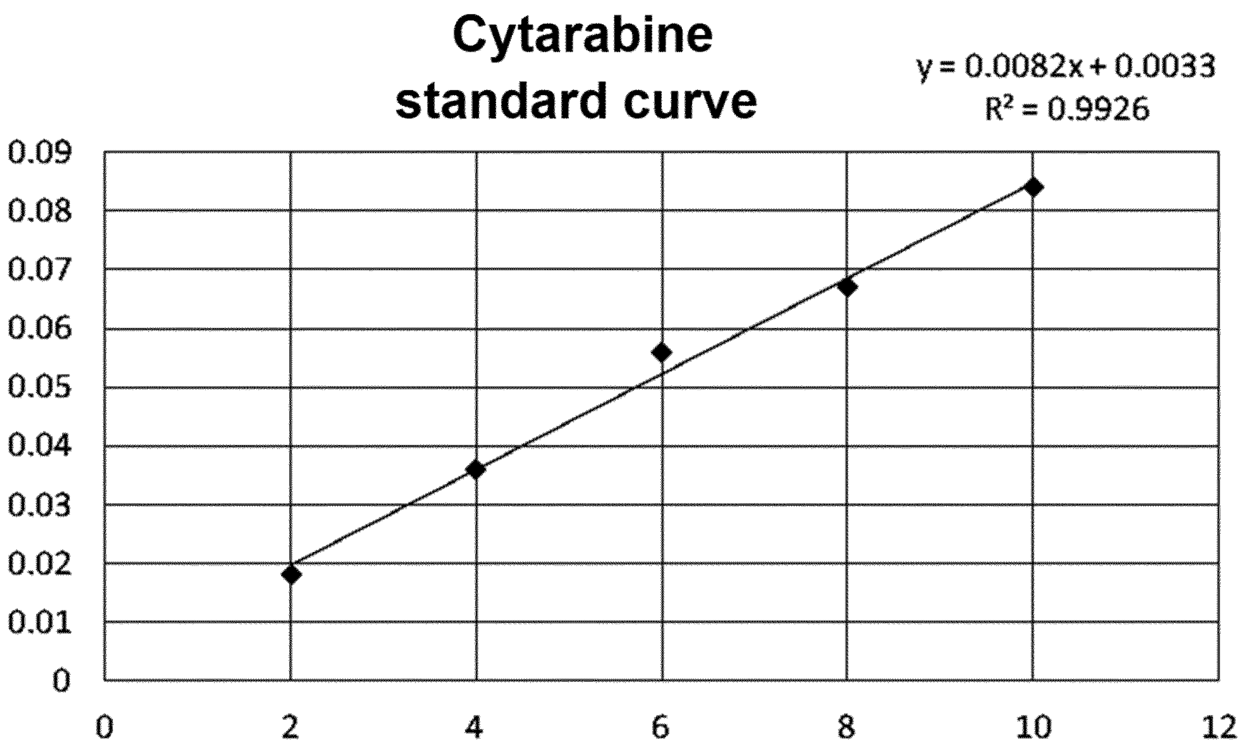


Fig. 50

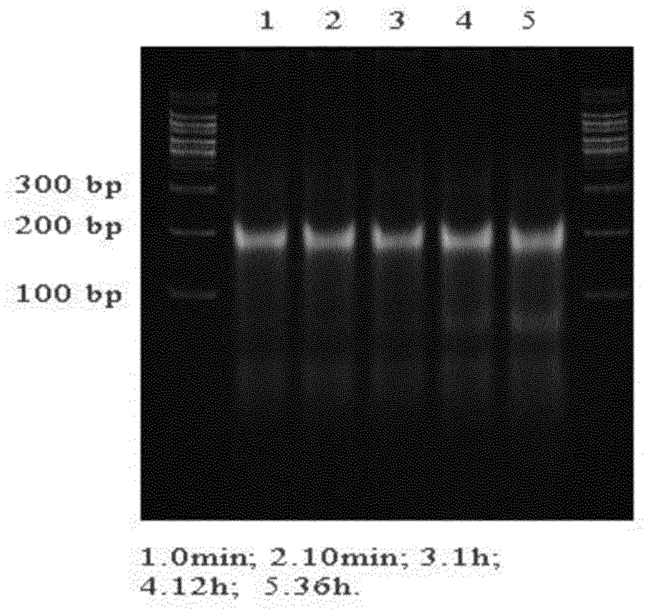


Fig. 51

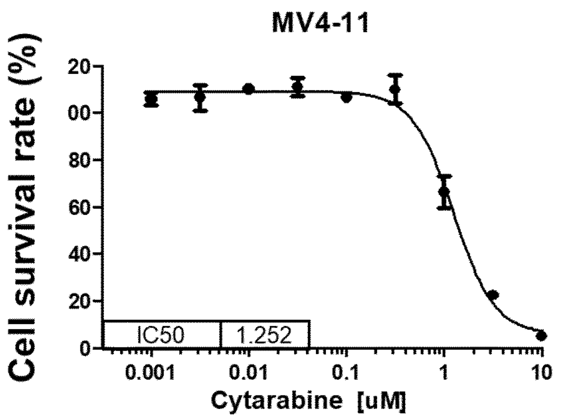


Fig. 52a

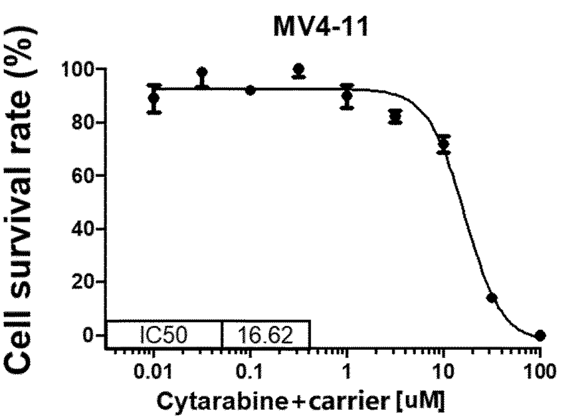


Fig. 52b

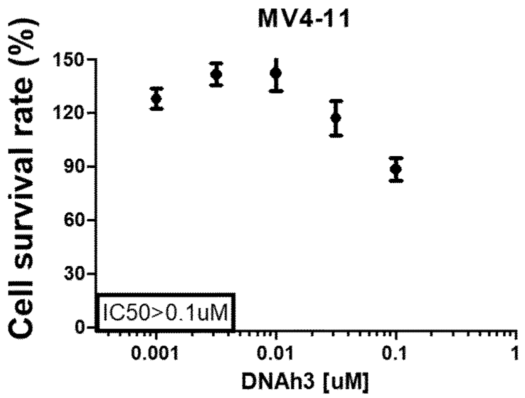


Fig. 52c

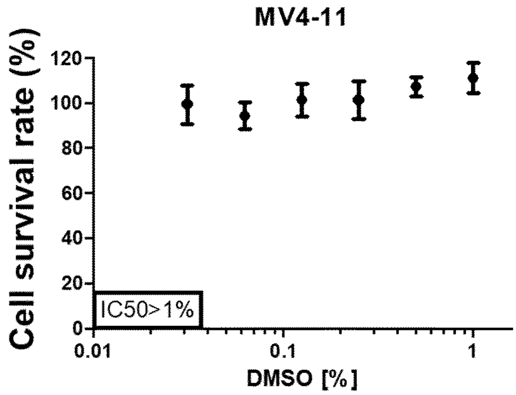


Fig. 52d

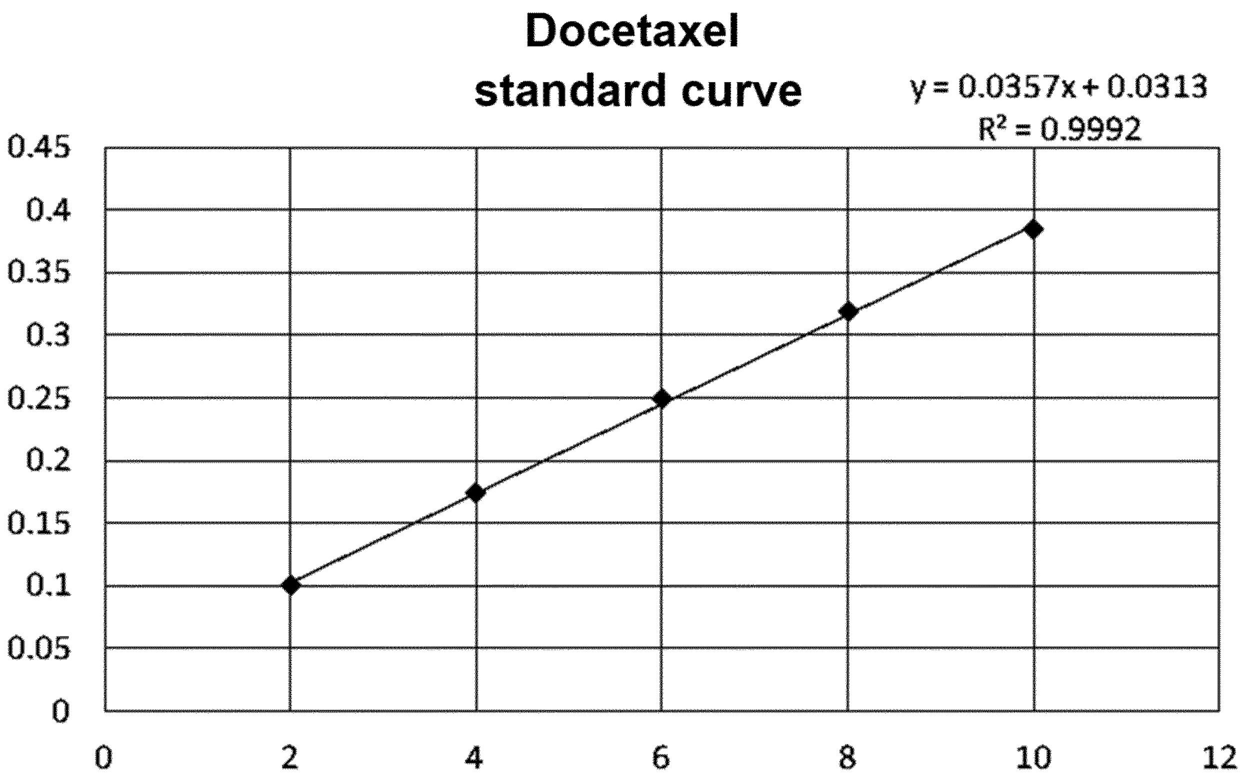


Fig. 53a

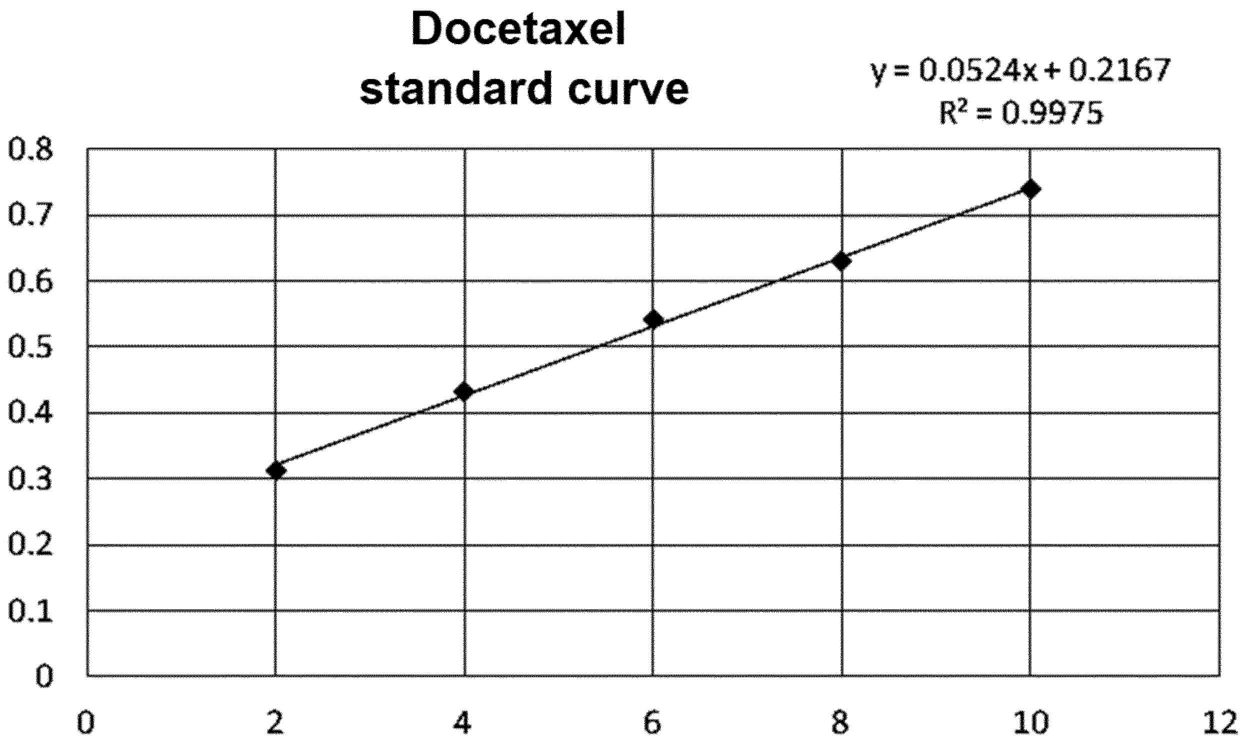


Fig. 53b

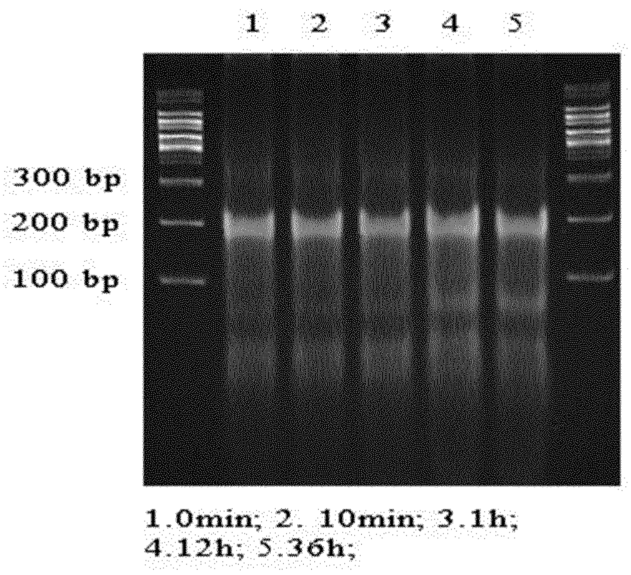


Fig. 54

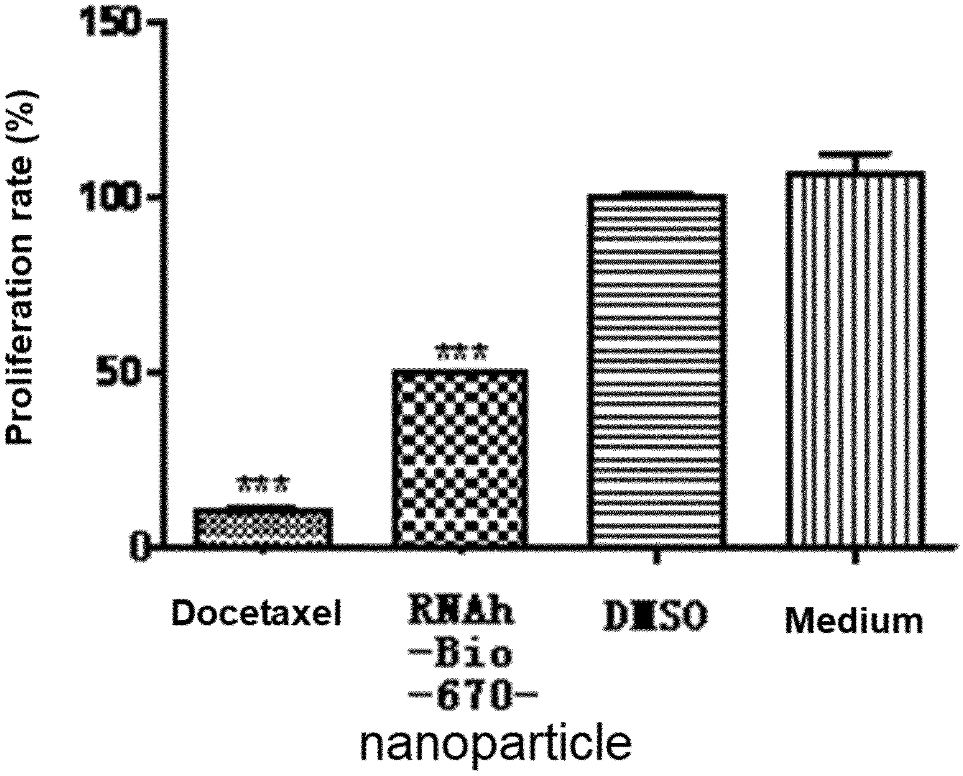


Fig. 55

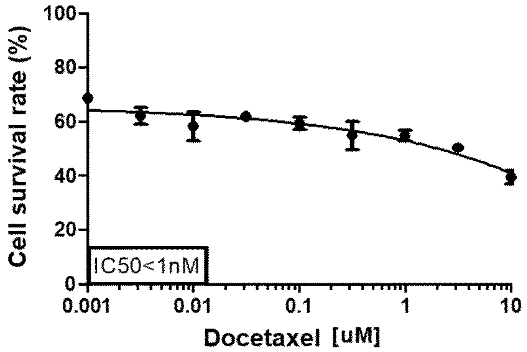


Fig. 56a

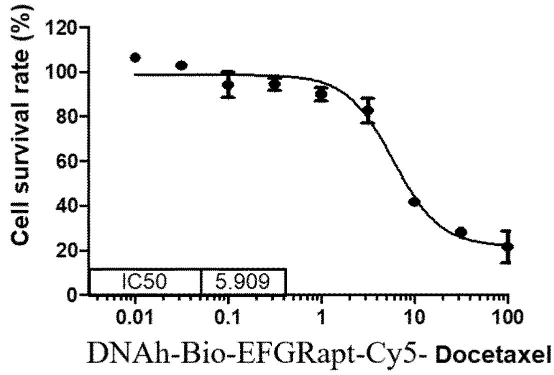


Fig. 56b

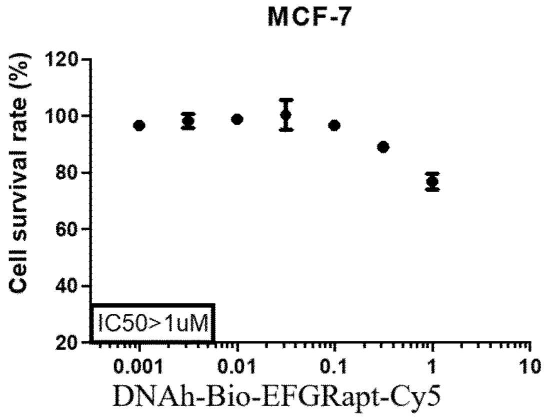


Fig. 56c

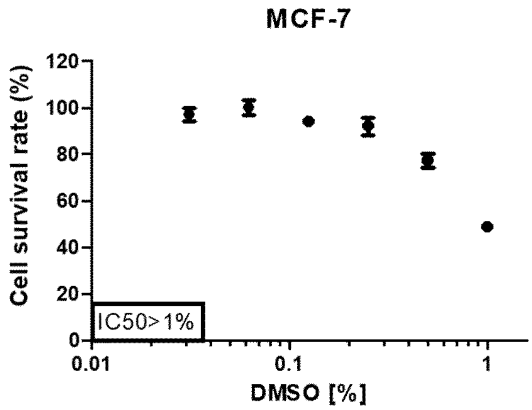


Fig. 56d

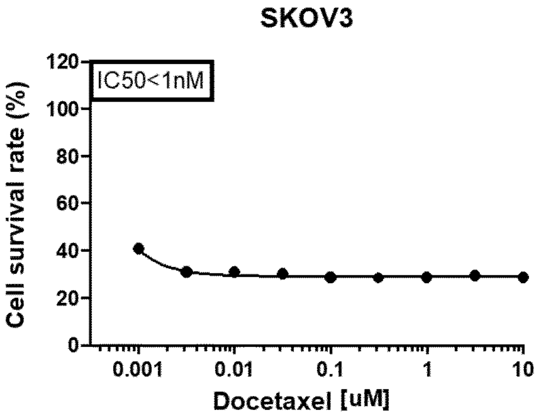


Fig. 57a

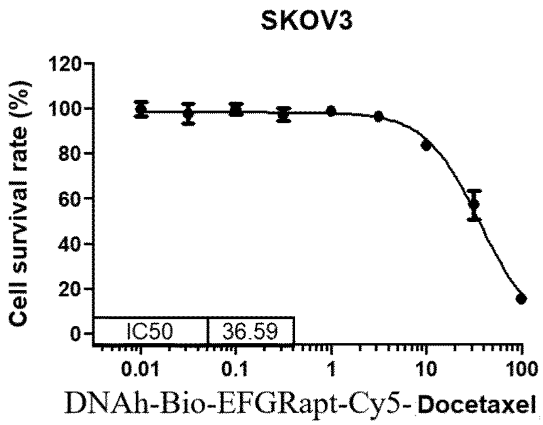


Fig. 57b

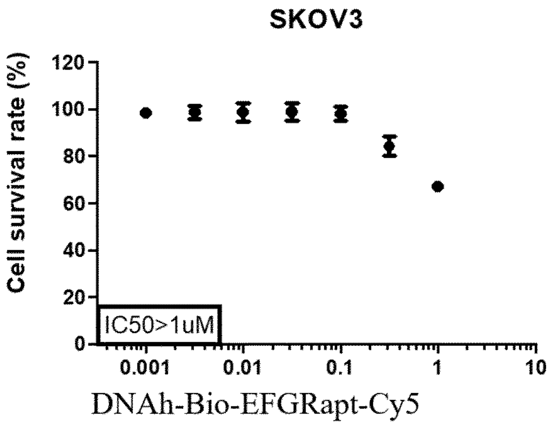


Fig. 57c

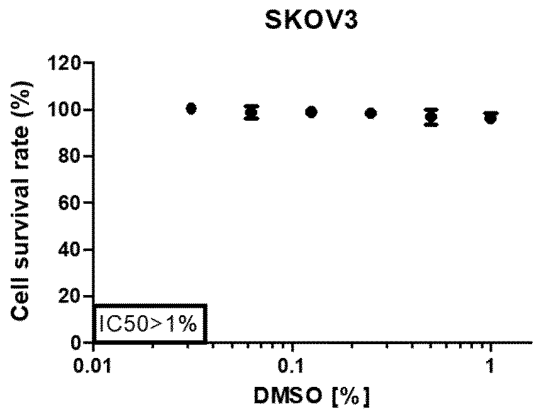


Fig. 57d

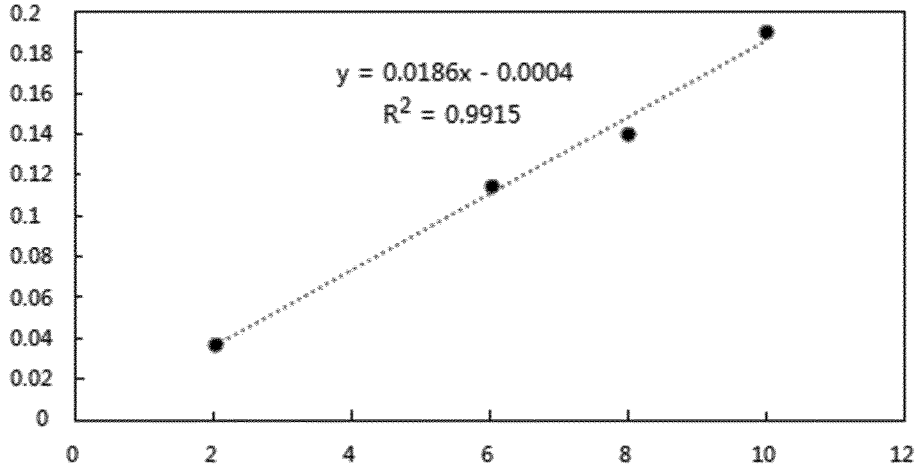
y = 0.0186x - 0.0004
$R^2$ = 0.9915
0.2
0.18
0.16
0.14
0.12
0.1
0.08
0.06
0.04
0.02
0
0
2
4
6
8
10
12

Fig. 58

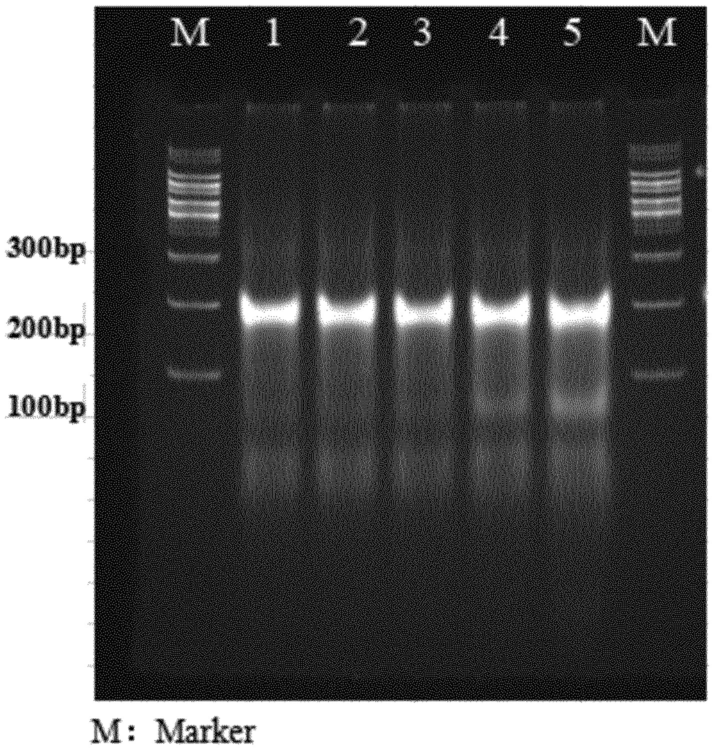
M 1 2 3 4 5 M
300bp
200bp
100bp
M： Marker

Fig. 59

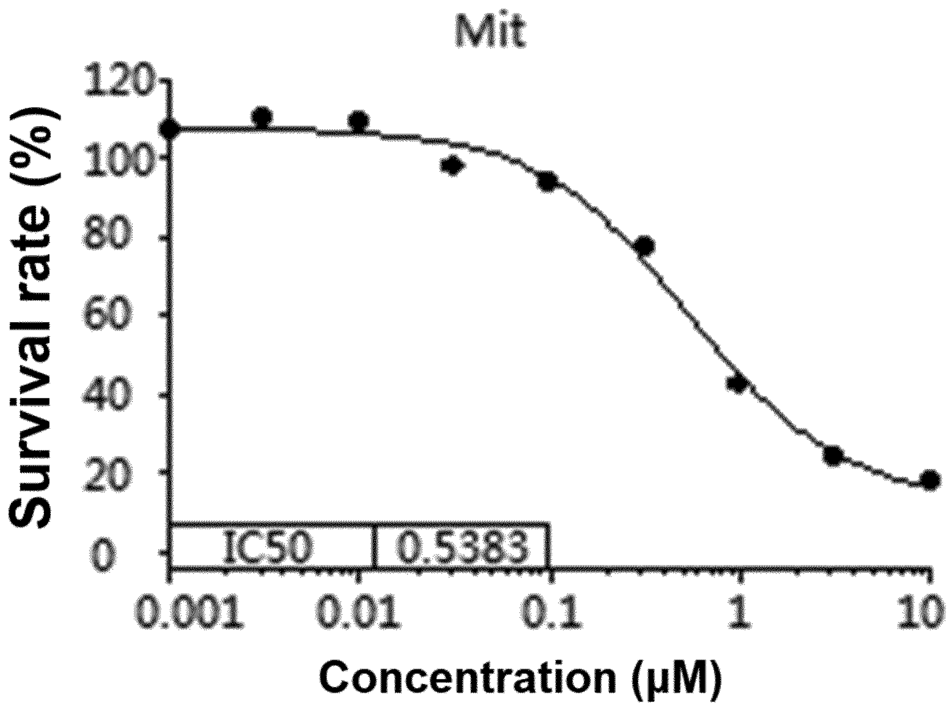
Mit
Survival rate (%)
120
100
80
60
40
20
0
IC50
0.5383
0.001
0.01
0.1
1
10
Concentration (μM)

Fig. 60

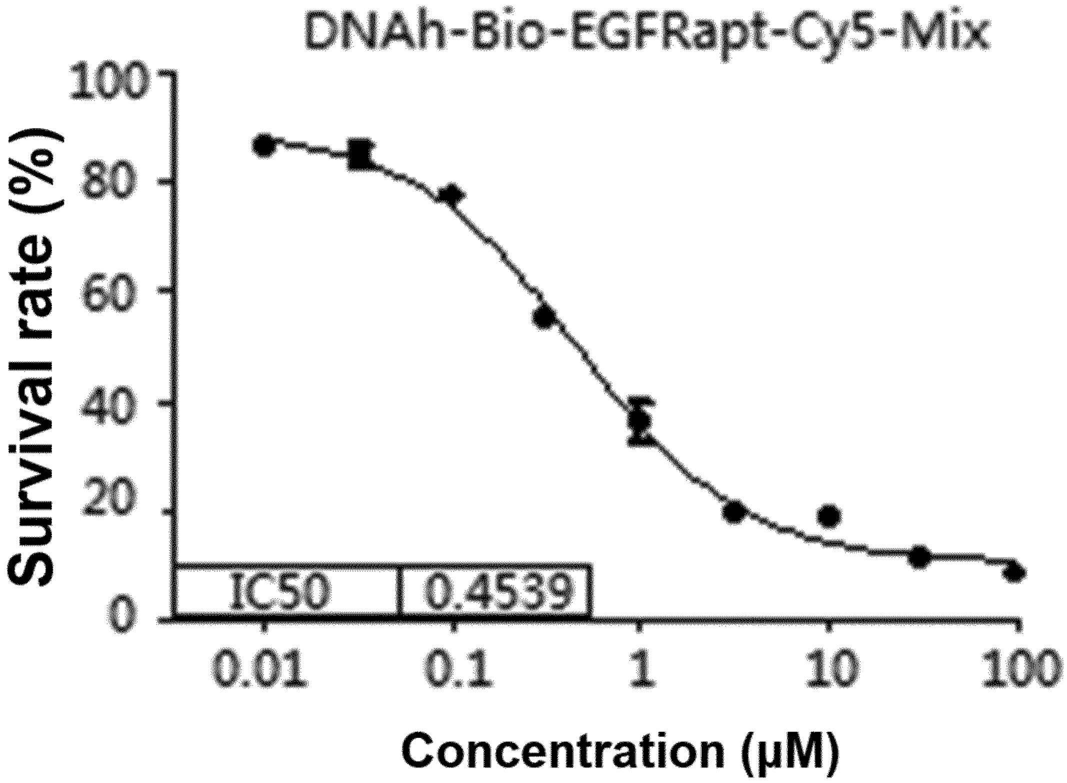
DNAh-Bio-EGFRapt-Cy5-Mix
Survival rate (%)
100
80
60
40
20
0
IC50
0.4539
0.01
0.1
1
10
100
Concentration (μM)

Fig. 61

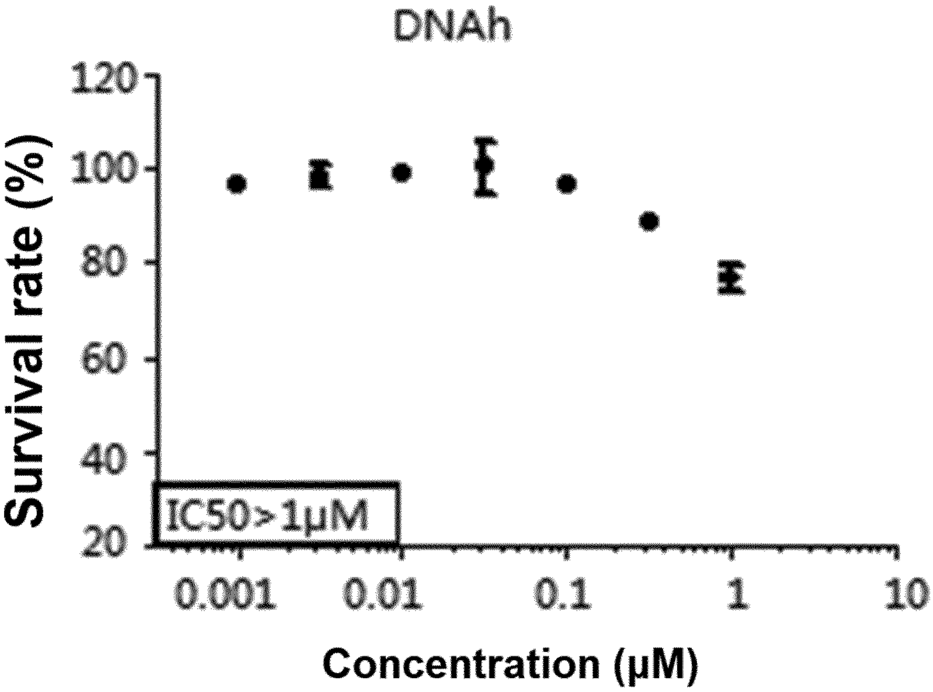


Fig. 62

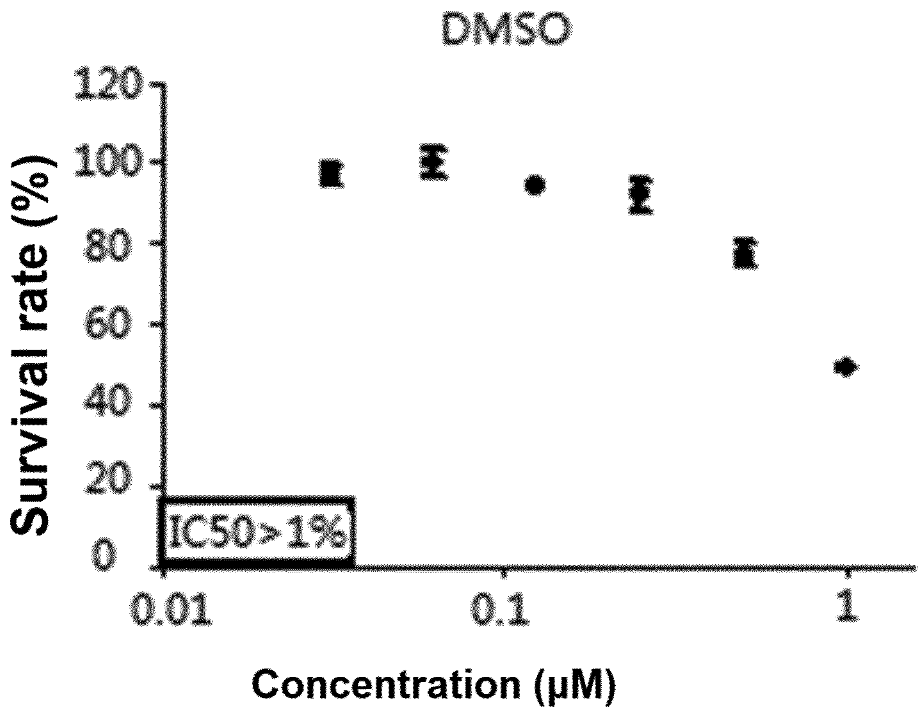


Fig. 63

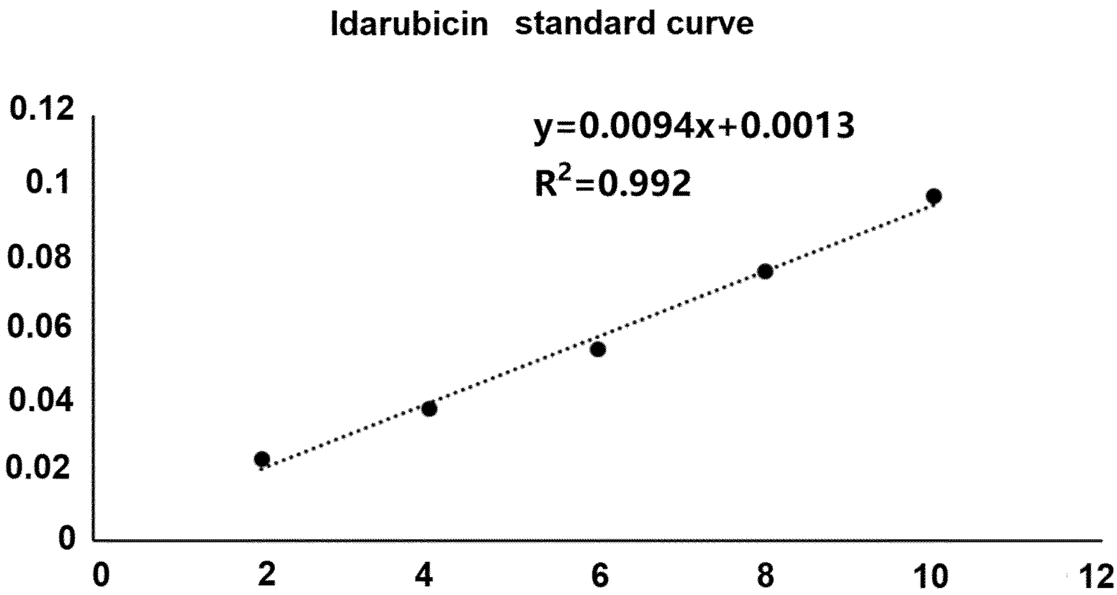
Idarubicin standard curve
y=0.0094x+0.0013
R2=0.992
0.12
0.1
0.08
0.06
0.04
0.02
0
0
2
4
6
8
10
12

Fig. 64

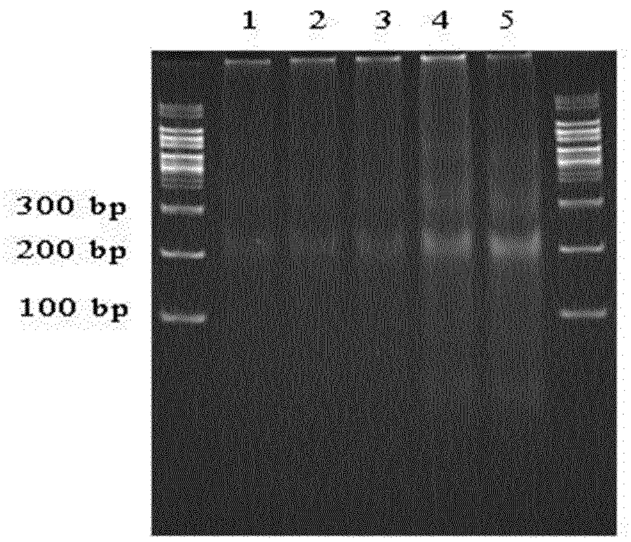
1
2
3
4
5
300 bp
200 bp
100 bp

Fig. 65

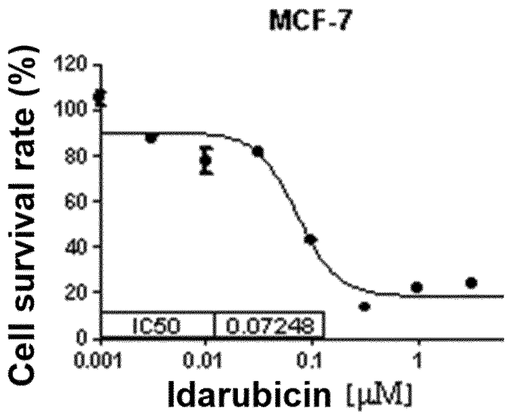


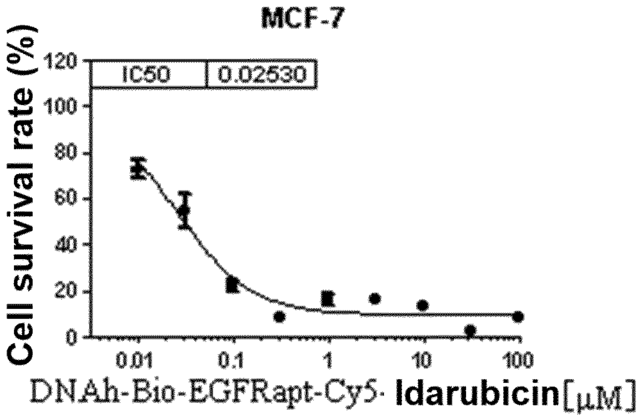


Fig. 66a

Fig. 66b

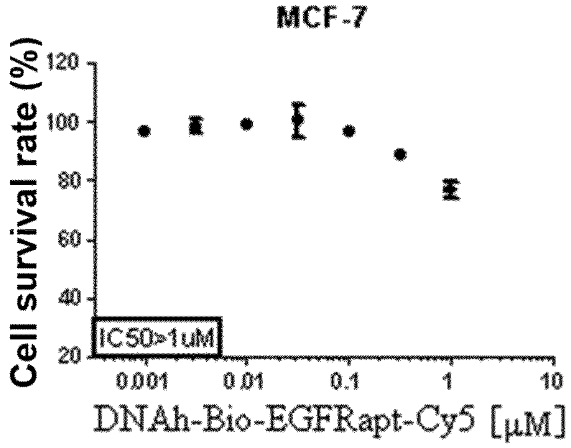


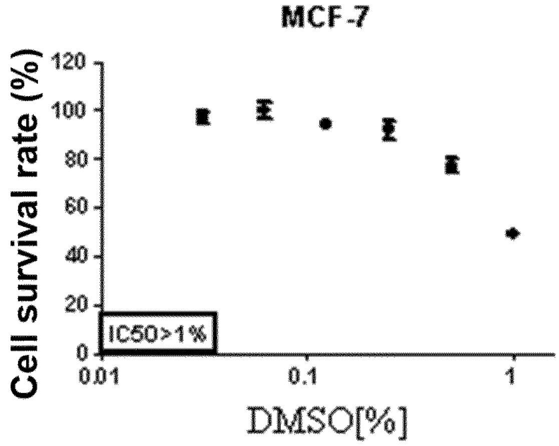


Fig. 66c

Fig. 66d

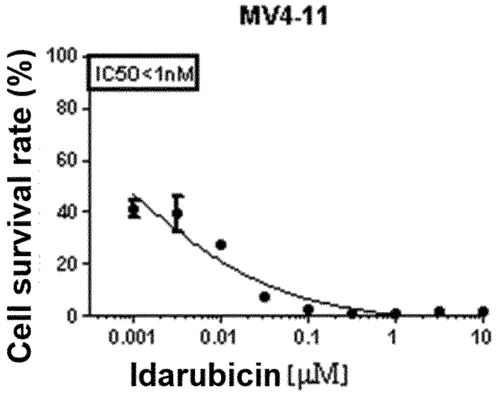


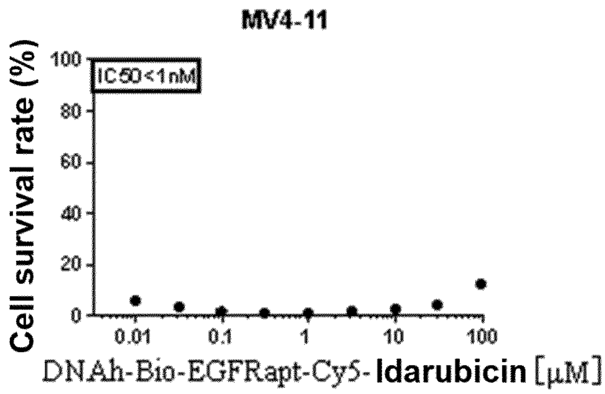


Fig. 67a

Fig. 67b

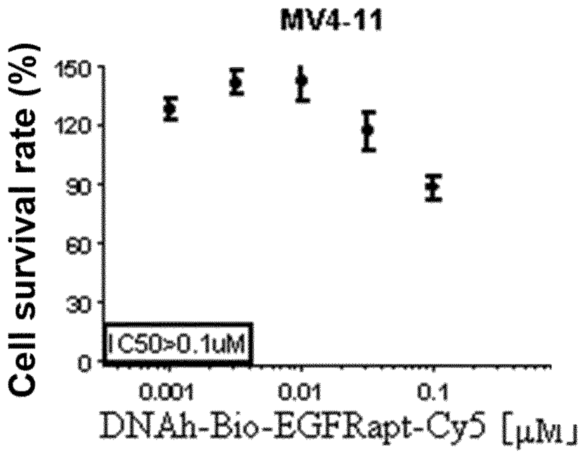


Fig. 67c

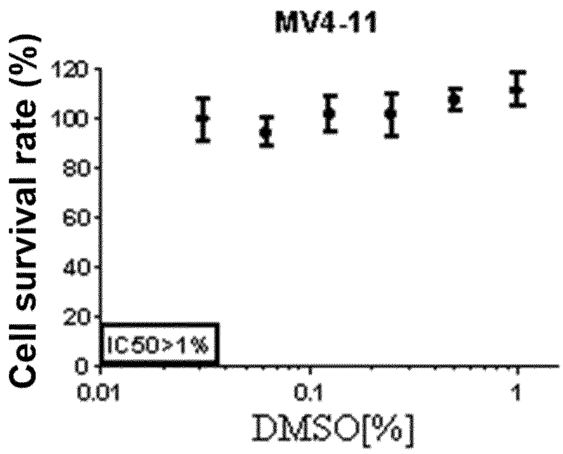


Fig. 67d

## INTERNATIONAL SEARCH REPORT

International application No.
**PCT/CN2019/109616**

### A. CLASSIFICATION OF SUBJECT MATTER

C12N 15/115(2010.01)i; A61K 47/56(2017.01)i; A61K 31/713(2006.01)i; C07H 21/04(2006.01)i

According to International Patent Classification (IPC) or to both national classification and IPC

### B. FIELDS SEARCHED

Minimum documentation searched (classification system followed by classification symbols)

C12N; A61K; C07H

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, EPTXT, USTXT, CNKI, 万方数据资源系统, Wanfang Data Resource System, 中国专利生物序列检索系统, Chinese Patent Biological Sequence Retrieval System, PubMed, ISI Web of Knowledge, EMBL, Patentics: 申请人/发明人, applicant/inventor, 纳米颗粒, nanoparticle, RNA scaffold, 3WJ, three-way junction, H.marismortui 5S rRNA, 紫杉醇, 来那度胺, 阿糖胞苷, 多西他赛, 伊达比星, 米托蒽醌, taxol, paclitaxel, lenalidomide, cytarabine, docetaxel, mitoxantrone, 小分子药物, 适体, aptamer, SEQ ID Nos: 1-6

### C. DOCUMENTS CONSIDERED TO BE RELEVANT

| Category* | Citation of document, with indication, where appropriate, of the relevant passages | Relevant to claim No. |
|---|---|---|
| X | CN 103403189 A (UNIVERSITY OF CINCINNATI) 20 November 2013 (2013-11-20) description, paragraphs [0148], [0155], [0189]-[0197] and [0207], and figures 10E and 10G | 1-29 |
| X | WO 2005106035 A2 (CORNELL RESEARCH FOUNDATION, INC.) 10 November 2005 (2005-11-10) claims 1-26, and description, figures 4A, 4B and 5A-C | 1-29 |
| Y | CN 104327141 A (SHANGHAI JIAO TONG UNIVERSITY) 04 February 2015 (2015-02-04) claims 1-11, and description, figure 1 | 1-29 |
| Y | CN 106795517 A (UNIVERSITY OF KENTUCKY RESEARCH FOUNDATION) 31 May 2017 (2017-05-31) description, figures 19 and 23 | 1-29 |
| Y | CN 107429251 A (UNIVERSITY OF KENTUCKY RESEARCH FOUNDATION) 01 December 2017 (2017-12-01) claims 1-51, and description, figures 1A and 2A | 1-29 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 December 2019** | **27 December 2019** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No. **PCT/CN2019/109616**

**C. DOCUMENTS CONSIDERED TO BE RELEVANT**

| Category* | Citation of document, with indication, where appropriate, of the relevant passages | Relevant to claim No. |
|---|---|---|
| Y | CN 107531740 A (UNIVERSITY OF KENTUCKY RESEARCH FOUNDATION) 02 January 2018 (2018-01-02)<br>claims 1-51, and description, figure 1A | 1-29 |
| Y | CN 107614685 A (UNIVERSITY OF KENTUCKY RESEARCH FOUNDATION) 19 January 2018 (2018-01-19)<br>claims 1-130, and description, figures 1A, 4B, 6E, 6F, 12, 19, 28A and 28B | 1-29 |
| Y | CN 108602849 A (OHIO STATE INNOVATION FOUNDATION) 28 September 2018 (2018-09-28)<br>claims 1-20, and description, figure 2A | 1-29 |
| Y | WO 2017197009 A1 (OHIO STATE INNOVATION FOUNDATION et al.) 16 November 2017 (2017-11-16)<br>claims 1-26, and description, figures 1A and 13A | 1-29 |
| Y | BAN, N. et al. "The Complete Atomic Structure of the Large Ribosomal Subunit at 2.4 Å Resolution."<br>*Science.*, Vol. 289, No. 5481, 11 August 2000 (2000-08-11),<br>pp. 905-920 | 1-29 |
| Y | DIAMOND, J. M. et al. "Thermodynamics of Three-Way Multibranch Loops in RNA."<br>*Biochemistry.*, Vol. 40, No. 23, 18 May 2001 (2001-05-18),<br>pp. 6971-6981 | 1-29 |

Form PCT/ISA/210 (second sheet) (January 2015)

**INTERNATIONAL SEARCH REPORT**
**Information on patent family members**

International application No.
**PCT/CN2019/109616**

| Patent document cited in search report | | | Publication date (day/month/year) | Patent family member(s) | | | Publication date (day/month/year) |
|---|---|---|---|---|---|---|---|
| CN | 103403189 | A | 20 November 2013 | US | 9297013 | B2 | 29 March 2016 |
| | | | | WO | 2012170372 | A2 | 13 December 2012 |
| | | | | WO | 2012170372 | A3 | 18 April 2013 |
| | | | | CN | 103403189 | B | 25 November 2015 |
| | | | | US | 2014179758 | A1 | 26 June 2014 |
| WO | 2005106035 | A2 | 10 November 2005 | WO | 2005106035 | A3 | 06 July 2006 |
| | | | | US | 2005282190 | A1 | 22 December 2005 |
| CN | 104327141 | A | 04 February 2015 | CN | 104327141 | B | 04 August 2017 |
| CN | 106795517 | A | 31 May 2017 | US | 10378018 | B2 | 13 August 2019 |
| | | | | WO | 2015196146 | A2 | 23 December 2015 |
| | | | | WO | 2015196146 | A3 | 25 February 2016 |
| | | | | US | 2017175122 | A1 | 22 June 2017 |
| CN | 107429251 | A | 01 December 2017 | US | 2019119682 | A1 | 25 April 2019 |
| | | | | WO | 2016145008 | A3 | 03 November 2016 |
| | | | | WO | 2016145008 | A2 | 15 September 2016 |
| CN | 107531740 | A | 02 January 2018 | WO | 2016145005 | A1 | 15 September 2016 |
| | | | | US | 2019106452 | A1 | 11 April 2019 |
| CN | 107614685 | A | 19 January 2018 | WO | 2016168784 | A2 | 20 October 2016 |
| | | | | WO | 2016168784 | A3 | 24 November 2016 |
| | | | | US | 2018092997 | A1 | 05 April 2018 |
| CN | 108602849 | A | 28 September 2018 | WO | 2017176894 | A1 | 12 October 2017 |
| | | | | US | 2019024085 | A1 | 24 January 2019 |
| | | | | EP | 3440090 | A1 | 13 February 2019 |
| WO | 2017197009 | A1 | 16 November 2017 | CN | 109196103 | A | 11 January 2019 |
| | | | | US | 2019127739 | A1 | 02 May 2019 |

Form PCT/ISA/210 (patent family annex) (January 2015)